(19) Europäisches Patentamt
European Patent Office
Office européen des brevets

(11) EP 4 219 461 A1

(12) **EUROPEAN PATENT APPLICATION**
published in accordance with Art. 153(4) EPC

(43) Date of publication:
**02.08.2023 Bulletin 2023/31**

(21) Application number: **21871464.0**

(22) Date of filing: **18.09.2021**

(51) International Patent Classification (IPC):
***C07D 401/12*** (2006.01) ***C07D 413/12*** (2006.01)
***C07D 217/04*** (2006.01) ***A61K 31/4725*** (2006.01)
***A61P 35/00*** (2006.01)

(52) Cooperative Patent Classification (CPC):
**A61K 31/4725; A61P 35/00; C07D 217/04; C07D 401/12; C07D 413/12**

(86) International application number:
**PCT/CN2021/119445**

(87) International publication number:
**WO 2022/063094 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.09.2020 CN 202011000192**
**08.02.2021 CN 202110187630**
**08.03.2021 CN 202110251658**
**14.05.2021 CN 202110529022**
**15.07.2021 CN 202110802850**

(71) Applicant: **Medshine Discovery Inc.**
**Nanjing, Jiangsu 210032 (CN)**

(72) Inventors:
- **CHEN, Kevin X**
  **Shanghai 200131 (CN)**
- **GUO, Zuhao**
  **Shanghai 200131 (CN)**
- **YU, Yanxin**
  **Shanghai 200131 (CN)**
- **HU, Boyu**
  **Shanghai 200131 (CN)**
- **WANG, Jingjing**
  **Shanghai 200131 (CN)**
- **CHEN, Zhaoguo**
  **Shanghai 200131 (CN)**
- **XIE, Cheng**
  **Shanghai 200131 (CN)**
- **XIONG, Jian**
  **Shanghai 200131 (CN)**
- **FANG, Yongbo**
  **Shanghai 200131 (CN)**
- **LIU, Yingtao**
  **Shanghai 201299 (CN)**
- **LI, Jian**
  **Shanghai 200131 (CN)**
- **CHEN, Shuhui**
  **Shanghai 200131 (CN)**

(74) Representative: **Dehns**
**St. Bride's House**
**10 Salisbury Square**
**London EC4Y 8JD (GB)**

# (54) TETRAHYDROISOQUINOLINE DERIVATIVE AND USE THEREOF

(57) A series of tetrahydroisoquinoline derivatives and the crystal forms thereof. Specifically disclosed are a compound as represented by formula (VII), a crystal form thereof, and a pharmaceutically acceptable salt thereof.

EP 4 219 461 A1

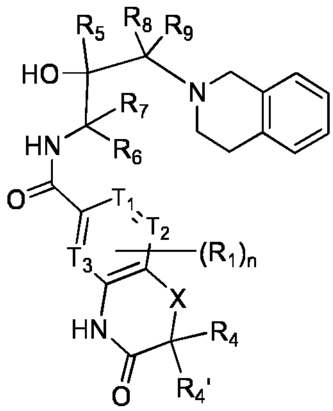

( VII )

## Description

The present invention claims the following priorities:

CN202011000192.8, filed on September 22, 2020;
CN202110187630.4, filed on February 8, 2021;
CN202110251658.X, filed on March 8, 2021;
CN202110529022.7, filed on May 14, 2021;
CN202110802850.3, filed on July 15, 2021.

TECHNICAL FIELD

The present disclosure relates to a series of tetrahydroisoquinoline derivatives and crystal forms thereof, specifically to a compound represented by formula (VII), a crystal form thereof and a pharmaceutically acceptable salt thereof.

BACKGROUND

Epigenetic regulation of gene expression is an important factor in protein production and cell differentiation, and plays an important role in many diseases.

Epigenetic regulation is a heritable change in gene expression without a change in nucleotide sequence. For example, the conformational transition between transcriptionally active and inactive states of chromatin is controlled by selective and reversible modifications (such as methylation) of DNA and proteins (such as histones). These modifications can be carried out by, for example, methyltransferases (such as the protein arginine methyltransferase family). Thus, these enzymes are associated with specific genetic alterations that cause human diseases.

Arginine methylation involving the protein arginine methyltransferase (PRMT) family is a widespread post-translational modification in the nucleus and cytoplasm. PRMTs utilize S-adenosyl-methionine as a methyl donor to methylate the nitrogen atom of the side chain of the protein arginine, and generate S-adenosylhomocysteine and methylarginine. The substrates of PRMTs are proteins rich in glycine and arginine domains. A total of 10 PRMTs have been found in mammals, eight of which are biologically active. They are classified into type I and type II according to the methylation products: type I PRMT catalyzes the formation of monomethylarginine and asymmetric dimethylarginine, and type II PRMT catalyzes the formation of symmetric dimethylarginine. Protein arginine methyltransferase 5 (PRMT5) belongs to type II PRMT.

PRMT5 can methylate different proteins involved in the regulation of physiological processes. For example, PRMT5 can affect the gene transcription process by methylating histones and transcription elongation factors; it can also methylate tumor suppressor gene p53 to change the activation state of p53. PRMT5 and its molecular chaperone protein MEP50 can form macromolecular complexes with various proteins, and catalyze the methylation of various substrate proteins in the cytoplasm and nucleus such as Sm protein, nucleolin, p53, histones H2A, H3 and H4, SPT5 and MBD2, playing a key role in RNA processing, chromatin remodeling, and regulation of gene expression. PRMT5 can regulate MAPK/ERK signaling pathway by methylating RAF protein, regulate ribosome biosynthesis by methylating ribosomal protein S10, and play an important role in apoptosis by regulating the expression of eIF4E and the translation of p53. In embryonic stem cells, gene differentiation is suppressed by methylating H2A in the cytoplasm.

It has been found that PRMT5 is highly expressed in mantle cell lymphoma and diffuse large B-cell lymphoma, and it is directly associated with the proliferation and survival of malignant B cells. Therefore, PRMT5 is a very promising target for tumor therapy. The PRMT5 inhibitor GSK3326595 disclosed in patent WO2014100719 has entered phase II clinical trials for clinical indications including non-Hodgkin's lymphoma, acute lymphoblastic leukemia, myelodysplastic syndrome, adenoid cystic carcinoma and breast cancer.

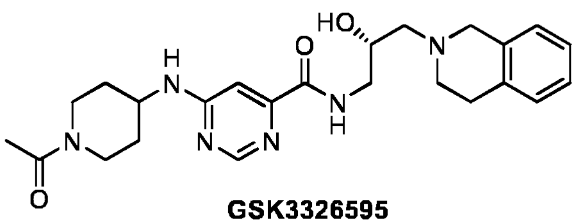

**GSK3326595**

In summary, there is an urgent need in the art to develop a novel inhibitor of protein arginine methyltransferase 5.

CONTENT OF THE PRESENT INVENTION

The present disclosure provides a compound represented by formula (VII) or a pharmaceutically acceptable salt thereof,

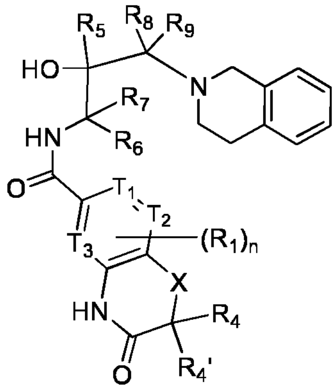

( VII )

wherein,

$T_1$, $T_2$ and $T_3$ are each independently selected from CH and N;
$R_1$ is each independently selected from H, F, Cl, Br, I, $OCH_3$ and $CH_3$, and the $OCH_3$ and $CH_3$ are optionally substituted by 1, 2 or 3 F;
$R_4$' is selected from H and $C_{1-3}$ alkyl;
$R_5$ is selected from H, F, Cl and $C_{1-3}$ alkyl, and the $C_{1-3}$ alkyl is optionally substituted by 1, 2 or 3 F;
$R_6$ and $R_8$ are each independently selected from H, F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
$R_7$ and $R_9$ are each independently selected from H, F, Cl and $C_{1-3}$ alkyl, and the $C_{1-3}$ alkyl is optionally substituted by 1, 2 or 3 F;
alternatively, $R_6$ and $R_7$ form $C_{3-6}$ cycloalkyl or 4- to 6-membered heterocycloalkyl together with the atoms to which they are attached, and the $C_{3-6}$ cycloalkyl and the 4- to 6-membered heterocycloalkyl are optionally substituted by 1, 2 or 3 F;
alternatively, $R_8$ and $R_9$ form $C_{3-6}$ cycloalkyl or 4- to 6-membered heterocycloalkyl together with the atoms to which they are attached, and the $C_{3-6}$ cycloalkyl and the 4- to 6-membered heterocycloalkyl are optionally substituted by 1, 2 or 3 F;
when X is selected from a single bond, $CH_2$ and O, $R_4$ and $R_4$' form moiety

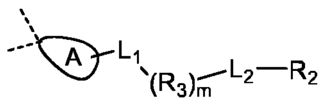

together with the atoms to which they are attached;
alternatively, X and $R_4$ are attached together to form a ring B;
the ring B is selected from $C_{3-6}$ cycloalkyl, and the $C_{3-6}$ cycloalkyl is optionally substituted by 1, 2 or 3 $R_c$;
$L_1$ and $L_2$ are each independently selected from a single bond and -C(=O)-;
$R_2$ is selected from H, OH,

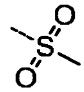,

$C_{1-3}$ alkyl, $C_{1-3}$ alkoxy, -NH-$C_{3-6}$ cycloalkyl, $C_{3-6}$ cycloalkyl and phenyl, and the $C_{1-3}$ alkyl, $C_{1-3}$ alkoxy, -NH-$C_{3-6}$ cycloalkyl, $C_{3-6}$ cycloalkyl and phenyl are optionally substituted by 1, 2 or 3 $R_a$;

$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
m is selected from 0 and 1;
n is selected from 0, 1, 2 and 3;
$R_a$ and $R_c$ are each independently selected from H, F, Cl, Br, I, OH, $-OCH_3$ and $NH_2$;
$R_b$ is each independently selected from H, F, Cl, Br, I, =O, OH, $NH_2$, $C_{1-3}$ alkyl and $C_{1-3}$ alkoxy;
the 4- to 6-membered heterocycloalkyl contains 1, 2, 3 or 4 heteroatoms or heteroatom groups independently selected from -NH-, -O-, -S- and N.

In some embodiments of the present disclosure, the $R_1$ is each independently selected from H, F, Cl, Br, I and $CH_3$, and other variables are as defined in the present disclosure.
In some embodiments of the present disclosure, the $R_1$ is each independently selected from H, F, $OCH_3$ and $CH_3$, and other variables are as defined in the present disclosure.
In some embodiments of the present disclosure, the $R_1$ is each independently selected from H, F and $CH_3$, and other variables are as defined in the present disclosure.
In some embodiments of the present disclosure, the $R_2$ is selected from H, OH,

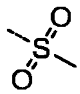,

$C_{1-3}$ alkyl, $C_{1-3}$ alkoxy, phenyl, cyclobutyl, -NH-cyclobutyl and -NH-cyclopropyl, and the $C_{1-3}$ alkyl, $C_{1-3}$ alkoxy, phenyl, cyclobutyl, -NH-cyclobutyl and -NH-cyclopropyl are optionally substituted by 1, 2 or 3 $R_a$, and other variables are as defined in the present disclosure.
In some embodiments of the present disclosure, the $R_2$ is selected from H, OH,

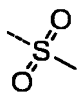,

$CH_3$,

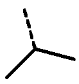, 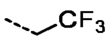, 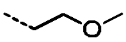, 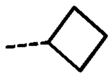, 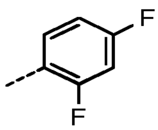, 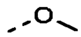, 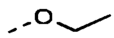, 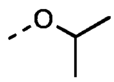,

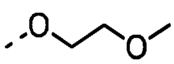, 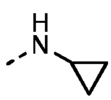 and 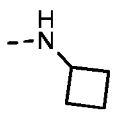,

and other variables are as defined in the present disclosure.
In some embodiments of the present disclosure, the $R_3$ is selected from cyclopropyl, cyclobutyl, cyclopentyl and cyclohexyl, and other variables are as defined in the present disclosure.
In some embodiments of the present disclosure, the $R_3$ is selected from

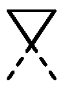,

and other variables are as defined in the present disclosure.
In some embodiments of the present disclosure, the structural moiety

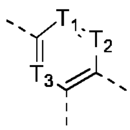

is selected from phenyl, pyridyl, pyrimidinyl, pyrazinyl and pyridazinyl, and other variables are as defined in the present disclosure.

In some embodiments of the present disclosure, the structural moiety

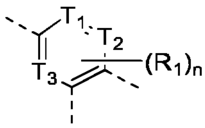

is selected from

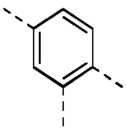 , 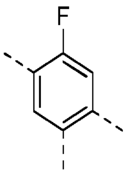 , 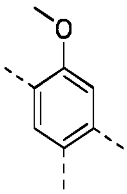 , 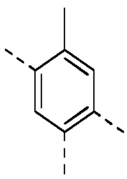 , 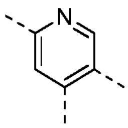 , 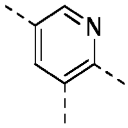 ,

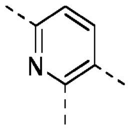 and 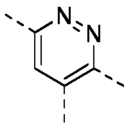 ,

and other variables are as defined in the present disclosure.

In some embodiments of the present disclosure, the structural moiety

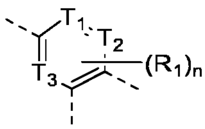

is selected from

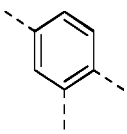 , 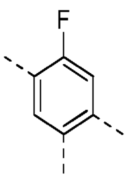 , 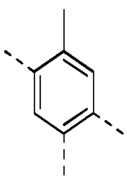 , 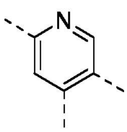 , 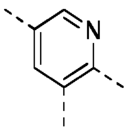 , 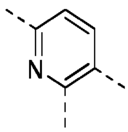

and

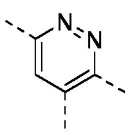 ,

and other variables are as defined in the present disclosure.

In some embodiments of the present disclosure, the ring A is selected from cyclopropyl, cyclobutyl, cyclopentyl, cyclohexyl, oxetanyl, tetrahydrofuranyl, tetrahydropyranyl, azetidinyl, piperidinyl and thietanyl, and the cyclopropyl, cyclobutyl, cyclopentyl, cyclohexyl, oxetanyl, tetrahydrofuranyl, tetrahydropyranyl, azetidinyl, piperidinyl and thietanyl are optionally substituted by 1, 2 or 3 $R_b$, and other variables are as defined in the present disclosure.

In some embodiments of the present disclosure, the ring A is selected from

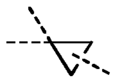,

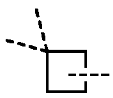, 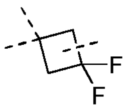, 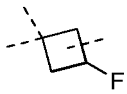, 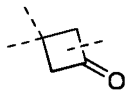, 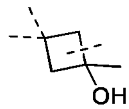, 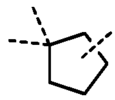, 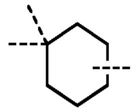, 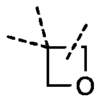,

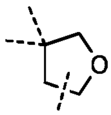, 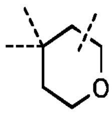, 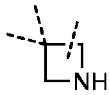, 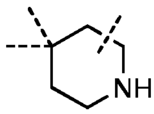, 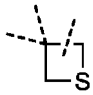 and 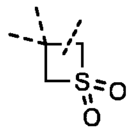,

and other variables are as defined in the present disclosure.

In some embodiments of the present disclosure, the $R_5$ is selected from H and $CH_3$, and other variables are as defined in the present disclosure.

In some embodiments of the present disclosure, the $R_6$ is selected from H, and other variables are as defined in the present disclosure.

In some embodiments of the present disclosure, the $R_7$ is selected from H, and other variables are as defined in the present disclosure.

In some embodiments of the present disclosure, the $R_6$ is selected from H, the $R_7$ is selected from H, and other variables are as defined in the present disclosure.

In some embodiments of the present disclosure, the $R_6$ is selected from H and $CH_3$, the $R_7$ is selected from H and $CH_3$, and other variables are as defined in the present disclosure.

In some embodiments of the present disclosure, the $R_8$ is selected from H and $CH_3$, the $R_9$ is selected from H and $CH_3$, and other variables are as defined in the present disclosure.

In some embodiments of the present disclosure, the $R_6$ and $R_7$ are attached together to form cyclopropyl, cyclobutyl, cyclopentyl and oxetanyl, and the cyclopropyl, cyclobutyl, cyclopentyl and oxetanyl are optionally substituted by 1, 2 or 3 F, and other variables are as defined in the present disclosure.

In some embodiments of the present disclosure, the $R_6$ and $R_7$ are attached together to form

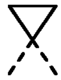 or 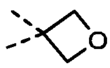.

In some embodiments of the present disclosure, the ring B is selected from cyclopropyl, cyclobutyl, cyclopentyl and cyclohexyl, and the cyclopropyl, cyclobutyl, cyclopentyl and cyclohexyl are optionally substituted by 1, 2 or 3 $R_c$, and other variables are as defined in the present disclosure.

In some embodiments of the present disclosure, the ring B is selected from

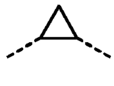,

and other variables are as defined in the present disclosure.

In some embodiments of the present disclosure, the structural moiety

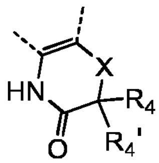

is selected from

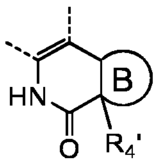 , 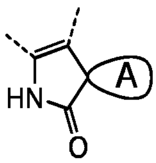 , 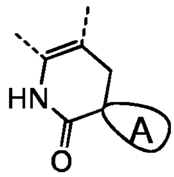 and 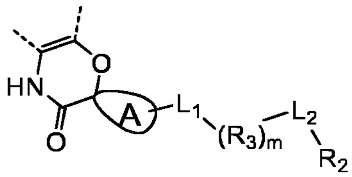 ,

and other variables are as defined in the present disclosure.

In some embodiments of the present disclosure, the structural moiety

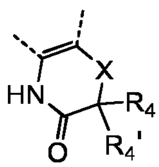

is selected from

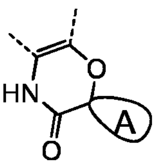 ,

and other variables are as defined in the present disclosure.

In some embodiments of the present disclosure, the structural moiety

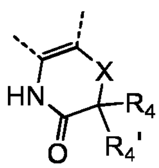

is selected from

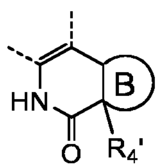 and 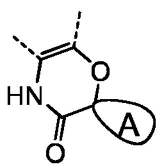 ,

and other variables are as defined in the present disclosure.

In some embodiments of the present disclosure, the structural moiety

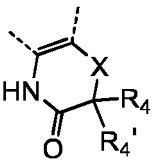

is selected from

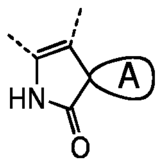 and 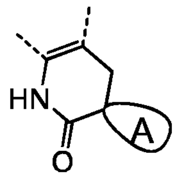,

and other variables are as defined in the present disclosure.

In some embodiments of the present disclosure, the $-L_1-(R_3)_m-L_2-R_2$ is selected from H, OH,

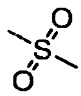,

$CH_3$,

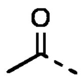, 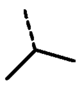, 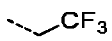, 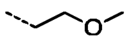, 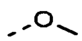, 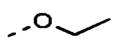, 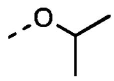,

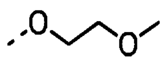, 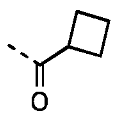, 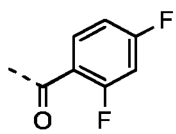, 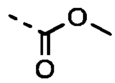, 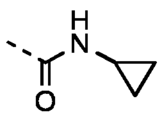 and 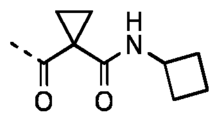,

and other variables are as defined in the present disclosure.

In some embodiments of the present disclosure, the structural moiety

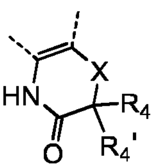

is selected from

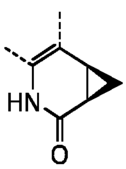, 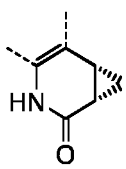, 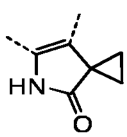, 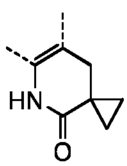, 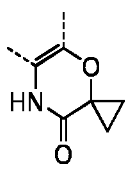, 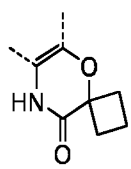,

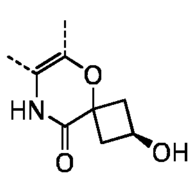 , 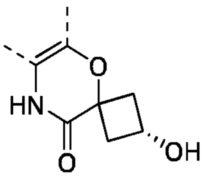 , 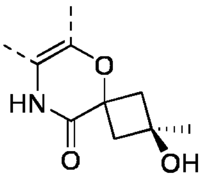 , 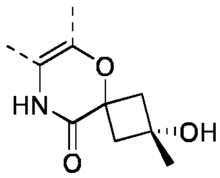 , 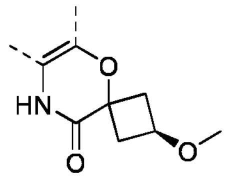 ,

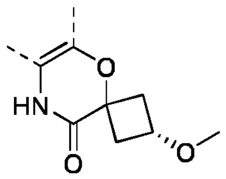 , 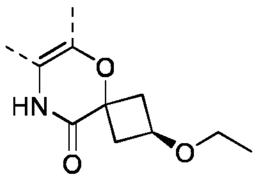 , 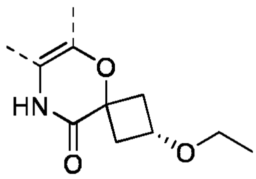 , 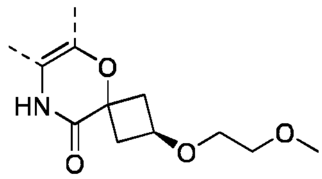 ,

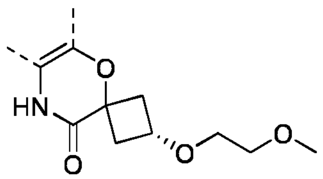 , 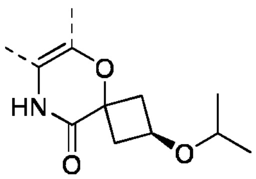 , 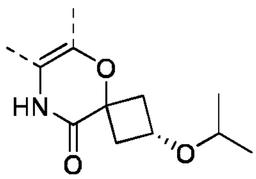 , 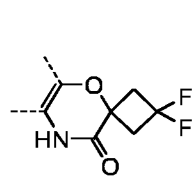 ,

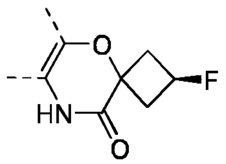 , 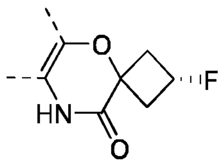 , 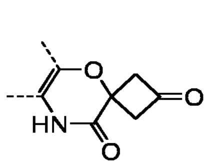 , 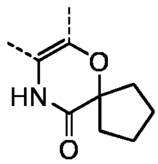 , 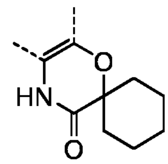 ,

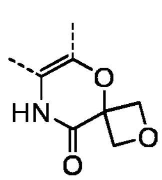 , 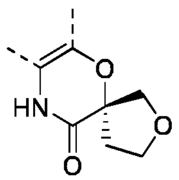 , 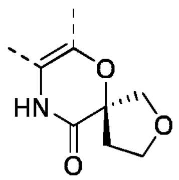 , 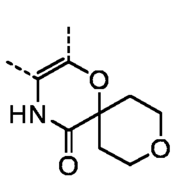 , 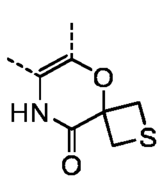 , 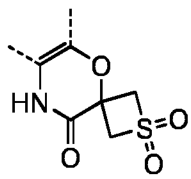 ,

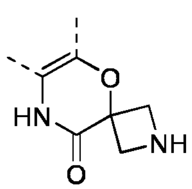 , 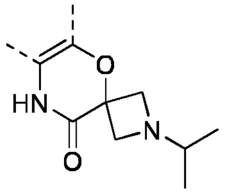 , 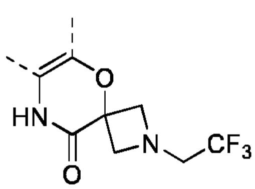 , 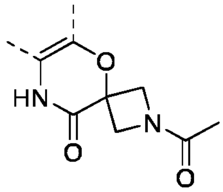 ,

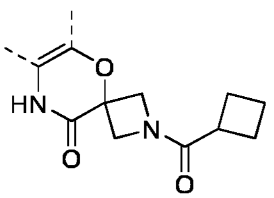 , 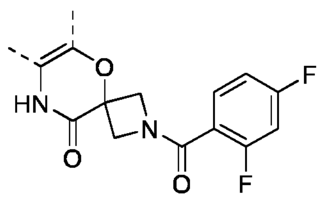 , 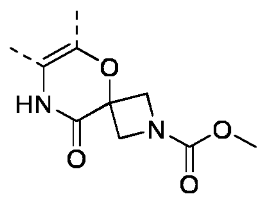 ,

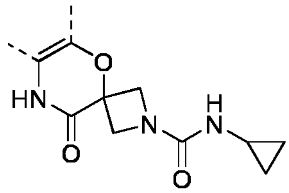 , 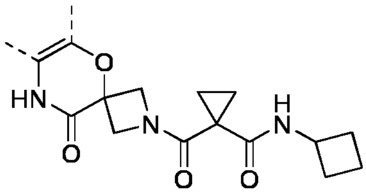 , 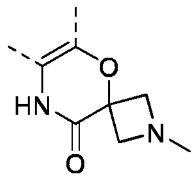 ,

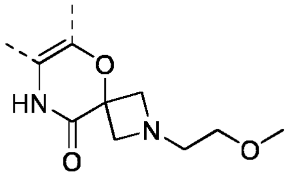 , 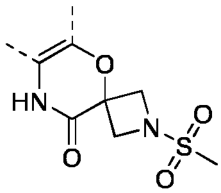 and 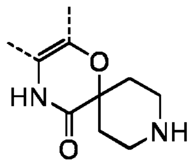 ,

and other variables are as defined in the present disclosure.

In some embodiments of the present disclosure, the compound or the pharmaceutically acceptable salt thereof is selected from:

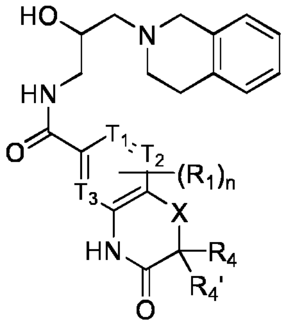

(IV) ,

wherein, $T_1$, $T_2$, $T_3$, $R_1$, $R_4$, $R_4$', X and n are as defined in the present disclosure.

In some embodiments of the present disclosure, the compound or the pharmaceutically acceptable salt thereof is selected from:

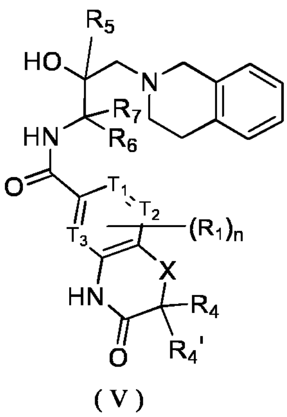

(V) ,

wherein, $T_1$, $T_2$, $T_3$, $R_1$, $R_4$, $R_4$', $R_5$, $R_6$, $R_7$, X and n are as defined in the present disclosure.

In some embodiments of the present disclosure, the compound or the pharmaceutically acceptable salt thereof is selected from:

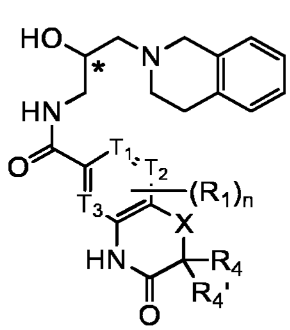
( IV-1) ,
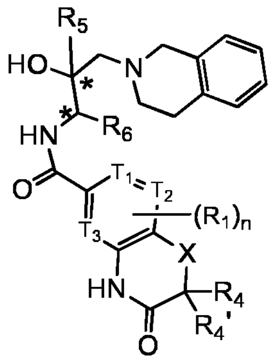
( V-1 ) and
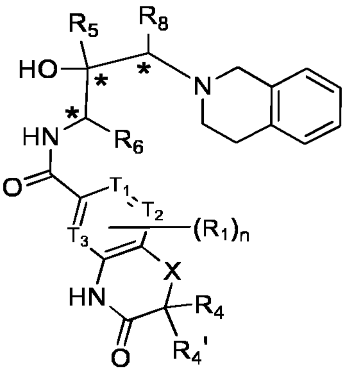
( VII-1 ) ,

wherein,

$T_1$, $T_2$, $T_3$, $R_1$, $R_4$, $R_4$', $R_5$, X and n are as defined in the present disclosure;
$R_6$ is selected from F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
$R_8$ is selected from F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
the carbon atom with "*" is a chiral carbon atom, which exists in a (*R*) or (*S*) single enantiomer form or a (*R*) or (*S*) single enantiomer-rich form.

In some embodiments of the present disclosure, the compound or the pharmaceutically acceptable salt thereof is selected from:

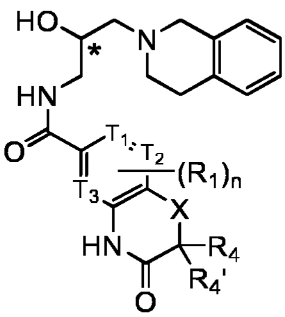
( IV-1) and
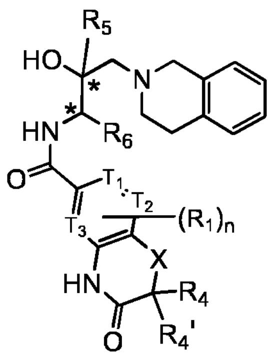
( V-1 ) ,

wherein,

$T_1$, $T_2$, $T_3$, $R_1$, $R_4$, $R_4$', $R_5$, $R_6$, X and n are as defined in the present disclosure;
the carbon atom with "*" is a chiral carbon atom, which exists in a *(R)* or (*S*) single enantiomer form or a (*R*) or (*S*) single enantiomer-rich form.

In some embodiments of the present disclosure, the compound or the pharmaceutically acceptable salt thereof is selected from:

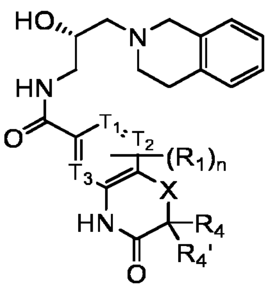
( IV-1-1) ,
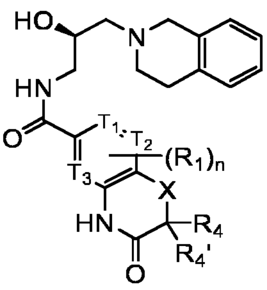
( IV-1-2) and
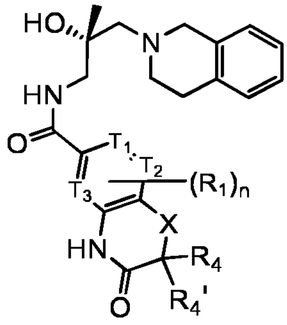
( V-1-1 ) ,

wherein,
$T_1$, $T_2$, $T_3$, $R_1$, $R_4$, $R_4$', X and n are as defined in the present disclosure.

In some embodiments of the present disclosure, the compound or the pharmaceutically acceptable salt thereof is selected from:

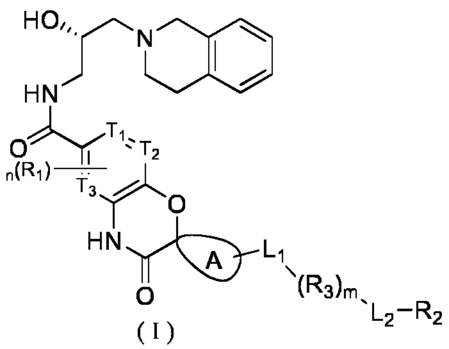
( I ) ,
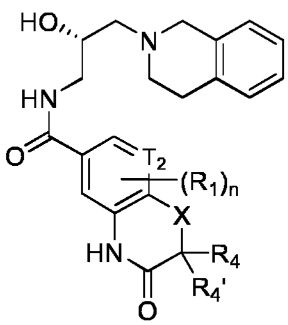
( IV-1-1-1) and
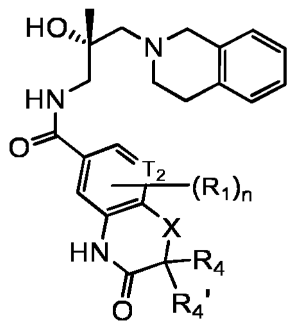
( V-1-1-1 ) ,

wherein, $T_1$, $T_2$, $T_3$, $R_1$, $R_2$, $R_3$, $R_4$, $R_4$', $L_1$, $L_2$, X, ring A, m and n are as defined in the present disclosure.

In some embodiments of the present disclosure, the compound or the pharmaceutically acceptable salt thereof is selected from:

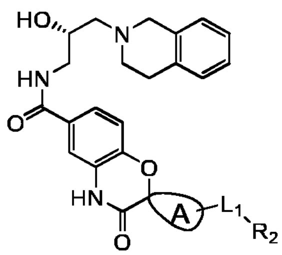
( I-a ) ,
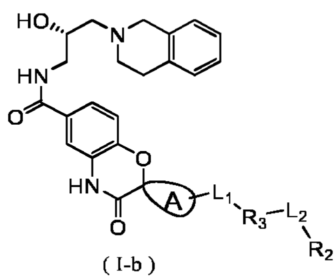
( I-b ) ,
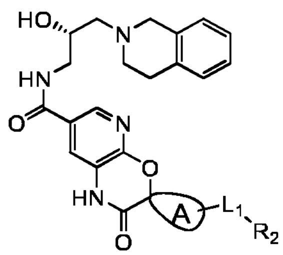
( II-a ) ,

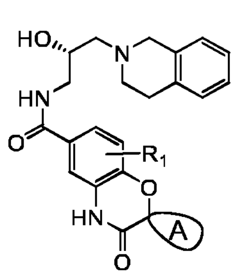
( IV-a ) ,

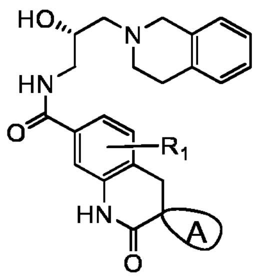
( IV-b ) ,

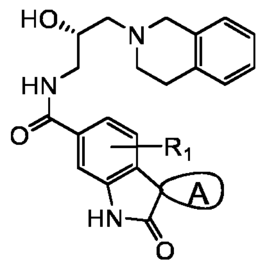
( IV-c ) ,

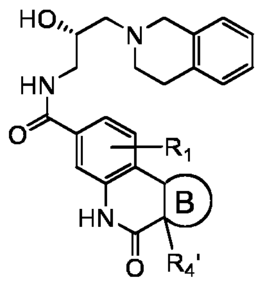
( IV-d ) ,

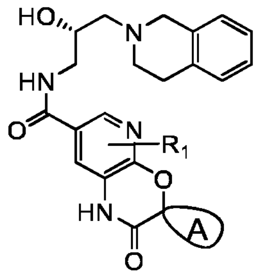
( IV-e ) ,

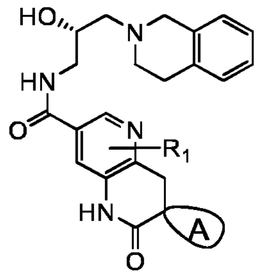
( IV-f ) ,

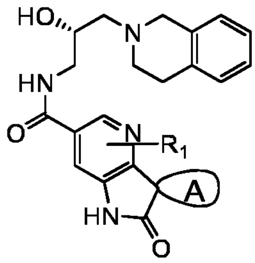
( IV-g ) ,

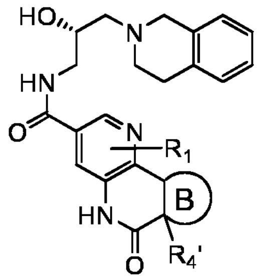
( IV-h ) ,

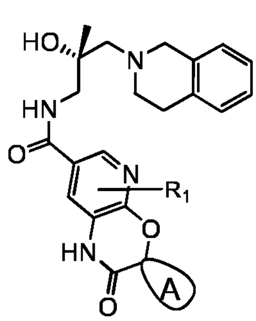
( V-a ) ,

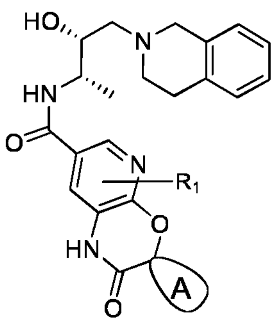
( VII-a ) ,

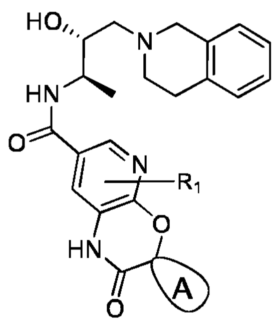
( VII-b ) ,

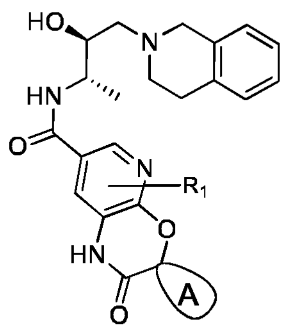
( VII-c ) ,

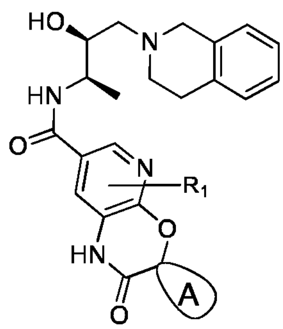
( VII-d ) ,

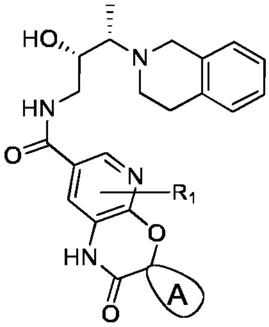
( VII-e ) ,

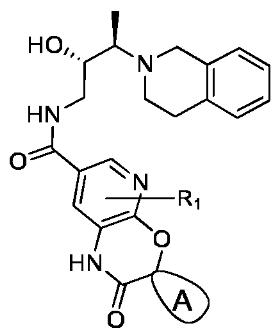
( VII-f ) ,

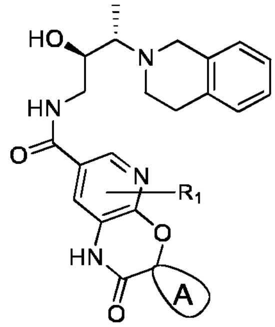
( VII-g )

and

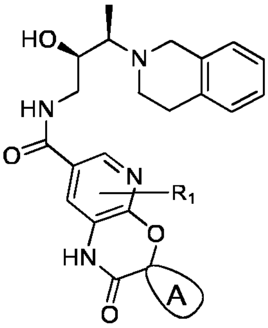

( VII-h ) ,

wherein, ring A, ring B, $R_1$, $R_2$, $R_3$, $R_4$', $L_1$ and $L_2$ are as defined in the present disclosure.

The present disclosure provides a compound represented by formula (VII) or a pharmaceutically acceptable salt thereof,

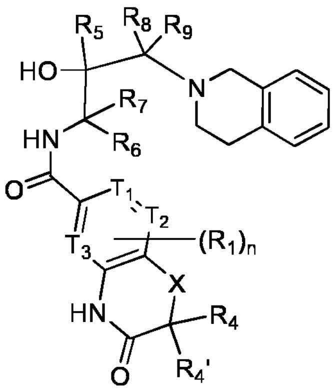

( VII ) ,

wherein,

$T_1$, $T_2$ and $T_3$ are each independently selected from CH and N;
$R_1$ is each independently selected from H, F, Cl, Br, I and $CH_3$;
$R_4$' is selected from H and $C_{1-3}$ alkyl;
$R_5$ is selected from H, F, Cl and $C_{1-3}$ alkyl, and the $C_{1-3}$ alkyl is optionally substituted by 1, 2 or 3 F;
$R_6$ and $R_8$ are each independently selected from H, F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
$R_7$ and $R_9$ are each independently selected from H, F, Cl and $C_{1-3}$ alkyl, and the $C_{1-3}$ alkyl is optionally substituted by 1, 2 or 3 F;
alternatively, $R_6$ and $R_7$ form $C_{3-6}$ cycloalkyl or 4- to 6-membered heterocycloalkyl together with the atoms to which they are attached, and the $C_{3-6}$ cycloalkyl and the 4- to 6-membered heterocycloalkyl are optionally substituted by 1, 2 or 3 F;
alternatively, $R_8$ and $R_9$ form $C_{3-6}$ cycloalkyl or 4- to 6-membered heterocycloalkyl together with the atoms to which they are attached, and the $C_{3-6}$ cycloalkyl and the 4- to 6-membered heterocycloalkyl are optionally substituted by 1, 2 or 3 F;
when X is selected from a single bond, $CH_2$ and O, $R_4$ and $R_4$' form moiety

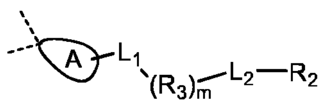

together with the atoms to which they are attached;

alternatively, X and $R_4$ are attached together to form a ring B;
the ring B is selected from $C_{3-6}$ cycloalkyl, and the $C_{3-6}$ cycloalkyl is optionally substituted by 1, 2 or 3 $R_c$;
$L_1$ and $L_2$ are each independently selected from a single bond and -C(=O)-;
$R_2$ is selected from H, OH,

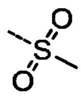

,

$C_{1-3}$ alkyl, $C_{1-3}$ alkoxy, -NH-$C_{3-6}$ cycloalkyl, $C_{3-6}$ cycloalkyl and phenyl, and the $C_{1-3}$ alkyl, $C_{1-3}$ alkoxy, -NH-$C_{3-6}$ cycloalkyl, $C_{3-6}$ cycloalkyl and phenyl are optionally substituted by 1, 2 or 3 $R_a$;
$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
m is selected from 0 and 1;
n is selected from 0, 1, 2 and 3;
$R_a$ and $R_c$ are each independently selected from H, F, Cl, Br, I, OH, -$OCH_3$ and $NH_2$;
$R_b$ is each independently selected from H, F, Cl, Br, I, =O, OH, $NH_2$, $C_{1-3}$ alkyl and $C_{1-3}$ alkoxy;
the 4- to 6-membered heterocycloalkyl contains 1, 2, 3 or 4 heteroatoms or heteroatom groups independently selected from -NH-, -O-, -S- and N.

The present disclosure also provides a compound represented by formula (V) or a pharmaceutically acceptable salt thereof,

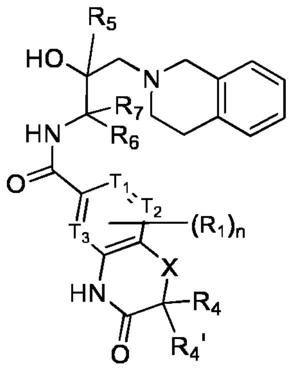

(V)

,

wherein,

$T_1$, $T_2$ and $T_3$ are each independently selected from CH and N;
$R_1$ is each independently selected from H, F, Cl, Br, I and $CH_3$;
$R_4$' is selected from H and $C_{1-3}$ alkyl;
$R_5$ is selected from H, F, Cl and $C_{1-3}$ alkyl, and the $C_{1-3}$ alkyl is optionally substituted by 1, 2 or 3 F;
$R_6$ is selected from H, F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
$R_7$ is selected from H, F, Cl and $C_{1-3}$ alkyl, and the $C_{1-3}$ alkyl is optionally substituted by 1, 2 or 3 F;
alternatively, $R_6$ and $R_7$ are attached together to form $C_{3-6}$ cycloalkyl, and the $C_{3-6}$ cycloalkyl is optionally substituted by 1, 2 or 3 F;
X is selected from a single bond, $CH_2$ and O, and $R_4$ and $R_4$' form moiety

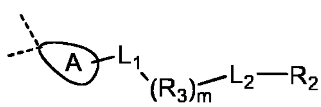

together with the atoms to which they are attached;
alternatively, X and $R_4$ are attached together to form a ring B;
the ring B is selected from $C_{3-6}$ cycloalkyl, and the $C_{3-6}$ cycloalkyl is optionally substituted by 1, 2 or 3 $R_c$;
$L_1$ and $L_2$ are each independently selected from a single bond and -C(=O)-;
$R_2$ is selected from H, OH,

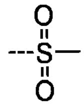

,

$C_{1-3}$ alkyl, $C_{1-3}$ alkoxy, -NH-$C_{3-6}$ cycloalkyl, $C_{3-6}$ cycloalkyl and phenyl, and the $C_{1-3}$ alkyl, $C_{1-3}$ alkoxy, -NH-$C_{3-6}$ cycloalkyl, $C_{3-6}$ cycloalkyl and phenyl are optionally substituted by 1, 2 or 3 $R_a$;
$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
m is selected from 0 and 1;
n is selected from 0, 1, 2 and 3;
$R_a$ and $R_c$ are each independently selected from H, F, Cl, Br, I, OH, $-OCH_3$ and $NH_2$;
$R_b$ is each independently selected from H, F, Cl, Br, I, =O, OH, $NH_2$, $C_{1-3}$ alkyl and $C_{1-3}$ alkoxy;
the 4- to 6-membered heterocycloalkyl contains 1, 2, 3 or 4 heteroatoms or heteroatom groups independently selected from -NH-, -O-, -S- and N.

The present disclosure provides a compound represented by formula (IV) or a pharmaceutically acceptable salt thereof,
wherein,

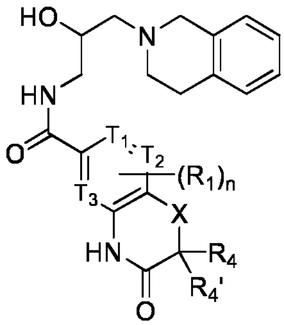

(IV)

,

$T_1$, $T_2$ and $T_3$ are each independently selected from CH and N;
$R_1$ is each independently selected from H, F, Cl, Br, I and $CH_3$;
$R_4$' is selected from H and $C_{1-3}$ alkyl;
X is selected from a single bond, $CH_2$ and O, and $R_4$ and $R_4$' form moiety

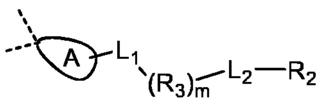

together with the atoms to which they are attached;
alternatively, X and $R_4$ are attached together to form a ring B;
the ring B is selected from $C_{3-6}$ cycloalkyl, and the $C_{3-6}$ cycloalkyl is optionally substituted by 1, 2 or 3 $R_c$;
$L_1$ and $L_2$ are each independently selected from a single bond and -C(=O)-;
$R_2$ is selected from H, OH,

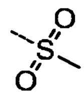

,

$C_{1-3}$ alkyl, $C_{1-3}$ alkoxy, -NH-$C_{3-6}$ cycloalkyl, $C_{3-6}$ cycloalkyl and phenyl, and the $C_{1-3}$ alkyl, $C_{1-3}$ alkoxy, -NH-$C_{3-6}$ cycloalkyl, $C_{3-6}$ cycloalkyl and phenyl are optionally substituted by 1, 2 or 3 $R_a$;
$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
m is selected from 0 and 1;
n is selected from 0, 1, 2 and 3;
$R_a$ and $R_c$ are each independently selected from H, F, Cl, Br, I, OH, $-OCH_3$ and $NH_2$;
$R_b$ is selected from H, F, Cl, Br, I, =O, OH, $NH_2$, $C_{1-3}$ alkyl and $C_{1-3}$ alkoxy;
the 4- to 6-membered heterocycloalkyl contains 1, 2, 3 or 4 heteroatoms or heteroatom groups independently selected from -NH-, -O-, -S- and N.

The present disclosure provides a compound represented by formula (I) or a pharmaceutically acceptable salt thereof,

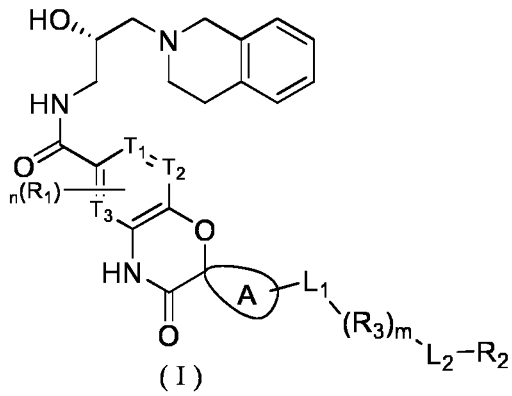

(I)

,

wherein,

$L_1$ and $L_2$ are each independently selected from a single bond and -C(=O)-;
$R_1$ is each independently selected from H, F, Cl, Br, I and $CH_3$;
m is selected from 0 and 1;
n is selected from 0, 1, 2 and 3;
$R_2$ is selected from H, OH,

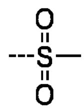

,

$C_{1-3}$ alkyl, $C_{1-3}$ alkoxy, $C_{3-6}$ cycloalkyl-NH-, $C_{3-6}$ cycloalkyl and phenyl, and the $C_{1-3}$ alkyl, $C_{1-3}$ alkoxy, $C_{3-6}$ cycloalkyl-NH-, $C_{3-6}$ cycloalkyl and phenyl are optionally substituted by 1, 2 or 3 $R_a$;
$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
$T_1$, $T_2$ and $T_3$ are each selected from CH and N;
$R_a$ is selected from H, F, Cl, Br, I, OH, $-OCH_3$ and $NH_2$;
$R_b$ is selected from H, F, Cl, Br, I, =O, OH, $NH_2$, $C_{1-3}$ alkyl and $C_{1-3}$ alkoxy;
the 4- to 6-membered heterocycloalkyl contains 1, 2, 3 or 4 heteroatoms or heteroatom groups independently selected from -NH-, -O-, -S- and N.

The present disclosure provides a compound represented by formula (I) or a pharmaceutically acceptable salt thereof,

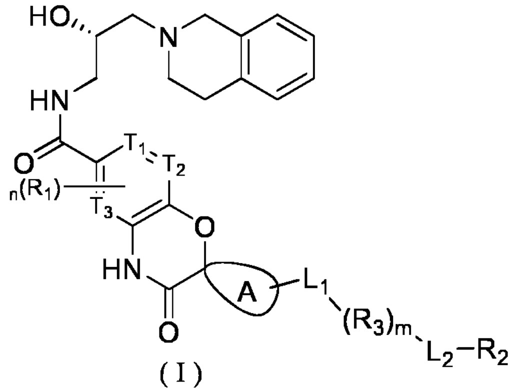

(I)

,

wherein,

- $L_1$ and $L_2$ are each independently selected from a single bond and -C(=O)-;
- $R_1$ is each independently selected from H, F, Cl, Br, I;
- m is selected from 0 and 1;
- n is selected from 0, 1, 2 and 3;
- $R_2$ is selected from H, $C_{1-3}$ alkyl, $C_{1-3}$ alkoxy, $C_{3-6}$ cycloalkyl-NH-, $C_{3-6}$ cycloalkyl and phenyl, and the $C_{1-3}$ alkyl, $C_{1-3}$ alkoxy, $C_{3-6}$ cycloalkyl-NH-, $C_{3-6}$ cycloalkyl and phenyl are optionally substituted by 1, 2 or 3 $R_a$;
- $R_3$ is selected from $C_{3-6}$ cycloalkyl;
- ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
- $T_1$, $T_2$ and $T_3$ are each selected from CH and N;
- $R_a$ is selected from H, F, Cl, Br, I, OH, $NH_2$;
- $R_b$ is selected from H, F, Cl, Br, I, OH, $NH_2$, $C_{1-3}$ alkyl and $C_{1-3}$ alkoxy;
- the 4- to 6-membered heterocycloalkyl each independently contains 1, 2, 3 or 4 heteroatoms or heteroatom groups independently selected from -NH-, -O-, -S- and N.

The present disclosure provides a compound represented by formula (I) or a pharmaceutically acceptable salt thereof,

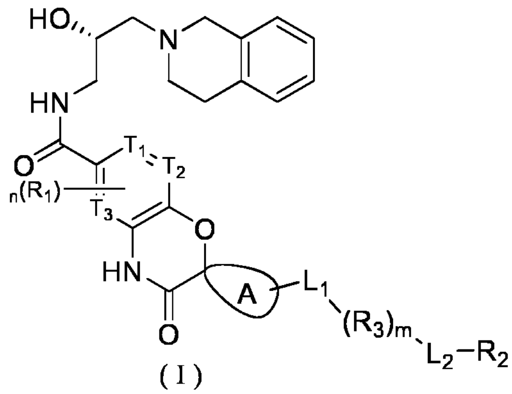

(I)

,

wherein,

- $L_1$ and $L_2$ are each independently selected from a single bond and -C(=O)-;
- $R_1$ is each independently selected from H, F, Cl, Br, I;
- m is selected from 0 and 1;
- n is selected from 0, 1, 2 and 3;
- $R_2$ is selected from H, $C_{1-3}$ alkoxy, $C_{3-6}$ cycloalkyl-NH-, $C_{3-6}$ cycloalkyl and phenyl, and the $C_{1-3}$ alkoxy, $C_{3-6}$ cycloalkyl-

NH-, $C_{3-6}$ cycloalkyl and phenyl are optionally substituted by 1, 2 or 3 $R_a$;
$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
$T_1$, $T_2$ and $T_3$ are each selected from CH and N;
$R_a$ is selected from H, F, Cl, Br, I, OH, $NH_2$;
$R_b$ is selected from H, F, Cl, Br, I, OH, $NH_2$, $C_{1-3}$ alkyl and $C_{1-3}$ alkoxy;
the 4- to 6-membered heterocycloalkyl each independently contains 1, 2, 3 or 4 heteroatoms or heteroatom groups independently selected from -NH-, -O-, -S- and N.
In some embodiments of the present disclosure, the $R_2$ is selected from H, $C_{1-3}$ alkyl, $C_{1-3}$ alkoxy, phenyl, cyclobutyl, cyclobutyl-NH- and cyclopropyl-NH-, and the $C_{1-3}$ alkyl, $C_{1-3}$ alkoxy, phenyl, cyclobutyl, cyclobutyl-NH- and cyclopropyl-NH- are optionally substituted by 1, 2 or 3 $R_a$, and $R_a$ and other variables are as defined in the present disclosure.

In some embodiments of the present disclosure, the $R_2$ is selected from H, $C_{1-3}$ alkoxy, phenyl, cyclobutyl, cyclobutyl-NH- and cyclopropyl-NH-, and the $C_{1-3}$ alkoxy, phenyl, cyclobutyl, cyclobutyl-NH- and cyclopropyl-NH- are optionally substituted by 1, 2 or 3 $R_a$, and $R_a$ and other variables are as defined in the present disclosure.
In some embodiments of the present disclosure, the $R_2$ is selected from H, OH,

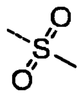,

$CH_3$,

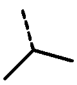, 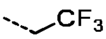, 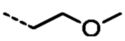, 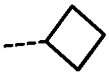, 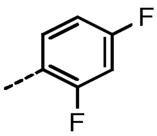, 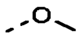, 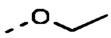, 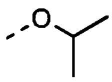,

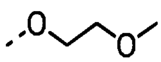, 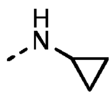 and 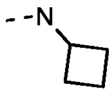,

and other variables are as defined in the present disclosure.
In some embodiments of the present disclosure, the $R_2$ is selected from H, $CH_3$,

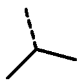,

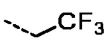, 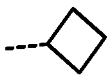, 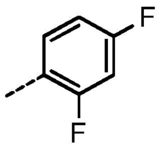, 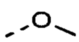, 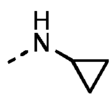 and 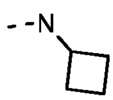,

and other variables are as defined in the present disclosure.
In some embodiments of the present disclosure, the $R_2$ is selected from H,

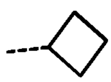,

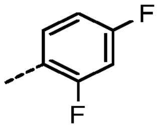, 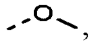, 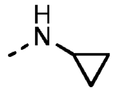 and 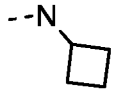,

and other variables are as defined in the present disclosure.

In some embodiments of the present disclosure, the $R_b$ is selected from H, F, Cl, Br, I, =O, OH and $NH_2$, and other variables are as defined in the present disclosure.

In some embodiments of the present disclosure, the $R_b$ is selected from H, F, Cl, Br, I, OH and $NH_2$, and other variables are as defined in the present disclosure.

In some embodiments of the present disclosure, the ring A is selected from cyclopropyl, cyclobutyl, cyclopentyl, cyclohexyl, oxetanyl, azetidinyl, thietanyl, piperidinyl, tetrahydrofuranyl and tetrahydropyranyl, and the cyclopropyl, cyclobutyl, cyclopentyl, cyclohexyl, oxetanyl, azetidinyl, thietanyl, piperidinyl, tetrahydrofuranyl and tetrahydropyranyl are optionally substituted by 1, 2 or 3 $R_b$, and $R_b$ and other variables are as defined in the present disclosure.

In some embodiments of the present disclosure, the ring A is selected from cyclopropyl, cyclobutyl, cyclopentyl, cyclohexyl, oxetanyl, azetidinyl, thietanyl, piperidinyl and tetrahydropyranyl, and the cyclopropyl, cyclobutyl, cyclopentyl, cyclohexyl, oxetanyl, azetidinyl, thietanyl, piperidinyl and tetrahydropyranyl are optionally substituted by 1, 2 or 3 $R_b$, and $R_b$ and other variables are as defined in the present disclosure.

In some embodiments of the present disclosure, the ring A is selected from

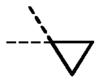,

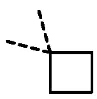, 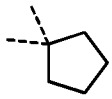, 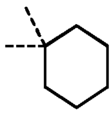, 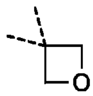, 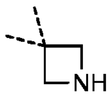, 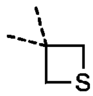, 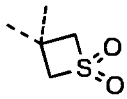, 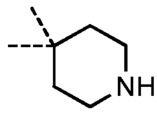,

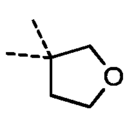 and 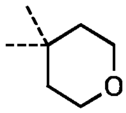,

and the

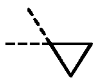, 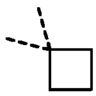, 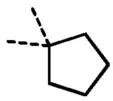, 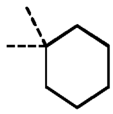, 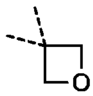, 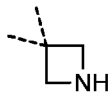,

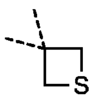, 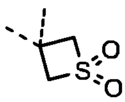, 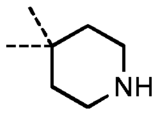, 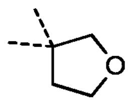 and 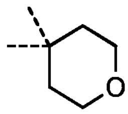

are optionally substituted by 1, 2 or 3 $R_b$, and $R_b$ and other variables are as defined in the present disclosure.

In some embodiments of the present disclosure, the ring A is selected from

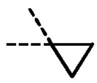,

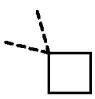, 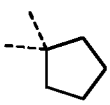, 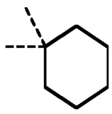, 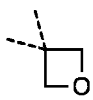, 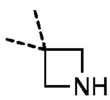, 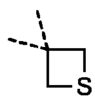, 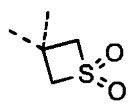, 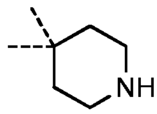

and

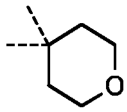,

and the

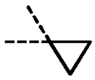, 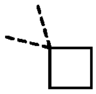, 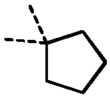, 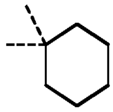, 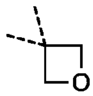, 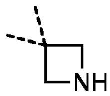, 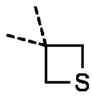,

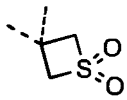, 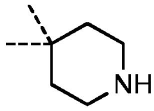 and 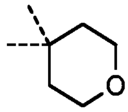

are optionally substituted by 1, 2 or 3 $R_b$, and other variables are as defined in the present disclosure.

In some embodiments of the present disclosure, the structural moiety

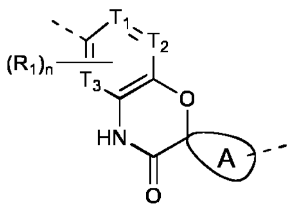

is selected from

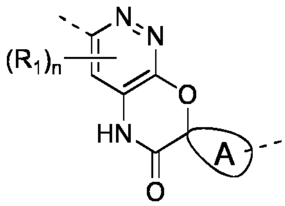, 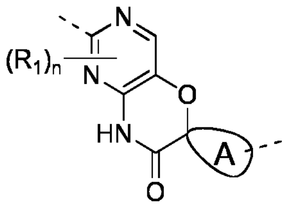,

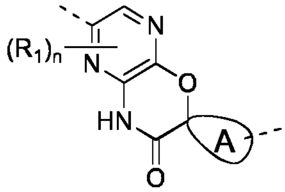, 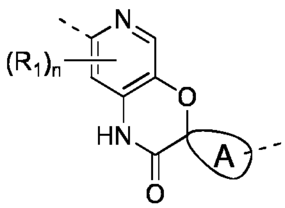, 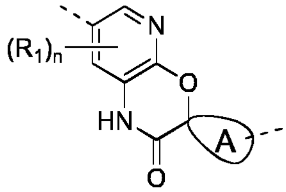,

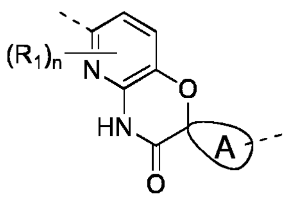 and 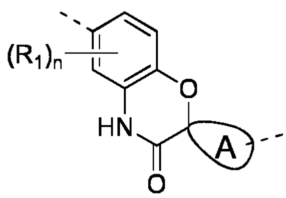,

and ring A, $R_1$ and n are as defined in the present disclosure.

In some embodiments of the present disclosure, the structural moiety

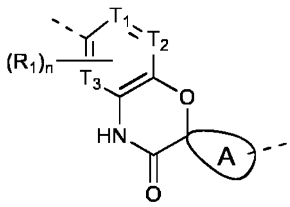

is selected from

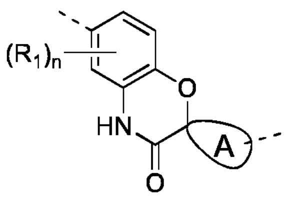
,
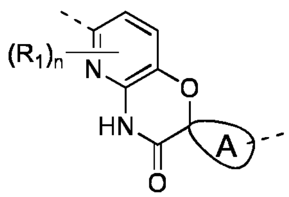
,

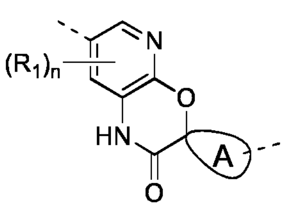
,
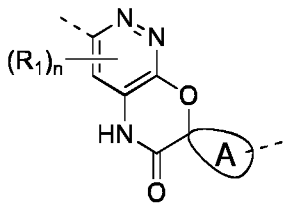
and
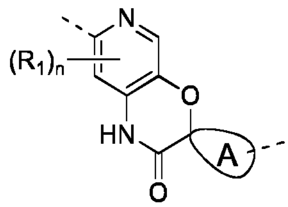
,

and ring A, $R_1$ and n are as defined in the present disclosure.

In some embodiments of the present disclosure, the structural moiety

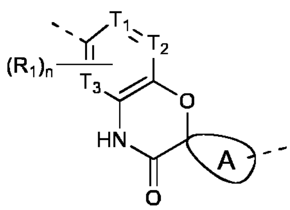

is selected from

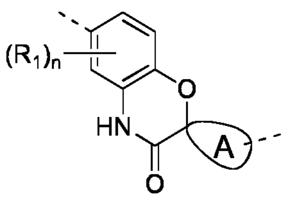
,

and ring A, $R_1$ and n are as defined in the present disclosure.

In some embodiments of the present disclosure, the structural moiety

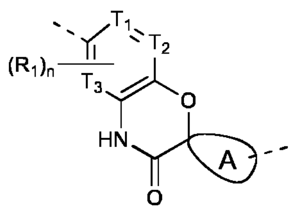

is selected from

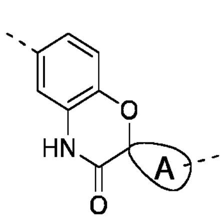 , 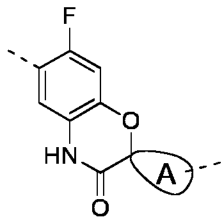 ,

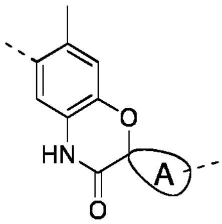 , 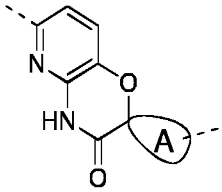 , 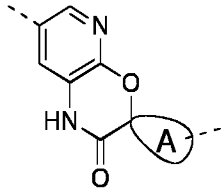 , 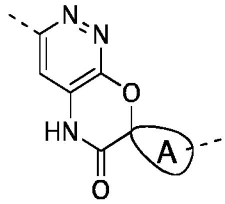

and

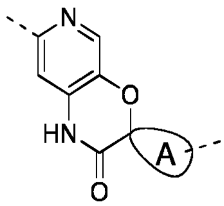 ,

and ring A and other variables are as defined in the present disclosure.

In some embodiments of the present disclosure, the structural moiety

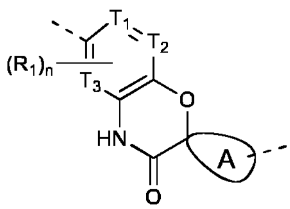

is selected from

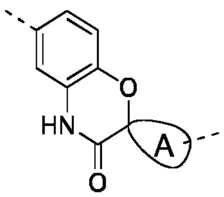 ,

and ring A and other variables are as defined in the present disclosure.

In some embodiments of the present disclosure, the $R_2$-$L_2$-$(R_3)_m$-$L_1$- is selected from H, OH,

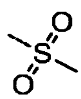 ,

$CH_3$,

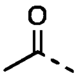, 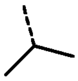, 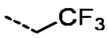, 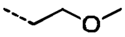, 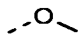, 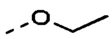, 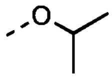,

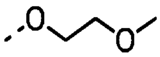, 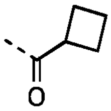, 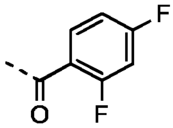, 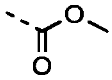, 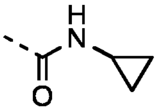 and 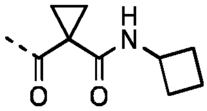,

and other variables are as defined in the present disclosure.

In some embodiments of the present disclosure, the $R_2$-$L_2$-$(R_3)_m$-$L_1$- is selected from H,

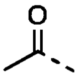, 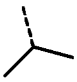, 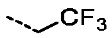, 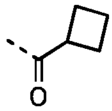, 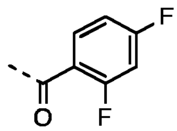, 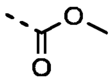, 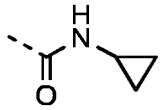

and

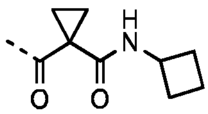,

and other variables are as defined in the present disclosure.

In some embodiments of the present disclosure, the $R_2$-$L_2$-$(R_3)_m$-$L_1$- is selected from

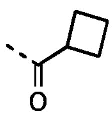, 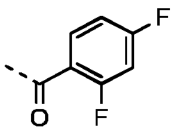, 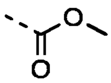, 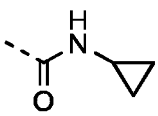 and 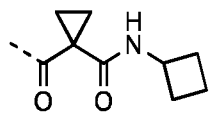,

and other variables are as defined in the present disclosure.

In some embodiments of the present disclosure, the structural moiety

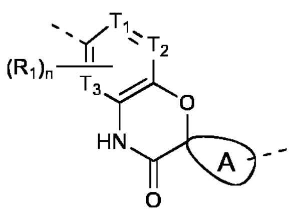

is selected from

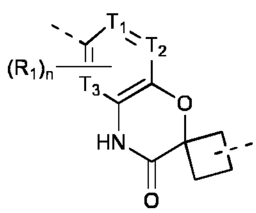, 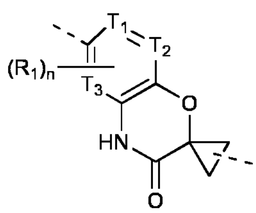,

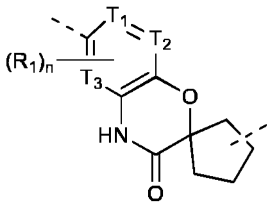, 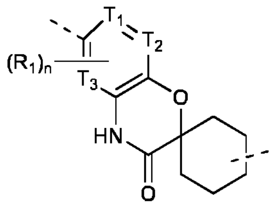, 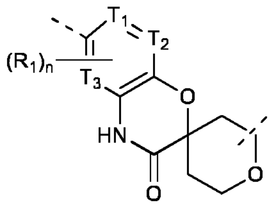, 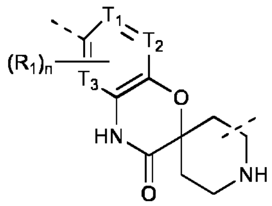,

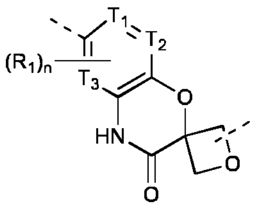, 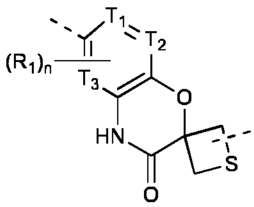, 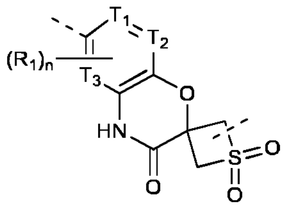, 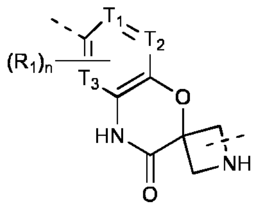

and

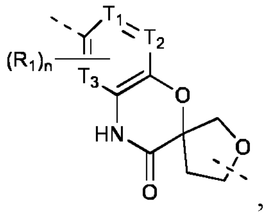,

and $T_1$, $T_2$, $T_3$, $R_1$ and n are as defined in the present disclosure.

In some embodiments of the present disclosure, the structural moiety

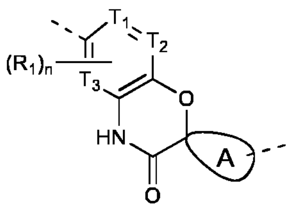

is selected from

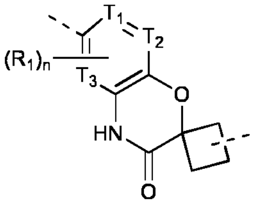, 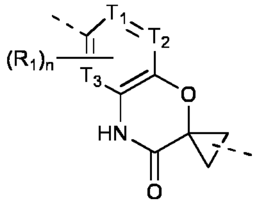,

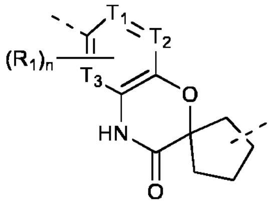, 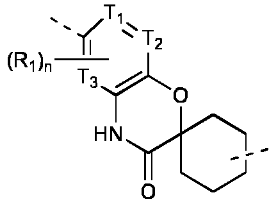, 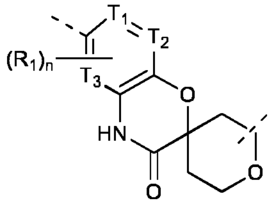, 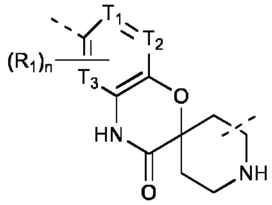,

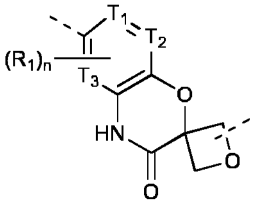,
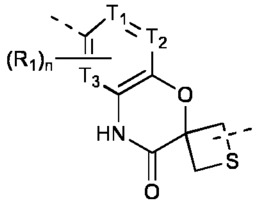,
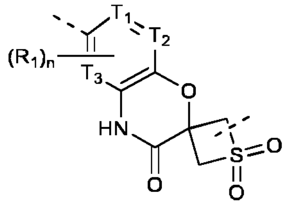 and
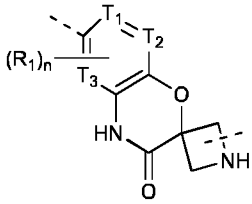,

and $T_1$, $T_2$, $T_3$, $R_1$ and n are as defined in the present disclosure.

In some embodiments of the present disclosure, the structural moiety

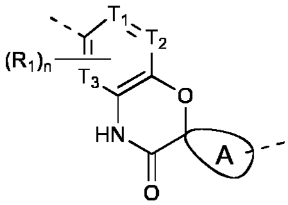

is selected from

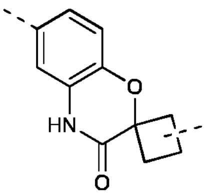,
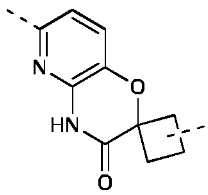,
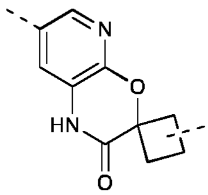,

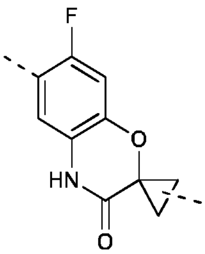,
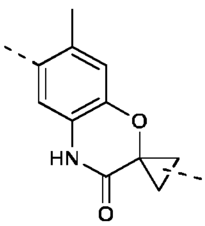,
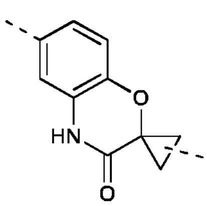,
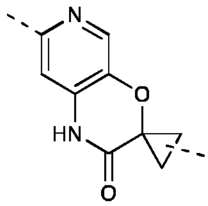,
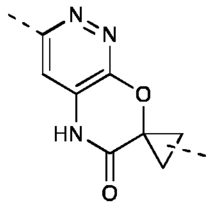,

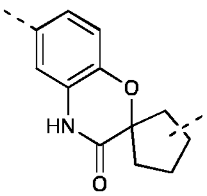,
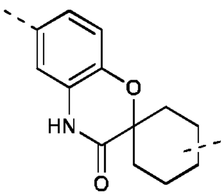,
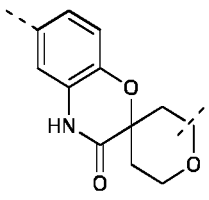,
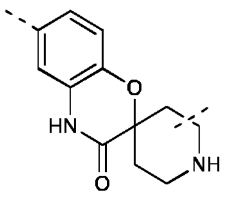,
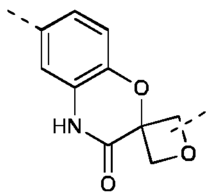,

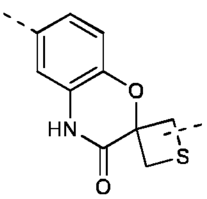,
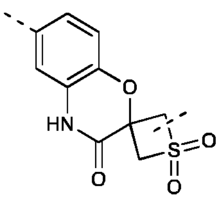,
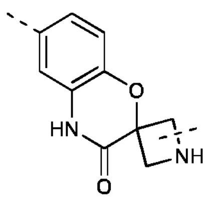 and
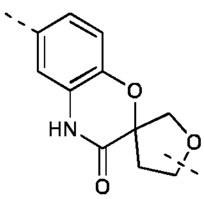,

and other variables are as defined in the present disclosure.

In some embodiments of the present disclosure, the compound or the pharmaceutically acceptable salt thereof is selected from:

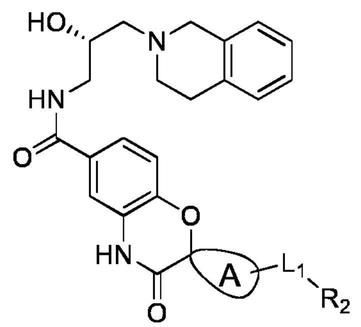

( I-a ) or

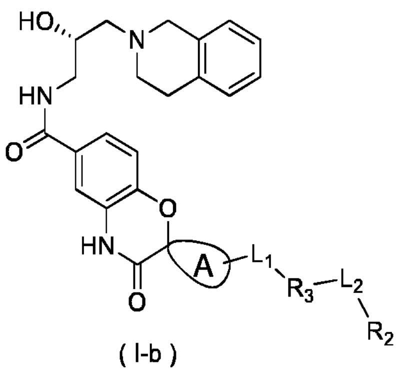

( I-b )

wherein, $L_1$, $L_2$, $R_2$, $R_3$ and ring A are as defined in the present disclosure.

In some embodiments of the present disclosure, the compound or the pharmaceutically acceptable salt thereof is selected from:

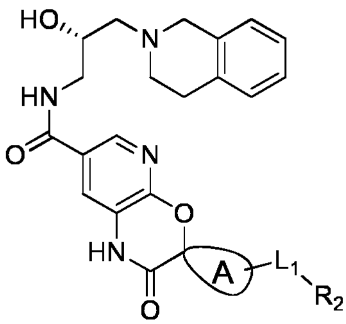

( II -a ) ,

wherein, $L_1$, $R_2$ and ring A are as defined in the present disclosure.

There are still some embodiments of the present disclosure which are obtained by any combination of the above variables.

The present disclosure also provides a compound represented by the following formulas or a pharmaceutically acceptable salt thereof:

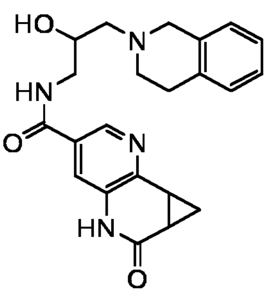

,

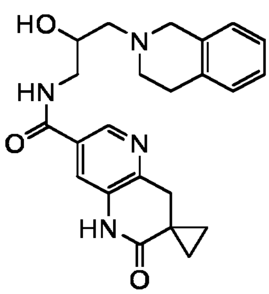

,

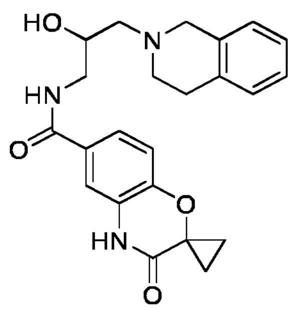

,

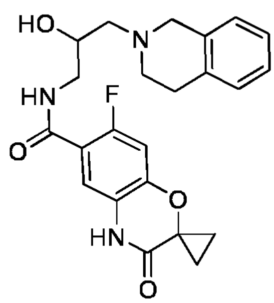

,

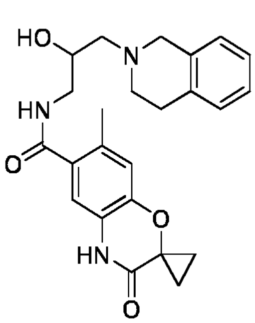 , 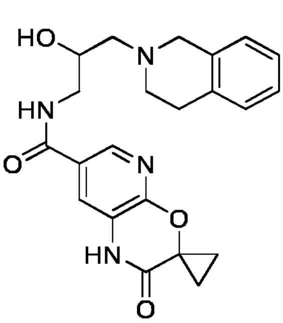 , 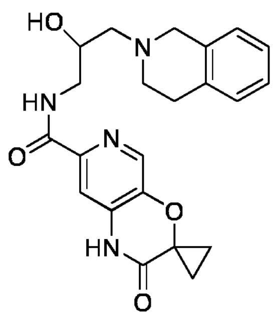 , 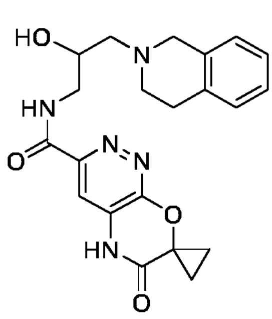 ,

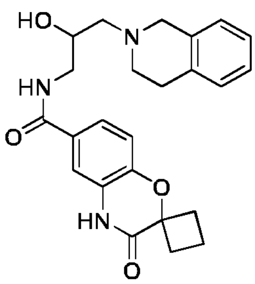 , 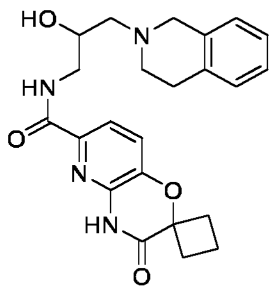 , 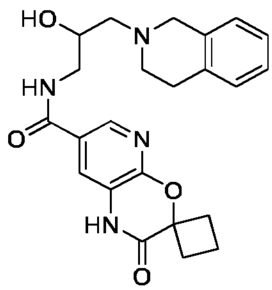 , 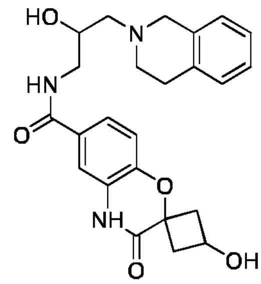 ,

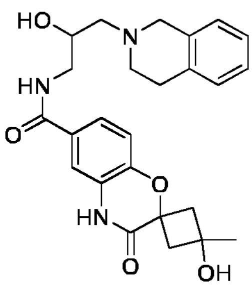 , 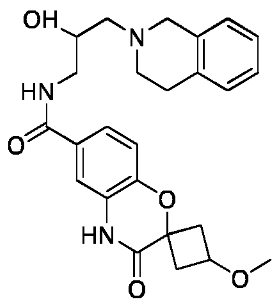 , 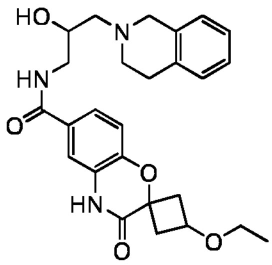 , 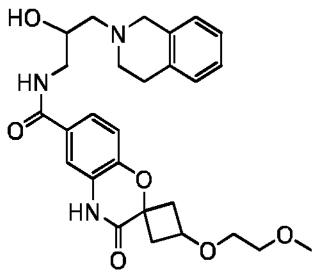 ,

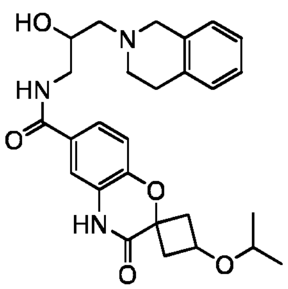 , 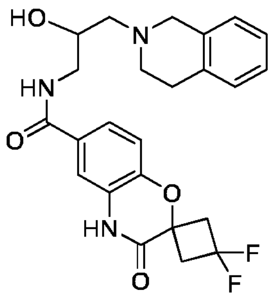 , 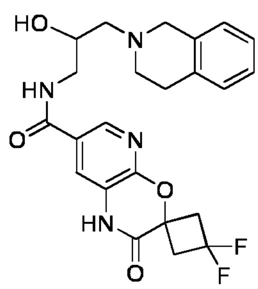 , 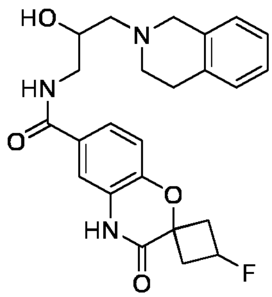 ,

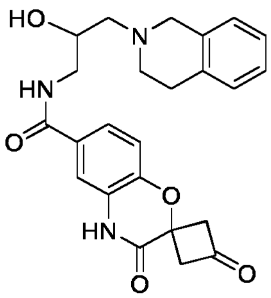 , 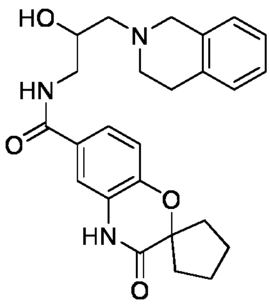 , 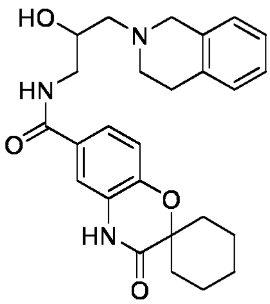 , 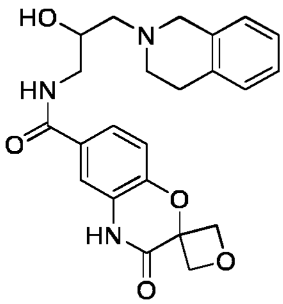 ,

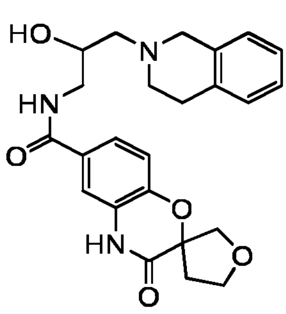 ,

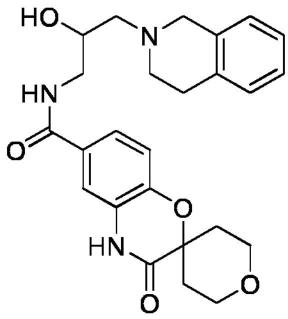 ,

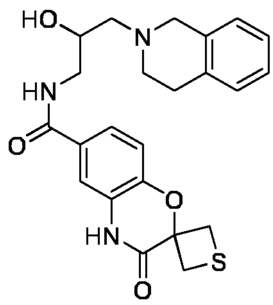 ,

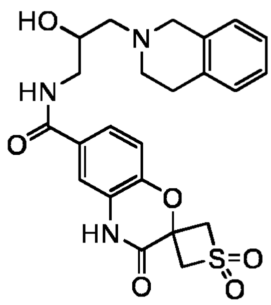 ,

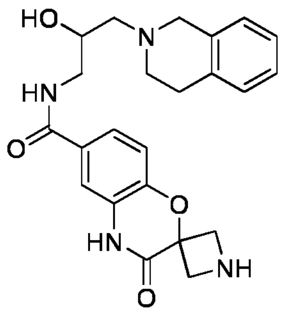 ,

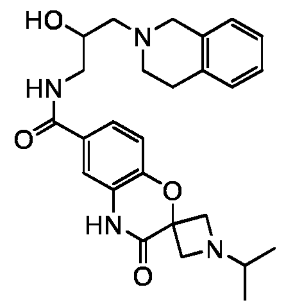 ,

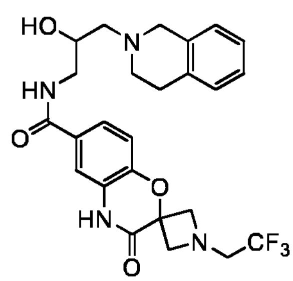 ,

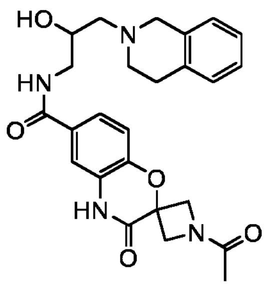 ,

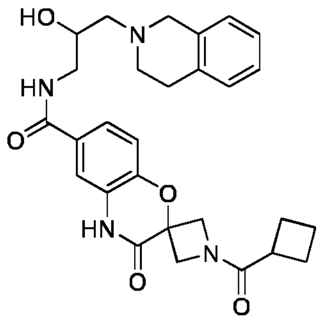 ,

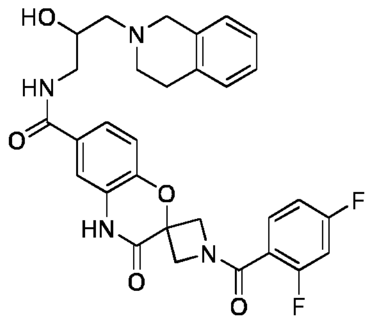 ,

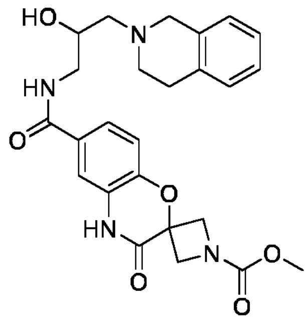 ,

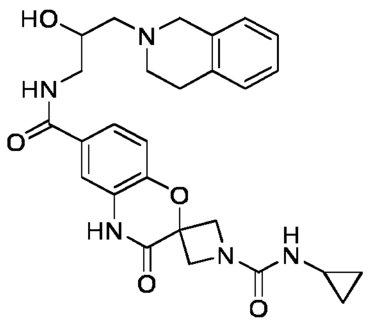 ,

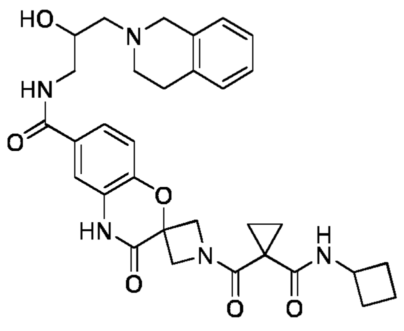 ,

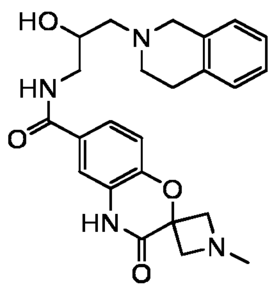 ,

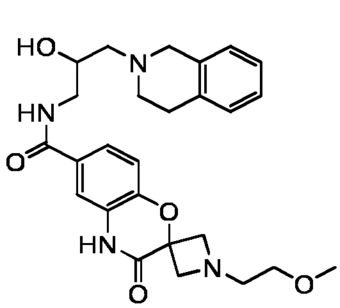, 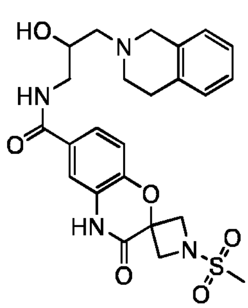, 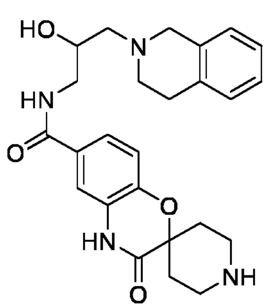, 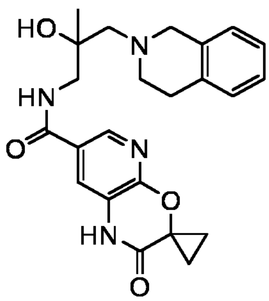,

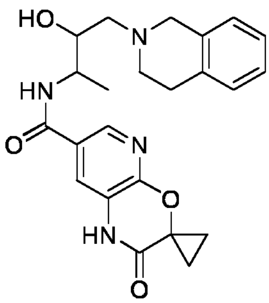, 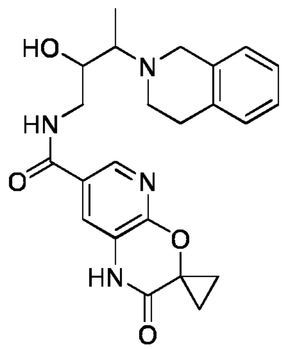, 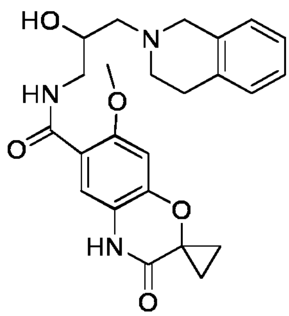,

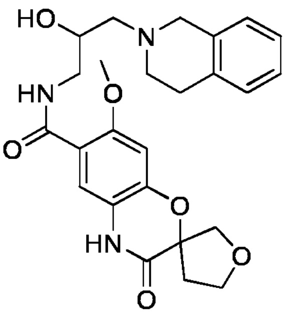 and 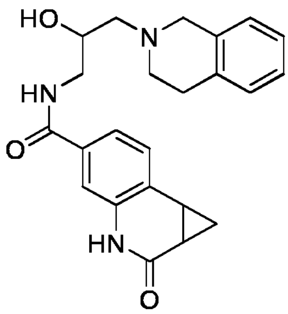.

The present disclosure also provides a compound represented by the following formulas or a pharmaceutically acceptable salt thereof:

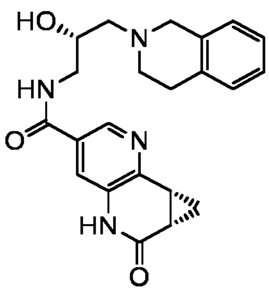, 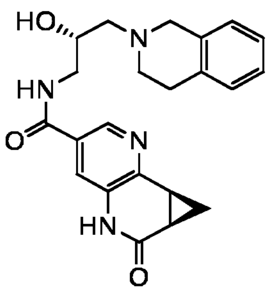, 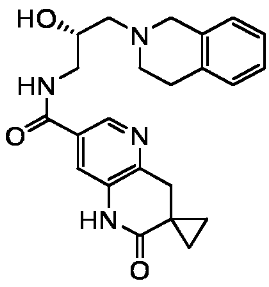, 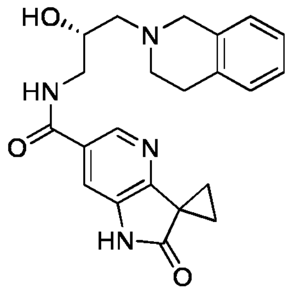,

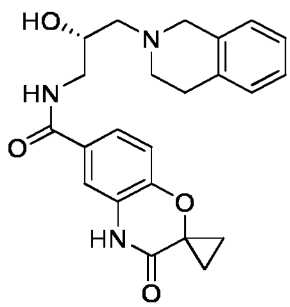,
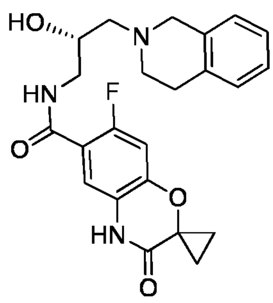,
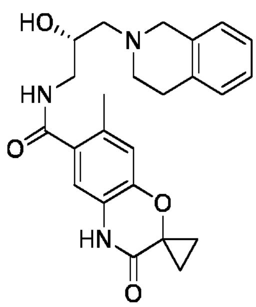,
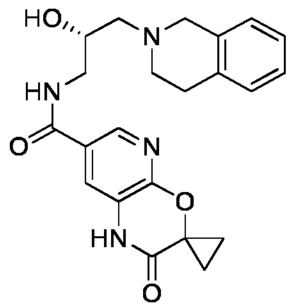,

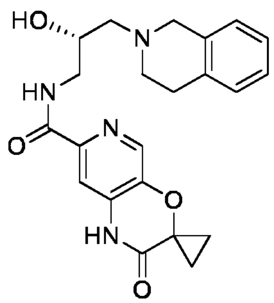,
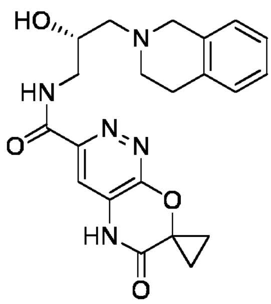,
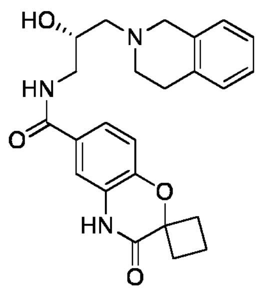,
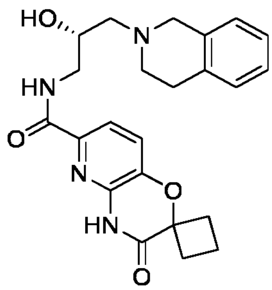,

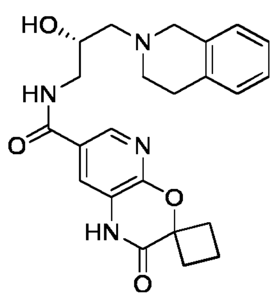,
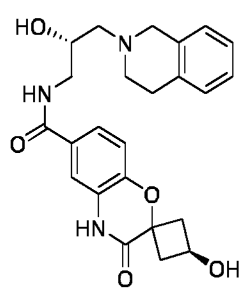,
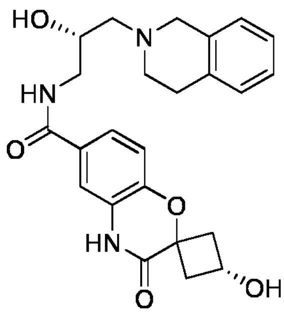,
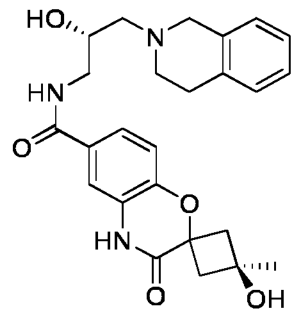,

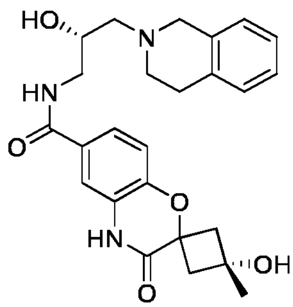,
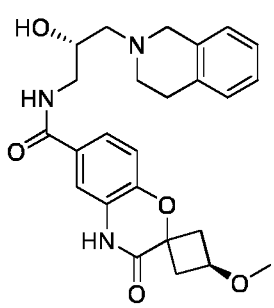,
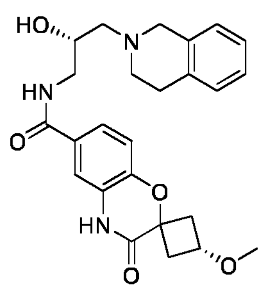,
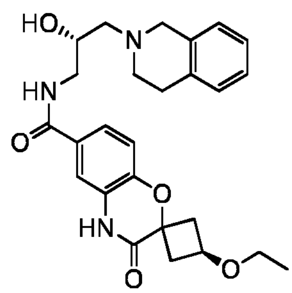,

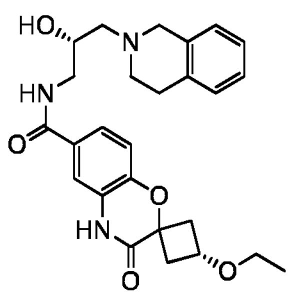,
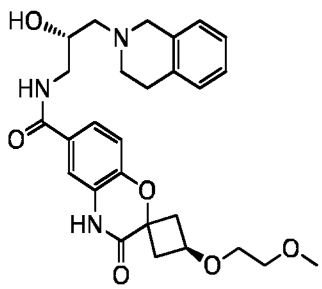,
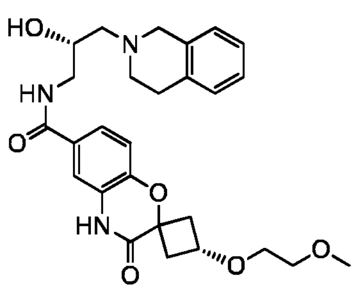,

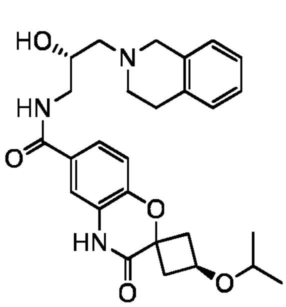,
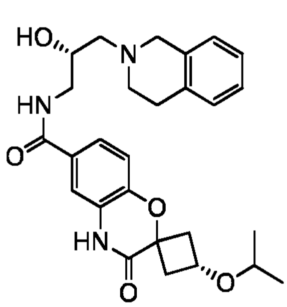,
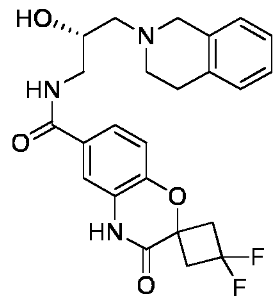,
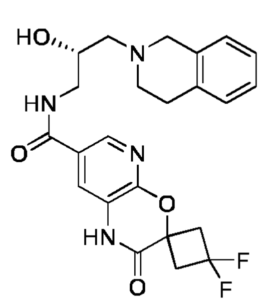,

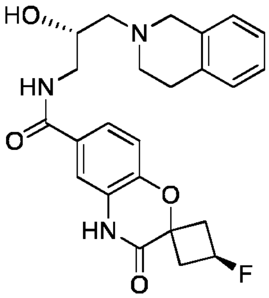,
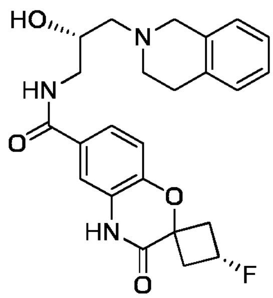,
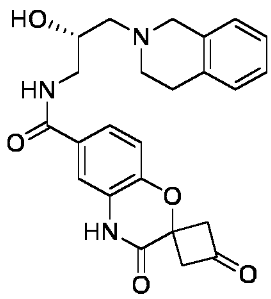,
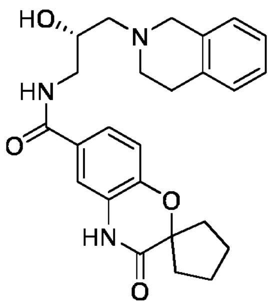,

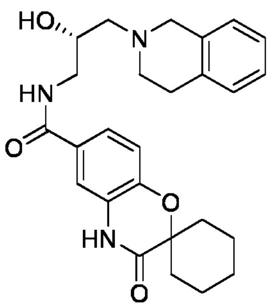,
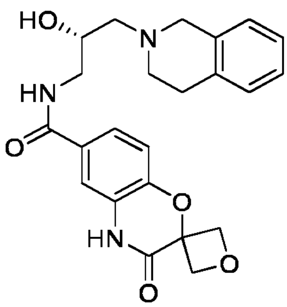,
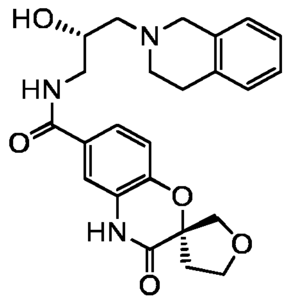,
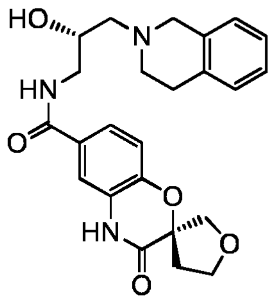,

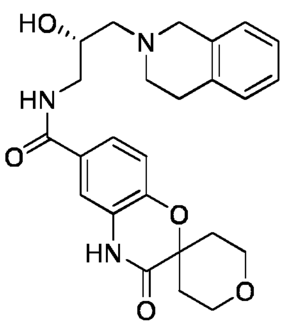,
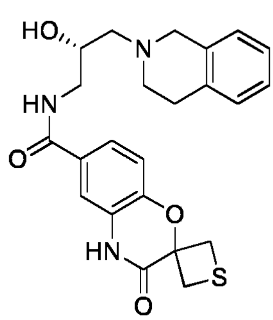,
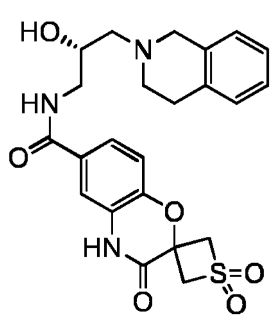,
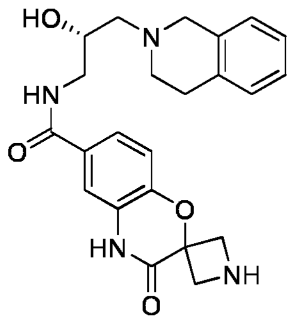,

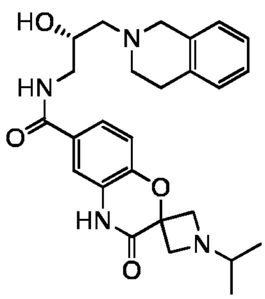,
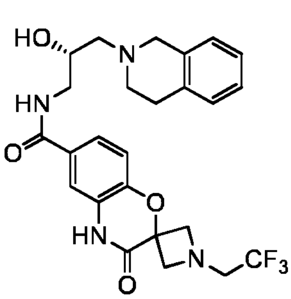,
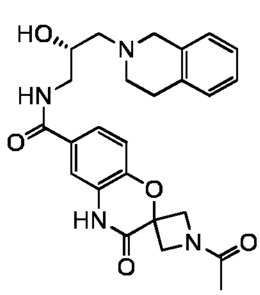,
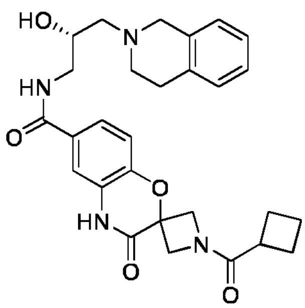,

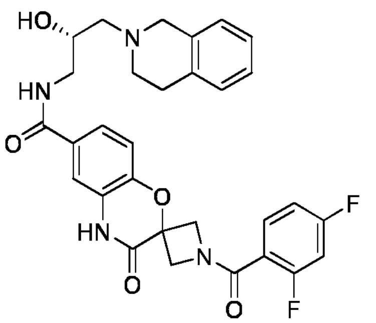 , 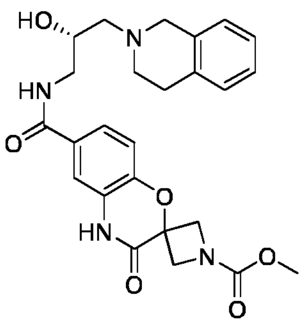 , 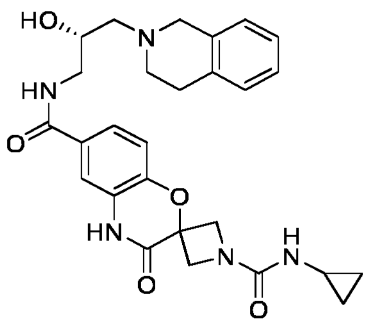 ,

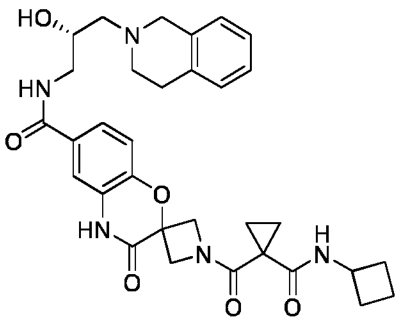 , 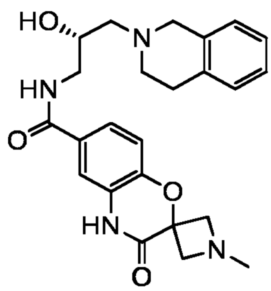 , 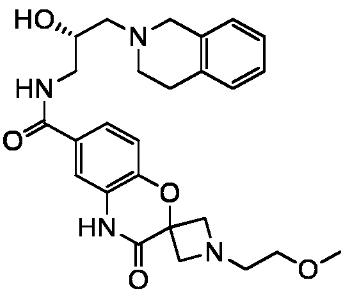 ,

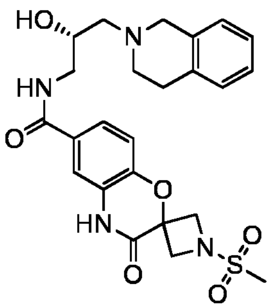 , 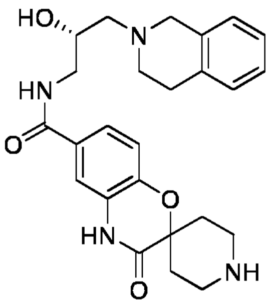 , 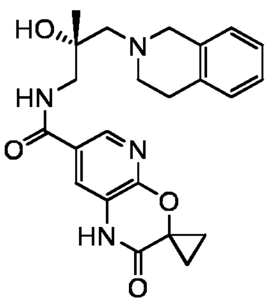 , 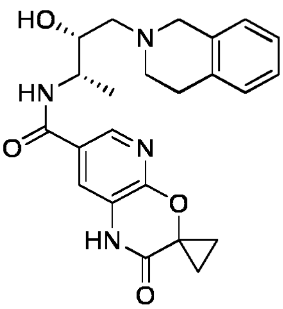 ,

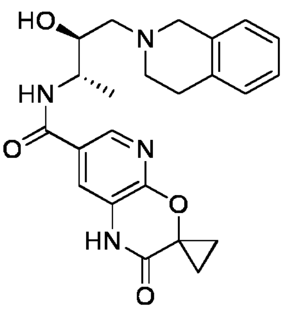 , 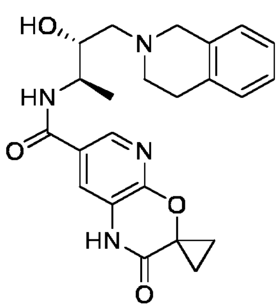 , 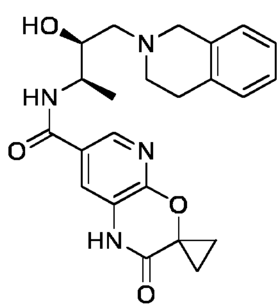 , 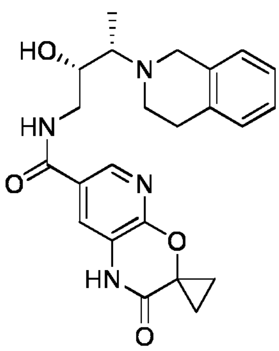 ,

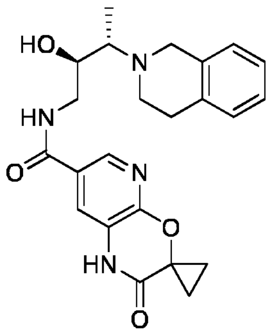 , 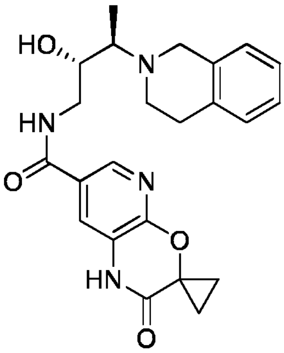 , 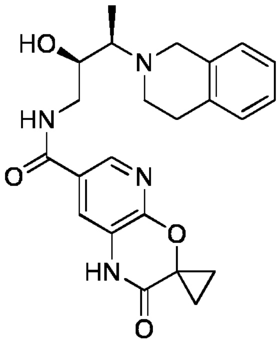 , 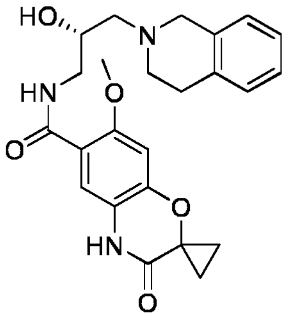 ,

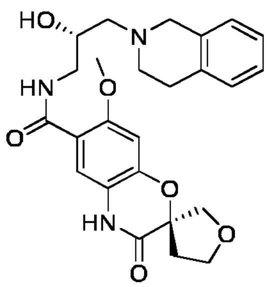 , 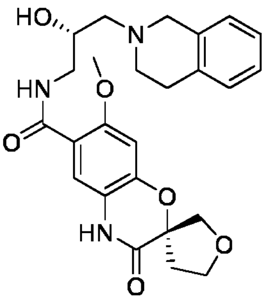 , 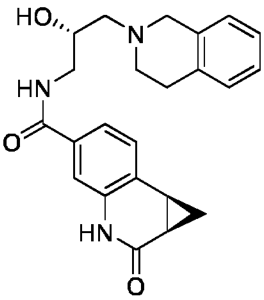 and 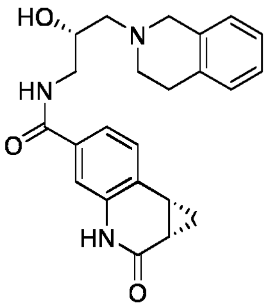 .

The present disclosure also provides a crystal form A of compound 46a, and the crystal form A has an X-ray powder diffraction pattern using Cu Kα radiation and having characteristic diffraction peaks at the following 2θ angles: 4.70±0.20°, 11.32±0.20°, 13.35±0.20°, 16.25±0.20°, 17.36±0.20°.

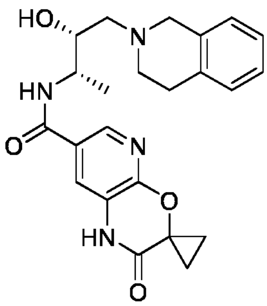

**46a**

In some embodiments of the present disclosure, the X-ray powder diffraction pattern of the crystal form A using Cu Kα radiation has characteristic diffraction peaks at the following 2θ angles: 4.70±0.20°, 8.68±0.20°, 9.35±0.20°, 11.32±0.20°, 13.35±0.20°, 16.25±0.20°, 17.36±0.20°, 23.22±0.20°.

In some embodiments of the present disclosure, the X-ray powder diffraction pattern of the crystal form A using Cu Kα radiation has characteristic diffraction peaks at the following 2θ angles: 4.70±0.20°, 8.68±0.20°, 9.35±0.20°, 9.99±0.20°, 11.32±0.20°, 13.35±0.20°, 14.05±0.20°, 16.25±0.20°, 17.36±0.20°, 20.08±0.20°, 23.22±0.20°, 25.65±0.20°.

In some embodiments of the present disclosure, the X-ray powder diffraction pattern of the crystal form A using Cu Kα radiation has characteristic diffraction peaks at the following 2θ angles: 4.70±0.20° and 8.68±0.20°, and also has characteristic diffraction peaks at 9.35±0.20°, and/or 9.99±0.20°, and/or 11.32±0.20°, and/or 13.35±0.20°, and/or 14.05±0.20°, and/or 16.25±0.20°, and/or 17.36±0.20°, and/or 20.08±0.20°, and/or 23.22±0.20°, and/or 25.65±0.20°.

In some embodiments of the present disclosure, the X-ray powder diffraction pattern of the crystal form A using Cu Kα radiation has characteristic diffraction peaks at the following 2θ angles: 4.70°, 8.68°, 9.35°, 9.99°, 11.32°, 13.35°, 14.05°, 16.25°, 17.36°, 17.65°, 20.08°, 23.22°, 23.80°, 24.13°, 25.65°, 28.34°, 30.32°, 33.54°.

In some embodiments of the present disclosure, the XRPD pattern of the crystal form A is shown in FIG. 3.

In some embodiments of the present disclosure, the peak position and relative intensity of the diffraction peak in the XRPD pattern of the crystal form A using Cu Kα radiation are shown in the following table:

Table 1: XRPD diffraction data of crystal form A of compound 46a

| No. | **Diffraction angle** 2θ[°] | Peak height [cts] | d-Spacing [Å] | Relative intensity [%] |
|---|---|---|---|---|
| 1 | 4.70 | 1642.29 | 18.81 | 73.93 |
| 2 | 8.68 | 321.39 | 10.19 | 14.47 |
| 3 | 9.35 | 184.12 | 9.46 | 8.29 |
| 4 | 9.99 | 116.78 | 8.85 | 5.26 |
| 5 | 11.32 | 642.15 | 7.81 | 28.91 |
| 6 | 13.35 | 427.55 | 6.63 | 19.25 |
| 7 | 14.05 | 195.32 | 6.30 | 8.79 |
| 8 | 16.25 | 2221.35 | 5.45 | 100.00 |
| 9 | 17.36 | 1306.02 | 5.11 | 58.79 |
| 10 | 17.65 | 1012.97 | 5.03 | 45.60 |
| 11 | 20.08 | 122.14 | 4.42 | 5.50 |
| 12 | 23.22 | 260.04 | 3.83 | 11.71 |
| 13 | 23.80 | 66.11 | 3.74 | 2.98 |
| 14 | 24.13 | 69.23 | 3.69 | 3.12 |
| 15 | 25.65 | 113.25 | 3.47 | 5.10 |
| 16 | 28.34 | 75.91 | 3.15 | 3.42 |
| 17 | 30.32 | 83.46 | 2.95 | 3.76 |
| 18 | 33.54 | 72.76 | 2.67 | 3.28 |

In some embodiments of the present disclosure, the differential scanning calorimetry (DSC) curve of the crystal form A shows an endothermic peak at 241.2°C±3°C.

In some embodiments of the present disclosure, the DSC pattern of the crystal form A is shown in FIG. 4.

In some embodiments of the present disclosure, the thermogravimetric analysis (TGA) curve of the crystal form A has a weight loss of 2.55% at 200.0°C±3°C.

In some embodiments of the present disclosure, the TGA pattern of the crystal form A is shown in FIG. 5.

In some embodiments of the present disclosure, the dynamic vapor sorption (DVS) of the crystal form A exhibits slight hygroscopicity.

In some embodiments of the present disclosure, the DVS pattern of the crystal form A is shown in FIG. 6.

The present disclosure also provides a method for preparing the crystal form A, comprising the following steps:

(a) adding compound **46a** to a mixed solvent of solvent X and solvent Y;
(b) stirring the suspension at 25 to 80°C for 3 to 6 days;
(c) filtering at room temperature to collect the solid;
wherein,

the solvent X is selected from ethyl acetate, acetonitrile, 2-methyltetrahydrofuran, tetrahydrofuran, acetone, dichloromethane, 1,4-dioxane and isopropanol;
the solvent Y does not exist;
alternatively, the solvent Y is selected from *n*-heptane, *n*-hexane, cyclohexane, water and methyl *tert*-butyl ether, and the volume ratio of the solvent X to the solvent Y is 1:1 to 1:10.

The present disclosure also provides a method for preparing the crystal form A, comprising the following steps:

(a) adding compound **46a** to solvent P to fully dissolve;
(b) adding solvent Q dropwise under stirring at 5 to 25°C until a solid precipitates;
(c) centrifuging to collect the solid;

wherein,

the solvent P is selected from tetrahydrofuran, chloroform, dimethylacetamide and dimethyl sulfoxide;
the solvent Q is selected from toluene, *n*-heptane, water and methyl isobutyl ketone.

The present disclosure also provides a crystal form B of compound **46a,** and the crystal form B has an X-ray powder diffraction pattern using Cu Kα radiation and having characteristic diffraction peaks at the following 2θ angles: 7.12±0.20°, 10.69±0.20°, 12.74±0.20°, 14.28±0.20°, 15.18±0.20°.

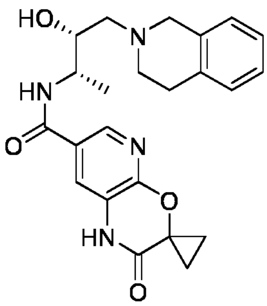

**46a**

In some embodiments of the present disclosure, the X-ray powder diffraction pattern of the crystal form B using Cu Kα radiation has characteristic diffraction peaks at the following 2θ angles: 7.12±0.20°, 10.15±0.20°, 10.69±0.20°, 12.74±0.20°, 14.28±0.20°, 15.18±0.20°, 17.87±0.20°, 25.12±0.20°.

In some embodiments of the present disclosure, the X-ray powder diffraction pattern of the crystal form B using Cu Kα radiation has characteristic diffraction peaks at the following 2θ angles: 3.61±0.20°, 7.12±0.20°, 10.15±0.20°, 10.69±0.20°, 12.74±0.20°, 14.28±0.20°, 15.18±0.20°, 17.87±0.20°, 20.39±0.20°, 21.09±0.20°, 23.86±0.20°, 25.12±0.20°.

In some embodiments of the present disclosure, the X-ray powder diffraction pattern of the crystal form B using Cu Kα radiation has characteristic diffraction peaks at the following 2θ angles: 3.61°, 7.12°, 10.16°, 10.69°, 10.95°, 12.08°, 12.74°, 14.28°, 15.18°, 17.07°, 17.87°, 20.08°, 20.39°, 21.09°, 22.79°, 23.29°, 23.86°, 24.77°, 25.12°, 26.37°, 30.02°, 30.68°, 31.23°, 32.46°, 34.80°, 36.18°, 37.13°.

In some embodiments of the present disclosure, the XRPD pattern of the crystal form B is shown in FIG. 7.

In some embodiments of the present disclosure, the peak position and relative intensity of the diffraction peak in the XRPD pattern of the crystal form B using Cu Kα radiation are shown in the following table:

Table 2: XRPD diffraction data of crystal form B of compound **46a**

| No. | 2θ [°] | Peak height [cts] | d-Spacing [Å] | Relative intensity [%] |
|---|---|---|---|---|
| 1 | 3.61 | 780.18 | 24.47 | 9.54 |
| 2 | 7.12 | 4261.88 | 12.42 | 52.09 |
| 3 | 10.16 | 908.56 | 8.71 | 11.10 |
| 4 | 10.69 | 8181.84 | 8.27 | 100.00 |
| 5 | 10.95 | 905.59 | 8.08 | 11.07 |
| 6 | 12.08 | 287.78 | 7.33 | 3.52 |
| 7 | 12.74 | 1557.18 | 6.95 | 19.03 |
| 8 | 14.28 | 6485.95 | 6.20 | 79.27 |
| 9 | 15.18 | 6610.63 | 5.84 | 80.80 |
| 10 | 17.07 | 184.10 | 5.19 | 2.25 |
| 11 | 17.87 | 1485.29 | 4.96 | 18.15 |
| 12 | 20.08 | 487.44 | 4.42 | 5.96 |
| 13 | 20.39 | 713.15 | 4.35 | 8.72 |

(continued)

| No. | 2θ [°] | Peak height [cts] | d-Spacing [Å] | Relative intensity [%] |
|---|---|---|---|---|
| 14 | 21.09 | 843.60 | 4.21 | 10.31 |
| 15 | 22.79 | 126.23 | 3.90 | 1.54 |
| 16 | 23.29 | 126.26 | 3.82 | 1.54 |
| 17 | 23.86 | 384.55 | 3.73 | 4.70 |
| 18 | 24.77 | 297.45 | 3.59 | 3.64 |
| 19 | 25.12 | 1884.55 | 3.54 | 23.03 |
| 20 | 26.37 | 148.75 | 3.38 | 1.82 |
| 21 | 30.02 | 139.14 | 2.98 | 1.70 |
| 22 | 30.68 | 78.55 | 2.91 | 0.96 |
| 23 | 31.23 | 60.03 | 2.86 | 0.73 |
| 24 | 32.46 | 318.50 | 2.76 | 3.89 |
| 25 | 34.80 | 65.66 | 2.58 | 0.80 |
| 26 | 36.18 | 502.39 | 2.48 | 6.14 |
| 27 | 37.13 | 105.48 | 2.42 | 1.29 |

In some embodiments of the present disclosure, the differential scanning calorimetry (DSC) curve of the crystal form B shows an endothermic peak at 241.4°C±3°C.

In some embodiments of the present disclosure, the DSC pattern of the crystal form B is shown in FIG. 8.

In some embodiments of the present disclosure, the thermogravimetric analysis (TGA) curve of the crystal form B has a weight loss of 2.98% at 140.0°C±3°C.

In some embodiments of the present disclosure, the TGA pattern of the crystal form B is shown in FIG. 9.

The present disclosure also provides a use of the compound or the pharmaceutically acceptable salt thereof in the manufacture of a medicament related to an inhibitor of treating protein arginine methyltransferase 5.

The present disclosure also provides a use of the crystal form A or crystal form B in the manufacture of a medicament related to an inhibitor of treating protein arginine methyltransferase 5.

In some embodiments of the present disclosure, the medicament related to the inhibitor of protein arginine methyltransferase 5 is a medicament for the prevention and/or treatment of diseases related to lymphoma and solid tumor.

In some embodiments of the present disclosure, the lymphoma is non-Hodgkin's lymphoma, and the solid tumor is breast cancer.

In some embodiments of the present disclosure, the lymphoma is non-Hodgkin's lymphoma, such as mantle cell lymphoma.

In some embodiments of the present disclosure, the solid tumor is breast cancer.

**Technical effect**

As a series of inhibitors of protein arginine methyltransferase 5 (PRMT5), these compounds of the present disclosure has good PRMT5 enzyme inhibitory activity and can effectively inhibit the proliferation of non-Hodgkin's lymphoma cells and/or triple-negative breast cancer cells with good plasma exposure and reasonable oral bioavailability. The efficacy results show that these compounds of the present disclosure have significant antitumor effects *in vivo.* As a drug inhibitor of lymphoma and solid tumor, the compound of the present disclosure has great application prospects for the prevention and/or treatment of diseases related to lymphoma and solid tumor.

**Definition and description**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereomers isomers, (*D*)-isomers, (*L*)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( ) and a wedged dashed bond ( ), and the relative configuration of a stereogenic center is represented by a straight solid bond ( ) and a straight dashed bond ( ), a wave line ( ) is used to represent a wedged solid bond ( ) or a wedged dashed bond ( ), or the wave line ( ) is used to represent a straight solid bond ( ) or a straight dashed bond ( ).

Unless otherwise specified, when there is a double bond structure in the compound, such as a carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by

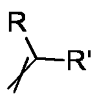,

this refers to the (*Z*) isomer, (*E*) isomer or a mixture of two isomers of the compound.

Unless otherwise specified, the terms "enriched in one isomer", "enriched in isomers", "enriched in one enantiomer" or "enriched in enantiomers" refer to the content of one of the isomers or enantiomers is less than 100%, and

the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomer, *or D* and *L isomer* can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to obtian the pure enantiomer. In addition, the enantiomer and the diastereoisomer are generally isolated through chromatography which uses a chiral stationary phase and optionally combines with a chemical derivative method (such as carbamate generated from amine). The compound of the present disclosure may contain an unnatural proportion of atomic isotope at one or more than one atom(s) that constitute the compound. For example, the compound can be radiolabeled with a radioactive isotope, such as tritium ($^3H$), iodine-125 ($^{125}I$) or C-14 ($^{14}C$). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When the number of a substituent is 0, it means that the substituent does not exist, for example, $-A-(R)_0$ means that the structure is actually A.

When a substituent is vacant, it means that the substituent does not exist, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, such substituent can be bonded to any atom on the ring, for example, the structural moiety

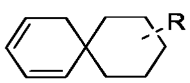

or

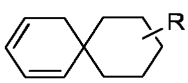

means that its substituent R can be substituted in any position on cyclohexyl or cyclohexadiene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in

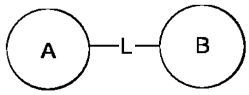

is -M-W-, then -M-W- can link ring A and ring B to form

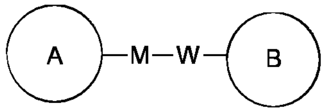

in the direction same as left-to-right reading order, and form

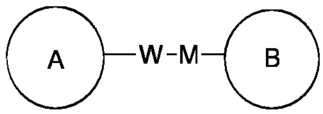

in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atom at the site will decrease correspondingly with the number of the chemical bond linking thereto so as to meet the corresponding valence. The chemical bond between the site and other groups can be represented by a straight solid bond (/), a straight dashed bond (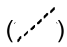) or a wavy line (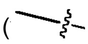). For example, the straight solid bond in $-OCH_3$ means that it is linked to other groups through the oxygen atom in the group; the straight dashed bond in

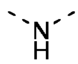

means that it is linked to other groups through the two ends of nitrogen atom in the group; the wave line in

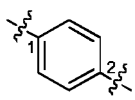

means that the phenyl group is linked to other groups through carbon atoms at position 1 and position 2;

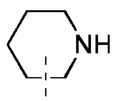

means that it can be linked to other groups through any linkable sites on the piperidinyl by one chemical bond, including at least four types of linkage, including

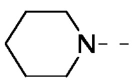, 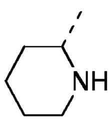, 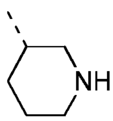 and 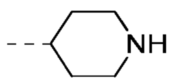,

even though the H atom is drawn on the -N-,

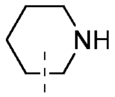

still includes the linkage of

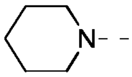

,

merely when one chemical bond was connected, the H of this site will be reduced by one to the corresponding monovalent piperidinyl;

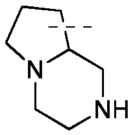

means that it can be linked to other groups through any linkable sites on the group by one chemical bond, including at least eight types of linkage, including

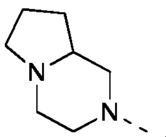

,

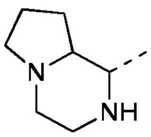

,

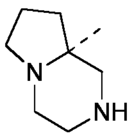

,

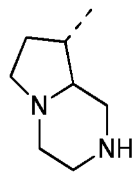

,

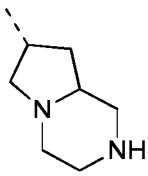

,

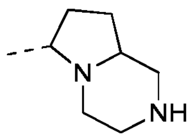

,

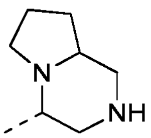

and

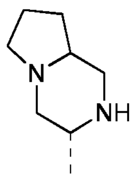

.

Unless otherwise specified, the term "$C_{1-3}$ alkyl" by itself or in combination with other terms refers to a linear or branched saturated hydrocarbon group consisting of 1 to 3 carbon atoms. The $C_{1-3}$ alkyl includes $C_{1-2}$ and $C_{2-3}$ alkyl and the like; it can be monovalent (such as methyl), divalent (such as methylene) or multivalent (such as methine). Examples of $C_{1-3}$ alkyl include but are not limited to methyl (Me), ethyl (Et), propyl (including *n*-propyl and isopropyl) and the like.

Unless otherwise specified, the term "$C_{1-3}$ alkoxy" by itself or in combination with other terms refers to an alkyl consisting of 1 to 3 carbon atoms that are connected to the rest of the molecule through an oxygen atom. The $C_{1-3}$ alkoxy includes $C_{1-2}$, $C_{2-3}$, $C_3$ and $C_2$ alkoxy and the like. Examples of $C_{1-3}$ alkoxy include, but are not limited to, methoxy, ethoxy, propoxy (including *n*-propoxy and isopropoxy) and the like.

Unless otherwise specified, "$C_{3-6}$ cycloalkyl" by itself or in combination with other terms refers to a saturated cyclic hydrocarbon group consisting of 3 to 6 carbon atoms, which is a monocyclic and bicyclic system, and the $C_{3-6}$ cycloalkyl includes $C_{3-5}$, $C_{4-5}$ and $C_{5-6}$ cycloalkyl, etc.; it can be monovalent, divalent or polyvalent. Examples of $C_{3-6}$ cycloalkyl include, but are not limited to, cyclopropyl, cyclobutyl, cyclopentyl, cyclohexyl, etc.

Unless otherwise specified, the term "4- to 6-membered heterocycloalkyl" by itself or in combination with other terms refers to a saturated cyclic group consisting of 4 to 6 ring atoms, wherein 1, 2, 3 or 4 ring atoms are heteroatoms independently selected from O, S and N, and the rest are carbon atoms, wherein nitrogen atoms are optionally quaternized, and nitrogen and sulfur heteroatoms can be optionally oxidized (i.e., NO and $S(O)_p$, p is 1 or 2). It includes monocyclic and bicyclic systems, wherein the bicyclic systems include a spiro ring, fused ring and bridged ring. In addition, with regard to the "4- to 6-membered heterocycloalkyl", a heteroatom may occupy the connection position of the heterocycloalkyl with the rest of the molecule. The 4- to 6-membered heterocycloalkyl includes 5- to 6-membered, 4-membered, 5-membered, and 6-membered heterocycloalkyl, etc. Examples of 4- to 6-membered heterocycloalkyl

include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl or hexahydropyridazinyl.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$ and $C_{12}$, and any range from n to n+m is also included, for example $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, $C_{9-12}$ and the like; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring and the like.

The term "protecting group" includes, but is not limited to "amino protecting group", "hydroxyl protecting group" or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (e.g., acetyl, trichloroacetyl or trifluoroacetyl); alkoxycarbonyl, such as *tert*-butoxycarbonyl (Boc); arylmethoxycarbonyl, such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and *tert*-butyldimethylsilyl (TBS), etc. The term "hydroxyl protecting group" refers to a protecting group suitable for preventing the side reactions of hydroxyl. Representative hydroxyl protecting groups include, but are not limited to: alkyl, such as methyl, ethyl, and *tert*-butyl; acyl, such as alkanoyl (e.g., acetyl); arylmethyl, such as benzyl (Bn), *p*-methoxybenzyl (PMB), 9-fluorenylmethyl (Fm), and diphenylmethyl (benzhydryl, DPM); silyl, such as trimethylsilyl (TMS) and *tert*-butyldimethylsilyl (TBS), etc.

The compounds of the present disclosure can be prepared by a variety of synthetic methods known to those skilled in the art, including the specific implementations listed below, the implementations formed by their combination with other chemical synthesis methods, and equivalent alternatives known to those skilled in the art, preferred implementations include but are not limited to the embodiments of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with Cu $K\alpha$ radiation as the light source and scanning mode: $\varphi/\omega$ scan, and after collecting the relevant data, the crystal structure can be further analyzed by direct method (Shelxs97), so that the absolute configuration can be confirmed.

The solvent used in the present disclosure is commercially available.

The following abbreviations are used in the present disclosure: $N_2$ refers to nitrogen; DMSO refers to dimethyl sulfoxide; HATU refers to 2-(7-azabenzotriazol-1-yl)-*N*,*N*,*N'*,*N'*-tetramethyluronium hexafluorophosphate; HOBt refers to 1-hydroxybenzotriazole; EDCI refers to 1-(3-dimethylaminopropyl)-3-ethylcarbodiimide hydrochloride; TMSCN refers to trimethylsilyl cyanide; DAST refers to diethylaminosulfur trifluoride; DPPF refers to 1,1'-bis(diphenylphosphino)ferrocene; DMAP refers to 4-dimethylaminopyridine; TBSCl refers to *tert*-butyldimethylsilyl chloride; DIEA refers to N,N-diisopropylethylamine; Pd/C refers to palladium on carbon; Pd(dppf)$Cl_2$ refers to [1,1'-bis(diphenylphosphino)ferrocene]palladium dichloride; Pd(dppf)$Cl_2 \cdot CH_2Cl_2$ refers to [1,1'-bis(diphenylphosphino)ferrocene]palladium dichloride dichloromethane complex; TBAF refers to tetrabutylammonium fluoride; TosCl refers to *p*-toluenesulfonyl chloride; DEA refers to diethylamine; ACN refers to acetonitrile; $CO_2$ refers to carbon dioxide; MPa refers to pressure unit Megapascal; psi refers to pounds per square inch; PDA refers to photo-diode array detector; RCF refers to relative centrifugal force; RLU refers to relative light unit; AUC refers to area under the curve; PO refers to per os; PEG400 refers to polyethylene glycol 400; RH refers to relative humidity; lux refers to Lux; $\mu w/cm^2$ refers to microwatts per square centimeter; rpm refers to revolutions per minute; XRPD refers to X-ray powder diffraction; DSC refers to differential scanning calorimetry; DVS refers to dynamic vapor sorption; TGA refers to thermogravimetric analysis; $^1H$ NMR refers to hydrogen nuclear magnetic resonance spectroscopy.

The compounds of the present disclosure are named according to the conventional naming principles in the art or by ChemDraw® software, and the commercially available compounds use the supplier catalog names.

**Instruments and analytical methods of the present disclosure**

**(1) X-ray powder diffractometer (XRPD) method of the present disclosure**

Detailed XRPD instrument information and parameters are shown in the following table:

Table 3: XRPD instrument information and test parameters

| Instrument model | **X' Pert3** (Reflection) |
|---|---|
| X-ray | Cu, Kα;<br>Kα1 (Å): 1.540598, Kα2 (Å): 1.544426;<br>Intensity ratio Kα2/Kα1: 0.50 |
| X-ray tube settings | 45 kV, 40 mA |
| Divergence slit | 1/8° |
| Scanning mode | Continuous |
| Scanning range (°2θ) | 3 to 40 |
| Scanning step (°2θ) | 0.0263 |
| Scanning time per step (s) | 46.665 |
| Scanning time (s) | 5 min 03 s |

**(2) Differential Scanning Calorimeter (DSC) method and Thermal Gravimetric Analyzer (TGA) method of the present disclosure**

Detailed TGA and DSC instrument information and parameters are shown in the following table:

Table 4: TGA and DSC instrument information and test parameters

| | **TGA** | **DSC** |
|---|---|---|
| Instrument model | TA Q5000/5500 Thermal Gravimetric Analyzer | TA Q2500 Differential Scanning Calorimeter |
| Method | Linear temperature increase | Linear temperature increase |
| Sample tray | Aluminum tray, open | Aluminum tray, capped/uncapped |
| Temperature range | Room temperature - Setting terminal temperature | 25°C - Setting terminal temperature |
| Scanning rate (°C/min) | 10 | 10 |
| Protective gas | Nitrogen | Nitrogen |

**(3) Dynamic Vapor Sorption (DVS) method**

Detailed DVS instrument information and parameters are shown in Table 5, and the classification of hygroscopicity evaluation is shown in Table 6:

Table 5: DVS instrument information and test parameters

| Instrument model | SMS DVS intrinsic Dynamic Vapor Sorption |
|---|---|
| Temperature | 25°C |
| Sample weight | 10 to 20 mg |
| Protective gas and flow | $N_2$, 200 mL/min |
| dm/dt | 0.002%/min |
| Minimum dm/dt equilibrium time | 10 min |

(continued)

| | |
|---|---|
| Maximum equilibrium time | 180 min |
| RH range | 0% RH to 95% RH |
| RH gradient | 10% (0% RH to 90% RH, 90% RH to 0% RH)<br>5% (90% RH to 95% RH, 95% RH to 90% RH) |

Table 6: Classification of hygroscopicity evaluation

| **Hygroscopicity classification** | $\Delta$W% |
|---|---|
| Deliquescence | Absorption of sufficient water to form a liquid |
| Extremely hygroscopic | $\Delta W\% \geq 15\%$ |
| Hygroscopic | $15\% > \Delta W\% \geq 2\%$ |
| Slightly hygroscopic | $2\% > \Delta W\% \geq 0.2\%$ |
| No or almost no hygroscopicity | $\Delta W\% < 0.2\%$ |
| Note: $\Delta$W% represents the moisture absorption weight gain of the test sample at 25$\pm$1°C and 80$\pm$2% RH. | |

BRIEF DESCRIPTION OF THE DRAWINGS

FIG. 1 shows the tumor growth curve of the subcutaneous xenograft tumor model of human mantle cell lymphoma Z-138 cells in tumor-bearing mice after administration of compounds 1 and 4.
FIG. 2 shows the tumor growth curve of the subcutaneous xenograft tumor model of human mantle cell lymphoma Z-138 cells in tumor-bearing mice after administration of compound 8.
FIG. 3 is the XRPD pattern of the crystal form A of compound **46a.**
FIG. 4 is the DSC pattern of the crystal form A of compound **46a.**
FIG. 5 is the TGA pattern of the crystal form A of compound **46a.**
FIG. 6 is the DVS pattern of the crystal form A of compound **46a.**
FIG. 7 is the XRPD pattern of the crystal form B of compound **46a.**
FIG. 8 is the DSC pattern of the crystal form B of compound **46a.**
FIG. 9 is the TGA pattern of the crystal form B of compound **46a.**
FIG. 10 is the three-dimensional ellipsoid diagram of compound **46a** dihydrate.

DETAILED DESCRIPTION OF THE PREFERRED EMBODIMENT

The present disclosure is described in detail by the embodiments below, but it does not mean that there are any adverse restrictions on the present disclosure. The present disclosure has been described in detail herein, and its specific embodiments have also been disclosed; for one skilled in the art, it is obvious to make various modifications and improvements to the embodiments of the present disclosure without departing from the spirit and scope of the present disclosure.

**Reference embodiment 1: Moiety BB-1**

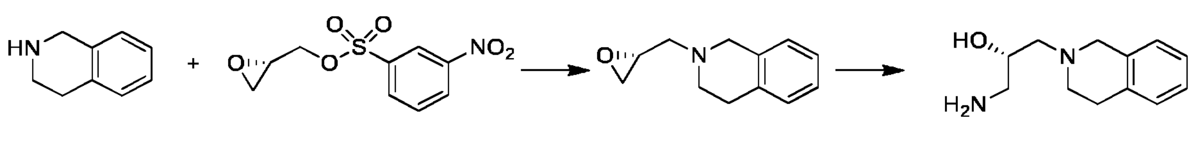

**BB-1-1** **BB-1-2** **BB-1-3** **BB-1**

Step **1:** Synthesis of compound **BB-1-3**

To a solution of compound **BB-1-1** (50.00 g, 375.40 mmol, 47.17 mL, 1.00 *eq*) in tetrahydrofuran (500.00 mL) was added potassium fluoride (87.24 g, 1.50 mol, 35.18 mL, 4.00 *eq*) at 0°C, the reaction mixture was stirred at 0°C for 1 hour, then added with compound **BB-1-2** (107.05 g, 412.94 mmol, 1.10 *eq*), and the reaction mixture was stirred at 20°C for 16 hours. The reaction mixture was filtered through diatomite, then the filtrate was concentrated under reduced pressure, and the crude product was purified by silica gel column chromatography (petroleum ether: ethyl acetate = 10:1 to 6:1) to obtain compound **BB-1-3.** MS (ESI) m/z: 190.2 $[M+H]^+$.

Step 2: Synthesis of compound **BB-1**

Ammonia gas was introduced into ethanol (1000.00 mL) at -70°C for 50 min, and the solution was then added with compound **BB-1-3** (20.00 g, 105.68 mmol, 1.00 *eq).* The reaction mixture was transferred to an autoclave, heated to 40°C, and stirred for 48 hours under pressure (0.5 MPa). The reaction mixture was concentrated under reduced pressure to obtain a crude product of compound **BB-1.** MS (ESI) m/z: 207.3 $[M+H]^+$.

**Embodiment 1: Preparation of compound 1**

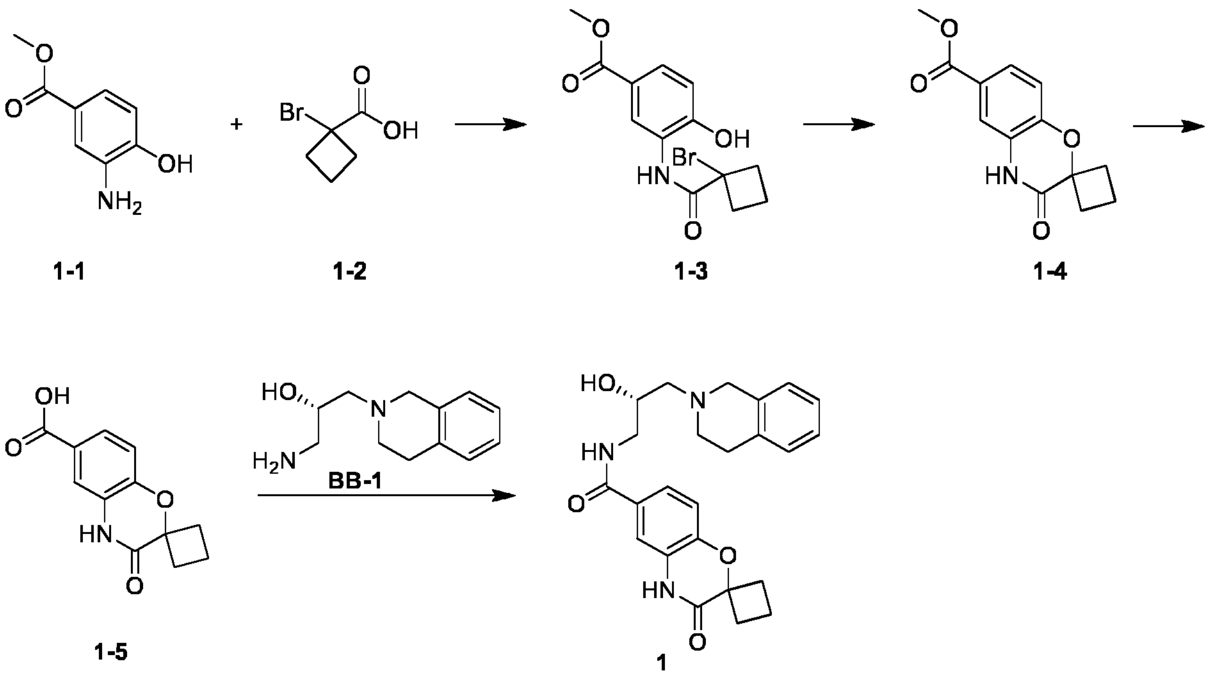


**1-1** **1-2** **1-3** **1-4**

**1-5** **1**

**Step 1: Synthesis of compound 1-3**

To a solution of compound **1-2** (1.10 g, 6.14 mmol, 1.00 *eq*) in dichloromethane (3.00 mL) was added *N,N*-dimethylformamide (22.40 mg, 307.24 μmol, 0.05 *eq*) in an ice bath, then thionyl chloride (877.20 mg, 7.37 mmol, 1.20 *eq*) was slowly added dropwise, and the reaction mixture was heated to 40°C and stirred for 6 hours. When the temperature was lowered to 0°C, the above reaction mixture was slowly added dropwise with a solution of compound **1-1** (1.03 g, 6.14 mmol, 1.00 *eq*) and *N*-methylmorpholine (1.55 g, 15.36 mmol, 2.5 *eq*) in dichloromethane (3.00 mL), and the reaction mixture was warmed to room temperature and stirred for 0.5 hours. The reaction mixture was added with water (100 mL) to quench, and extracted with ethyl acetate (100 mL × 2). The organic phases were combined, dried over anhydrous sodium sulfate, filtered, and the filtrate was concentrated under reduced pressure to obtain compound **1-3.** MS (ESI) m/z: 327.8 $[M+H]^+$.

**Step 2: Synthesis of compound 1-4**

To a solution of compound **1-3** (1.60 g, 4.88 mmol, 1.00 *eq*) in *N,N*-dimethylformamide (10.00 mL) was added

potassium carbonate (3.37 g, 24.38 mmol, 5.00 *eq*), and the reaction mixture was heated to 80°C and stirred for 16 hours. The reaction mixture was then cooled to room temperature, added with water (100 mL) to quench, and extracted with ethyl acetate (100 mL × 3). The organic phases were combined, washed with water (100 mL), dried over anhydrous sodium sulfate, filtered, and the filtrate was concentrated under reduced pressure to obtain compound **1-4.** MS (ESI) m/z: 248.1 $[M+H]^+$.

**Step 3: Synthesis of compound 1-5**

To a solution of compound **1-4** (400.00 mg, 1.62 mmol, 1.00 *eq*) in tetrahydrofuran (4.00 mL), methanol (2.00 mL) and water (1.00 mL) was added lithium hydroxide monohydrate (203.60 mg, 4.85 mmol, 3.00 *eq*), and the reaction mixture was heated to 60°C and stirred for 5 hours. The reaction mixture was then added with water (20 mL), and washed with ethyl acetate (20 mL). The aqueous phase was added with dilute hydrochloric acid to adjust the pH to 6, and filtered to obtain a crude product of compound **1-5,** which was directly used in the next reaction step. MS (ESI) m/z: 234.0 $[M+H]^+$.

**Step 4: Synthesis of compound 1**

To a solution of compound **1-5** (380.00 mg, 1.63 mmol, 1.00 *eq*) in *N,N*-dimethylformamide (6.00 mL) was added 2-(7-azabenzotriazol-1-yl)-*N,N,N',N'*-tetramethyluronium hexafluorophosphate (619.50 mg, 1.63 mmol, 1.00 *eq)* and *N,N*-diisopropylethylamine (421.17 mg, 3.26 mmol, 2.00 *eq*) in an ice bath, and the reaction mixture was stirred in an ice bath for 10 min. The reaction mixture was then added with compound **BB-1** (336.11 mg, 1.63 mmol, 1.00 *eq*), and stirred for 20 min. The reaction mixture was added with ethyl acetate (100 mL), and washed with water (30 mL × 3). The organic phase was dried over anhydrous sodium sulfate, filtered, and the filtrate was concentrated under reduced pressure. The crude product was purified by preparative high performance liquid chromatography (chromatographic column: Waters Xbridge 150×25 mm×5 μm; mobile phase: [water (10 mM ammonium bicarbonate)-acetonitrile], the retention time of the HPLC column was 11.5 min) to obtain compound **1.** $^1$H NMR (400 MHz, $CDCl_3$): δ 9.07 (m, 1H), 7.68 (d, *J* = 2. 0 Hz, 1H), 7.31 (br t, *J* = 5.6 Hz, 1H), 7.24-7.13 (m, 4H), 7.03-7.01 (m, 1H), 6.88 (d, *J* = 8.4 Hz, 1H), 4.13-4.05 (m, 1H), 3.85 (d, *J* = 14.8 Hz, 1H), 3.80-3.72 (m, 1H), 3.71-3.60 (m, 2H), 3.00-2.90 (m, 3H), 2.82-2.62 (m, 5H), 2.40-2.29 (m, 2H), 2.08-1.91 (m, 3H). MS (ESI) m/z: 422.4 $[M+H]^+$.

**Embodiment 2: Preparation of compound 2**

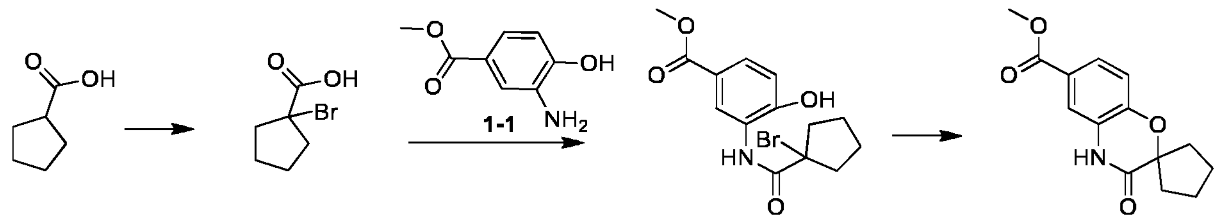


**2-1** **2-2** **2-3** **2-4**

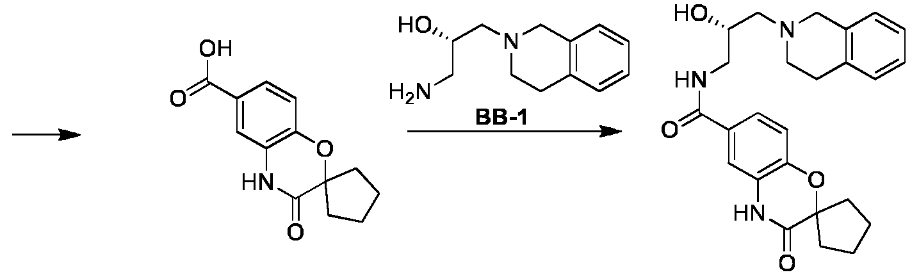


**2-5** **2**

**Step 1: Synthesis of compound 2-2**

To compound **2-1** (20 g, 175.22 mmol, 19.05 mL, 1 *eq*) was added phosphorus tribromide (7.59 g, 28.04 mmol,

0.16 *eq*), the reaction mixture was stirred at 100°C for 1 hour, then added with liquid bromine (70.00 g, 438.05 mmol, 22.58 mL, 2.5 *eq*), and the reaction mixture was stirred continuously at 100°C for 12 hours. The reaction mixture was cooled to 0-15°C, then added with dichloromethane (200 mL), and the reaction mixture was added with sodium bisulfite solution (10%, 200 mL) to quench. The reaction mixture was stirred for 0.5 hours, and extracted with dichloromethane (200 mL × 3). The organic phases were combined, washed with saturated brine (200 mL), dried over anhydrous sodium sulfate, filtered, and the filtrate was concentrated under reduced pressure to obtain compound **2-2.**

$^{1}$H NMR (400 MHz, $CDCl_3$): δ 10.70 (br s, 1H), 2.36-2.13 (m, 4H), 2.00-1.84 (m, 2H), 1.82-1.64 (m, 2H).

**Step 2: Synthesis of compound 2-3**

Compound **2-2** (1.73 g, 8.97 mmol, 1.5 *eq)* was dissolved in dichloromethane (20 mL), then **1-1** (1 g, 5.98 mmol, 1 *eq*), *N*,*N*-diisopropylethylamine (2.55 g, 19.74 mmol, 3.44 mL, 3.3 *eq*) and 2-(7-azabenzotriazol-1-yl)-*N*,*N*,*N*',*N*'-tetramethyluronium hexafluorophosphate (3.41 g, 8.97 mmol, 1.5 *eq*) were added, and the reaction mixture was stirred at 25°C for 1 hour. The reaction mixture was added with water (20 mL), and extracted with dichloromethane (30 mL × 3). The organic phases were combined, washed with saturated brine (30 mL × 3), dried over anhydrous sodium sulfate, filtered, and the filtrate was concentrated under reduced pressure. The crude product was purified by preparative HPLC (chromatographic column: Phenomenex luna C18 250×80 mm×10 μm; mobile phase: [water (0.05% hydrochloric acid)-acetonitrile]; acetonitrile%: 28% to 58%, the retention time of the HPLC column was 28 min) to obtain compound **2-3.** MS (ESI) m/z: 342.1 $[M+1]^+$.

**Step 3: Synthesis of compound 2-4**

Compound **2-3** (0.64 g, 1.87 mmol, 1 *eq)* was dissolved in *N*,*N*-dimethylformamide (10 mL), then potassium carbonate (1.29 g, 9.35 mmol, 5 *eq)* was added, and the reaction mixture was stirred at 80°C for 8 hours. The reaction mixture was added with water (50 mL), and extracted with ethyl acetate (50 mL × 3). The organic phases were combined, washed with saturated brine (50 mL), dried over anhydrous sodium sulfate, filtered, and the filtrate was concentrated under reduced pressure. A crude product of compound **2-4** was obtained. MS (ESI) m/z: 262.1 $[M+1]^+$.

**Step 4: Synthesis of compound 2-5**

Compound **2-4** (2.2 g, 8.42 mmol, 1 *eq*) was dissolved in a solution of tetrahydrofuran (12 mL) and methanol (6 mL), and then a solution of lithium hydroxide monohydrate (1.41 g, 33.68 mmol, 4 *eq*) dissolved in water (3 mL) was added. The reaction mixture was stirred at 60°C for 5 hours. The reaction mixture was concentrated under reduced pressure. The reaction mixture was then added with hydrochloric acid (2 M) to adjust the pH to 2 to 3, stirred at 25°C for 1 hour, filtered, and the filter cake was collected and dried under reduced pressure. Compound **2-5** was obtained. MS (ESI) m/z: 248.2 $[M+1]^+$.

**Step 5: Synthesis of compound 2**

Compound **2-5** (0.1 g, 404.46 μmol, 1 *eq*) was dissolved in *N*,*N*-dimethylformamide (6 mL) at 0°C, then 2-(7-azabenzotriazol-1-yl)-*N*,*N*,*N*',*N*'-tetramethyluronium hexafluorophosphate (153.79 mg, 404.46 μmol, 1 *eq*) and *N*,*N*-diisopropylethylamine (104.55 mg, 808.91 μmol, 140.90 μL, 2 *eq*) were added, and the reaction mixture was stirred for 10 min. The reaction mixture was added with compound **BB-1** (83.43 mg, 404.46 μmol, 1 *eq*), and stirred continuously at 0°C for 20 min. The reaction mixture was added with water (20 mL), and extracted with ethyl acetate (20 mL × 3). The organic phases were combined, washed with saturated brine (20 mL), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile%: 25% to 55%, the retention time of the HPLC column was 10 min) to obtain compound **2.**

$^{1}$H NMR (400 MHz, $CDCl_3$): δ 8.00 (br s, 1H), 7.38 (d, *J* = 2.0 Hz, 1H), 7.33 (dd, *J* = 2.0, 8.0 Hz, 1H), 7.25-7.08 (m, 4H), 7.04 (d, *J* = 6.8 Hz, 1H), 6.91 (d, *J* = 8.0 Hz, 1H), 4.07 (m, 1H), 3.89-3.83 (m, 1H), 3.79-3.64 (m, 2H), 3.53 (m, 1H), 3.01-2.90 (m, 3H), 2.83-2.58 (m, 3H), 2.26-2.15 (m, 2H), 2.05-1.77 (m, 6H). MS (ESI) m/z: 436.3 $[M+1]^+$.

**Embodiment 3: Preparation of compound 3**

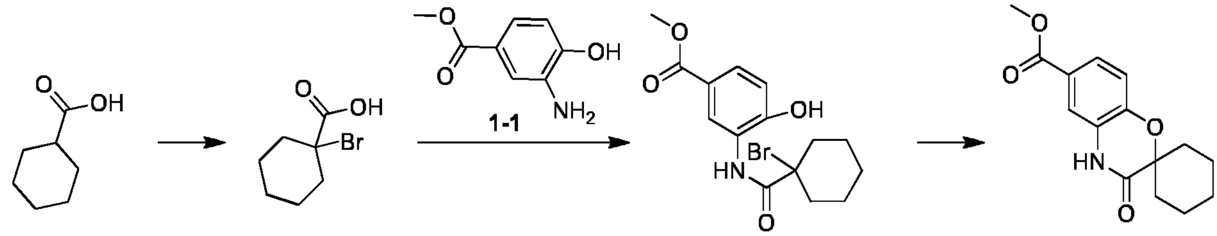


3-1 3-2 3-3 3-4

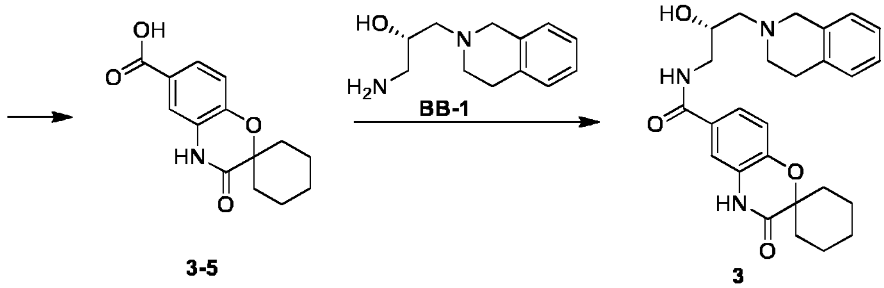


3-5 3

**Step 1: Synthesis of compound 3-2**

To compound **3-1** (20 g, 156.04 mmol, 19.42 mL, 1 *eq*) was added phosphorus tribromide (8.45 g, 31.21 mmol, 0.2 *eq*), the reaction mixture was stirred at 100°C for 1 hour, then added with liquid bromine (62.34 g, 390.11 mmol, 20.11 mL, 2.5 *eq*), and the reaction mixture was stirred continuously at 100°C for 48 hours. The reaction mixture was cooled to 0-15°C, then added with dichloromethane (200 mL), and the reaction mixture was added with sodium bisulfite solution (10%, 200 mL) to quench. The reaction mixture was stirred for 0.5 hours, and extracted with dichloromethane (200 mL × 3). The organic phases were combined, washed with saturated brine (200 mL), dried over anhydrous sodium sulfate, filtered, and the filtrate was concentrated under reduced pressure. Compound **3-2** was obtained.

$^1H$ NMR (400 MHz, $CDCl_3$): δ 12.11 (br s, 1H), 2.16-2.01 (m, 3H), 1.91-1.80 (m, 1H), 1.74-1.61 (m, 2H), 1.59-1.30 (m, 4H).

**Step 2: Synthesis of compound 3-3**

Compound **3-2** (1.86 g, 8.97 mmol, 1.5 *eq)* was dissolved in dichloromethane (20 mL), then **1-1** (1 g, 5.98 mmol, 1 *eq), N,N*-diisopropylethylamine (2.55 g, 19.73 mmol, 3.44 mL, 3.3 *eq*) and 2-(7-azabenzotriazol-1-yl)-*N,N,N',N'*-tetramethyluronium hexafluorophosphate (3.41 g, 8.97 mmol, 1.5 *eq*) were added, and the reaction mixture was stirred at 25°C for 1 hour. The reaction mixture was added with water (20 mL), and extracted with dichloromethane (30 mL × 3). The organic phases were combined, washed with saturated brine (30 mL × 3), dried over anhydrous sodium sulfate, filtered, and the filtrate was concentrated under reduced pressure. The crude product was purified by preparative HPLC (chromatographic column: Phenomenex luna C18 150×25 mm×10 μm; mobile phase: [water (0.05% hydrochloric acid)-acetonitrile]; acetonitrile%: 45% to 75%, the retention time of the HPLC column was 10 min) to obtain compound **3-3.** MS (ESI) m/z: 356.1 $[M+1]^+$.

**Step 3: Synthesis of compound 3-4**

Compound **3-3** (0.24 g, 673.76 μmol, 1 *eq*) was dissolved in N,N-dimethylformamide (10 mL), then potassium carbonate (465.60 mg, 3.37 mmol, 5 *eq*) was added, and the reaction mixture was stirred at 80°C for 2 hours. The reaction mixture was then added with water (50 mL), and extracted with ethyl acetate (50 mL × 3). The organic phases were combined, washed with saturated brine (50 mL), dried over anhydrous sodium sulfate, filtered, and the filtrate was concentrated under reduced pressure. A crude product of compound **3-4** was obtained. MS (ESI) m/z: 276.1 $[M+1]^+$.

**Step 4: Synthesis of compound 3-5**

Compound **3-4** (0.2 g, 726.48 μmol, 1 *eq*) was dissolved in a solution of tetrahydrofuran (12 mL) and methanol (6 mL), and then a solution of lithium hydroxide monohydrate (121.93 mg, 2.91 mmol, 4 *eq*) in water (3 mL) was added.

The reaction mixture was stirred at 60°C for 6 hours. The reaction mixture was concentrated under reduced pressure. The reaction mixture was then added with HCl (2 M) to adjust the pH to 2 to 3, stirred at 25°C for 1 hour, filtered, and the filter cake was collected and dried under reduced pressure. A crude product of compound **3-5** was obtained. MS (ESI) m/z: 262.0 $[M+1]^+$.

**Step 5: Synthesis of compound 3**

Compound **3-5** (0.15 g, 574.11 μmol, 1 *eq*) was dissolved in *N*,*N*-dimethylformamide (6 mL) at 0°C, then 2-(7-azabenzotriazol-1-yl)-*N*,*N*,*N'*,*N'*-tetramethyluronium hexafluorophosphate (218.29 mg, 574.11 μmol, 1 *eq*) and A, A-diisopropylethylamine (148.40 mg, 1.15 mmol, 200.00 μL, 2 *eq*) were added, and the reaction mixture was stirred for 10 min. The reaction mixture was added with compound **BB-1** (118.43 mg, 574.11 μmol, 1 *eq*), and stirred continuously at 0°C for 20 min. The reaction mixture was added with water (20 mL), and extracted with ethyl acetate (20 mL × 3). The organic phases were combined, washed with saturated brine (20 mL), dried over anhydrous sodium sulfate, filtered, and the filtrate was concentrated under reduced pressure. The crude product was purified by preparative HPLC (chromatographic column: Waters Xbridge C18 150×50 mm×10 μm; mobile phase: [water (10 mM ammonium bicarbonate)-acetonitrile]; acetonitrile%: 33% to 63%, the retention time of the HPLC column was 11.5 min) to obtain compound 3.

$^1H$ NMR (400 MHz, $CDCl_3$): δ 8.01 (br s, 1H), 7.43 (d, *J* = 2.0 Hz, 1H), 7.36 (dd, *J* = 1.6, 8.4 Hz, 1H), 7.24-7.13 (m, 4H), 7.07-7.01 (m, 1H), 6.99 (d, *J* = 8.4 Hz, 1H), 4.10 (br dd, *J* = 4.8, 8.6 Hz, 1H), 3.97-3.85 (m, 1H), 3.81-3.69 (m, 2H), 3.64-3.49 (m, 1H), 3.07-2.92 (m, 3H), 2.91-2.60 (m, 4H), 1.96-1.72 (m, 10H). MS (ESI) m/z: 450.3 $[M+1]^+$.

**Embodiment 4: Preparation of compound 4**

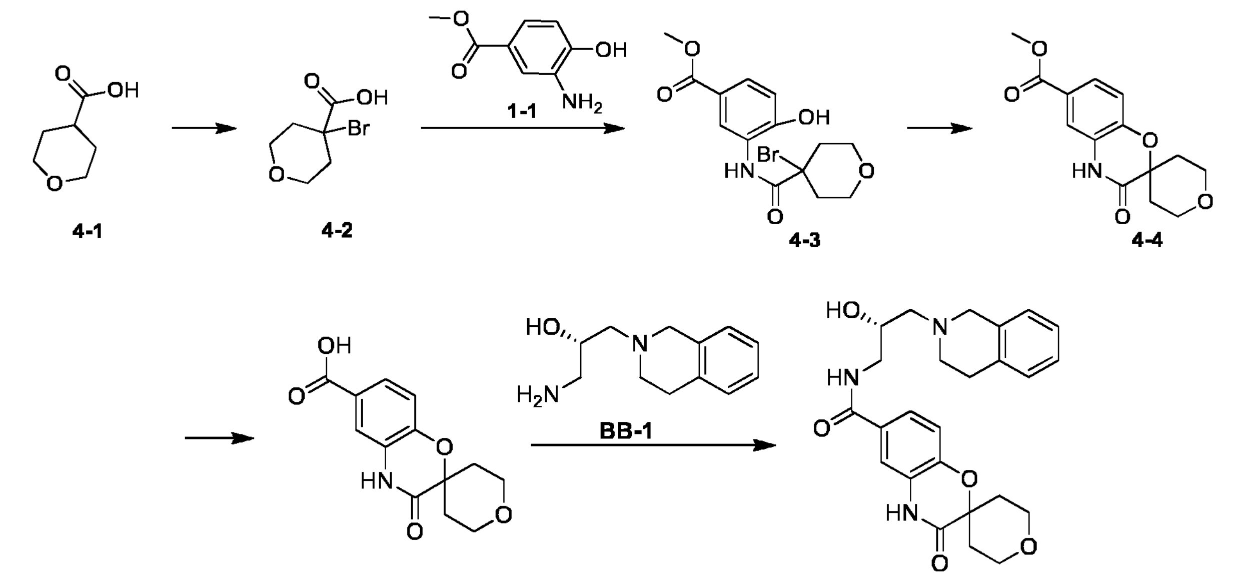


4-1 4-2 4-3 4-4

4-5 4

**Step 1: Synthesis of compound 4-2**

Compound **4-1** (10 g, 76.84 mmol, 19.42 mL, 1 *eq*) was dissolved in 1,2-dichloroethane (100 mL), then phosphorus tribromide (4.16 g, 15.37 mmol, 0.2 *eq*) was added, and the reaction mixture was stirred at 100°C for 1 hour. The reaction mixture was then added with liquid bromine (30.70 g, 192.10 mmol, 9.90 mL, 2.5 *eq*), and stirred continuously at 100°C for 48 hours. The reaction mixture was cooled to 0-15°C, then added with dichloromethane (200 mL), and the reaction mixture was added with sodium bisulfite solution (10%, 200 mL) to quench. The reaction mixture was stirred for 0.5 hours, and extracted with dichloromethane (200 mL × 3). The organic phases were combined, washed with saturated brine (200 mL), dried over anhydrous sodium sulfate, filtered, and the filtrate was concentrated under reduced pressure to obtain compound **4-2.**

$^1H$ NMR (400 MHz, $CDCl_3$): δ 11.18 (br s, 1H), 4.00-3.69 (m, 4H), 2.26-2.07 (m, 3H), 1.86-1.72 (m, 1H).

**Step 2: Synthesis of compound 4-3**

Compound **4-2** (2.5 g, 11.96 mmol, 1 *eq*) was dissolved in *N*,*N*-dimethylformamide (20 mL), then 2-(7-azabenzotriazol-1-yl)-*N*,*N*,*N'*,*N'*-tetramethyluronium hexafluorophosphate (4.55 g, 11.96 mmol, 1 *eq*), compound **1-1** (2.00 g, 11.96 mmol, 1 *eq*) and *N,N*-diisopropylethylamine (3.09 g, 23.92 mmol, 4.17 mL, 2 *eq*) were added, and the reaction mixture was stirred at 25°C for 2 hours. The reaction mixture was then added with water (50 mL), and extracted with ethyl acetate (50 mL × 3). The organic phases were combined, washed with saturated brine (50 mL × 1), dried over anhydrous sodium sulfate, filtered, and the filtrate was concentrated under reduced pressure. The crude product was purified by preparative HPLC (chromatographic column: Phenomenex luna C18 250×50 mm×15 μm; mobile phase: [water (0.05% hydrochloric acid)-acetonitrile]; acetonitrile%: 20% to 50%, the retention time of the HPLC column was 25 min) to obtain compound **4-3.** MS (ESI) m/z: 358.1 $[M+1]^+$.

**Step 3: Synthesis of compound 4-4**

Compound **4-3** (1.2 g, 3.35 mmol, 1 *eq*) was dissolved in A, A-dimethylformamide (24 mL), then potassium carbonate (2.32 g, 16.75 mmol, 5 *eq*) was added, and the reaction mixture was stirred at 80°C for 2 hours. The reaction mixture was then added with water (50 mL), and extracted with ethyl acetate (50 mL × 3). The organic phases were combined, washed with saturated brine (50 mL), dried over anhydrous sodium sulfate, filtered, and the filtrate was concentrated under reduced pressure. Compound **4-4** was obtained. MS (ESI) m/z: 278.1 $[M+1]^+$.

**Step 4: Synthesis of compound 4-5**

Compound **4-4** (0.6 g, 2.16 mmol, 1 *eq*) was dissolved in a solution of tetrahydrofuran (12 mL) and methanol (6 mL), and then a solution of lithium hydroxide monohydrate (363.20 mg, 8.66 mmol, 4 *eq*) dissolved in water (3 mL) was added. The reaction mixture was stirred at 60°C for 3 hours. The reaction mixture was concentrated under reduced pressure. The reaction mixture was then added with hydrochloric acid (2 M) to adjust the pH to 2 to 3, stirred at 25°C for 1 hour, filtered, and the filter cake was collected and dried under reduced pressure. A crude product of compound **4-5** was obtained. MS (ESI) m/z: 264.2 $[M+1]^+$.

**Step 5: Synthesis of compound 4**

Compound **4-5** (0.1 g, 379.87 μmol, 1 *eq*) was dissolved in *N*,*N*-dimethylformamide (3 mL) at 0°C, then 2-(7-azabenzotriazol-1-yl)-*N*,*N*,*N'*,*N'*-tetramethyluronium hexafluorophosphate (144.44 mg, 379.87 μmol, 1 *eq*) and *N*,*N*-diisopropylethylamine (98.19 mg, 759.75 μmol, 132.33 μL, 2 *eq*) were added, and the reaction mixture was stirred for 10 min. The reaction mixture was added with compound **BB-1** (78.36 mg, 379.87 μmol, 1 *eq*), and stirred continuously at 0°C for 20 min. The reaction mixture was added with water (20 mL), and extracted with ethyl acetate (20 mL × 3). The organic phases were combined, washed with saturated brine (20 mL), dried over anhydrous sodium sulfate, filtered, and the filtrate was concentrated under reduced pressure. The crude product was purified by preparative HPLC (chromatographic column: Waters Xbridge C18 150×50 mm×10 μm; mobile phase: [water (10 mM ammonium bicarbonate)-acetonitrile]; acetonitrile%: 17% to 47%, the retention time of the HPLC column was 10 min) to obtain compound **4.**

$^1$H NMR (400 MHz, $CDCl_3$): δ 8.71 (br s, 1H), 7.60 (d, *J* = 2.0 Hz, 1H), 7.34-7.29 (m, 1H), 7.25-7.12 (m, 4H), 7.03 (d, *J* = 7.4 Hz, 1H), 6.97 (d, *J* = 8.4 Hz, 1H), 4.09 (m, 1H), 3.95-3.81 (m, 5H), 3.77 (m, 1H), 3.72-3.53 (m, 2H), 3.03-2.89 (m, 3H), 2.87-2.59 (m, 3H), 2.25 (m, 2H), 1.79 (br d, *J* = 12.0 Hz, 3H). MS (ESI) m/z: 452.3 $[M+1]^+$.

**Embodiment 5: Preparation of compound 5**

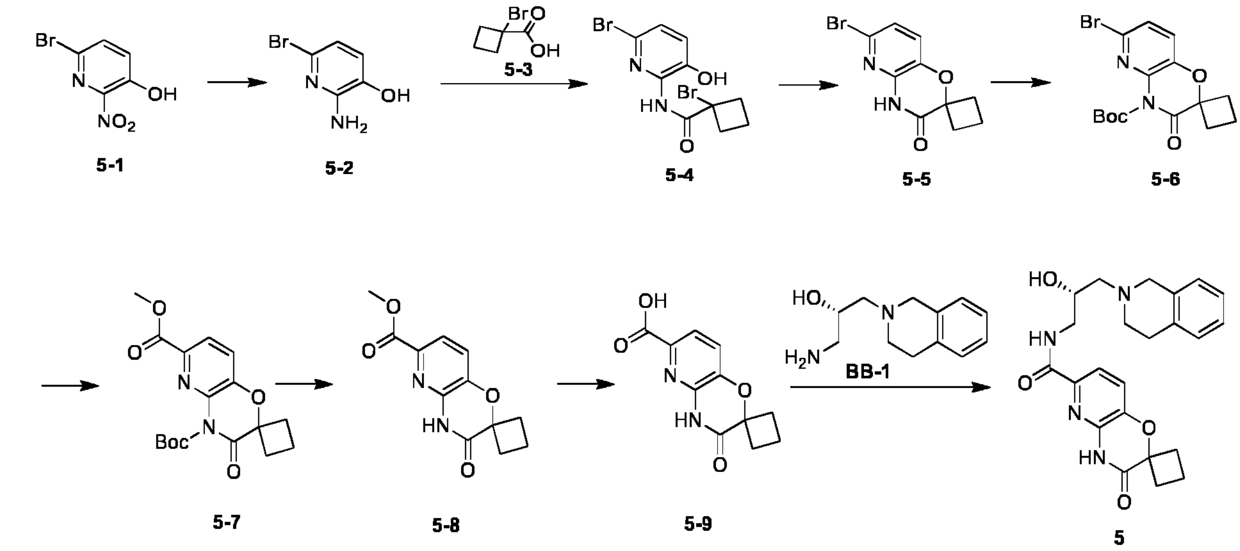


**Step 1: Synthesis of compound 5-2**

To a solution of compound **5-1** (1 g, 4.57 mmol, 1 *eq*) and ammonium chloride (2.44 g, 45.66 mmol, 10 *eq*) in ethanol (20 mL) and water (20 mL) was added iron powder (2.55 g, 45.66 mmol, 10 *eq*) at 75°C, and the reaction mixture was stirred continuously for 2 hours. The reaction mixture was cooled to room temperature, filtered, and the filtrate was concentrated under reduced pressure to obtain a crude product of compound **5-2.**

**Step 2: Synthesis of compound 5-4**

To a solution of compound **5-2** (691.38 mg, 3.86 mmol, 1 *eq*) in *N,N*-dimethylformamide (10 mL) was added 2-(7-azabenzotriazol-1-yl)-*N,N,N',N'*-tetramethyluronium hexafluorophosphate (1.76 g, 4.63 mmol, 1.2 *eq)* and diisopropylethylamine (748.73 mg, 5.79 mmol, 1.01 mL, 1.5 *eq*) respectively at 25°C, the reaction mixture was stirred for 10 min, then compound **5-3** (0.730 g, 3.86 mmol, 1 *eq*) was added thereto, and the reaction mixture was stirred continuously for 12 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 3:1) to obtain compound **5-4.**

**Step 3: Synthesis of compound 5-5**

To a solution of compound **5-4** (0.7 g, 2.00 mmol, 1 *eq*) in *N*,*N*-dimethylformamide (2 mL) was added potassium carbonate (414.61 mg, 3.00 mmol, 1.5 *eq*) at 25°C, and the reaction mixture was heated to 80°C and stirred for 2 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **5-5.**

**Step 4: Synthesis of compound 5-6**

To a solution of compound **5-5** (0.3 g, 1.11 mmol, 1 *eq*) and di-*tert*-butyl dicarbonate (364.97 mg, 1.67 mmol, 384.18 μL, 1.5 *eq*) in tetrahydrofuran (10 mL) was added 4-dimethylaminopyridine (13.62 mg, 111.49 μmol, 0.1 *eq*) at 25°C, and the reaction mixture was stirred continuously for 1 hour. The reaction mixture was added with saturated citric acid solution (15 mL) to quench, and extracted with ethyl acetate (15 mL × 3). The organic phases were combined, washed with saturated brine (15 mL × 2), dried over anhydrous sodium sulfate, filtered, and the filtrate was concentrated under reduced pressure. A crude product of compound **5-6** was obtained.

**Step 5: Synthesis of compound 5-7**

A solution of compound **5-6** (0.4 g, 1.08 mmol, 1 *eq*), [1,1'-bis(diphenylphosphino)ferrocene]palladium dichloride (237.82 mg, 325.02 μmol, 0.3 *eq*) and triethylamine (328.88 mg, 3.25 mmol, 452.38 μL, 3 *eq*) in methanol (20 mL) was

stirred and reacted at 80°C for 12 hours under carbon monoxide (50 psi) atmosphere. The reaction mixture was cooled to room temperature, filtered, and the filtrate was concentrated under reduced pressure. The crude product was purified by silica gel column chromatography (petroleum ether: ethyl acetate = 3:1 to 1:1) to obtain compound **5-7.**

**Step 6: Synthesis of compound 5-8**

To a solution of compound **5-7** (0.2 g, 574.14 μmol, 1 *eq*) in ethyl acetate (5 mL) was added hydrochloric acid/ethyl acetate (4 M, 5 mL, 34.84 *eq*) at 25°C, and the reaction mixture was stirred continuously for 12 hours. The reaction mixture was concentrated under reduced pressure. The crude product was added with saturated sodium bicarbonate solution (10 mL) to adjust the pH to 9, and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 3:1 to 0:1) to obtain compound **5-8.**

**Step 7: Synthesis of compound 5-9**

To a solution of compound **5-8** (0.055 g, 221.57 μmol, 1 *eq*) in methanol (5 mL), tetrahydrofuran (3 mL) and water (1 mL) was added lithium hydroxide monohydrate (27.89 mg, 664.70 μmol, 3 *eq*) at 25°C, and the reaction mixture was stirred continuously for 2 hours. The reaction mixture was diluted with water (10 mL), added with dilute hydrochloric acid (1 M) to adjust the pH to 3, and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **5-9.**

**Step 8: Synthesis of compound 5**

To a solution of compound **5-9** (0.038 g, 162.25 μmol, 1 *eq*) in *N,N*-dimethylformamide (2 mL) was added 2-(7-azabenzotriazol-1-yl)-*N,N,N',N'*-tetramethyluronium hexafluorophosphate (74.03 mg, 194.70 μmol, 1.2 *eq*) and *N,N*-diisopropylethylamine (31.45 mg, 243.37 μmol, 42.39 μL, 1.5 *eq*) respectively at 25°C, the reaction mixture was stirred for 10 min, then compound **BB-1** (33.47 mg, 162.25 μmol, 1 *eq*) was added thereto, and the reaction mixture was stirred continuously for 1 hour. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mm×5 μm; mobile phase: [water (10 mM ammonium bicarbonate)-acetonitrile]; acetonitrile: 25% to 55%, the retention time of the HPLC column was 10 min) to obtain compound **5**.

$^1$H NMR (400 MHz, DMSO-$d_6$): δ 11.38 (s, 1H), 8.10 (br s, 1H), 7.65 (d, *J* = 8.2 Hz, 1H), 7.55 (d, *J* = 8.2 Hz, 1H), 7.22-6.99 (m, 4H), 5.05 (br s, 1H), 4.25-3.41 (m, 3H), 3.01-2.73 (m, 4H), 2.63-2.52 (m, 4H), 2.44-2.17 (m, 4H), 2.01-1.90 (m, 1H), 1.90-1.74 (m, 1H). MS (ESI) m/z: 423.4 [M+H]$^+$.

**Embodiment 6: Preparation of compound 6**

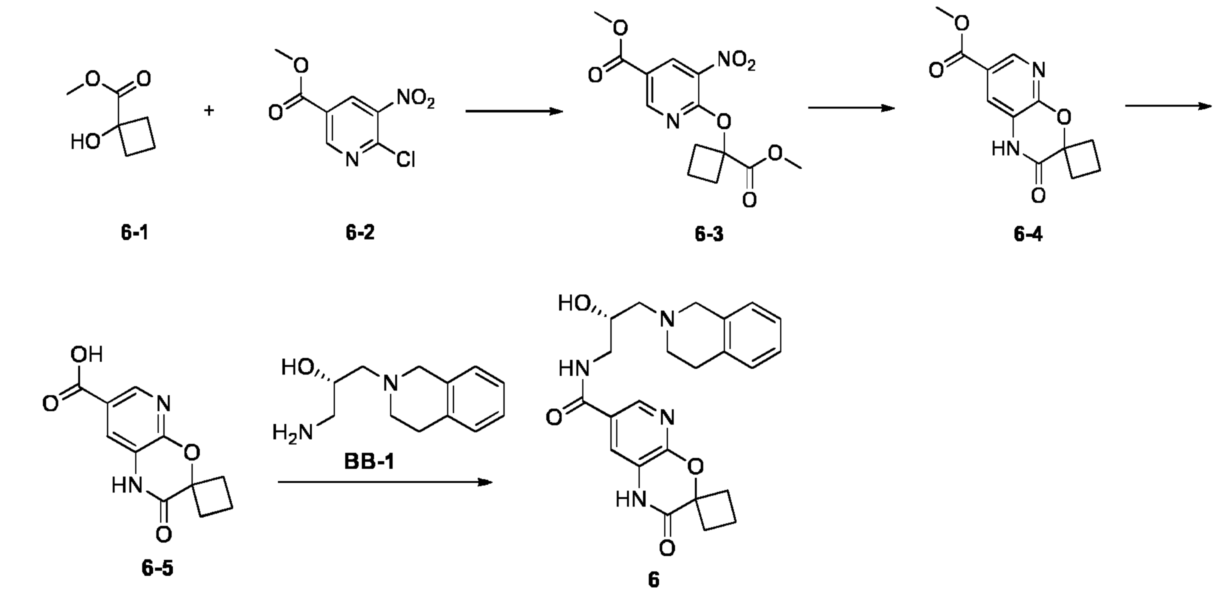

6-1 6-2 6-3 6-4

6-5 BB-1 6

**Step 1: Synthesis of compound 6-3**

To a solution of compound **6-2** (332.8 mg, 1.54 mmol, 1 *eq*) in dioxane (5.00 mL) was added sodium hydride (92.2 mg, 2.31 mmol, purity: 60%, 1.5 *eq*) and compound **6-1** (200.0 mg, 1.54 mmol, 1 *eq*), and the reaction mixture was stirred continuously at 20°C for 16 hours. The reaction mixture was added with aqueous solution (50 mL) to quench, and extracted with ethyl acetate (50 mL × 4). The organic phases were combined, dried over anhydrous sodium sulfate, filtered, and the filtrate was concentrated under reduced pressure. The crude product was purified by a preparative thin-layer plate (petroleum ether: ethyl acetate = 5:1) to obtain compound **6-3.** MS (ESI) m/z: 311.0 $[M+H]^+$.

**Step 2: Synthesis of compound 6-4**

To a solution of compound **6-3** (194.0 mg, 625.28 μmol, 1 *eq*) in glacial acetic acid (2.00 mL) was added reduced iron powder (139.6 mg, 2.50 mmol, 4 *eq*) at room temperature, and the reaction mixture was stirred at 70°C for 2 hours. The reaction mixture was added with saturated sodium bicarbonate aqueous solution (2 mL) to quench, then the suspension was filtered through diatomite, and the filter cake was washed with ethyl acetate (50 mL). The filtrates were combined, then the phases were separated, and the aqueous phase was extracted with ethyl acetate (50 mL × 3). The organic phases were combined, washed with water (50 mL × 3) and saturated brine (50 mL × 1) successively, dried over anhydrous sodium sulfate, filtered, and the filtrate was concentrated under reduced pressure to obtain a crude product of compound **6-4,** which was directly used in the next reaction step.

$^1H$ NMR (400 MHz, $CDCl_3$): δ 8.60 (d, *J* = 2.0 Hz, 1H), 8.50-8.40 (m, 1H), 7.76-7.73 (m, 1H), 3.95 (s, 3H), 2.83-2.70 (m, 2H), 2.59-2.48 (m, 2H), 2.17-1.95 (m, 2H). MS (ESI) m/z: 249.0 $[M+H]^+$.

**Step 3: Synthesis of compound 6-5**

To a mixed solution of compound **6-4** (86.0 mg, 346.45 μmol, 1 *eq*) in tetrahydrofuran (1.00 mL) and water (0.20 mL) was added lithium hydroxide monohydrate (43.6 mg, 1.04 mmol, 3 *eq*) at room temperature, and the reaction mixture was stirred at 20°C for 3 hours. The reaction mixture was added with dilute hydrochloric acid to adjust the pH to 4, and extracted with ethyl acetate (20 mL × 3). The organic phases were combined, dried over anhydrous sodium sulfate, filtered, and the filtrate was concentrated under reduced pressure to obtain a crude product of compound **6-5,** which was directly used in the next reaction step. MS (ESI) m/z: 235.0 $[M+H]^+$.

**Step 4: Synthesis of compound 6**

To a solution of compound **6-5** (79.0 mg, 337.31 μmol, 1 *eq*) in *N,N*-dimethylformamide (1.00 mL) was added 2-(7-azabenzotriazol-1-yl)-*N,N,N',N'*-tetramethyluronium hexafluorophosphate (153.9 mg, 404.77 μmol, 1.2 *eq*) and *N,N*-diisopropylethylamine (87.2 mg, 674.61 μmol, 117.51 μL, 2 *eq*) respectively at 0°C, the reaction mixture was stirred for 0.5 hours, then a solution of compound **BB-1** (69.6 mg, 337.31 μmol, 1 *eq)* in *N,N*-dimethylformamide (0.50 mL) was added, and the reaction mixture was stirred continuously at 0°C for 0.5 hours. The reaction mixture was diluted with

water (20 mL), and extracted with ethyl acetate (20 mL × 3). The organic phases were combined, washed with water (20 mL) and saturated brine (20 mL), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (0.05% ammonia water)-acetonitrile], acetonitrile: 18% to 48%, the retention time of the HPLC column was 10 min) to obtain compound **6.**

$^1$H NMR (400 MHz, $CDCl_3$): δ 8.29 (d, *J* = 2.4 Hz, 1H), 7.72 (d, *J* = 2.0 Hz, 1H), 7.23-7.09 (m, 4H), 7.05-7.01 (m, 1H), 4.12-4.04 (m, 1H), 3.87 (d, *J* = 15.2 Hz, 1H), 3.81-3.74 (m, 1H), 3.67 (d, *J* = 14.8 Hz, 1H), 3.52 (dt, *J* = 5.8, 13.6 Hz, 1H), 3.01-2.90 (m, 3H), 2.83-2.67 (m, 4H), 2.65-2.58 (m, 1H), 2.56-2.45 (m, 2H), 2.14-1.95 (m, 2H); MS (ESI) m/z: 423.2 [M+H]$^+$.

**Embodiment 7: Preparation of compound 7**

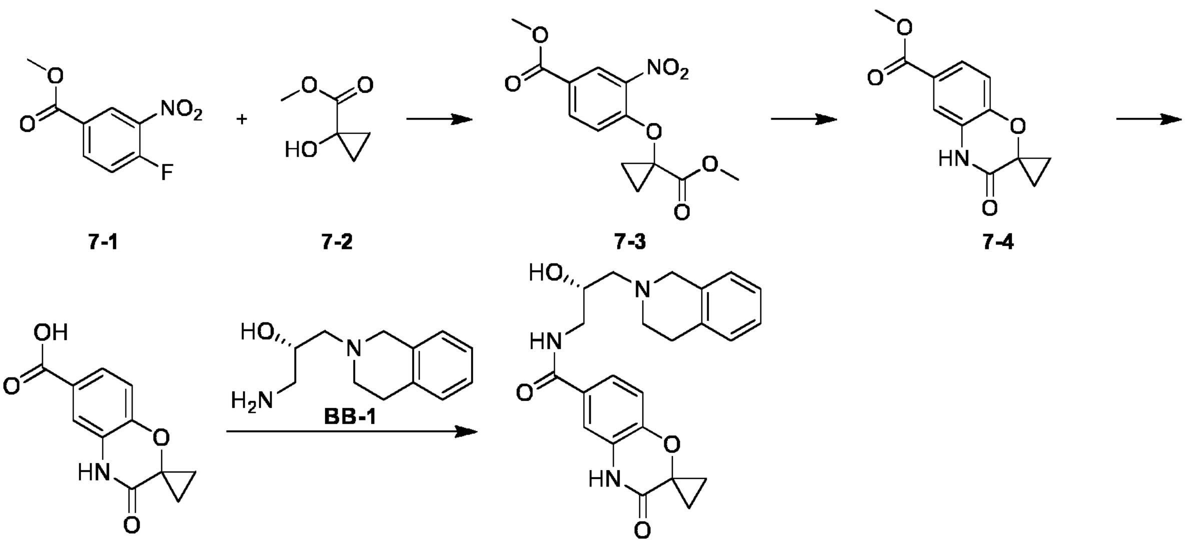

7-1 7-2 7-3 7-4

BB-1

7-5 7

**Step 1: Synthesis of compound 7-3:**

To a solution of compound **7-1** (300 mg, 2.58 mmol, 1 *eq*) in *N*,*N*-dimethylformamide (3.00 mL) was added sodium hydride (206.69 mg, 5.17 mmol, purity: 60%, 2 *eq*) at 0°C, the reaction mixture was stirred at 0°C for 10 min, then compound **7-2** (514.50 mg, 2.58 mmol, 1 *eq*) was added dropwise, and the reaction mixture was stirred continuously at 10°C for 2 hours. The reaction mixture was then added with saturated ammonium chloride aqueous solution (50 mL) to quench, and extracted with ethyl acetate (50 mL × 3). The organic phases were combined, washed with water (50 mL) and saturated brine (50 mL), dried over anhydrous sodium sulfate, filtered, and the filtrate was concentrated under reduced pressure to obtain a crude product of compound **7-3,** which was directly used in the next reaction step. MS (ESI) m/z: 296.0 [M+1]$^+$.

**Step 2: Synthesis of compound 7-4:**

To a solution of compound **7-3** (660 mg, 2.24 mmol, 1 *eq*) in glacial acetic acid (6.00 mL) was added reduced iron powder (624.19 mg, 11.18 mmol, 5 *eq*) at room temperature, and the reaction mixture was stirred at 80°C for 1 hour. The reaction mixture was added with saturated sodium bicarbonate aqueous solution (50 mL) to quench, then the suspension was filtered through diatomite, and the filter cake was washed with ethyl acetate (50 mL). The filtrates were combined and the phases were separated. The organic phase was washed with water (50 mL), dried over anhydrous sodium sulfate, filtered, and the filtrate was concentrated under reduced pressure to obtain a crude product of compound **7-4,** which was directly used in the next reaction step. MS (ESI) m/z: 234.0 [M+1]$^+$.

**Step 3: Synthesis of compound 7-5:**

To a mixed solution of compound **7-4** (469 mg, 2.01 mmol, 1 *eq*) in tetrahydrofuran (4.00 mL), methanol (2.00

mL) and water (1.00 mL) was added lithium hydroxide monohydrate (253.16 mg, 6.03 mmol, 3 *eq*) at room temperature, and the reaction mixture was stirred at 17°C for 16 hours. The reaction mixture was added with dilute hydrochloric acid to adjust the pH to 4, and extracted with ethyl acetate (50 mL × 2). The organic phases were combined, washed with water (50 mL), dried over anhydrous sodium sulfate, filtered, and the filtrate was concentrated under reduced pressure to obtain a crude product of compound **7-5,** which was directly used in the next reaction step. MS (ESI) m/z: 220.0 $[M+1]^+$.

**Step 4: Synthesis of compound 7:**

To a solution of compound **7-5** (130 mg, 593.08 μmol, 1 *eq*) in *N,N*-dimethylformamide (2.00 mL) was added 2-(7-azabenzotriazol-1-yl)-*N,N,N',N'*-tetramethyluronium hexafluorophosphate (248.06 mg, 652.39 μmol, 1.1 *eq*) and *N,N*-diisopropylethylamine (229.96 mg, 1.78 mmol, 309.91 μL, 3 *eq*) respectively at 0°C, the reaction mixture was stirred for 0.5 hours, then compound **BB-1** (122.34 mg, 593.08 μmol, 1 *eq*) was added, and the reaction mixture was stirred continuously at 0°C for 0.5 hours. The reaction mixture was diluted with water (10 mL), then the suspension was stirred for 10 min, filtered, and the filter cake was washed with water (30 mL) and concentrated under reduced pressure. The crude product was purified by preparative HPLC (chromatographic column: Waters Xbridge 150×25 mmx5 μm; mobile phase: [water (0.05% ammonia water)-acetonitrile], acetonitrile%: 25% to 55%, the retention time of the HPLC column was 10 min). Compound 7 was obtained.

$^1H$ NMR (400 MHz, $CDCl_3$): δ 9.54 (s, 1H), 7.75 (s, 1H), 7.47-7.39 (m, 1H), 7.21-7.10 (m, 4H), 7.02-6.97 (m, 1H), 6.69 (d, *J* = 8.4 Hz, 1H), 4.12-4.03 (m, 1H), 3.82 (d, *J* = 14.8 Hz, 1H), 3.77-3.69 (m, 1H), 3.69-3.58 (m, 2H), 2.97-2.88 (m, 3H), 2.83-2.75 (m, 1H), 2.71 (dd, *J* = 12.4, 4.8 Hz, 1H), 2.65 (dd, *J* = 12.4, 8.4 Hz, 1H), 1.48-1.43 (m, 2H), 1.25-1.19 (m, 2H). MS (ESI) m/z: 408.3 $[M+1]^+$.

**Embodiment 8: Preparation of compound 8**

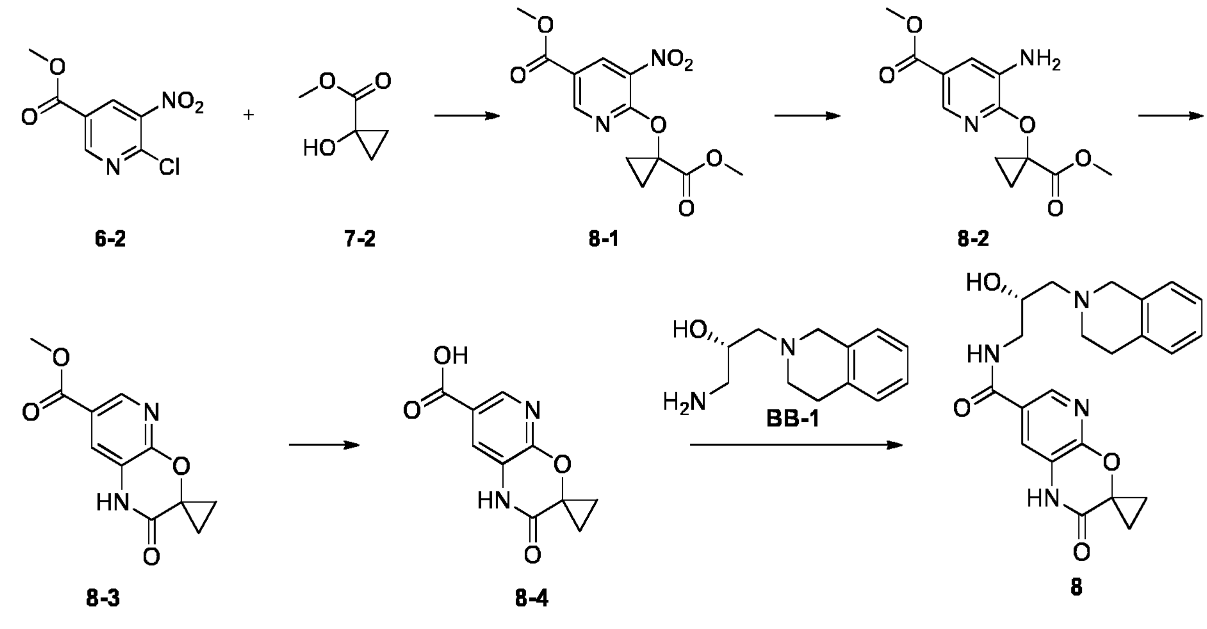

6-2 7-2 8-1 8-2

8-3 8-4 BB-1 8

**Step 1: Synthesis of compound 8-1:**

To a solution of **6-2** (1.60 g, 13.78 mmol, 1 *eq*) and compound **7-2** (2.98 g, 13.78 mmol, 1 *eq*) in tetrahydrofuran (30.00 mL) was added triethylenediamine (3.09 g, 27.56 mmol, 3.03 mL, 2 *eq*) at 20°C, the reaction mixture was stirred at 20°C for 16 hours, then added with water (100 mL), and extracted with ethyl acetate (100 mL × 2). The organic phases were combined, washed with water (100 mL), dried over anhydrous sodium sulfate, filtered, and the filtrate was concentrated under reduced pressure. The crude product was purified by silica gel column chromatography (petroleum ether: ethyl acetate = 10:1) to obtain compound **8-1.**

**Step 2: Synthesis of compound 8-2:**

To a mixed solution of compound **8-1** (1.14 g, 3.85 mmol, 1 *eq*) in ethanol (10.00 mL) and water (10.00 mL) was added ammonium chloride (1.03 g, 19.24 mmol, 5 *eq*) at room temperature, the reaction mixture was heated to 80°C, then reduced iron powder (1.07 g, 19.24 mmol, 5 *eq*) was slowly added, and the reaction mixture was stirred at 80°C for 3 hours. The reaction mixture was filtered through diatomite while hot, then the filtrate was concentrated under reduced pressure to remove the organic solvent, and extracted with ethyl acetate (50 mL × 3). The organic phases were combined, washed with water (20 mL), dried over anhydrous sodium sulfate, filtered, and the filtrate was concentrated under reduced pressure to obtain a crude product of compound **8-2,** which was directly used in the next reaction step. MS (ESI) m/z: 267.0 $[M+1]^+$.

**Step 3: Synthesis of compound 8-3:**

To a mixture of compound **8-2** (657.0 mg, 2.47 mmol, 1 *eq*) in methanol (7.00 mL) was added *p*-toluenesulfonic acid monohydrate (234.69 mg, 1.23 mmol, 0.5 *eq*), the reaction mixture was stirred at 70°C for 3 hours, and concentrated under reduced pressure. The reaction mixture was added with saturated sodium bicarbonate solution (5 mL), filtered, then the filter cake was washed with water (10 mL), and concentrated under reduced pressure to obtain a crude product of compound **8-3,** which was directly used in the next reaction step. MS (ESI) m/z: 235.1 [M+1].

**Step 4: Synthesis of compound 8-4:**

To a mixed solution of compound **8-3** (533.0 mg, 2.28 mmol, 1 *eq*) in tetrahydrofuran (8.00 mL), methanol (4.00 mL) and water (3.00 mL) was added lithium hydroxide monohydrate (286.5 mg, 6.83 mmol, 3 *eq*) at room temperature, and the reaction mixture was stirred at 50°C for 5 hours. The reaction mixture was concentrated under reduced pressure to remove the organic solvent, then the aqueous phase was added with dilute hydrochloric acid to adjust the pH to about 6, filtered, and the filter cake was concentrated under reduced pressure to obtain a crude product of compound **8-4,** which was directly used in the next reaction step.

$^1$H NMR (400 MHz, DMSO-$d_6$): δ 11.10 (s, 1H), 8.31 (d, *J* = 2.0 Hz, 1H), 7.71 (d, *J* = 2.4 Hz, 1H), 1.40-1.33 (m, 2H), 1.33-1.27 (m, 2H). MS (ESI) m/z: 221.1 $[M+1]^+$.

**Step 5: Synthesis of compound 8:**

To a solution of compound **8-4** (497.0 mg, 2.26 mmol, 1 *eq*) in *N,N*-dimethylformamide (10.00 mL) was added 2-(7-azabenzotriazol-1-yl)-*N,N,N',N'*-tetramethyluronium hexafluorophosphate (944.09 mg, 2.48 mmol, 1.1 *eq*) and *N,N*-diisopropylethylamine (583.46 mg, 4.51 mmol, 786.34 μL, 2 *eq*) respectively at 0°C, and the reaction mixture was stirred for 1 hour, then compound **BB-1** (512.19 mg, 2.48 mmol, 1.1 *eq*) was added, and the reaction mixture was stirred continuously at 0°C for 1 hour. The reaction mixture was diluted with water (30 mL), and extracted with ethyl acetate (30 mL × 3). The organic phases were combined, washed with water (30 mL), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (0.05% ammonia water)-acetonitrile], acetonitrile%: 15% to 45%, the retention time of the HPLC column was 10 min). Compound **8** was obtained.

$^1$H NMR (400 MHz, $CDCl_3$): δ 8.25 (d, *J* = 2.0 Hz, 1H), 7.81 (d, *J* = 2.0 Hz, 1H), 7.26-7.23 (m, 1H), 7.21-7.10 (m, 3H), 7.03-6.99 (m, 1H), 4.12-4.03 (m, 1H), 3.84 (d, *J*= 14.8 Hz, 1H), 3.80-3.72 (m, 1H), 3.65 (br d, *J* = 14.8 Hz, 1H), 3.50 (td, *J* = 6.0, 13.6 Hz, 1H), 3.00-2.88 (m, 3H), 2.81-2.73 (m, 1H), 2.69 (dd, *J* = 4.4, 12.4 Hz, 1H), 2.61 (dd, *J* = 9.2, 12.4 Hz, 1H), 1.58-1.52 (m, 2H), 1.43-1.37 (m, 2H). MS (ESI) m/z: 409.3 $[M+1]^+$.

**Embodiment 9: Preparation of compound 9**

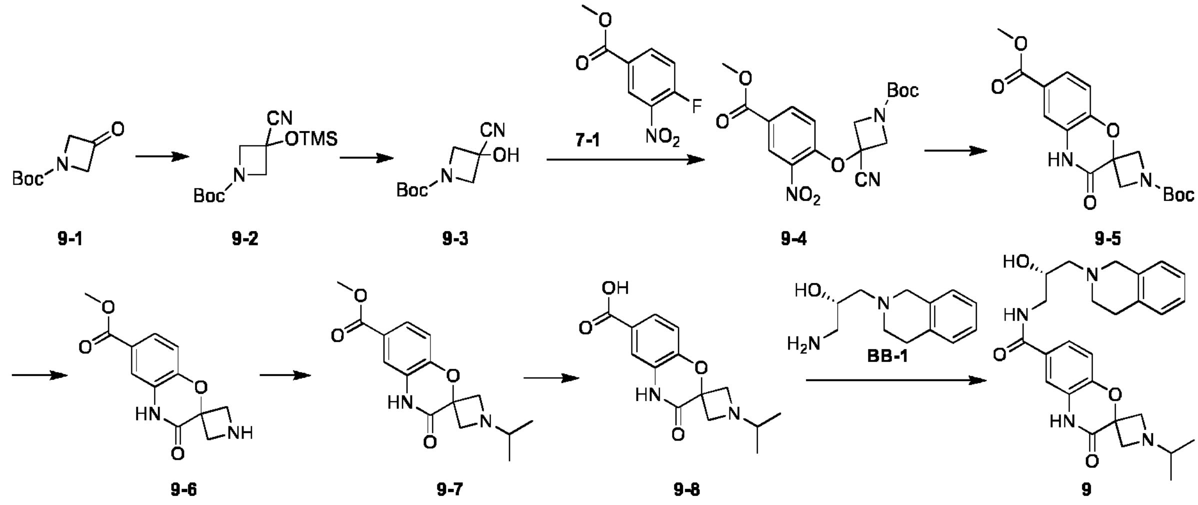


**Step 1: Synthesis of compound 9-2**

To a solution of compound **9-1** (2 g, 11.68 mmol, 1 *eq*) and trimethylsilyl cyanide (1.62 g, 16.36 mmol, 2.05 mL, 1.4 *eq*) in tetrahydrofuran (30 mL) was added zinc iodide (745.82 mg, 2.34 mmol, 0.2 *eq*) at 25°C, and the reaction mixture was stirred continuously for 12 hours. The reaction mixture was concentrated under reduced pressure. The crude product was diluted with ethyl acetate (25 mL), then washed with saturated sodium bicarbonate solution (15 mL) and saturated brine (15 mL × 2) respectively, dried over anhydrous sodium sulfate, filtered, and the filtrate was concentrated under reduced pressure to obtain a crude product of compound **9-2.**

$^1$H NMR (400 MHz, $CDCl_3$): δ 4.70 (s, 4H), 1.49 (s, 9H), 0.32-0.11 (m, 9H).

**Step 2: Synthesis of compound 9-3**

To a solution of compound **9-2** (1 g, 3.70 mmol, 1 *eq*) in ethyl acetate (10 mL) was added dilute hydrochloric acid (0.5 M, 11.09 mL, 1.5 *eq*) at 0°C, and the reaction mixture was stirred for 11 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **9-3.**

$^1$H NMR (400 MHz, $CDCl_3$): δ 4.38 (dd, *J* = 1.0, 9.6 Hz, 2H), 4.08 (dd, *J* = 1.0, 9.8 Hz, 2H), 1.46 (s, 9H).

**Step 3: Synthesis of compound 9-4**

To a solution of compound **9-3** (0.1 g, 504.49 μmol, 1 *eq*) in *N,N*-dimethylformamide (2 mL) was added sodium hydride (30.27 mg, 756.74 μmol, purity: 60%, 1.5 *eq*) at 0°C, and the reaction mixture was stirred for 30 min, then compound **7-1** (100.46 mg, 504.49 μmol, 1 *eq)* was added thereto, and the reaction mixture was stirred at 25°C for 12 hours. The reaction mixture was added with saturated ammonium chloride solution (10 mL) to quench, and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 5:1 to 1:1) to obtain compound **9-4.**

**Step 4: Synthesis of compound 9-5**

To a solution of compound **9-4** (0.05 g, 132.50 μmol, 1 *eq*) and ammonium chloride (35.44 mg, 662.52 μmol, 5 *eq*) in ethanol (2 mL) and water (2 mL) was added iron powder (37.00 mg, 662.52 μmol, 5 *eq*) at 75°C, and the reaction mixture was stirred continuously for 2 hours. The reaction mixture was filtered, then the filtrate was concentrated under reduced pressure, and the crude product was purified by silica gel column chromatography (petroleum ether: ethyl acetate = 3:1 to 1:1) to obtain compound **9-5.**

**Step 5: Synthesis of compound 9-6**

To a solution of compound **9-5** (0.040 g, 114.83 μmol, 1 *eq*) in ethyl acetate (2 mL) was added hydrochloric acid/ethyl acetate (4 M, 4 mL, 138.55 *eq*) at 25°C, and the reaction mixture was stirred for 2 hours. The reaction mixture

was concentrated under reduced pressure to obtain a crude product of compound **9-6** hydrochloride.

**Step 6: Synthesis of compound 9-7**

To a solution of compound **9-6** (0.035 g, crude hydrochloride) and acetone (10.71 mg, 184.41 μmol, 13.56 μL, 1.5 *eq*) in methanol (5 mL) and acetic acid (0.5 mL) was added sodium triacetoxyborohydride (52.11 mg, 245.88 μmol, 2.0 *eq*) at 25°C, and the reaction mixture was stirred continuously for 2 hours. The reaction mixture was added with saturated sodium bicarbonate solution to adjust the pH to 9, and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 3:1 to 0:1) to obtain compound **9-7.**

**Step 7: Synthesis of compound 9-8**

To a solution of compound **9-7** (0.030 g, 103.34 μmol, 1 *eq*) in methanol (5 mL), water (1 mL) and tetrahydrofuran (1.5 mL) was added lithium hydroxide monohydrate (21.68 mg, 516.68 μmol, 5 *eq*) at 25°C, and the reaction mixture was stirred for 1 hour, then heated to 45°C, and stirred for 12 hours. The reaction mixture was diluted with water (10 mL), added with dilute hydrochloric acid (1 M) to adjust the pH to 6, and extracted with ethyl acetate (10 mL×3), showing that the product was in the aqueous phase. The aqueous phase was concentrated under reduced pressure and evaporated to dryness by rotary evaporation to obtain a crude product of compound **9-8.**

**Step 8: Synthesis of compound 9**

To a solution of compound **9-8** (0.028 g, 103.30 μmol, 1 *eq*) in *N,N*-dimethylformamide (2 mL) was added HATU (41.24 mg, 108.46 μmol, 1.05 *eq*) and diisopropylethylamine (16.02 mg, 123.96 μmol, 21.59 μL, 1.2 *eq*) respectively at 25°C, and the reaction mixture was stirred for 10 min, then compound **BB-1** (21.31 mg, 103.30 μmol, 1 *eq*) was added thereto, and the reaction mixture was stirred continuously for 30 min. The reaction mixture was diluted with water (10 mL), and extracted with ethyl acetate/tetrahydrofuran (4:1, V/V, 10 mL × 3). The organic phases were combined, washed with saturated brine (10 mL × 3), dried over anhydrous sodium sulfate, filtered, and the filtrate was concentrated under reduced pressure. The crude product was purified by preparative HPLC (chromatographic column: Waters Xbridge C18 150×50 mm×10 μm; mobile phase: [water (10 mM ammonium bicarbonate)-acetonitrile]; acetonitrile%: 25% to 55%, the retention time of the HPLC column was 10 min) to obtain compound **9.**

$^1$H NMR (400 MHz, $CD_3OD$): δ 7.40-7.38 (m, 1H), 7.36 (s, 1H), 7.17-7.09 (m, 3H), 7.06-7.00 (m, 1H), 7.01-6.97 (m, 1H), 4.17-4.05 (m, 1H), 3.82-3.68 (m, 4H), 3.55-3.40 (m, 4H), 2.99-2.85 (m, 4H), 2.78-2.65 (m, 2H), 2.60-2.48 (m, 1H), 0.98 (d, *J* = 6.0 Hz, 6H). MS (ESI) m/z: 465.4 $[M+H]^+$.

**Embodiment 10: Preparation of compound 10**

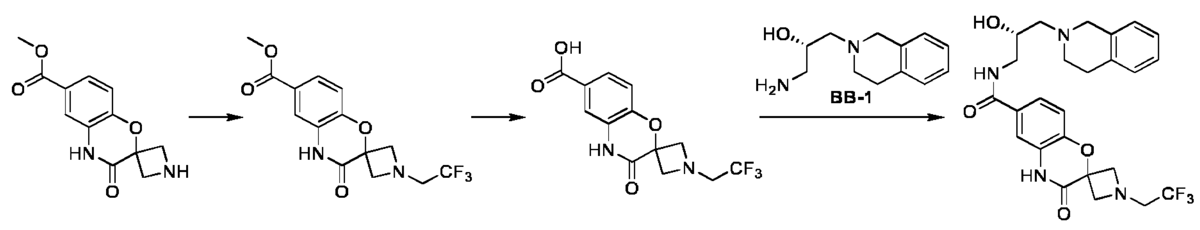

9-6 10-1 10-2 10

**Step 1: Synthesis of compound 10-1:**

To a solution of compound **9-6** (0.120 g, 483.41 μmol, 1 *eq,* crude hydrochloride) in *N,N*-dimethylformamide (5 mL) was added *N,N*-diisopropylethylamine (374.86 mg, 2.90 mmol, 505.20 μL, 6 *eq*) and trifluoroethyl trifluoromethanesulfonate (145.86 mg, 628.44 μmol, 1.3 *eq*) at 25°C, and the reaction mixture was stirred continuously for 2 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by column chromatography (petroleum ether: ethyl acetate = 3:1 to 1:1) to obtain compound **10-1.**

**Step 2: Synthesis of compound 10-2**

To a solution of compound **10-1** (110 mg, 333.07 μmol, 1 *eq*) in methanol (5 mL), water (1 mL) and tetrahydrofuran (1.5 mL) was added lithium hydroxide monohydrate (69.88 mg, 1.67 mmol, 5 *eq*) at 25°C, and the reaction mixture was heated to 50°C and stirred for 12 hours. The reaction mixture was diluted with water (10 mL), added with dilute hydrochloric acid (1 M) to adjust the pH to 6, and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **10-2.**

**Step 3: Synthesis of compound 10**

To a solution of compound **10-2** (0.100 g, 316.22 μmol, 1 *eq*) in *N,N*-dimethylformamide (5 mL) was added 2-(7-azabenzotriazol-1-yl)-*N,N,N',N'*-tetramethyluronium hexafluorophosphate (126.25 mg, 332.03 μmol, 1.05 *eq*) and DIEA (49.04 mg, 379.47 μmol, 66.09 μL, 1.2 *eq*) respectively at 25°C, and the reaction mixture was stirred for 10 min, then compound **BB-1** (65.23 mg, 316.22 μmol, 1 *eq*) was added thereto, and the reaction mixture was stirred continuously for 30 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (0.05% ammonia water v/v)-acetonitrile]; acetonitrile%: 28% to 58%, the retention time of the HPLC column was 10 min), then purified by column chromatography (petroleum ether: ethyl acetate = 1.5:1, then dichloromethane: methanol = 10:1), and finally purified by preparative HPLC (chromatographic column: Phenomenex Gemini NX-C18 (75×30 mm×3 μm); mobile phase: [water (10 mM ammonium bicarbonate)-acetonitrile]; acetonitrile%: 24% to 54%, the retention time of the HPLC column was 8 min) to obtain compound **10.**

$^1$H NMR (400 MHz, DMSO-$d_6$): δ 10.96 (br s, 1H), 8.56-8.31 (m, 1H), 7.53-7.27 (m, 2H), 7.17-6.93 (m, 5H), 4.82 (d, *J* = 4.4 Hz, 1H), 3.96-3.75 (m, 3H), 3.67-3.53 (m, 4H), 3.32-3.27 (m, 5H), 3.27-3.17 (m, 1H), 2.85-2.63 (m, 4H). MS (ESI) m/z: 505.4 [M+1]$^+$.

**Embodiment 11: Preparation of compound 11**

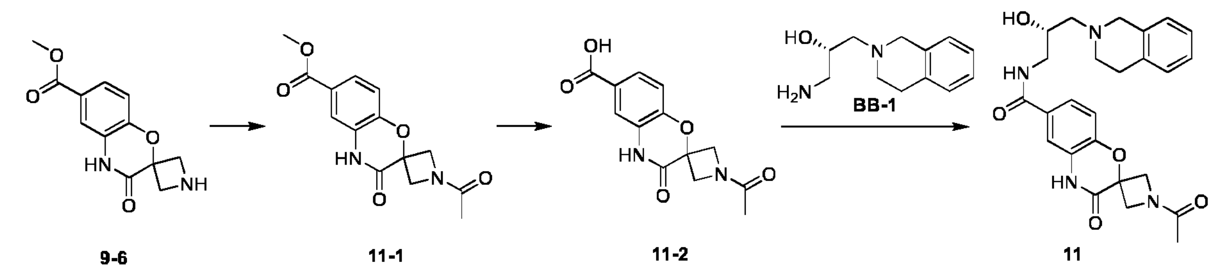

9-6 11-1 11-2 BB-1 11

**Step 1: Synthesis of compound 11-1**

To a solution of compound **9-6** (150 mg, crude hydrochloride) in dichloromethane (5.00 mL) was added triethylamine (91.72 mg, 906.40 μmol, 126.16 μL, 1.5 *eq*) and acetic anhydride (74.03 mg, 725.12 μmol, 67.91 μL, 1.2 *eq*) at 25°C, and the reaction mixture was stirred at 25°C for 1 hour. The reaction mixture was diluted with water (10 mL), and extracted with dichloromethane (20 mL × 3). The organic phases were combined, washed with saturated brine (10 mL × 2), dried over anhydrous sodium sulfate, filtered, and the filtrate was concentrated under reduced pressure. The crude product was purified by column chromatography (petroleum ether: ethyl acetate = 1:0 to 0:1) to obtain compound **11-1.** MS (ESI) m/z: 291.1 [M+1]$^+$.

**Step 2: Synthesis of compound 11-2**

To a solution of compound **11-1** (139 mg, 478.86 μmol, 1 *eq*) in tetrahydrofuran (2 mL), methanol (2 mL) and water (2 mL) was added lithium hydroxide monohydrate (60.28 mg, 1.44 mmol, 3 *eq*) at 25°C, and the reaction mixture was stirred continuously at 25°C for 48 hours. The reaction mixture was added with dilute hydrochloric acid (1 mol/L) to adjust the pH to 5, and extracted with ethyl acetate (20 mL × 3). The organic phases were combined, washed with

saturated brine (10 mL × 2), dried over anhydrous sodium sulfate, filtered, and the filtrate was concentrated under reduced pressure to obtain a crude product of compound **11-2.** MS (ESI) m/z: 277.0 $[M+1]^+$.

**Step 3: Synthesis of compound 11**

To a solution of compound **11-2** (112 mg, 405.44 μmol, 1 *eq*) in *N,N*-dimethylformamide (3.00 mL) was added 2-(7-azabenzotriazol-1-yl)-*N,N,N',N'*-tetramethyluronium hexafluorophosphate (200.41 mg, 527.07 μmol, 1.3 *eq)* and *N,N*-diisopropylethylamine (78.60 mg, 608.16 μmol, 105.93 μL, 1.5 *eq*) respectively at 25°C, and the reaction mixture was stirred for 0.5 hours, then compound **BB-1** (83.64 mg, 405.44 μmol, 1 *eq*) was added thereto, and the reaction mixture was stirred continuously for 0.5 hours. The reaction mixture was diluted with water (10 mL), and extracted with ethyl acetate (20 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (0.05% ammonia water)-acetonitrile]; acetonitrile%: 13% to 43%, the retention time of the HPLC column was 10 min) to obtain compound **11.**

$^1$H NMR (400 MHz, DMSO-$d_6$): δ 8.47-8.33 (s, 1H), 7.44-7.37 (m, 2H), 7.15-7.06 (m, 4H), 7.06-6.98 (m, 2H), 4.85-4.78 (m, 1H), 4.60-4.53 (m, 1H), 4.31-4.22 (m, 2H), 4.04-3.76 (m, 2H), 3.64-3.60 (m, 2H), 3.25-3.16 (m, 2H), 2.83-2.68 (m, 6H), 1.82-1.81 (s, 3H). MS (ESI) m/z: 465.3 $[M+1]^+$.

**Embodiment 12: Preparation of compound 12**

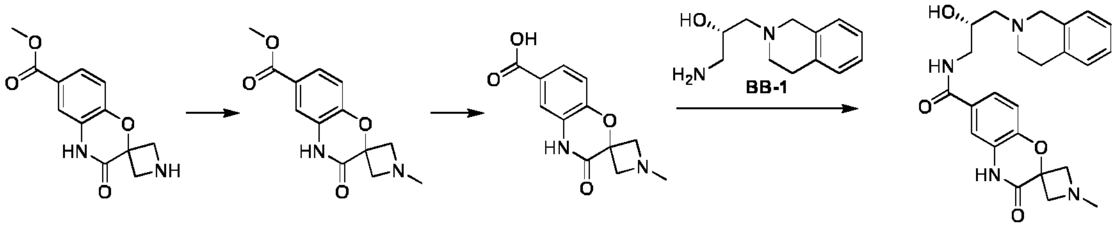


9-6 12-1 12-2 12

**Step 1: Synthesis of compound 12-1:**

To a solution of compound **9-6** (0.070 g, 245.88 μmol, 1 *eq,* crude hydrochloride) and formaldehyde aqueous solution (59.87 mg, 737.63 μmol, 54.92 μL, purity: 37%, 3 *eq*) in dichloromethane (5 mL) was added sodium triacetoxyborohydride (312.67 mg, 1.48 mmol, 6 *eq*) at 25°C, and the reaction mixture was stirred continuously for 2 hours. The reaction mixture was added with saturated sodium bicarbonate solution (10 mL) to quench, and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **12-1.**

**Step 2: Synthesis of compound 12-2:**

To a solution of compound **12-1** (0.095 g, 362.23 μmol, 1 *eq*) in methanol (2 mL), tetrahydrofuran (2 mL) and water (1 mL) was added lithium hydroxide monohydrate (76.00 mg, 1.81 mmol, 5 *eq*) at 25°C, and the reaction mixture was heated to 45°C and stirred for 12 hours. The reaction mixture was added with dilute hydrochloric acid to adjust the pH to 3, extracted with ethyl acetate (10 mL×2), and the aqueous phase was concentrated under reduced pressure to obtain a crude product of compound **12-2.**

**Step 3: Synthesis of compound 12:**

To a solution of compound **12-2** (0.020 g, 80.57 μmol, 1 *eq*) in *N,N*-dimethylformamide (2 mL) was added 2-(7-azabenzotriazol-1-yl)-*N,N,N',N'*-tetramethyluronium hexafluorophosphate (45.95 mg, 120.85 μmol, 1.5 *eq*) and *N,N*-diisopropylethylamine (20.83 mg, 161.14 μmol, 28.07 μL, 2.0 *eq*) respectively at 25°C, and the reaction mixture was stirred for 10 min, then compound **BB-1** (21.61 mg, 104.74 μmol, 1.3 *eq*) and 4-dimethylaminopyridine (1.97 mg, 16.11 μmol, 0.2 *eq*) were added thereto, and the reaction mixture was stirred for 12 hours. The reaction mixture was diluted

with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile%: 15% to 45%, the retention time of the HPLC column was 10 min) to obtain compound **12.**

$^1H$ NMR (400 MHz, $CD_3OD$): δ 7.41-7.33 (m, 2H), 7.15-7.03 (m, 4H), 7.01-6.90 (m, 1H), 4.12-4.05 (m, 1H), 3.81-3.70 (m, 4H), 3.58-3.40 (m, 4H), 2.93-2.90 (m, 2H), 2.88-2.83 (m, 2H), 2.70-2.62 (m, 2H), 2.43 (s, 3H). MS (ESI) m/z: 437.5 $[M+1]^+$.

**Embodiment 13: Preparation of compound 13**

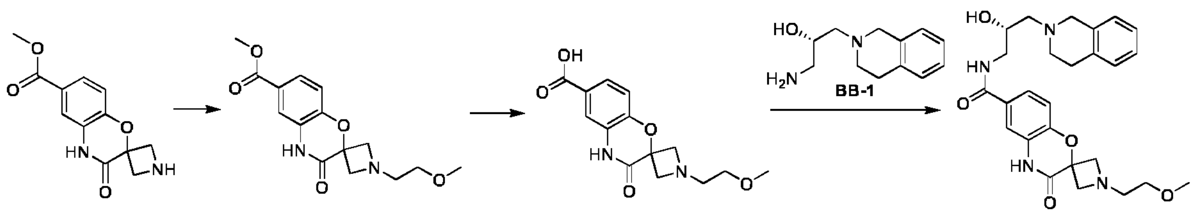


9-6 13-1 13-2 13

**Step 1: Synthesis of compound 13-1:**

To a solution of compound **9-6** (0.170 g, crude hydrochloride) in *N,N*-dimethylformamide (5 mL) was added *N,N*-diisopropylethylamine (531.05 mg, 4.11 mmol, 715.70 μL, 6 *eq*) and 2-bromoethyl methyl ether (123.74 mg, 890.29 μmol, 83.61 μL, 1.3 *eq*) at 25°C, and the reaction mixture was stirred continuously for 1 hour. The reaction mixture was diluted with water (25 mL), and extracted with ethyl acetate (20 mL × 3). The organic phases were combined, washed with saturated brine (20 mL × 2), dried over anhydrous sodium sulfate, filtered, and the filtrate was concentrated under reduced pressure to obtain a crude product of compound **13-1.**

$^1H$ NMR (400 MHz, DMSO-$d_6$): δ 10.94 (br s, 1H), 7.56 (dd, *J* = 2.0, 8.4 Hz, 1H), 7.50 (d, *J* = 1.6 Hz, 1H), 7.20-7.13 (m, 1H), 3.95 (d, *J* = 9.8Hz, 2H), 3.85-3.79 (m, 3H), 3.62 (d, *J* = 9.8 Hz, 2H), 3.55 (d, *J* = 9.8 Hz, 2H), 3.33-3.26 (m, 3H), 2.61 (t, *J* = 5.6 Hz, 2H).

**Step 2: Synthesis of compound 13-2:**

To a solution of compound **13-1** (0.250 g, 816.16 μmol, 1 *eq*) in methanol (4 mL), tetrahydrofuran (4 mL) and water (2 mL) was added lithium hydroxide monohydrate (171.24 mg, 4.08 mmol, 5 *eq*) at 25°C, and the reaction mixture was stirred for 1 hour, then heated to 40°C, and stirred for 12 hours. The reaction mixture was added with dilute hydrochloric acid (1 M) to adjust the pH to 5, extracted with ethyl acetate (15 mL × 3), and the aqueous phase was concentrated under reduced pressure. The crude product was purified by column chromatography (dichloromethane: methanol =10:1), and then purified by preparative HPLC (chromatographic column: Phenomenex Synergi C18 150×25 mm×10 μm; mobile phase: [water (0.1% trifluoroacetic acid)-acetonitrile]; acetonitrile%: 1% to 25%, the retention time of the HPLC column was 40 min) to obtain compound **13-2.**

**Step 3: Synthesis of compound 13:**

To a solution of compound **13-2** (0.030 g, 102.64 μmol, 1 *eq*) in *N,N*-dimethylformamide (2 mL) was added 2-(7-azabenzotriazol-1-yl)-*N,N,N',N'*-tetramethyluronium hexafluorophosphate (46.83 mg, 123.17 μmol, 1.2 *eq*) and *N,N*-diisopropylethylamine (19.90 mg, 153.96 μmol, 26.82 μL, 1.5 *eq*) respectively at 25°C, and the reaction mixture was stirred for 10 min, then compound **BB-1** (21.17 mg, 102.64 μmol, 1 *eq*) was added thereto, and the reaction mixture was stirred for 30 min. The reaction mixture was diluted with water (15 mL), and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile%: 14% to 47%, the retention time of the HPLC column was 9 min) to obtain compound **13.**

$^1H$ NMR (400 MHz, $CDCl_3$): δ 8.06 (br s, 1H), 7.64 (s, 1H), 7.42 (dd, *J* = 1.8, 8.4 Hz, 1H), 7.25-7.12 (m, 3H), 7.06 (d, *J* = 7.2 Hz, 1H), 6.94 (d, *J* = 8.4 Hz, 1H), 4.46-4.34 (m, 1H), 4.33-4.17 (m, 2H), 3.95-3.80(m, 2H), 3.71-3.53 (m,

4H), 3.48-3.43 (m, 2H), 3.37 (br s, 2H), 3.31 (s, 3H), 3.22-3.15 (m, 2H), 3.14-3.05 (m, 4H), 2.88-2.81 (m, 2H). MS (ESI) m/z: 481.4 [M+1]$^+$.

**Embodiment 14: Preparation of compound 14**

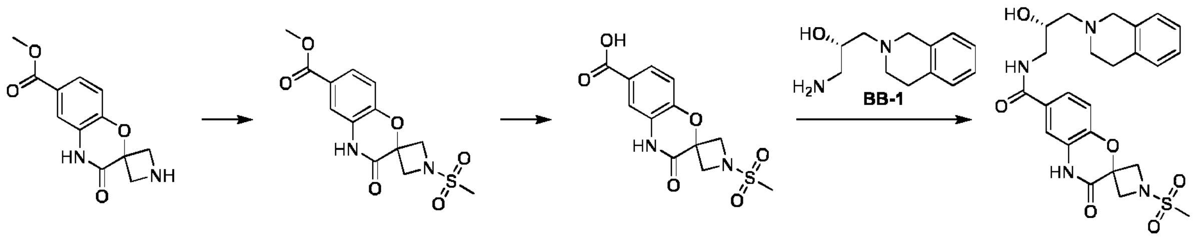


9-6 14-1 14-2 14

**Step 1: Synthesis of compound 14-1:**

To a solution of compound **9-6** (100 mg, crude hydrochloride) and methanesulfonic anhydride (70.17 mg, 402.85 μmol, 1 *eq*) in dichloromethane (5 mL) was added DMAP (24.61 mg, 201.42 μmol, 0.5 *eq*) and triethylamine (61.15 mg, 604.27 μmol, 84.11 μL, 1.5 *eq*), and the reaction mixture was stirred at 25°C for 1 hour. The reaction mixture was diluted with water (10 mL), and extracted with dichloromethane (20 mL × 3). The organic phases were combined, washed with saturated brine (10mL × 2), dried over anhydrous sodium sulfate, filtered, and the filtrate was concentrated under reduced pressure to obtain a crude product of compound **14-1.**

**Step 2: Synthesis of compound 14-2:**

To a solution of compound **14-1** (0.079 g, 242.09 μmol, 1 *eq*) in methanol (6 mL), tetrahydrofuran (6 mL) and water (3 mL) was added lithium hydroxide monohydrate (101.59 mg, 2.42 mmol, 10 *eq*) at 25°C, and the reaction mixture was stirred at 45°C for 8 hours. The reaction mixture was added with dilute hydrochloric acid (1 M) to adjust the pH to 5, and extracted with ethyl acetate (10 mL × 2). The organic phases were combined, washed with saturated brine (10 mL × 2), dried over anhydrous sodium sulfate, filtered, and the filtrate was concentrated under reduced pressure to obtain a crude product of compound **14-2.**

**Step 3: Synthesis of compound 14:**

To a solution of compound **14-2** (0.050 g, 160.10 μmol, 1 *eq*) in *N,N*-dimethylformamide (5 mL) was added HATU (73.05 mg, 192.12 μmol, 1.2 *eq*) and DIEA (31.04 mg, 240.16 μmol, 41.83 μL, 1.5 *eq*) respectively at 25°C, and the reaction mixture was stirred for 10 min, then compound **BB-1** (33.03 mg, 160.10 μmol, 1 *eq*) was added thereto, and the reaction mixture was stirred for 1 hour. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile%: 15% to 49%, the retention time of the HPLC column was 10 min) to obtain compound **14.**

$^1$H NMR(400 MHz, DMSO-$d_6$): δ 11.16 (brs, 1H), 8.44 (t, *J* = 5.4 Hz, 1H), 7.47-7.37 (m, 2H), 7.13-6.99 (m, 5H), 4.86-4.77 (m, 1H), 4.40 (d, *J* = 10.4 Hz, 2H), 4.04 (d, *J* = 10.4 Hz, 2H), 3.96-3.84 (m, 1H), 3.62 (d, *J* = 4.4 Hz, 2H), 3.47-3.39 (m, 1H), 3.32-3.27 (m, 2H), 3.26-3.16 (m, 1H), 3.10 (s, 3H), 2.85-2.77 (m, 2H), 2.75-2.64 (m, 2H). MS (ESI) m/z: 501.4 [M+1]$^+$.

**Embodiment 15: Preparation of compound 15**

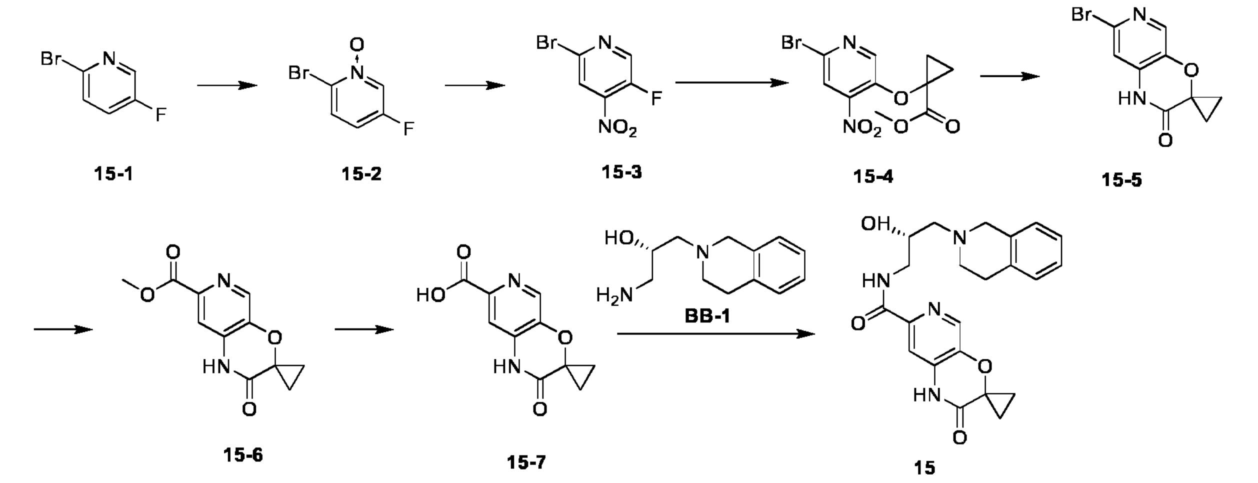

15-1 15-2 15-3 15-4 15-5

BB-1

15-6 15-7 15

**Step 1: Synthesis of compound 15-2**

To a solution of compound **15-1** (2 g, 11.36 mmol, 1 *eq*) in chloroform (22 mL) was added urea hydrogen peroxide (1.07 g, 11.36 mmol, 1 *eq*) and trifluoroacetic anhydride (4.77 g, 22.73 mmol, 3.16 mL, 2 *eq*) at 0°C, and the reaction mixture was stirred at 20°C for 15 hours. The reaction mixture was added with saturated sodium sulfite solution (10 mL) to quench in an ice bath, then added with saturated sodium bicarbonate solution (10 mL), and extracted with chloroform. The organic phases were combined, dried over anhydrous sodium sulfate, filtered, and the filtrate was concentrated under reduced pressure to obtain a crude product of compound **15-2.**

$^1$H NMR (400 MHz, $CDCl_3$): δ 8.36-8.33 (m, 1H), 7.70-7.60 (m, 1H), 7.01-6.96 (m, 1H).

**Step 2: Synthesis of compound 15-3**

To a solution of compound **15-2** (2.67 g, 13.91 mmol, 1 *eq*) in concentrated sulfuric acid (20 mL) was added fuming nitric acid (90% to 97.5%, 3.5 g, 55.54 mmol, 5.8 mL, 3.99 *eq*) at 0°C, and the reaction mixture was heated to 120°C and stirred continuously for 8 hours. The reaction mixture was poured into ice water, and extracted with ethyl acetate (20 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 1:0 to 5:1) to obtain compound **15-3.**

$^1$H NMR (400 MHz, $CDCl_3$): δ 9.17 (d, *J* = 6.8 Hz, 1H), 8.79 (d, *J* = 8.8 Hz, 1H).

**Step 3: Synthesis of compound 15-4**

To a solution of compound **15-3** (0.112 g, 506.82 μmol, 1 *eq*) and methyl 1-hydroxycyclopropane-1-carboxylate (58.85 mg, 506.82 μmol, 1 *eq*) in tetrahydrofuran (5 mL) was added sodium hydride (40.55 mg, 1.01 mmol, purity: 60%, 2.0 *eq*) in an ice bath at 0°C, and the reaction mixture was warmed to 25°C and stirred continuously for 15 min. The reaction mixture was added with saturated ammonium chloride solution (10 mL) to quench, and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **15-4.**

**Step 4: Synthesis of compound 15-5**

To a solution of compound **15-4** (0.490 g, 1.55 mmol, 1 *eq*) and ammonium chloride (413.30 mg, 7.73 mmol, 5 *eq*) in ethanol (10 mL) and water (2 mL) was added iron powder (431.48 mg, 7.73 mmol, 5 *eq*) at 70°C, and the reaction mixture was stirred continuously for 30 min. The reaction mixture was filtered, and the filtrate was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **15-5.**

$^1$H NMR (400 MHz, $CDCl_3$): δ 8.78 (br s, 1H), 7.95 (s, 1H), 7.09 (s, 1H), 1.56-1.49 (m, 2H), 1.38-1.32 (m, 2H).

**Step 5: Synthesis of compound 15-6**

A solution of compound **15-5** (0.260 g, 1.02 mmol, 1 *eq*), triethylamine (206.29 mg, 2.04 mmol, 283.76 μL, 2.0 *eq*) and 1,1'-bis(diphenylphosphino)ferrocene palladium(II) dichloromethane complex (166.49 mg, 203.87 μmol, 0.2 *eq*) in methanol (5 mL) was stirred and reacted at 80°C for 12 hours under carbon monoxide atmosphere. The reaction mixture was filtered, and the filtrate was concentrated under reduced pressure and evaporated to dryness by rotary evaporation. The crude product was purified by silica gel column chromatography (petroleum ether: ethyl acetate = 4:1 to 1:1) to obtain compound **15-6.**

**Step 6: Synthesis of compound 15-7**

To a solution of compound **15-6** (0.050 g, 213.49 μmol, 1 *eq*) in tetrahydrofuran (2 mL) and water (0.5 mL) was added lithium hydroxide monohydrate (26.88 mg, 640.46 μmol, *3 eq*) at 25°C, and the reaction mixture was stirred continuously for 1 hour. The reaction mixture was diluted with water (10 mL), and extracted with ethyl acetate (10 mL×3). The aqueous phase was added with dilute hydrochloric acid (1 M) to adjust the pH to 5, concentrated under reduced pressure, added with water (2 mL), stirred for 2 min, filtered, and the filter cake was dried under reduced pressure to obtain compound **15-7.**

**Step 7: Synthesis of compound 15**

To a solution of compound **15-7** (0.010 g, 45.42 μmol, 1 *eq)* in *N,N*-dimethylformamide (1 mL) was added HATU (20.72 mg, 54.50 μmol, 1.2 *eq*) and DIEA (8.80 mg, 68.13 μmol, 11.87 μL, 1.5 *eq*) respectively at 25°C, and the reaction mixture was stirred for 10 min, then compound **BB-1** (9.37 mg, 45.42 μmol, 1 *eq*) was added thereto, and the reaction mixture was stirred for 30 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: W Phenomenex Gemini-NX C18 75×30 mm×3 μm; mobile phase: [water (10 mM ammonium bicarbonate)-acetonitrile]; acetonitrile: 22% to 52%, the retention time of the HPLC column was 8 min) to obtain compound **15.**

$^1H$ NMR (400 MHz, $CDCl_3$): δ 9.81 (br s, 1H), 8.47 (br t, *J* = 6.1 Hz, 1H), 7.99 (s, 2H), 7.26-7.14 (m, 3H), 7.10-7.03 (m, 1H), 4.57-4.44(m, 1H), 4.38-4.17 (m, 2H), 3.85-3.64 (m, 1H), 3.55-3.45 (m, 1H), 3.44-3.33 (m, 2H), 3.29-3.02 (m, 4H), 1.55-1.31 (m, 4H); MS (ESI) m/z: 409.3 $[M+H]^+$.

**Embodiment 16: Preparation of compound 16**

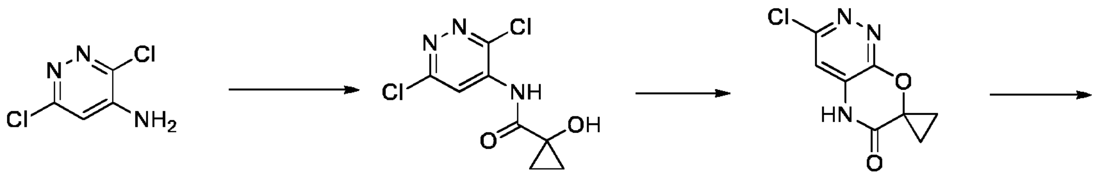

**16-1** **16-2** **16-3**

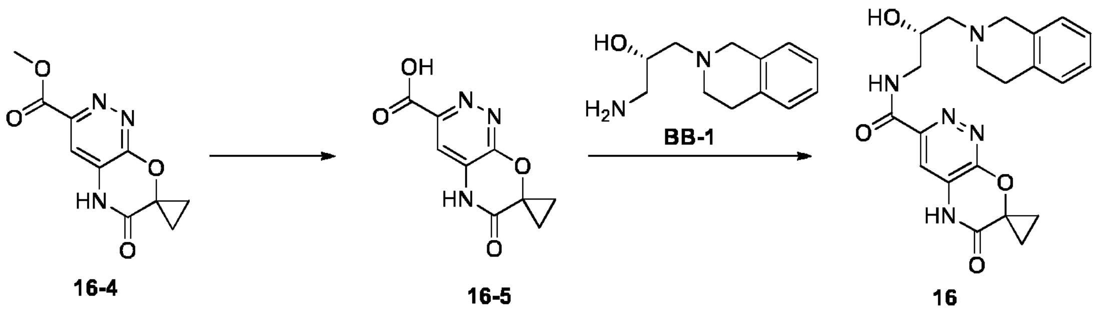

**BB-1**

**16-4** **16-5** **16** 

**Step 1: Synthesis of compound 16-2**

To a solution of compound methyl 1-hydroxycyclopropane-1-carboxylate (3.00 g, 29.39 mmol, 1 *eq*) in dichloromethane (20 mL) and *N,N*-dimethylformamide (10.74 mg, 146.93 μmol, 11.30 μL, 0.005 *eq*) was added thionyl chloride (10 mL) at room temperature, and the reaction mixture was stirred at 80°C for 1 hour. After the reaction was completed, the reaction mixture was concentrated under reduced pressure to obtain acyl chloride. To a solution of compound **16-1** (2.86 g, 17.42 mmol, 0.6 *eq*) and diisopropylethylamine (18.76 g, 145.19 mmol, 25.29 mL, 5 *eq*) in dichloromethane (35 mL) was added the above acyl chloride, and the reaction mixture was stirred at 20°C for 0.5 hours. The reaction mixture was added with water (100 mL), and extracted with dichloromethane (100 mL × 2). The organic phases were combined, washed with water (100 mL × 2) and saturated brine (100 mL), dried over anhydrous sodium sulfate, filtered, and the filtrate was concentrated under reduced pressure. The crude product was purified by silica gel column chromatography (petroleum ether: ethyl acetate = 4:1) to obtain compound **16-2.**

**Step 2: Synthesis of compound 16-3**

To a solution of compound **16-2** (2.00 g, 8.06 mmol, 1 *eq)* in *N,N*-dimethylformamide (10.00 mL) was added sodium hydroxide (644.94 mg, 16.12 mmol, 2 *eq*), and the reaction mixture was stirred at 85°C for 2 hours, then added with water (50 mL), and extracted with ethyl acetate (50 mL × 3). The organic phases were combined, washed with water (50 mL × 3) and saturated brine (50 mL), dried over anhydrous sodium sulfate, filtered, and the filtrate was concentrated under reduced pressure to obtain a crude product. The crude product was stirred with (petroleum ether: ethyl acetate = 1:2) at 25°C for 2 min, and filtered to obtain compound **16-3.** MS (ESI) m/z: 212.0 $[M+H]^+$.

**Step 3: Synthesis of compound 16-4**

To a mixed solution of compound **16-3** (620.0 mg, 2.93 mmol, 1 *eq*) in *N,N*-dimethylformamide (5 mL), methanol (5 mL) and triethylamine (0.5 mL) was added palladium acetate (131.56 mg, 586.00 μmol, 0.2 *eq*) and 1,1'-bis(diphenylphosphino)ferrocene (324.86 mg, 586.00 μmol, 0.2 *eq*), and the reaction flask was replaced with carbon monoxide three times, heated to 80°C, and stirred for 16 hours under carbon monoxide (50 psi) atmosphere. The reaction mixture was filtered through diatomite, and the filtrate was concentrated under reduced pressure, added with water (50 mL), and extracted with ethyl acetate (50 mL × 3). The organic phases were combined, washed with water (50 mL), dried over anhydrous sodium sulfate, filtered, and the filtrate was concentrated under reduced pressure. The crude product was slurried with (petroleum ether: ethyl acetate = 1:1) at 20°C for 5 min, and then purified by preparative HPLC (chromatographic column: Phenomenex Luna C18 75×30 mm×3 μm; mobile phase: [water (0.05% hydrochloric acid)-acetonitrile], acetonitrile: 4% to 24%, the retention time of the HPLC column was 7 min) to obtain compound **16-4.**

$^1H$ NMR (400 MHz, DMSO-$d_6$): δ 11.67 (s, 1H), 7.53 (s, 1H), 3.92 (s, 3H), 1.50-1.39 (m, 4H). MS (ESI) m/z: 236.0 $[M+H]^+$.

**Step 4: Synthesis of compound 16-5**

To a mixed solution of compound **16-4** (45.0 mg, 191.33 μmol, 1 *eq*) in tetrahydrofuran (1 mL), methanol (1 mL) and water (0.5 mL) was added lithium hydroxide monohydrate (16.06 mg, 382.66 μmol, 2 *eq*) at room temperature, and the reaction mixture was stirred at 25°C for 1 hour. The reaction mixture was concentrated under reduced pressure to remove the organic solvent. The aqueous phase was extracted with ethyl acetate (10 mL), then added with hydrochloric acid to adjust the pH to 6, concentrated under reduced pressure, added with water (1 mL), stirred for 5 min, and filtered to obtain a crude product of compound **16-5,** which was directly used in the next reaction step.

**Step 5: Synthesis of compound 16**

To a solution of compound **16-5** (42.0 mg, 189.90 μmol, 1 *eq*) in *N,N*-dimethylformamide (1 mL) was added compound **BB-1** (77.37 mg, 375.08 μmol, 1 *eq*), 1-hydroxybenzotriazole (30.79 mg, 227.88 μmol, 1.2 *eq*), diisopropylethylamine (73.63 mg, 569.70 μmol, 99.23 μL, 3 *eq*) and 1-ethyl-3(3-dimethylpropylamine)carbodiimide (43.68 mg, 227.88 μmol, 1.2 *eq*) respectively at 0°C, and the reaction mixture was stirred at 25°C for 16 hours. The reaction mixture was then diluted with water (10 mL), and extracted with ethyl acetate (10 mL × 2). The organic phases were combined, washed with water (10 mL), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile], acetonitrile: 18% to 48%, the retention time of the HPLC column was 10 min) to obtain compound **16.**

$^1H$ NMR (400 MHz, $CDCl_3$): δ 8.53 (brt, *J* = 5.8 Hz, 1H), 8.08 (s, 1H), 7.19-7.09 (m, 3H), 7.04-6.99 (m, 1H),

4.19-4.11 (m, 1H), 3.90 (d, *J* = 15.2 Hz, 1H), 3.81-3.68 (m, 2H), 3.61-3.51 (m, 1H), 3.05-2.89 (m, 3H), 2.89-2.80 (m, 1H), 2.76-2.61 (m, 2H), 1.65-1.59 (m, 2H), 1.58-1.51 (m, 2H); MS (ESI) m/z: 410.4 $[M+H]^+$.

**Embodiment 17: Preparation of compounds 17 and 18**

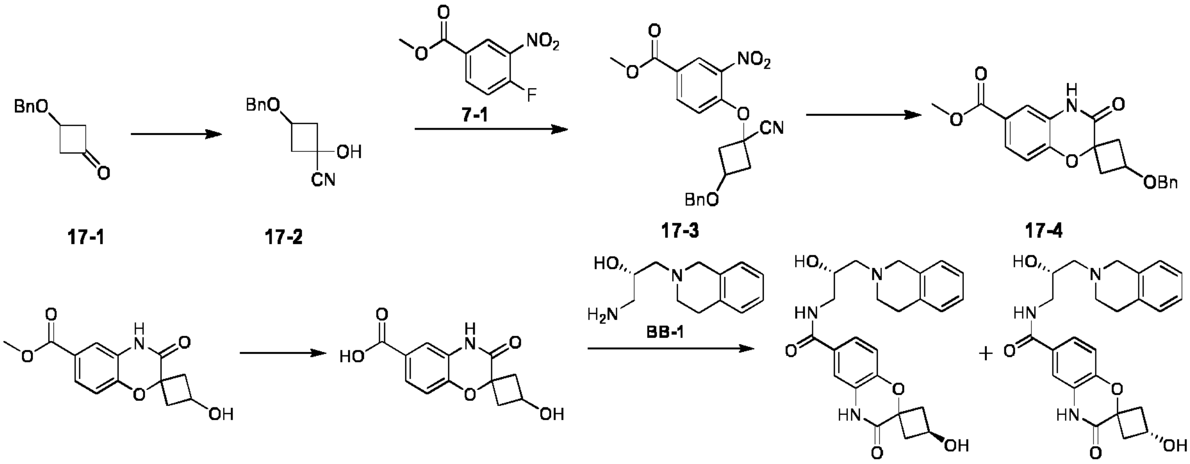


17-1 17-2 17-3 17-4 17-5 17-6 17 or 18 18 or 17

**Step 1: Synthesis of compound 17-2**

To a solution of compound **17-1** (5.00 g, 28.38 mmol, 1 *eq*) in dichloromethane (50 mL) was added trimethylsilyl cyanide (4.22 g, 42.56 mmol, 5.32 mL, 1.5 *eq*) at 0°C, then boron trifluoride diethyl etherate solution (4.03 g, 28.38 mmol, 3.50 mL, 1 *eq*) was added dropwise, and the reaction mixture was stirred continuously at 0°C for 0.5 hours. The reaction mixture was poured into saturated sodium bicarbonate aqueous solution (100 mL) to quench, then the phases were separated, and the aqueous phase was extracted with dichloromethane (100 mL). The organic phases were combined, washed with saturated brine (100 mL × 2), dried over anhydrous sodium sulfate, filtered, and the filtrate was concentrated under reduced pressure to obtain a crude product of compound **17-2,** which was directly used in the next reaction step.

$^1H$ NMR (400 MHz, $CDCl_3$): δ 7.38-7.31 (m, 6H), 4.46 (s, 2H), 4.00 (q, *J* = 7.0 Hz, 1H), 3.05-2.98 (m, 2H), 2.41-2.33 (m, 2H).

**Step 2: Synthesis of compound 17-3**

To a solution of compound **17-2** (5.24 g, 25.78 mmol, 2.1 *eq*) and compound **7-1** (2.44 g, 12.28 mmol, 1 *eq*) in tetrahydrofuran (60.00 mL) was added sodium hydride (638.37 mg, 15.96 mmol, purity: 60%, 1.3 *eq*) at -10°C, and the reaction mixture was slowly warmed to 15°C and stirred for 16 hours. The reaction mixture was poured into saturated ammonium chloride aqueous solution (100 mL) to quench, and extracted with ethyl acetate (100 mL × 3). The organic phases were combined, washed with water (100 mL), dried over anhydrous sodium sulfate, filtered, and the filtrate was concentrated under reduced pressure. The crude product was purified by silica gel column chromatography (petroleum ether: ethyl acetate = 10:1) to obtain compound **17-3.**

$^1H$ NMR (400 MHz, $CDCl_3$): δ 8.50-8.42 (m, 1H), 8.21-8.12 (m, 1H), 7.31-7.27 (m, 5H), 7.04 (d, *J* = 8.8 Hz, 1H), 4.42 (s, 2H), 3.88 (s, 3H), 3.23-3.15 (m, 2H), 2.65-2.57 (m, 2H), 2.35-2.24 (m, 1H).

**Step 3: Synthesis of compound 17-4**

To a solution of compound **17-3** (2.00 g, 5.23 mmol, 1 *eq*) in glacial acetic acid (10 mL) was added reduced iron powder (1.75 g, 31.38 mmol, 6 *eq*) at room temperature, and the reaction mixture was stirred at 80°C for 3.5 hours. The reaction mixture was poured into saturated sodium bicarbonate (250 mL), then filtered through diatomite, and the phases were separated, and the aqueous phase was extracted with ethyl acetate (50 mL × 2). The organic phases were combined, washed with water (100 mL) and saturated brine (100 mL), dried over anhydrous sodium sulfate, filtered, and the filtrate was concentrated under reduced pressure to obtain a crude product. The crude product was purified by silica gel column chromatography (petroleum ether: ethyl acetate = 9:1 to 2:1) to obtain compound **17-4.**

$^1H$ NMR (400 MHz, $CDCl_3$): δ 8.07 (br s, 1H), 7.78-7.68 (m, 1H), 7.55-7.50 (m, 1H), 7.43-7.31 (m, 5H), 7.10-7.02

(m, 1H), 4.52-4.47 (m, 2H), 4.16-4.07 (m, 1H), 3.93 (s, 3H), 3.09-2.96 (m, 2H), 2.51-2.38 (m, 2H); MS (ESI) m/z: 354.1 $[M+H]^+$.

**Step 4: Synthesis of compound 17-5**

To a solution of compound **17-4** (1.00 g, 2.83 mmol, 1 *eq*) in methanol was added Pd/C (50.0 mg, purity: 10%) and concentrated hydrochloric acid (14.3 mg, 141.50 μmol, 14.05 μL, purity: 36 to 38%, 0.05 *eq*) at room temperature, then the reaction flask was replaced with hydrogen three times, and the reaction mixture was heated to 70°C and stirred for 2 hours under hydrogen (15 psi) atmosphere. The reaction mixture was filtered through diatomite, and the filtrate was concentrated under reduced pressure to obtain a crude product of compound **17-5,** which was directly used in the next reaction step. MS (ESI) m/z: 263.9 $[M+H]^+$.

**Step 5: Synthesis of compound 17-6**

To a mixed solution of compound **17-5** (200.0 mg, 759.75 μmol, 1 *eq*) in tetrahydrofuran (4 mL), methanol (2 mL) and water (2 mL) was added lithium hydroxide monohydrate (95.65 mg, 2.28 mmol, 3 *eq*) at room temperature, and the reaction mixture was stirred at 60°C for 3 hours. The reaction mixture was concentrated under reduced pressure to remove the organic solvent. The aqueous phase was added with hydrochloric acid to adjust the pH to 4, and extracted with ethyl acetate (10 mL × 2). The organic phases were combined, dried over anhydrous sodium sulfate, filtered, and the filtrate was concentrated under reduced pressure to obtain a crude product of compound **17-6,** which was directly used in the next reaction step.

**Step 6: Synthesis of compounds 17 and 18**

To a solution of compound **17-6** (165.0 mg, 662.07 μmol, 1 *eq*) in *N,N*-dimethylformamide (2.00 mL) was added HATU (251.74 mg, 662.07 μmol, 1 *eq*) and diisopropylethylamine (256.70 mg, 1.99 mmol, 345.96 μL, 3 *eq*) respectively at 0°C, and the reaction mixture was stirred for 1 hour, then compound **BB-1** (136.57 mg, 662.07 μmol, 1 *eq*) was added, and the reaction mixture was stirred continuously at 0°C for 10 min. The reaction mixture was then diluted with water (20 mL), and extracted with ethyl acetate (30 mL × 3). The organic phases were combined, washed with water (30 mL) and saturated brine (30 mL), dried over anhydrous sodium sulfate, filtered, and the filtrate was concentrated under reduced pressure. The crude product was purified by preparative HPLC (chromatographic column: Phenomenex Gemini-NX C18 75×30 mm×3 μm; mobile phase: [water (0.05% ammonia water)-acetonitrile], acetonitrile: 12% to 42%, the retention time of the HPLC column was 8 min), and the resulting mixture was further chirally purified by SFC [chromatographic column: DAICEL CHIRALPAK IC (250 mm×30 mm×10 μm); mobile phase: mobile phase A was $CO_2$, mobile phase B was ethanol (0.1% ammonia water); gradient: B%: 60% to 60%] to obtain compounds **17** and **18.**

**Compound 17:** SFC analysis conditions: chromatographic column: Chiralpak IC-3 50x4.6 mm I.D, 3 μm, mobile phase: mobile phase A was $CO_2$, mobile phase B was ethanol (0.05% diethylamine); gradient: B%: 40% to 40%; flow rate: 3 mL/min; detector: PDA; retention time: 1.896 min, chiral purity = 100%. $^1H$ NMR (400 MHz, $CD_3OD$): δ 7.37-7.32 (m, 2H), 7.15-7.06 (m, 3H), 7.05-7.00 (m, 1H), 6.91 (d, *J* = 8.8 Hz, 1H), 4.19-4.12 (m, 1H), 4.12-4.06 (m, 1H), 3.74 (s, 2H), 3.54-3.48 (m, 1H), 3.48-3.42 (m, 1H), 2.95-2.84 (m, 6H), 2.72-2.61 (m, 2H), 2.31-2.22 (m, 2H); MS (ESI) m/z: 438.3 $[M+H]^+$;

**compound 18:** SFC analysis conditions: chromatographic column: Chiralpak IC-3 50x4.6 mm I.D, 3 μm, mobile phase: mobile phase A was $CO_2$, mobile phase B was ethanol (0.05% diethylamine); gradient: B%: 40% to 40%; flow rate: 3 mL/min; detector: PDA; retention time: 2.762 min, chiral purity = 96.25%. $^1H$ NMR (400 MHz, $CD_3OD$): δ 7.36 (dd, *J* = 2.0, 8.4 Hz, 1H), 7.33 (d, *J* = 2.0 Hz, 1H), 7.14-7.07 (m, 3H), 7.05-7.00 (m, 1H), 6.93 (d, *J* = 8.0 Hz, 1H), 4.60-4.51 (m, 1H), 4.13-4.06 (m, 1H), 3.75 (s, 2H), 3.54-3.48 (m, 1H), 3.48-3.42 (m, 1H), 2.95-2.84 (m, 5H), 2.73-2.62 (m, 2H), 2.54 (d, *J* = 7.2 Hz, 4H). MS (ESI) m/z: 438.3 $[M+H]^+$.

**Embodiment 18: Preparation of compounds 19 and 20**

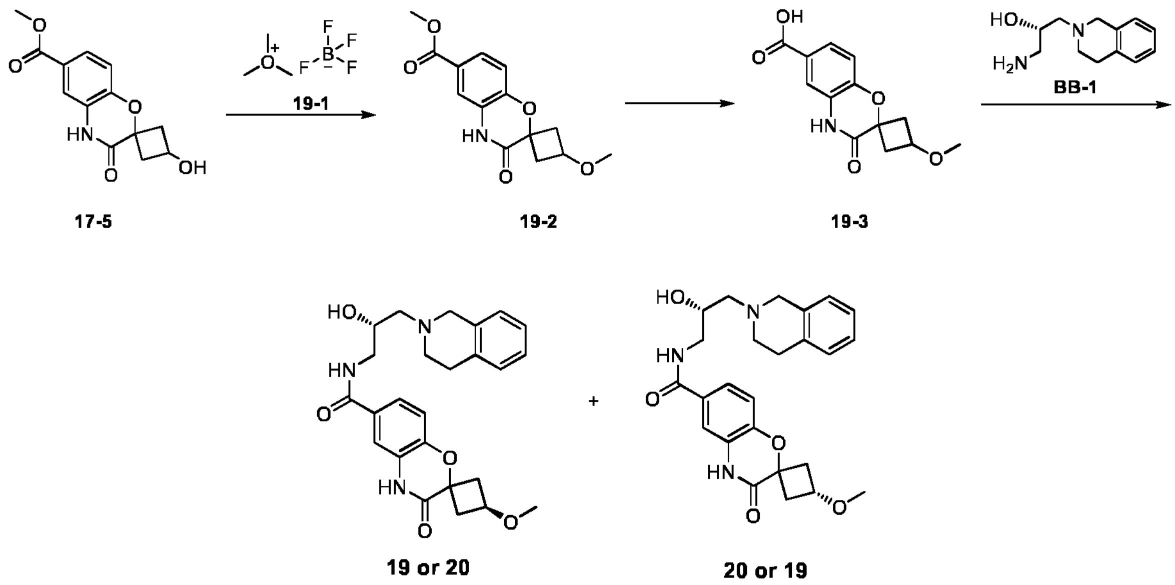


17-5 19-2 19-3

19 or 20 20 or 19

**Step 1: Synthesis of compound 19-2**

To a solution of compound **17-5** (200.0 mg, 759.75 μmol, 1 *eq*) in dichloromethane (30 mL) was added compound **19-1** at room temperature, and the reaction mixture was stirred at 20°C for 16 hours. After the reaction was completed, the reaction mixture was slowly poured into saturated sodium bicarbonate solution (50 mL), and extracted with ethyl acetate (50 mL × 2). The organic phases were combined, washed with water (50 mL), dried over anhydrous sodium sulfate, filtered, and the filtrate was concentrated under reduced pressure. The crude product was purified by a thin-layer chromatography silica gel plate (petroleum ether: ethyl acetate = 3:1) to obtain compound **19-2.**

$^1H$ NMR (400 MHz, $CDCl_3$): δ 7.95 (br s, 1H), 7.71 (dd, *J* = 2.0, 8.8 Hz, 1H), 7.51 (d, *J* = 2.0 Hz, 1H), 7.04 (d, *J* = 8.4 Hz, 1H), 3.91 (s, 3H), 3.30 (s, 3H), 3.04-2.97 (m, 2H), 2.39-2.33 (m, 2H); MS (ESI) m/z: 278.0 $[M+H]^+$.

**Step 2: Synthesis of compound 19-3**

To a mixed solution of compound **19-2** (75.0 mg, 270.49 μmol, 1 *eq*) in tetrahydrofuran (2 mL), methanol (2 mL) and water (2 mL) was added lithium hydroxide monohydrate (34.0 mg, 811.48 μmol, 3 *eq*) at room temperature, and the reaction mixture was stirred at 50°C for 3 hours. The reaction mixture was concentrated under reduced pressure to remove the organic solvent. The aqueous phase was added with hydrochloric acid to adjust the pH to 4, and extracted with ethyl acetate (30 mL × 3). The organic phases were combined, washed with water (30 mL), dried over anhydrous sodium sulfate, filtered, and the filtrate was concentrated under reduced pressure to obtain a crude product of compound **19-3,** which was directly used in the next reaction step.

**Step 3: Synthesis of compounds 19 and 20**

To a solution of compound **19-3** (74.0 mg, 281.11 μmol, 1 *eq*) in *N,N*-dimethylformamide (2.00 mL) was added HATU (106.89 mg, 281.11 μmol, 1 *eq*) and diisopropylethylamine (108.99 mg, 843.32 μmol, 146.89 μL, 3 *eq*) successively at 0°C, and the reaction mixture was stirred for 1 hour, then compound **BB-1** (57.99 mg, 281.11 μmol, 1 *eq*) was added, and the reaction mixture was stirred continuously at 0°C for 5 min. The reaction mixture was then diluted with water (30 mL), and extracted with ethyl acetate (30 mL × 2). The organic phases were combined, washed with water (30 mL) and saturated brine (30 mL), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (0.05% ammonia water)-acetonitrile], acetonitrile: 26% to 56%, the retention time of the HPLC column was 10 min), and the resulting mixture was further chirally purified by SFC [chromatographic column: DAICEL CHIRALPAK IC (250 mm×30 mm×10 μm); mobile phase: mobile phase A was $CO_2$, mobile phase B was isopropanol (0.1% ammonia water); isopropanol (0.1% ammonia water): 60% to 60%] to obtain compounds **19** and **20.**

**Compound 19:** SFC analysis conditions: chromatographic column: Chiralpak IC-3 50x4.6 mm I.D, 3 μm, mobile phase: mobile phase A was $CO_2$, mobile phase B was isopropanol+acetonitrile (0.05% diethylamine); mobile phase B: 40% to 40%; flow rate: 3 mL/min; detector: PDA; retention time: 1.338 min, chiral purity = 100%. $^1H$ NMR (400 MHz, $CDCl_3$): δ 8.44 (s, 1H), 7.54 (d, *J* = 2.0 Hz, 1H), 7.25 (dd, *J* = 2.0, 8.0 Hz, 1H), 7.23-7.12 (m, 4H), 7.02 (d, *J* = 7.6 Hz,

1H), 6.91 (d, *J* = 8.4 Hz, 1H), 4.12-4.02 (m, 1H), 3.93-3.83 (m, 2H), 3.78-3.72 (m, 1H), 3.71-3.65 (m, 1H), 3.59 (dt, *J* = 6.0, 13.6 Hz, 1H), 3.29 (s, 3H), 3.02-2.91 (m, 5H), 2.84-2.77 (m, 1H), 2.75-2.62 (m, 2H), 2.38-2.27 (m, 2H). MS (ESI) m/z: 452.3 $[M+H]^+$.

**Compound 20:** SFC analysis conditions: chromatographic column: Chiralpak IC-3 50x4.6 mm I.D, 3 μm, mobile phase: mobile phase A was $CO_2$, mobile phase B was isopropanol+acetonitrile (0.05% diethylamine); mobile phase B: 40% to 40%; flow rate: 3 mL/min; detector: PDA; retention time: 1.843 min, chiral purity = 94.29%. $^1H$ NMR (400 MHz, $CDCl_3$): δ 8.65 (s, 1H), 7.55 (d, *J* = 2.0 Hz, 1H), 7.27 (dd, *J* = 2.0, 8.0 Hz, 1H), 7.24-7.11 (m, 4H), 7.04-7.00 (m, 1H), 6.91 (d, *J* = 8.4 Hz, 1H), 4.26 (q, *J* = 7.2 Hz, 1H), 4.12-4.04 (m, 1H), 3.86 (d, *J* = 14.8 Hz, 1H), 3.78-3.71 (m, 1H), 3.68 (d, *J* = 14.8 Hz, 1H), 3.58 (dt, *J* = 5.6, 14.0 Hz, 1H), 3.28 (s, 3H), 3.02-2.90 (m, 3H), 2.84-2.76 (m, 1H), 2.75-2.70 (m, 1H), 2.69-2.61 (m, 3H), 2.60-2.51 (m, 3H); MS (ESI) m/z: 452.3 $[M+H]^+$.

**Embodiment 19: Preparation of compounds 21 and 22**

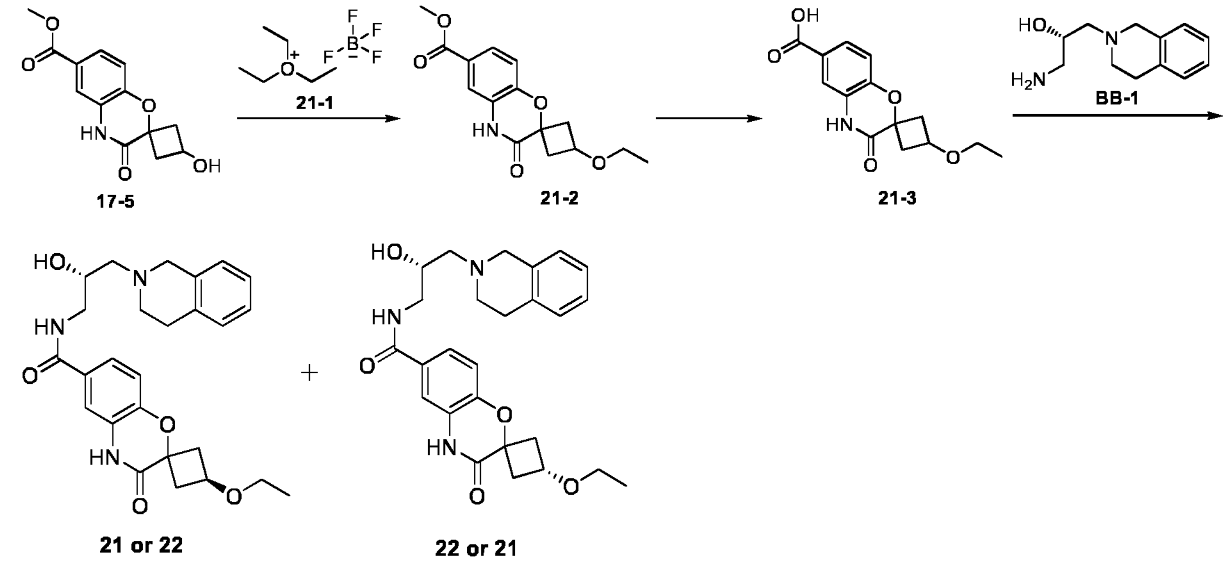

17-5
21-2
21-3
+
21 or 22
22 or 21

**Step 1: Synthesis of compound 21-2:**

To a solution of compound **17-5** (491.0 mg, 1.87 mmol, 1 *eq*) in dichloromethane (10.00 mL) was added compound **21-1** (531.54 mg, 2.80 mmol, 399.65 μL, 1.5 *eq*) at room temperature, and the reaction mixture was stirred at 25°C for 24 hours. After the reaction was completed, the reaction mixture was slowly poured into saturated sodium bicarbonate solution (20 mL), and extracted with ethyl acetate (20 mL × 2). The organic phases were combined, washed with water (50 mL), dried over anhydrous sodium sulfate, filtered, and the filtrate was concentrated under reduced pressure. The crude product was purified by a preparative plate (petroleum ether: ethyl acetate = 1:1) to obtain compound **21-2.**

MS (ESI) m/z: 292.2 $[M+1]^+$.

**Step 2: Synthesis of compound 21-3:**

To a mixed solution of compound **21-2** (98.0 mg, 336.42 μmol, 1 *eq*) in tetrahydrofuran (2.00 mL), methanol (2.00 mL) and water (1.00 mL) was added lithium hydroxide monohydrate (42.35 mg, 1.01 mmol, 3 *eq*) at room temperature, and the reaction mixture was stirred at 50°C for 18 hours. The reaction mixture was concentrated under reduced pressure to remove the organic solvent. The aqueous phase was added with hydrochloric acid to adjust the pH to 6, and extracted with ethyl acetate (30 mL × 3). The organic phases were combined, dried over anhydrous sodium sulfate, filtered, and the filtrate was concentrated under reduced pressure to obtain a crude product of compound **21-3,** which was directly used in the next reaction step.

**Step 3: Synthesis of compounds 21 and 22:**

To a solution of compound **21-3** (104.0 mg, 375.08 μmol, 1 *eq*) in *N,N*-dimethylformamide (2.00 mL) was added

HATU (156.88 mg, 412.59 μmol, 1 *eq*) and diisopropylethylamine (96.95 mg, 750.17 μmol, 130.67 μL, 2 *eq*) respectively at 0°C, and the reaction mixture was stirred for 0.5 hours, then compound **BB-1** (77.37 mg, 375.08 μmol, 1 *eq*) was added, and the reaction mixture was stirred continuously at 0°C for 0.2 hours. The reaction mixture was then diluted with water (50 mL), and extracted with ethyl acetate (50 mL × 3). The organic phases were combined, washed with water (50 mL) and saturated brine (50 mL), dried over anhydrous sodium sulfate, filtered, and the filtrate was concentrated under reduced pressure. The crude product was purified by preparative HPLC (chromatographic column: Phenomenex Gemini-NX C18 75×30 mm×3 μm; mobile phase: [water (0.05% ammonia water)-acetonitrile], acetonitrile%: 25% to 55%, the retention time of the HPLC column was 7 min), and the resulting mixture was further chirally purified by SFC [chromatographic column: DAICEL CHIRALCEL OJ (250 mm×30 mm×10 μm); mobile phase: mobile phase A: $CO_2$, mobile phase B: [0.1% ammonia water-methanol]; mobile phase B%: 30% to 30%] to obtain compounds **21** and **22.**

**Compound 21:** SFC analysis conditions: chromatographic column: Chiralcel OJ-3 50x4.6 mm I.D., 3 μm, mobile phase: mobile phase A was $CO_2$, mobile phase B was methanol (0.05% diethylamine); mobile phase B: 40% to 40%; flow rate: 3 mL/min; detector: PDA; retention time: 1.536 min, chiral purity = 100%. $^1$H NMR (400 MHz, $CDCl_3$): δ 8.82 (s, 1H), 7.60-7.51 (m, 2H), 7.39 (dd, *J* = 1.6, 8.4 Hz, 1H), 7.25-7.12 (m, 3H), 7.07-7.02 (m, 1H), 6.94 (d, *J* = 8.4 Hz, 1H), 4.39-4.29 (m, 1H), 4.24-4.16 (m, 1H), 4.03 (d, *J* = 15.2 Hz,1H), 3.91 (d, *J* = 14.4 Hz, 1H), 3.76-3.70 (m, 1H), 3.63-3.54 (m, 1H), 3.44 (q, *J* = 7.2 Hz, 2H), 3.18-2.96 (m, 4H), 2.94-2.80 (m, 2H), 2.69-2.59 (m, 2H), 2.58-2.49 (m, 2H), 1.28-1.18 (m, 4H). MS (ESI) m/z: 466.5 $[M+1]^+$.

**Compound 22:** SFC analysis conditions: chromatographic column: Chiralcel OJ-3 50x4.6 mm I.D., 3 μm, mobile phase: mobile phase A was $CO_2$, mobile phase B was methanol (0.05% diethylamine); mobile phase B: 40% to 40%; flow rate: 3 mL/min; detector: PDA; retention time: 1.722 min, chiral purity = 99.19%. $^1$H NMR (400 MHz, $CDCl_3$): δ 8.88 (s, 1H), 7.61 (d, *J* = 2.0 Hz, 1H), 7.57-7.49 (m, 1H), 7.34 (dd, *J* = 2.0, 8.4 Hz, 1H), 7.25-7.13 (m, 3H), 7.05-7.01 (m, 1H), 6.91 (d, *J* = 8.0 Hz, 1H), 4.23-4.15 (m, 1H), 4.02 (d, *J* = 15.2 Hz, 1H), 3.97-3.92 (m, 1H), 3.89 (d, *J* = 14.8 Hz, 1H), 3.78-3.69 (m, 1H), 3.65-3.56 (m, 1H), 3.44 (q, *J* = 6.8 Hz, 2H), 3.17-3.07 (m, 1H), 3.06-2.98 (m, 3H), 2.96-2.89 (m, 2H), 2.89-2.79 (m, 2H), 2.37-2.30 (m, 2H), 1.21 (t, *J* = 7.0 Hz, 4H); MS (ESI) m/z: 466.5 $[M+1]^+$.

**Embodiment 20: Preparation of compounds 23 and 24**

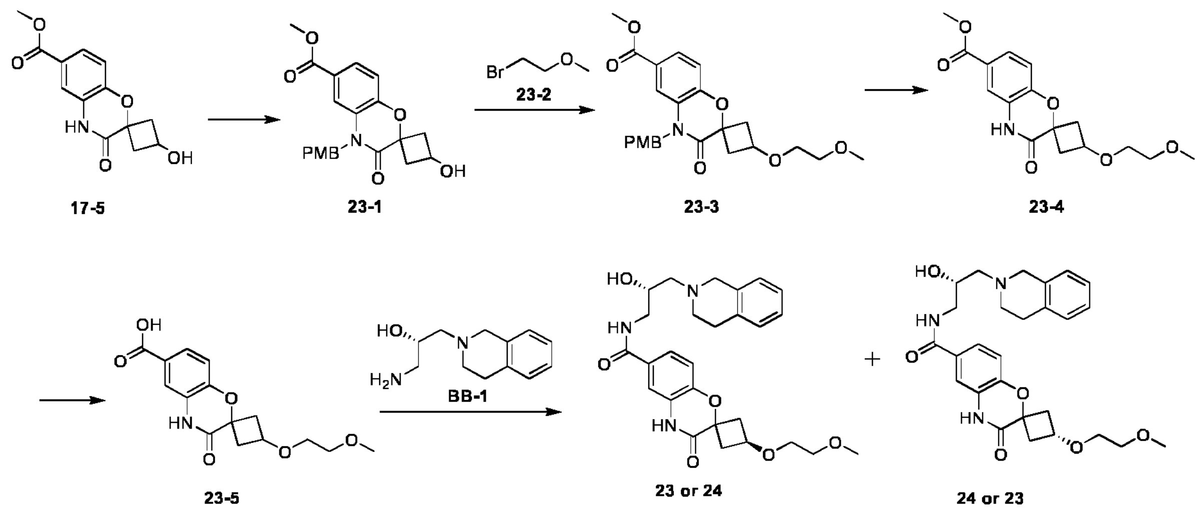


17-5 23-1 23-3 23-4

23-5 23 or 24 24 or 23

**Step 1: Synthesis of compound 23-1:**

To a solution of compound **17-5** (440 mg, 1.67 mmol, 1 *eq*) in *N,N*-dimethylformamide (4.00 mL) was added cesium carbonate (653.51 mg, 2.01 mmol, 1.2 *eq*) at room temperature, and the reaction mixture was stirred for 0.5 hours, then added with p-methoxybenzyl chloride (274.85 mg, 1.76 mmol, 239.00 μL, 1.05 *eq*), and the reaction mixture was stirred at 25°C for 16 hours. After the reaction was completed, the reaction mixture was added with water (50 mL), and extracted with ethyl acetate (50 mL × 2). The organic phases were combined, washed with water (50 mL), dried over anhydrous sodium sulfate, filtered, and the filtrate was concentrated under reduced pressure. The crude product was purified by a preparative plate (petroleum ether: ethyl acetate = 3:1) to obtain compound **23-1.**

MS (ESI) m/z: 384.2 $[M+1]^+$.

**Step 2: Synthesis of compound 23-3:**

To a mixed solution of compound **23-1** (495.0 mg, 1.29 mmol, 1 *eq*) in *N,N*-dimethylformamide (5.00 mL) was added sodium hydride (103.28 mg, 2.58 mmol, purity: 60%, *2 eq*) at 0°C, and the reaction mixture was stirred for 0.2 hours; compound **23-2** (3.59 g, 25.82 mmol, 2.43 mL, 20 *eq*) was added dropwise thereto, and the reaction mixture was stirred at 27°C for 1 hour. The reaction mixture was added with saturated ammonium chloride solution (50 mL) to quench, and extracted with ethyl acetate (50 mL of acetic acid). The organic phase was washed with water (50 mL), dried over anhydrous sodium sulfate, filtered, and the filtrate was concentrated under reduced pressure to obtain a crude product. The crude product was purified by silica gel column chromatography (petroleum ether: ethyl acetate = 4:1) to obtain compound **23-3.**

**Step 3: Synthesis of compound 23-4:**

Compound **23-3** (190.0 mg, 430.38 μmol, 1 *eq*) was dissolved in trifluoroacetic acid (6.00 mL), and the reaction mixture was heated to 100°C and stirred for 3 hours. The reaction mixture was poured into saturated sodium bicarbonate (50 mL) to quench, and extracted with ethyl acetate (50 mL × 2). The organic phases were combined, washed with water (50 mL), dried over anhydrous sodium sulfate, filtered, and the filtrate was concentrated under reduced pressure to obtain a crude product. The crude product was purified by silica gel column chromatography (petroleum ether: ethyl acetate = 1:1) to obtain compound **23-4.**

**Step 4: Synthesis of compound 23-5:**

To a mixed solution of compound **23-4** (108.0 mg, 336.11 μmol, 1 *eq*) in tetrahydrofuran (2.00 mL), methanol (2.00 mL) and water (1.00 mL) was added lithium hydroxide monohydrate (42.31 mg, 1.01 mmol, 3 *eq*) at room temperature, and the reaction mixture was stirred at 80°C for 3 hours. The reaction mixture was concentrated under reduced pressure to remove the organic solvent. The aqueous phase was added with hydrochloric acid to adjust the pH to 6, and extracted with ethyl acetate (50 mL × 3). The organic phases were combined, dried over anhydrous sodium sulfate, filtered, and the filtrate was concentrated under reduced pressure to obtain a crude product of compound **23-5,** which was directly used in the next reaction step. MS (ESI) m/z: 308.1 $[M+1]^+$.

**Step 5: Synthesis of compounds 23 and 24:**

To a solution of compound **23-5** (138.0 mg, 449.08 μmol, 1 *eq*) in *N,N*-dimethylformamide (3.00 mL) was added HATU (187.83 mg, 493.98 μmol, 1.1 *eq*) and diisopropylethylamine (116.08 mg, 898.15 μmol, 156.44 μL, 2 *eq*) respectively at 0°C, and the reaction mixture was stirred at 27°C for 0.5 hours, then compound **BB-1** (138.96 mg, 673.61 μmol, 1.5 *eq*) was added, and the reaction mixture was stirred continuously at 27°C for 0.5 hours. The reaction mixture was then diluted with water (50 mL), and extracted with ethyl acetate (50 mL × 3). The organic phases were combined, washed with water (50 mL), dried over anhydrous sodium sulfate, filtered, and the filtrate was concentrated under reduced pressure. The crude product was purified by preparative HPLC (chromatographic column: Phenomenex Gemini-NX C18 75×30 mm×3 μm; mobile phase: [water (0.05% ammonia water)-acetonitrile], acetonitrile%: 20% to 48%, the retention time of the HPLC column was 7 min), and the resulting mixture was further chirally purified by SFC (chromatographic column: DAICEL CHIRALPAK AD (250 mm×30 mm, 10 μm); mobile phase B: [0.1% ammonia water-methanol]; B%: 60% to 60%) to obtain compounds **23** and **24.**

Compound **23:** SFC analysis conditions: chromatographic column: Chiralpak AD-3 50x4.6 mm I.D., 3 μm, mobile phase: mobile phase A was $CO_2$, mobile phase B was methanol (0.05% diethylamine); methanol (0.05% diethylamine): 40% to 40%; flow rate: 3 mL/min; detector: PDA; retention time: 3.349 min, chiral purity = 100%. $^1H$ NMR (400 MHz, $CDCl_3$): δ 9.40 (s, 1H), 7.69 (s, 1H), 7.65-7.58 (m, 1H), 7.30-7.27 (m, 1H), 7.23-7.11 (m, 3H), 7.04-6.99 (m, 1H), 6.84 (d, *J* = 8.4 Hz, 1H), 4.21-4.12 (m, 1H), 4.01-3.91 (m, 2H), 3.83 (d, *J* = 14.8 Hz, 1H), 3.77-3.67 (m, 1H), 3.64-3.58 (m, 1H), 3.54 (s, 4H), 3.39 (s, 3H), 3.08-3.04 (m, 1H), 3.01-2.94 (m, 4H), 2.93-2.89 (m, 2H), 2.88-2.83 (m, 1H), 2.83-2.76 (m, 1H), 2.42-2.32 (m, 2H). MS (ESI) m/z: 496.5 $[M+1]^+$.

Compound **24:** SFC analysis conditions: chromatographic column: Chiralpak AD-3 50x4.6 mm I.D., 3 μm, mobile phase: mobile phase A was $CO_2$, mobile phase B was methanol (0.05% diethylamine); methanol (0.05% diethylamine): 40% to 40%; flow rate: 3 mL/min; detector: PDA; retention time: 4.942 min, chiral purity = 100%. $^1H$ NMR (400 MHz, $CDCl_3$): δ 9.48 (s, 1H), 7.67 (d, *J* = 1.2 Hz, 1H), 7.56 (brt, *J* = 5.2 Hz, 1H), 7.30-7.26 (m, 1H), 7.21-7.10 (m, 3H), 7.03-6.98 (m, 1H), 6.86 (d, *J* = 8.4 Hz, 1H), 4.38 (quin, *J* = 7.2 Hz, 1H), 4.16-4.08 (m, 1H), 3.87 (d, *J* = 15.2 Hz, 1H), 3.76-3.68 (m, 2H), 3.62-3.56 (m, 1H), 3.54 (s, 4H), 3.38 (s, 3H), 3.02-2.90 (m, 4H), 2.87-2.82 (m, 1H), 2.80-2.74 (m, 1H), 2.73-2.63 (m, 3H), 2.57-2.49 (m, 2H); MS (ESI) m/z: 496.5 $[M+1]^+$.

**Embodiment 21: Preparation of compounds 25 and 26**

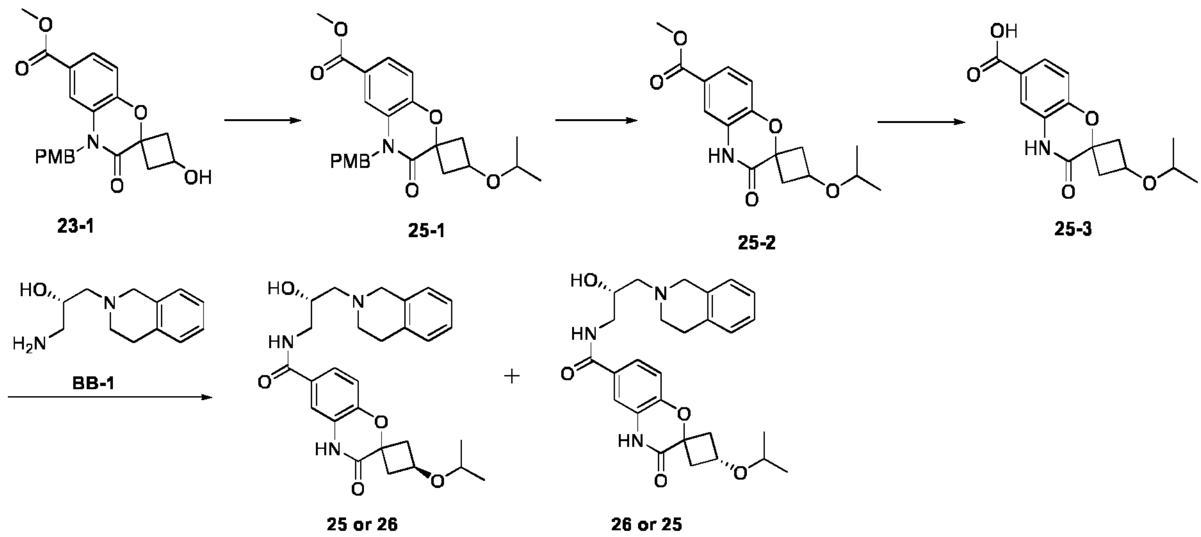

23-1 25-1 25-2 25-3

BB-1

25 or 26 + 26 or 25

**Step 1: Synthesis of compound 25-1:**

To a mixed solution of compound **23-1** (495.0 mg, 1.29 mmol, 1 *eq*) and 2-iodopropane (4.39 g, 25.82 mmol, 2.58 mL, 20 *eq*) in *N,N*-dimethylformamide (4.00 mL) was added sodium hydride (103.29 mg, 2.58 mmol, purity: 60%, 2 *eq*) at -5°C, and the reaction mixture was stirred at -5°C for 1 hour. The reaction mixture was added with saturated ammonium chloride solution (50 mL) to quench, and extracted with ethyl acetate (50 mL × 2). The organic phase was washed with water (50 mL), dried over anhydrous sodium sulfate, filtered, and the filtrate was concentrated under reduced pressure to obtain a crude product. The crude product was purified by silica gel column chromatography (petroleum ether: ethyl acetate = 15:1 to 3:1) to obtain **25-1.**

**Step 2: Synthesis of compound 25-2:**

Compound **25-1** was dissolved in trifluoroacetic acid (5.00 mL), and the reaction mixture was heated to 100°C and stirred for 3 hours. The reaction mixture was poured into saturated sodium bicarbonate (30 mL) to quench, and extracted with ethyl acetate (30 mL × 2). The organic phases were combined, washed with water (30 mL × 2), dried over anhydrous sodium sulfate, filtered, and the filtrate was concentrated under reduced pressure to obtain a crude product of compound **25-2,** which was directly used in the next reaction step.

$^1$H NMR (400 MHz, $CDCl_3$): δ 7.73-7.69 (m, 1H), 7.68-7.66 (m, 1H), 7.49-7.47 (m, 1H), 7.05 (d, *J* = 8.8 Hz, 1H), 4.48-4.39 (m, 1H), 4.07-3.98 (m, 1H), 3.92-3.90 (m, 3H), 2.73-2.64 (m, 2H), 2.62-2.55 (m, 2H), 1.18 (s, 3H), 1.16 (s, 3H).

**Step 3: Synthesis of compound 25-3:**

To a mixed solution of compound **25-2** (151.0 mg, 494.55 μmol, 1 *eq*) in tetrahydrofuran (2.00 mL), methanol (2.00 mL) and water (0.50 mL) was added lithium hydroxide monohydrate (62.26 mg, 1.48 mmol, 3 *eq*) at room temperature, and the reaction mixture was stirred at 60°C for 2 hours. The reaction mixture was concentrated under reduced pressure to remove the organic solvent. The aqueous phase was added with hydrochloric acid to adjust the pH to 6, and extracted with ethyl acetate (50 mL × 2). The organic phases were combined, dried over anhydrous sodium sulfate, filtered, and the filtrate was concentrated under reduced pressure to obtain a crude product of compound **25-3,** which was directly used in the next reaction step. MS (ESI) m/z: 292.1 $[M+1]^+$.

**Step 4: Synthesis of compound 25 and compound 26:**

To a solution of compound **25-3** (65 mg, 223.14 μmol, 1 *eq*) in *N,N*-dimethylformamide (1.00 mL) was added HATU (93.33 mg, 245.45 μmol, 1.1 *eq*) and diisopropylethylamine (57.68 mg, 446.28 μmol, 77.73 μL, 2 *eq*) respectively at 0°C, and the reaction mixture was stirred at 0°C for 0.5 hours, then compound **BB-1** (50.63 mg, 245.45 μmol, 1.1 *eq*)

was added, and the reaction mixture was stirred continuously at 0°C for 0.5 hours. The reaction mixture was then diluted with water (30 mL), and extracted with ethyl acetate (30 mL × 2). The organic phases were combined, washed with water (30 mL), dried over anhydrous sodium sulfate, filtered, and the filtrate was concentrated under reduced pressure. The crude product was purified by preparative HPLC (chromatographic column: Phenomenex Gemini-NX C18 75×30 mm×3 μm; mobile phase: [water (0.05% ammonia water)-acetonitrile], acetonitrile%: 26% to 56%, the retention time of the HPLC column was 8 min), and the resulting mixture was further chirally purified by SFC (chromatographic column: DAICEL CHIRALCEL OJ (250 mm×30 mm, 10 μm); mobile phase B: [0.1% ammonia water-methanol]; B%: 30% to 30%) to obtain compounds **25** and **26.**

Compound **25:** SFC analysis conditions: chromatographic column: Chiralpak IC-3 50x4.6 mm I.D., 3 μm, mobile phase: mobile phase A was $CO_2$, mobile phase B was methanol (0.05% diethylamine); methanol (0.05% diethylamine): 40% to 40%; flow rate: 3 mL/min; detector: PDA; retention time: 0.727 min, chiral purity = 100%. $^1$H NMR (400 MHz, $CDCl_3$): δ 9.28 (s, 1H), 7.96 (br s, 1H), 7.66 (s, 1H), 7.47 (br d, *J* = 8.4 Hz, 1H), 7.25-7.13 (m, 3H), 7.09-7.05 (m, 1H), 6.94 (d, *J* = 8.4 Hz, 1H), 4.43-4.36 (m, 1H), 4.28-4.13 (m, 2H), 3.79-3.69 (m, 1H), 3.66-3.57 (m, 2H), 3.36-3.28 (m, 2H), 3.18-3.01 (m, 4H), 2.63-2.47 (m, 5H), 1.16 (s, 3H), 1.15 (s, 3H). MS (ESI) m/z: 480.4 $[M+1]^+$.

Compound **26:** SFC analysis conditions: chromatographic column: Chiralpak IC-3 50x4.6 mm I.D., 3 μm, mobile phase: mobile phase A was $CO_2$, mobile phase B was methanol (0.05% diethylamine); methanol (0.05% diethylamine): 40% to 40%; flow rate: 3 mL/min; detector: PDA; retention time: 0.616 min, chiral purity = 94.36%. $^1$H NMR (400 MHz, $CDCl_3$): δ 9.83 (s, 1H), 8.43 (s, 1H), 7.75 (s, 1H), 7.57-7.50 (m, 1H), 7.26-7.14 (m, 3H), 7.13-7.06 (m, 1H), 6.91-6.85 (m, 1H), 4.82-4.68 (m, 1H), 4.64-4.51 (m, 1H), 4.37-4.23 (m, 1H), 3.92-3.80 (m, 2H), 3.78-3.64 (m, 2H), 3.62-3.54 (m, 1H), 3.50-3.29 (m, 4H), 3.17-3.00 (m, 1H), 2.88-2.74 (m, 2H), 2.31-2.20 (m, 2H), 1.15 (s, 3H), 1.13 (s, 3H); MS (ESI) m/z: 480.4 $[M+1]^+$.

**Embodiment 22: Preparation of compound 27**

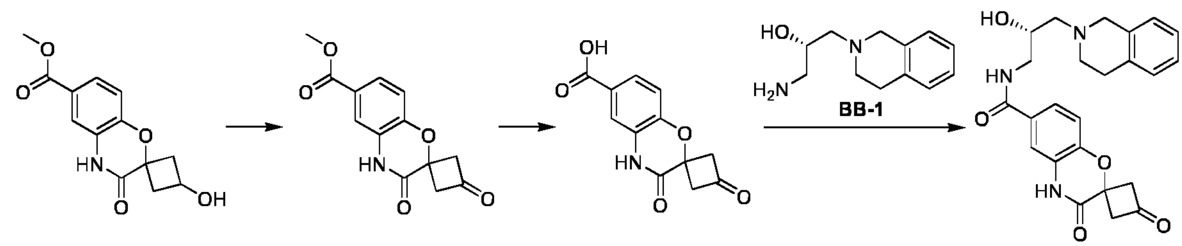


17-5 27-2 27-3 27

**Step 1: Synthesis of compound 27-2**

To a solution of compound **17-5** (0.350 g, 1.33 mmol, 1 *eq*) in dichloromethane (10 mL) was added Dess-Martin periodinane (676.70 mg, 1.60 mmol, 493.94 μL, 1.2 *eq*) at 25°C, and the reaction mixture was stirred continuously for 2 hours. The reaction mixture was added with saturated sodium sulfite (10 mL) and saturated sodium bicarbonate solution (10 mL) to quench, and extracted with dichloromethane (15 mL × 3). The organic phases were combined, washed with saturated brine (15 mL × 2), dried over anhydrous sodium sulfate, filtered, and the filtrate was concentrated under reduced pressure. The crude product was purified by silica gel column chromatography (petroleum ether: ethyl acetate = 1:1) to obtain compound **27-2.**

$^1$H NMR (400 MHz, $CDCl_3$): δ 8.15 (br s, 1H), 7.76 (dd, *J* = 2.0, 8.4 Hz, 1H), 7.59 (d, *J* = 2.0 Hz, 1H), 7.10 (d, *J* = 8.4 Hz, 1H), 3.93 (s, 3H), 3.90-3.79 (m, 2H), 3.46-3.30 (m, 2H).

**Step 2: Synthesis of compound 27-3**

A solution of compound **27-2** (0.050 g, 191.40 μmol, 1 *eq*) in acetic acid (2 mL) and concentrated hydrochloric acid (1 mL) was stirred at 100°C for 1 hour. The reaction mixture was diluted with water (15 mL), and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **27-3.**

**Step 3: Synthesis of compound 27**

To a solution of compound **27-3** (0.040 g, 161.81 μmol, 1 *eq*) in *N,N*-dimethylformamide (4 mL) was added HATU (92.29 mg, 242.72 μmol, 1.5 *eq*) and DIEA (41.83 mg, 323.62 μmol, 56.37 μL, 2.0 *eq*) respectively at 25°C, and the reaction mixture was stirred for 10 min, then compound **BB-1** (33.38 mg, 161.81 μmol, 1 *eq*) was added thereto, and the reaction mixture was stirred continuously for 20 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile: 18% to 48%, the retention time of the HPLC column was 9 min) to obtain compound **27.**

$^1H$ NMR (400 MHz, $CDCl_3$): δ 7.61 (br s, 1H), 7.24-7.10 (m, 5H), 7.08-6.88 (m, 2H), 4.08 (br s, 1H), 3.93-3.75 (m, 4H), 3.70-3.54 (m, 2H), 3.43-3.26 (m, 2H), 3.07 - 2.87 (m, 4H), 2.82-2.48 (m, 3H); MS (ESI) m/z: 436.4 $[M+H]^+$.

**Embodiment 23: Preparation of compound 28**

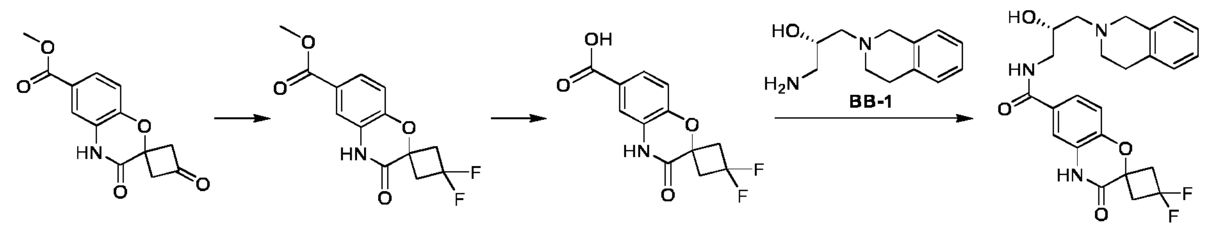


27-2 28-1 28-2 28

**Step 1: Synthesis of compound 28-1**

To a solution of compound **27-2** (0.1 g, 382.80 μmol, 1 *eq*) in dichloromethane (5 mL) was added diethylaminosulfur trifluoride (308.52 mg, 1.91 mmol, 252.88 μL, 5 *eq*) in an ice bath at 0°C, and the reaction mixture was warmed to 25°C and stirred continuously for 12 hours. The reaction mixture was poured into saturated sodium bicarbonate solution (10 mL), and extracted with dichloromethane (10 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 5:1) to obtain compound **28-1.**

$^1H$ NMR (400 MHz, $CDCl_3$): δ 8.02 (br s, 1H), 7.78-7.72 (m, 1H), 7.56 (d, *J* = 1.8 Hz, 1H), 7.12 (d, *J* = 8.4 Hz, 1H), 3.95-3.91 (m, 3H), 3.47-3.30 (m, 2H), 3.01-2.84 (m, 1H).

**Step 2: Synthesis of compound 28-2**

To a solution of compound **28-1** (0.030 g, 105.92 μmol, 1 *eq*) in methanol (2 mL) and water (0.5 mL) was added lithium hydroxide monohydrate (22.22 mg, 529.61 μmol, 5 *eq*) at 25°C, and the reaction mixture was heated to 40°C and stirred for 12 hours. The reaction mixture was added with dilute hydrochloric acid (1 M) to adjust the pH to 3, and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **28-2.**

**Step 3: Synthesis of compound 28**

To a solution of compound **28-2** (0.030 g, 111.44 μmol, 1 *eq*) in *N,N*-dimethylformamide (4 mL) was added HATU (63.56 mg, 167.16 μmol, 1.5 *eq*) and DIEA (28.81 mg, 222.88 μmol, 38.82 μL, 2.0 *eq*) respectively at 25°C, and the reaction mixture was stirred for 10 min, then compound **BB-1** (22.99 mg, 111.44 μmol, 1 *eq*) was added thereto, and the reaction mixture was stirred continuously for 20 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile: 25% to 55%, the retention time of the HPLC column

was 9 min) to obtain compound **28.**

$^1$H NMR (400 MHz, $CDCl_3$): δ 7.66 (s, 1H), 7.44-7.34 (m, 1H), 7.33-7.28 (m, 1H), 7.24-7.12 (m, 3H), 7.07-6.99 (m, 1H), 6.96 (d, *J* = 8.4 Hz, 1H), 4.19-4.05 (m, 1H), 3.99-3.86 (m, 1H), 3.82-3.68 (m, 2H), 3.65-3.53 (m, 1H), 3.44-3.26 (m, 2H), 3.08-2.65 (m, 8H). MS (ESI) m/z: 458.3 $[M+H]^+$.

**Embodiment 24: Preparation of compound 29**

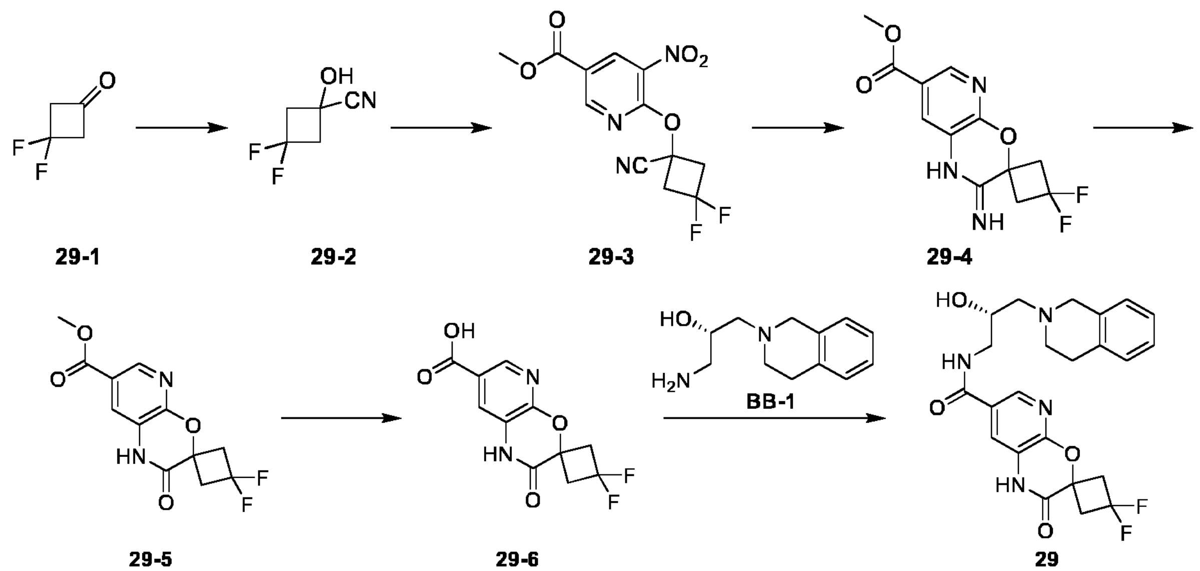


**29-1** **29-2** **29-3** **29-4**

**29-5** **29-6** **29**

**Step 1: Synthesis of compound 29-2**

To a solution of compound **29-1** (0.1 g, 942.76 μmol, 1 *eq*) in dichloromethane (5 mL) was added trimethylsilyl cyanide (112.23 mg, 1.13 mmol, 141.53 μL, 1.2 *eq*) at 0°C, then boron trifluoride diethyl etherate (133.81 mg, 942.76 μmol, 116.35 μL, 1 *eq*) was added, and the reaction mixture was stirred for 1 hour. The reaction mixture was added with saturated sodium bicarbonate solution to adjust the pH to 10, and extracted with dichloromethane (10 mL × 3). The organic phases were combined, washed with saturated brine (10 mL × 2), dried over anhydrous sodium sulfate, filtered, and the filtrate was concentrated under reduced pressure to obtain a crude product of compound **29-2.**

$^1$H NMR (400 MHz, $CDCl_3$): δ 4.21-3.92 (m, 1H), 3.38-3.20 (m, 2H), 3.07-2.91 (m, 2H).

**Step 2: Synthesis of compound 29-3**

To a solution of compound **29-2** (25.81 mg, 193.93 μmol, 1.2 *eq*) and methyl-6-chloro-5-nitronicotinate (0.035 g, 161.60 μmol, 1 *eq*) in tetrahydrofuran (4 mL) was added 1,4-diazabicyclo[2.2.2]octane (36.25 mg, 323.21 μmol, 35.54 μL, 2.0 *eq*) at 25°C, and the reaction mixture was heated to 60°C and stirred for 12 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by thin-layer chromatography (petroleum ether: ethyl acetate = 2:1) to obtain compound **29-3.**

**Step 3: Synthesis of compound 29-4**

To a solution of compound **29-3** (0.090 g, 287.34 μmol, 1 *eq*) and ammonium chloride (76.85 mg, 1.44 mmol, 5 *eq*) in ethanol (10 mL) and water (2 mL) was added iron powder (80.24 mg, 1.44 mmol, 5 *eq*) at 80°C, and the reaction mixture was stirred for 1 hour. The reaction mixture was filtered through diatomite, then the filtrate was diluted with water (15 mL), and extracted with ethyl acetate (15 mL × 3). The organic phases were combined, washed with saturated brine (15mL × 2), dried over anhydrous sodium sulfate, filtered, and the filtrate was concentrated under reduced pressure to obtain a crude product of compound **29-4.**

**Step 4: Synthesis of compound 29-5**

To a solution of compound **29-4** (0.090 g, 317.76 μmol, 1 *eq*) in tetrahydrofuran (2 mL) was added dilute hydrochloric acid (2 M, 794.41 μL, 5 *eq*) at 25°C, and the reaction mixture was heated to 70°C and stirred for 12 hours. The reaction mixture was diluted with water (15 mL), and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **29-5.**

**Step 5: Synthesis of compound 29-6**

To a solution of compound **29-5** (0.080 g, 281.48 μmol, 1 *eq*) in methanol (8 mL), tetrahydrofuran (8 mL) and water (2 mL) was added lithium hydroxide monohydrate (118.12 mg, 2.81 mmol, 10 *eq*) at 25°C, and the reaction mixture was heated to 45°C and stirred for 1 hour. The reaction mixture was added with dilute hydrochloric acid (1 M) to adjust the pH to 3, and extracted with ethyl acetate (15 mL × 2). The organic phases were combined, washed with saturated brine (15 mL × 2), dried over anhydrous sodium sulfate, filtered, and the filtrate was concentrated under reduced pressure to obtain a crude product of compound **29-6.**

**Step 6: Synthesis of compound 29**

To a solution of compound **29-6** (0.050 g, 185.06 μmol, 1 *eq*) in *N,N*-dimethylformamide (4 mL) was added HATU (84.44 mg, 222.07 μmol, 1.2 *eq*) and DIEA (35.87 mg, 277.58 μmol, 48.35 μL, 1.5 *eq*) respectively at 25°C, and the reaction mixture was stirred for 10 min, then compound **BB-1** (38.17 mg, 185.06 μmol, 1 *eq*) was added thereto, and the reaction mixture was stirred for 30 min. The reaction mixture was diluted with water (15 mL), and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile: 18% to 51%, the retention time of the HPLC column was 9 min) to obtain compound **29.**

$^1H$ NMR (400 MHz, $CD_3OD$): δ 8.35 (d, *J* = 2.0 Hz, 1H), 7.75 (d, *J* = 2.0 Hz, 1H), 7.29-7.18 (m, 3H), 7.13 (br d, *J* = 7.2 Hz, 1H), 4.33-4.19 (m, 3H), 3.62-3.51 (m, 1H), 3.49-3.35 (m, 5H), 3.18-2.95 (m, 6H).

MS (ESI) m/z: 459.3 $[M+H]^+$.

**Embodiment 25: Preparation of compound 30**

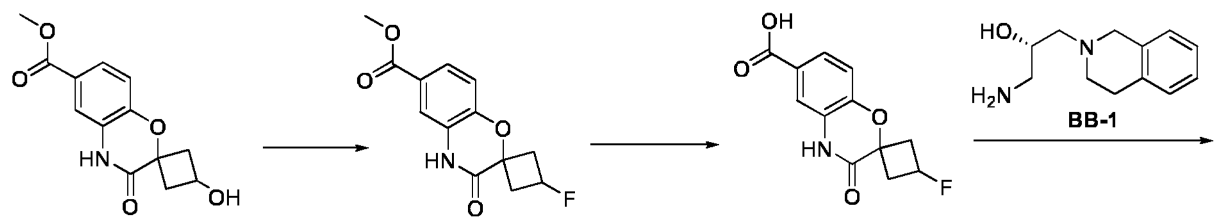


**17-5** **30-1** **30-2**

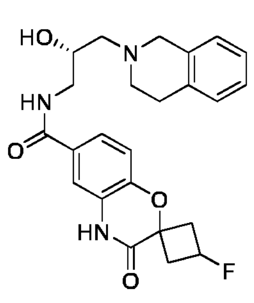

**30**

**Step 1: Synthesis of compound 30-1**

To a solution of compound **17-5** (70 mg, 265.91 μmol, 1 *eq*) in dichloromethane (5 mL) was added diethylaminosulfur trifluoride (50.15 mg, 311.12 μmol, 41.11 μL, 1.17 *eq*) at - 70°C, and the reaction mixture was stirred at 20°C for 12 hours. The reaction mixture was added with saturated sodium bicarbonate solution (3 mL) to quench, and extracted with dichloromethane (20 mL × 3). The organic phases were combined, washed with saturated brine (20 mL × 2), dried over anhydrous sodium sulfate, filtered, and the filtrate was concentrated under reduced pressure. The crude product was purified by silica gel column chromatography (petroleum ether: ethyl acetate = 1:0 to 0:1) to obtain compound **30-1.**

**Step 2: Synthesis of compound 30-2**

To a solution of compound **30-1** (303 mg, 1.14 mmol, 1 *eq*) in tetrahydrofuran (4 mL), methanol (4 mL) and water (2 mL) was added lithium hydroxide monohydrate (22.22 mg, 529.61 μmol, 5 *eq*), and the reaction mixture was stirred at 17°C for 16 hours. The reaction mixture was added with dilute hydrochloric acid (1 M) to adjust the pH to 4, and extracted with ethyl acetate (50 mL × 2). The organic phases were combined, washed with water (50 mL), dried over anhydrous sodium sulfate, filtered, and the filtrate was concentrated under reduced pressure to obtain a crude product of compound **30-2.**

**Step 3: Synthesis of compound 30**

To a solution of compound **30-2** (171 mg, 680.71 μmol, 1 *eq*) in *N,N*-dimethylformamide (3 mL) was added HATU (310.59 mg, 816.85 μmol, 1.2 *eq*) and diisopropylethylamine (131.96 mg, 1.02 mmol, 177.85 μL, 1.5 *eq*) respectively at 0°C, and the reaction mixture was stirred for 30 min, then compound **BB-1** (140.42 mg, 680.71 μmol, 1 *eq*) was added thereto, and the reaction mixture was stirred continuously for 30 min. The reaction mixture was diluted with water, and extracted with ethyl acetate (20 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Phenomenex Gemini-NX C18 75×30 mm×3 μm; mobile phase: [water (10 mM ammonium bicarbonate)-acetonitrile]; acetonitrile: 24% to 54%, the retention time of the HPLC column was 8 min) to obtain compound **30.**

$^1H$ NMR (400 MHz, $CDCl_3$): δ 8.22-8.17 (m, 1H), 7.47 (s, 1H), 7.29-7.27 (m, 1H), 7.22-7.11 (m, 5H), 7.05 (d, *J* = 8.0 Hz, 1H), 6.92 (d, *J* = 8.0 Hz, 1H), 5.11-4.95(m, 1H), 4.07-4.02 (m, 1H), 3.87-3.83 (m, 1H), 3.75-3.61 (m, 2H), 3.58-3.49 (m, 1H), 3.11-3.05 (m, 2H), 2.95-2.88 (m, 3H) , 2.80-2.78 (m, 1H), 2.70-2.52 (m, 4H); MS (ESI) m/z: 440.3$[M+H]^+$.

**Embodiment 26: Preparation of compound 32**

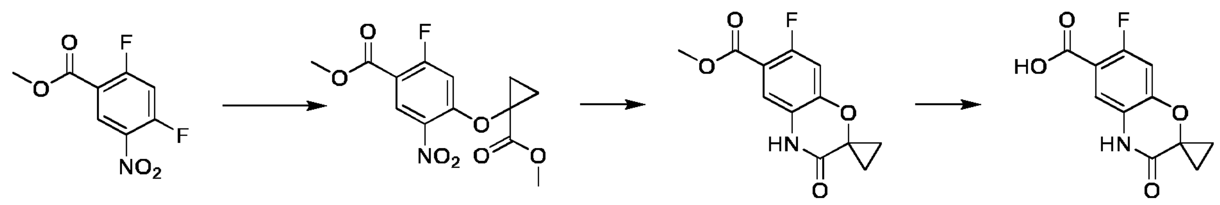

**32-1** **32-2** **32-3** **32-4**

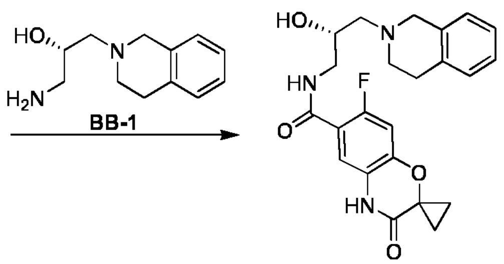


**32**

**Step 1: Synthesis of compound 32-2**

To a solution of compound **32-1** (0.1 g, 460.56 μmol, 1 *eq*) and methyl 1-hydroxycyclopropane-1-carboxylate (53.48 mg, 460.56 μmol, 1 *eq*) in tetrahydrofuran (5 mL) was added sodium hydride (27.63 mg, 690.84 μmol, purity: 60%, 1.5 *eq*) in an ice bath, and the reaction mixture was warmed to 25°C and stirred continuously for 1 hour. The reaction mixture was added with saturated ammonium chloride solution (10 mL) to quench, and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by thin-layer chromatography (petroleum ether: ethyl acetate = 3:1) to obtain compound **32-2.**

**Step 2: Synthesis of compound 32-3**

To a solution of compound **32-2** (0.080 g, 255.40 μmol, 1 *eq*) and ammonium chloride (68.31 mg, 1.28 mmol, 5 *eq*) in ethanol (3 mL) and water (1 mL) was added iron powder (71.32 mg, 1.28 mmol, 5 *eq*) at 75°C, and the reaction mixture was stirred continuously for 1 hour. The reaction mixture was filtered, then the filtrate was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by thin-layer chromatography (petroleum ether: ethyl acetate = 2:1) to obtain compound **32-3.**

$^1H$ NMR (400 MHz, $CDCl_3$): δ 8.02 (br s, 1H), 7.39 (d, *J* = 6.6 Hz, 1H), 6.66 (d, *J* = 10.8 Hz, 1H), 3.98-3.91 (m, 3H), 1.55-1.50 (m, 2H), 1.32-1.28 (m, 2H).

**Step 3: Synthesis of compound 32-4**

To a solution of compound **32-3** (0.035 g, 139.33 μmol, 1 *eq*) in methanol (4 mL), tetrahydrofuran (2 mL) and water (1 mL) was added lithium hydroxide monohydrate (29.23 mg, 696.63 μmol, 5 *eq*) at 25°C, and the reaction mixture was stirred for 1 hour, then heated to 45°C, and stirred for 12 hours. The reaction mixture was added with dilute hydrochloric acid (1 M) to adjust the pH to 3, and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **32-4.**

**Step 4: Synthesis of compound 32**

To a solution of compound **32-4** (0.030 g, 126.48 μmol, 1 *eq*) in *N,N*-dimethylformamide (4 mL) was added HATU (72.14 mg, 189.73 μmol, 1.5 *eq*) and diisopropylethylamine (32.69 mg, 252.97 μmol, 44.06 μL, 2.0 *eq*) respectively at 25°C, and the reaction mixture was stirred for 10 min, then compound **BB-1** (26.09 mg, 126.48 μmol, 1 *eq*) was added thereto, and the reaction mixture was stirred continuously for 30 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile: 26% to 56%, the retention time of the HPLC column was 10 min) to obtain compound **32.**

$^1H$ NMR (400 MHz, $CDCl_3$): δ 8.81 (br s, 1H), 7.84 (d, *J* = 7.6 Hz, 1H), 7.42-7.30 (m, 1H), 7.22-7.10 (m, 3H), 7.06-6.97 (m, 1H), 7.06-6.94 (m, 1H), 7.42-7.30 (d, *J* = 12.0 Hz, 1H), 4.15-3.99 (m, 1H), 3.85-3.73 (m, 2H), 3.68-3.49 (m, 2H), 3.00-2.88 (m, 3H), 2.80-2.71 (m, 1H), 2.69-2.53 (m, 2H), 1.53-1.49 (m, 2H), 1.31-1.20 (m, 2H). MS (ESI) m/z: 426.3 $[M+H]^+$.

**Embodiment 27: Preparation of compound 33**

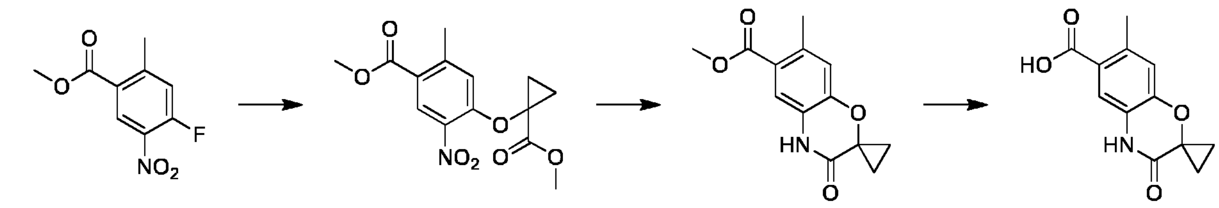

33-1 33-2 33-3 33-4

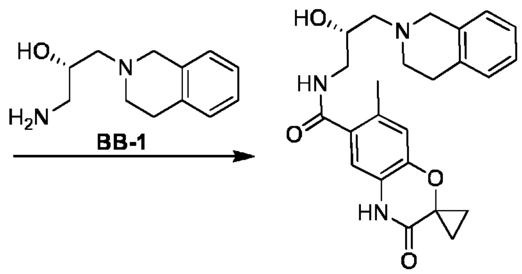


33

**Step 1: Synthesis of compound 33-2:**

To a solution of compound **33-1** (0.1 g, 469.12 μmol, 1 *eq*) and methyl 1-hydroxycyclopropane-1-carboxylate (55 mg, 473.67 μmol, 1.01 *eq*) in tetrahydrofuran (2 mL) was added sodium hydride (30 mg, 750.07 μmol, purity: 60%, 1.60 *eq*) at 0°C under nitrogen atmosphere, and the reaction mixture was stirred at 20°C to 25°C for 1 hour. The reaction mixture was added with saturated ammonium chloride solution (5 mL) to quench, and extracted with ethyl acetate (5 mL × 3). The organic phases were combined, washed with water (5 mL) and saturated brine (5 mL), dried over anhydrous sodium sulfate, filtered, and the filtrate was concentrated under reduced pressure to obtain a crude product of compound **33-2.**

**Step 2: Synthesis of compound 33-3**

To a solution of compound **33-2** (120 mg, 388.01 μmol, 1 *eq*) in acetic acid (3 mL) was added iron powder (120.00 mg, 2.15 mmol, 5.54 *eq*), and the reaction mixture was stirred at 80°C for 1 hour. The reaction mixture was added with saturated sodium bicarbonate (30 mL), and filtered through diatomite. The filter cake was washed with ethyl acetate (30 mL), and the phases of the filtrate were separated. The organic phase was washed with water (30 mL), dried over anhydrous sodium sulfate, filtered, and the filtrate was concentrated under reduced pressure to obtain a crude product of compound **33-3.**

**Step 3: Synthesis of compound 33-4**

To a solution of compound **33-3** (0.1 g, 404.46 μmol, 1 *eq*) in tetrahydrofuran (2 mL), methanol (1 mL) and water (0.5 mL) was added lithium hydroxide monohydrate (60.00 mg, 1.43 mmol, 3.54 *eq*), then the reaction mixture was stirred at 20°C to 25°C for 12 hours, and stirred at 60°C for 3 hours. The reaction mixture was added with hydrochloric acid (1 mol/L) to adjust the pH to 4, and extracted with ethyl acetate (10 mL × 2). The organic phases were combined, washed with water (10 mL), dried over anhydrous sodium sulfate, filtered, and concentrated under reduced pressure to obtain compound **33-4.**

**Step 4: Synthesis of compound 33**

To a solution of compound **33-4** (0.1 g, 428.78 μmol, 1 *eq*) in *N*,*N*-dimethylformamide (3 mL) was added HATU (180 mg, 473.40 μmol, 1.1 *eq*) and DIEA (170.66 mg, 1.32 mmol, 230 μL, 3.08 *eq*) at 0°C, and the reaction mixture was stirred at 0°C for 0.5 hours, then compound **BB-1** (90 mg, 436.29 μmol, 1.02 *eq*) was added thereto, and the reaction mixture was stirred at 0°C for 0.5 hours. The reaction mixture was added with water (10 mL), stirred for 10 min, filtered, and the filter cake was washed with water (30 mL) and evaporated to dryness by rotary evaporation. The crude product was purified by preparative HPLC (chromatographic column: Waters Xbridge 150×25 mmx5 μm; mobile phase: [water (10 mM ammonium bicarbonate)-acetonitrile]; acetonitrile%: 30% to 60%, the retention time of the HPLC column was

10 min) to obtain compound **33.**

$^1$H NMR (400 MHz, $CDCl_3$): δ 8.93 (br s, 1H), 7.19-7.07 (m, 3H), 7.04-6.93 (m, 2H), 6.71-6.64 (m, 2H), 4.08-4.00 (m, 1H), 3.82 (d, *J* = 14.9 Hz, 1H), 3.70 (ddd, *J* = 3.7, 5.9, 13.8 Hz, 1H), 3.63 (d, *J* = 14.9 Hz, 1H), 3.39 (td, *J* = 6.0, 13.8 Hz, 1H), 2.97-2.84 (m, 3H), 2.79-2.70 (m, 1H), 2.66-2.53 (m, 2H), 2.42-2.32 (m, 3H), 1.48-1.34 (m, 2H), 1.24-1.11 (m, 2H); MS (ESI) m/z: 422.3 $[M+1]^+$.

**Embodiment 28: Preparation of compound 34**

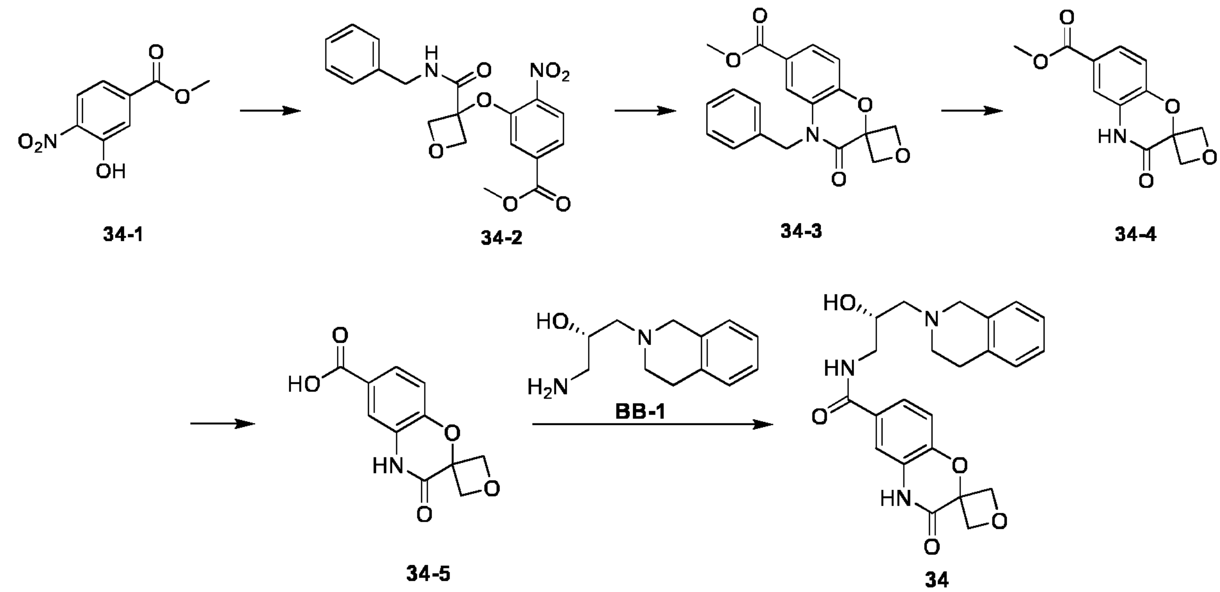

34-1 34-2 34-3 34-4

BB-1

34-5 34

**Step 1: Synthesis of compound 34-2**

To compound **34-1** (0.5 g, 2.54 mmol, 1 *eq*) was added 3-oxetanone (182.77 mg, 2.54 mmol, 1 *eq*) and benzyl isocyanide (297.11 mg, 2.54 mmol, 308.85 μL, 1 *eq*), and the reaction mixture was stirred at 55°C for 12 hours. The reaction mixture was concentrated under reduced pressure to obtain a crude product of compound **34-2.**

**Step 2: Synthesis of compound 34-3**

To a solution of compound **34-2** (0.980 g, 2.54 mmol, 1 *eq)* in *N,N*-dimethylformamide (10 mL) was added potassium *tert*-butoxide (569.25 mg, 5.07 mmol, 2 *eq)* at 25°C, and the reaction mixture was heated to 100°C and stirred for 1 hour. The reaction mixture was added with water (10 mL) and dilute hydrochloric acid (1 M, 5 mL) to quench, and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by silica gel column chromatography (petroleum ether: ethyl acetate = 4:1) to obtain compound **34-3.**

**Step 3: Synthesis of compound 34-4**

To a solution of compound **34-3** (0.536 g, 1.58 mmol, 1 *eq*) in acetic acid (10 mL) was added palladium on carbon (0.536 g, 1.58 mmol, purity: 10%, 1.00 *eq*) at 25°C, and the reaction mixture was stirred at 110°C for 12 hours under hydrogen atmosphere. The reaction mixture was filtered through diatomite, then the filtrate was concentrated under reduced pressure, and the crude product was purified by silica gel column chromatography (petroleum ether: ethyl acetate = 5:1 to 1:1) to obtain compound **34-4.**

$^1$H NMR (400 MHz, DMSO-$d_6$): δ 11.07 (br s, 1H), 7.57 (dd, *J* = 2.0, 8.4 Hz, 1H), 7.50 (d, *J* = 2.0 Hz, 1H), 7.19 (d, *J* = 8.4 Hz, 1H), 5.05-4.86 (m, 2H), 4.78-4.58 (m, 2H), 3.81 (s, 3H).

**Step 4: Synthesis of compound 34-5**

To a solution of compound **34-4** (0.240 g, 963.01 μmol, 1 *eq*) in methanol (10 mL) and water (2 mL) was added

lithium hydroxide monohydrate (202.04 mg, 4.82 mmol, 5 *eq)* at 25°C, and the reaction mixture was heated to 45°C and stirred for 2 hours. The reaction mixture was added with dilute hydrochloric acid (1 M) to adjust the pH to 3, and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **34-5.**

**Step 5: Synthesis of compound 34**

To a solution of compound **34-5** (0.226 g, 960.91 μmol, 1 *eq*) in *N,N*-dimethylformamide (5 mL) was added HATU (438.44 mg, 1.15 mmol, 1.2 *eq*) and DIEA (186.28 mg, 1.44 mmol, 251.06 μL, 1.5 *eq*) respectively at 25°C, and the reaction mixture was stirred for 10 min, then compound **BB-1** (198.22 mg, 960.91 μmol, 1 *eq)* was added thereto, and the reaction mixture was stirred for 30 min. The reaction mixture was diluted with water (10 mL), and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile: 25% to 55%, the retention time of the HPLC column was 10 min) to obtain compound **34.**

$^1$H NMR (400 MHz, DMSO-$d_6$): δ 11.01 (brs, 1H), 8.41 (t, *J* = 5.6 Hz, 1H), 7.46-7.34 (m, 2H), 7.18-6.95 (m, 5H), 5.03-4.88 (m, 2H), 4.87-4.79 (m, 1H), 4.68-4.51 (m, 2H), 4.01-3.85 (m, 1H), 3.64-3.59 (m, 2H), 3.35-3.17 (m, 4H), 2.85-2.76 (m, 2H), 2.74-2.64 (m, 2H); MS (ESI) m/z: 424.3 [M+H]$^+$.

**Embodiment 29: Preparation of compounds 35 and 36**

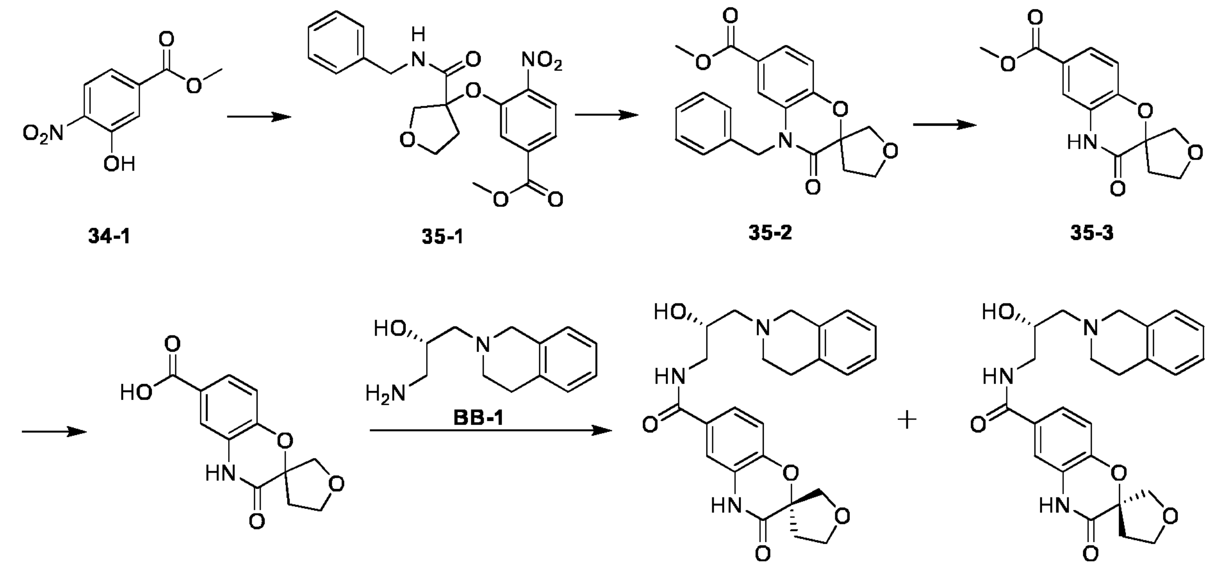


**34-1** **35-1** **35-2** **35-3**

**35-4** **35 or 36** **36 or 35**

**Step 1: Synthesis of compound 35-1:**

To compound **34-1** (0.5 g, 2.54 mmol, 1 *eq*) was added dihydrofuran-3(2*H*)-one (218.67 mg, 2.54 mmol, 1 *eq*) and benzyl isocyanide (297.55 mg, 2.54 mmol, 309.31 μL, 1 *eq),* and the reaction mixture was stirred at 55°C for 12 hours. The reaction mixture was concentrated under reduced pressure to obtain a crude product of compound **35-1.**

**Step 2: Synthesis of compound 35-2:**

To a solution of compound **35-1** (1 g, 2.50 mmol, 1 *eq*) in N,N-dimethylformamide (10 mL) was added potassium *tert*-butoxide (560.53 mg, 5.00 mmol, 2 *eq*) at 25°C, and the reaction mixture was heated to 100°C and stirred for 1 hour. The reaction mixture was added with water (10 mL) and dilute hydrochloric acid (1 M, 10 mL) to quench, and extracted with ethyl acetate (15 mL × 3). The organic phases were combined, washed with saturated brine (15 mL × 2), dried

over anhydrous sodium sulfate, filtered, and the filtrate was concentrated under reduced pressure. The crude product was purified by column chromatography (petroleum ether: ethyl acetate = 10:1 to 4:1) to obtain compound **35-2.**

**Step 3: Synthesis of compound 35-3:**

To a solution of compound **35-2** (0.262 g, 741.44 μmol, 1 *eq*) in acetic acid (10 mL) was added palladium on carbon (0.262 g, purity: 10%) at 25°C, and the reaction mixture was stirred at 115°C for 12 hours under hydrogen atmosphere. The reaction mixture was filtered through diatomite, then the filtrate was concentrated under reduced pressure, and the crude product was purified by column chromatography (petroleum ether: ethyl acetate = 10:1 to 2:1) to obtain compound **35-3.**

$^1$H NMR (400 MHz, DMSO-$d_6$): δ 11.08 (br s, 1H), 7.58 (dd, *J* = 2.0, 8.4 Hz, 1H), 7.54 (d, *J* = 2.0 Hz, 1H), 7.10 (d, *J* = 8.4 Hz, 1H), 4.02-3.84 (m, 4H), 3.82 (s, 3H), 2.43-2.35 (m, 1H), 2.21-2.10 (m, 1H).

**Step 4: Synthesis of compound 35-4:**

To a solution of compound **35-3** (0.110 g, 417.86 μmol, 1 *eq*) in methanol (10 mL) and water (2 mL) was added lithium hydroxide monohydrate (87.67 mg, 2.09 mmol, 5 *eq*) at 25°C, and the reaction mixture was heated to 45°C and stirred for 12 hours. The reaction mixture was added with dilute hydrochloric acid (1 M) to adjust the pH to 3, and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **35-4.**

**Step 5: Synthesis of compounds 35 and 36:**

To a solution of compound **35-4** (0.090 g, 361.13 μmol, 1 *eq)* in *N,N*-dimethylformamide (5 mL) was added 2-(7-azabenzotriazol-1-yl)-*N,N,N',N'*-tetramethyluronium hexafluorophosphate (164.77 mg, 433.35 μmol, 1.2 *eq*) and *N,N*-diisopropylethylamine (70.01 mg, 541.69 μmol, 94.35 μL, 1.5 *eq*) respectively at 25°C, and the reaction mixture was stirred for 10 min, then compound **BB-1** (111.74 mg, 541.69 μmol, 1.5 *eq*) was added thereto, and the reaction mixture was stirred for 30 min. The reaction mixture was diluted with water (10 mL), and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by preparative HPLC (chromatographic column: Waters Xbridge 150×25 mm×5 μm; mobile phase: [water (10 mM ammonium bicarbonate)-acetonitrile]; acetonitrile%: 17% to 50%, the retention time of the HPLC column was 9 min), and then purified by SFC (chromatographic column: DAICEL CHIRALPAK IC (250 mm×30 mm, 10 μm); mobile phase B: [0.1% ammonia water-isopropanol]; B%: 60% to 60%) to obtain compounds **35** and **36.**

**Compound 35:** SFC analysis conditions: chromatographic column: Chiralpak IC-3 50×4.6 mm I.D., 3 μm, mobile phase: mobile phase A was $CO_2$, mobile phase B was isopropanol (0.05% diethylamine); isopropanol (0.05% diethylamine): 40% to 40%; flow rate: 3 mL/min; detector: PDA; retention time: 2.317 min, chiral purity = 100%. $^1$H NMR (400 MHz, $CDCl_3$): δ 9.35 (br s, 1H), 7.81 (br s, 1H), 7.73 (s, 1H), 7.42 (dd, *J* = 2.0, 8.4 Hz, 1H), 7.26-7.12 (m, 3H), 7.05 (d, *J* = 7.2 Hz, 1H), 6.93 (d, *J* = 8.4 Hz, 1H), 4.34-4.22 (m, 1H), 4.19-4.08 (m, 3H), 4.07-3.96 (m, 3H), 3.82-3.70 (m, 1H), 3.67-3.55 (m, 1H), 3.29-3.13 (m, 2H), 3.11-2.91 (m, 4H), 2.59-2.47 (m, 1H), 2.32-2.20 (m, 1H); MS (ESI) m/z: 438.3 $[M+1]^+$.

**Compound 36:** SFC analysis conditions: chromatographic column: Chiralpak IC-3 50×4.6 mm I.D., 3 μm, mobile phase: mobile phase A was $CO_2$, mobile phase B was isopropanol (0.05% diethylamine); isopropanol (0.05% diethylamine): 40% to 40%; flow rate: 3 mL/min; detector: PDA; retention time: 4.500 min, chiral purity = 99.46%. $^1$H NMR (400 MHz, $CDCl_3$): δ 9.58-8.91 (m, 1H), 7.71 (s, 2H), 7.38 (dd, *J* = 1.8, 8.4 Hz, 1H), 7.26-7.11 (m, 3H), 7.08-7.01 (m, 1H), 6.92 (d, *J* = 8.4 Hz, 1H), 4.29-4.18 (m, 1H), 4.17-4.07 (m, 2H), 4.06-3.86 (m, 4H), 3.82-3.71 (m, 1H), 3.67-3.55 (m, 1H), 3.22-3.07 (m, 2H), 3.04-2.84 (m, 4H), 2.59-2.46(m, 1H), 2.26-2.22 (m, 1H); MS (ESI) m/z: 438.3 $[M+1]^+$.

**Embodiment 30: Preparation of compound 37**

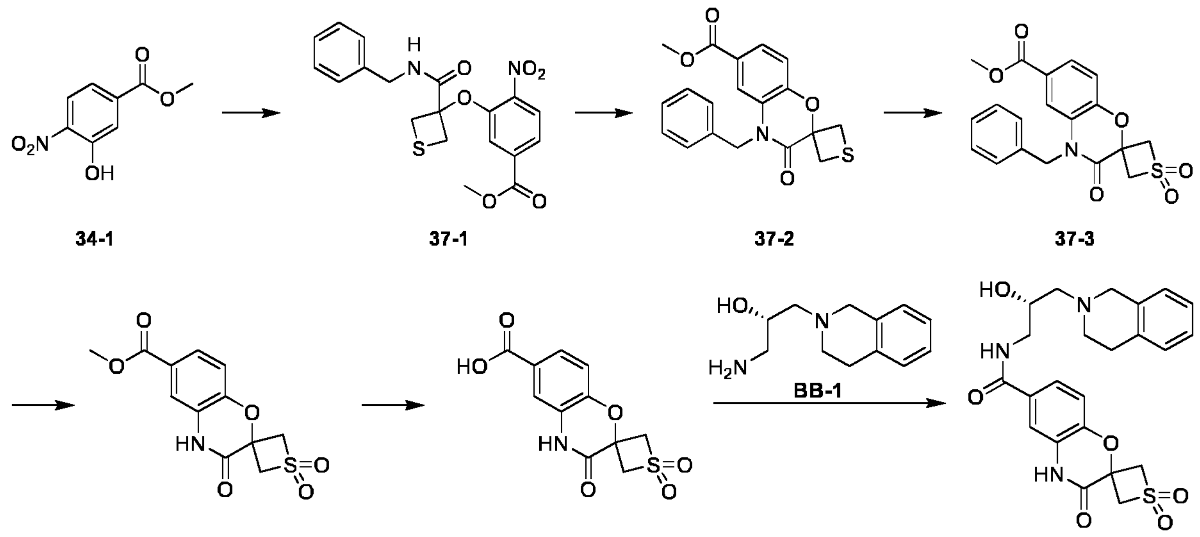


34-1 37-1 37-2 37-3

37-4 37-5 37

**Step 1: Synthesis of compound 37-1**

To compound **34-1** (0.5 g, 2.54 mmol, 1 *eq*) was added thietan-3-one (223.85 mg, 2.54 mmol, 1 *eq*) and benzyl isocyanide (297.55 mg, 2.54 mmol, 309.31 μL, 1 *eq*), and the reaction mixture was stirred at 55°C for 12 hours. The reaction mixture was concentrated under reduced pressure to obtain a crude product of compound **37-1.**

**Step 2: Synthesis of compound 37-2**

To a solution of compound **37-1** (1 g, 2.48 mmol, 1 *eq*) in *N,N*-dimethylformamide (10 mL) was added potassium *tert*-butoxide (557.68 mg, 4.97 mmol, 2 *eq*) at 25°C, and the reaction mixture was heated to 100°C and stirred for 1 hour. The reaction mixture was added with water (10 mL) and dilute hydrochloric acid (1 M, 10 mL) to quench, and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by silica gel column chromatography (petroleum ether: ethyl acetate = 20:1 to 4:1) to obtain compound **37-2.**

**Step 3: Synthesis of compound 37-3**

To a solution of compound **37-2** (0.235 g, 661.21 μmol, 1 *eq*) in dichloromethane (10 mL) was added *m*-chloroperoxybenzoic acid (402.73 mg, 1.98 mmol, purity: 85%, 3.0 *eq*) in an ice bath, and the reaction mixture was warmed to 25°C and stirred for 12 hours. The reaction mixture was added with saturated sodium sulfite (10 mL) to quench, and extracted with dichloromethane (10 mL × 3). The organic phases were combined, washed with saturated sodium bicarbonate solution (10 mL × 5) and saturated brine (10 mL × 2), dried over anhydrous sodium sulfate, filtered, and the filtrate was concentrated under reduced pressure to obtain a crude product of compound **37-3.**

**Step 4: Synthesis of compound 37-4**

To a solution of compound **37-3** (0.240 g, 619.51 μmol, 1 *eq*) in acetic acid (5 mL) was added palladium on carbon (0.050 g, purity: 10%) at 25°C, and the reaction mixture was stirred at 115°C for 12 hours under hydrogen (15 psi) atmosphere. The reaction mixture was filtered through diatomite, then the filtrate was concentrated under reduced pressure, and the crude product was purified by silica gel column chromatography (petroleum ether: ethyl acetate = 5:1 to 1:1) to obtain compound **37-4.**

$^1H$ NMR (400 MHz, DMSO-$d_6$): δ 11.44 (s, 1H), 7.63 (dd, *J* = 2.0, 8.4 Hz, 1H), 7.56 (d, *J* = 2.0 Hz, 1H), 7.22 (d, *J* = 8.4 Hz, 1H), 4.97-4.80 (m, 2H), 4.62-4.39 (m, 2H), 3.87-3.77 (m, 3H).

**Step 5: Synthesis of compound 37-5**

A solution of compound **37-4** (0.040 g, 134.55 μmol, 1 *eq)* in acetic acid (4 mL) and concentrated hydrochloric acid (4 mL) was stirred at 95°C for 4 hours. The reaction mixture was diluted with water (10 mL), and extracted with (ethyl acetate: tetrahydrofuran = 4:1 v/v, 10 mL × 3). The organic phases were combined, washed with saturated brine

(10 mL × 2), dried over anhydrous sodium sulfate, filtered, and the filtrate was concentrated under reduced pressure to obtain a crude product of compound **37-5.**

**Step 6: Synthesis of compound 37**

To a solution of compound **37-5** (0.040 g, 141.21 μmol, 1 *eq*) in *N,N*-dimethylformamide (5 mL) was added 2-(7-azabenzotriazol-1-yl)-*N,N,N',N'*-tetramethyluronium hexafluorophosphate (64.43 mg, 169.46 μmol, 1.2 *eq*) and *N,N*-diisopropylethylamine (27.38 mg, 211.82 μmol, 36.89 μL, 1.5 *eq*) respectively at 25°C, and the reaction mixture was stirred for 10 min, then compound **BB-1** (29.13 mg, 141.21 μmol, 1 *eq)* was added thereto, and the reaction mixture was stirred for 30 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 1150×25 mmx5 μm; mobile phase: [water (10 mM ammonium bicarbonate)-acetonitrile]; acetonitrile: 15% to 48%, the retention time of the HPLC column was 9 min) to obtain compound **37.**

$^1$H NMR(400 MHz, DMSO-$d_6$): δ 11.38 (brs, 1H), 8.47 (t, *J* = 5.6 Hz, 1H), 7.54-7.44 (m, 2H), 7.16-7.06 (m, 4H), 7.05-6.98 (m, 1H), 4.96-4.86 (m, 2H), 4.83-4.77 (m, 1H), 4.56-4.42 (m, 2H), 3.96-3.81 (m, 1H), 3.71-3.55 (m, 2H), 3.47-3.44 (m, 2H), 3.27-3.15 (m, 1H), 2.84-2.77 (m, 2H), 2.76-2.64 (m, 2H). MS (ESI) m/z: 472.2 [M+H]$^+$.

**Embodiment 31: Preparation of compound 38**

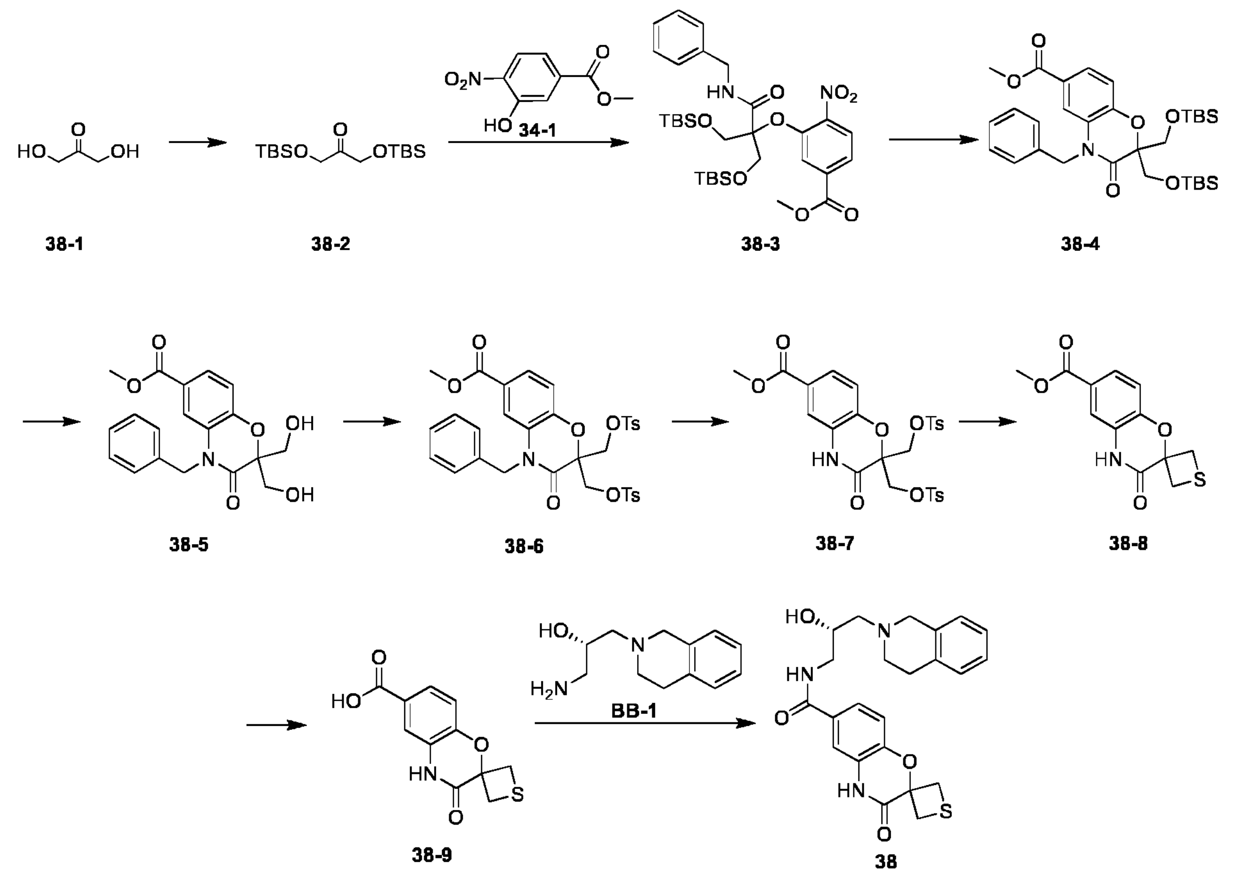


38-1 38-2 38-3 38-4

38-5 38-6 38-7 38-8

38-9 38

**Step 1: Synthesis of compound 38-2:**

To a solution of compound **38-1** (2 g, 22.20 mmol, 1 *eq*) in N,N-dimethylformamide (10 mL) was added imidazole (7.56 g, 111.02 mmol, 5 *eq*) and *tert*-butyldimethylsilyl chloride (8.37 g, 55.51 mmol, 6.80 mL, 2.5 *eq*) at 25°C, and the reaction mixture was stirred for 12 hours. The reaction mixture was diluted with water (20 mL), and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by

silica gel column chromatography (petroleum ether: ethyl acetate = 20:1) to obtain compound **38-2.**

$^1H$ NMR (400 MHz, $CDCl_3$): δ 4.51-4.36 (m, 4H), 0.98-0.92 (m, 18H), 0.10-0.08 (m, 12H).

**Step 2: Synthesis of compound 38-3**

To compound **34-1** (1 g, 5.07 mmol, 1 *eq)* was added compound **38-2** (1.62 g, 5.07 mmol, 1.0 *eq*) and benzyl isocyanide (594.22 mg, 5.07 mmol, 617.69 μL, 1.0 *eq*), and the reaction mixture was stirred at 55°C for 12 hours. The reaction mixture was concentrated under reduced pressure to obtain a crude product of compound **38-3.**

**Step 3: Synthesis of compound 38-4**

To a solution of compound **38-3** (3 g, 4.74 mmol, 1 *eq)* in *N*,*N*-dimethylformamide (40 mL) was added potassium *tert*-butoxide (1.06 g, 9.48 mmol, 2.0 *eq*) at 25°C, and the reaction mixture was heated to 100°C and stirred for 1 hour. The reaction mixture was added with dilute hydrochloric acid (1 M, 10 mL) to quench, and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **38-4.**

**Step 4: Synthesis of compound 38-5**

To a solution of compound **38-4** (3 g, 5.12 mmol, 1 *eq*) in tetrahydrofuran (20 mL) was added tetrabutylammonium fluoride (1 M, 12.80 mL, 2.5 *eq*) at 25°C, and the reaction mixture was stirred continuously for 1 hour. The reaction mixture was diluted with water (20 mL), and extracted with ethyl acetate (20 mL × 3). The organic phases were combined, washed with saturated brine (20 mL × 2), dried over anhydrous sodium sulfate, filtered, and the filtrate was concentrated under reduced pressure. The crude product was purified by silica gel column chromatography (petroleum ether: ethyl acetate = 10:1 to 0:1) to obtain compound **38-5.**

**Step 5: Synthesis of compound 38-6**

To a solution of compound **38-5** (1 g, 2.80 mmol, 1 *eq*) in dichloromethane (20 mL) was added triethylamine (1.13 g, 11.19 mmol, 1.56 mL, 4 *eq*), 4-dimethylaminopyridine (170.94 mg, 1.40 mmol, 0.5 *eq*) and *p*-toluenesulfonyl chloride (2.13 g, 11.19 mmol, 4.0 *eq)* at 25°C, and the reaction mixture was stirred for 24 hours. The reaction mixture was diluted with water (15 mL), and extracted with dichloromethane (15 mL × 3). The organic phases were combined, washed with saturated brine (15 mL × 2), dried over anhydrous sodium sulfate, filtered, and the filtrate was concentrated under reduced pressure. The crude product was purified by silica gel column chromatography (petroleum ether: ethyl acetate = 10:1 to 2:1) to obtain compound **38-6.**

**Step 6: Synthesis of compound 38-7**

To a solution of compound **38-6** (0.667 g, 1.00 mmol, 1 *eq*) in acetic acid (10 mL) was added palladium on carbon (0.667 g, purity: 10%) at 25°C, and the reaction mixture was stirred at 115°C for 12 hours under hydrogen atmosphere. The reaction mixture was filtered, then the filtrate was concentrated under reduced pressure, and the crude product was purified by silica gel column chromatography (petroleum ether: ethyl acetate = 4:1 to 1:1) to obtain compound **38-7.**

**Step 7: Synthesis of compound 38-8**

To a solution of compound **38-7** (0.090 g, 156.36 μmol,1 *eq*) and tetrabutylammonium iodide (11.55 mg, 31.27 μmol, 0.2 *eq*) in *N,N*-dimethylformamide (4 mL) was added sodium sulfide nonahydrate (187.77 mg, 781.78 μmol, 131.31 μL, 5 *eq*) at 25°C, and the reaction mixture was heated to 100°C and stirred continuously and reacted for 2 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by silica gel column chromatography (petroleum ether: ethyl acetate = 5:1) to obtain compound **38-8.**

$^1H$ NMR (400 MHz, $CD_3OD$): δ 7.73-7.67 (m, 1H), 7.56 (d, *J* = 2.0 Hz, 1H), 7.17 (d, *J* = 8.4 Hz, 1H), 3.88 (s, 3H), 3.70 (d, *J* = 11.0 Hz, 2H), 3.46 (d, *J* = 11.0 Hz, 2H).

**Step 8: Synthesis of compound 38-9**

To a solution of compound **38-8** (0.010 g, 37.70 μmol, 1 *eq*) in tetrahydrofuran (2 mL) and water (0.5 mL) was added lithium hydroxide monohydrate (7.91 mg, 188.48 μmol, 5 *eq*) at 25°C, and the reaction mixture was heated to 45°C and stirred for 2 hours. The reaction mixture was added with dilute hydrochloric acid (1 M) to adjust the pH to 5, and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude product of compound **38-9.**

**Step 9: Synthesis of compound 38**

To a solution of compound **38-9** (0.010 g, 39.80 μmol, 1 *eq)* in *N,N*-dimethylformamide (5 mL) was added 2-(7-azabenzotriazol-1-yl)-*N,N,N',N'*-tetramethyluronium hexafluorophosphate (18.16 mg, 47.76 μmol, 1.2 *eq*) and *N,N*-diisopropylethylamine (7.72 mg, 59.70 μmol, 10.40 μL, 1.5 *eq*) respectively at 25°C, and the reaction mixture was stirred for 10 min, then compound **BB-1** (8.21 mg, 39.80 μmol, 1 *eq*) was added thereto, and the reaction mixture was stirred for 30 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile: 29% to 59%, the retention time of the HPLC column was 10 min) to obtain compound **38.**

$^1H$ NMR (400 MHz, $CDCl_3$): δ 8.85-8.17 (m, 1H), 7.54 (s, 1H), 7.38-7.28 (m, 2H), 7.26-7.09 (m, 4H), 7.06-6.97 (m, 2H), 4.17-4.02 (m, 1H), 3.95-3.84 (m, 1H), 3.81-3.67 (m, 4H), 3.62-3.54 (m, 1H), 3.49-3.40 (m, 2H), 3.06-2.89 (m, 3H), 2.87-2.80 (m, 1H), 2.78-2.72 (m, 1H), 2.71-2.58 (m, 1H). MS (ESI) m/z: 440.3 $[M+H]^+$.

**Embodiment 32: Preparation of compound 39**

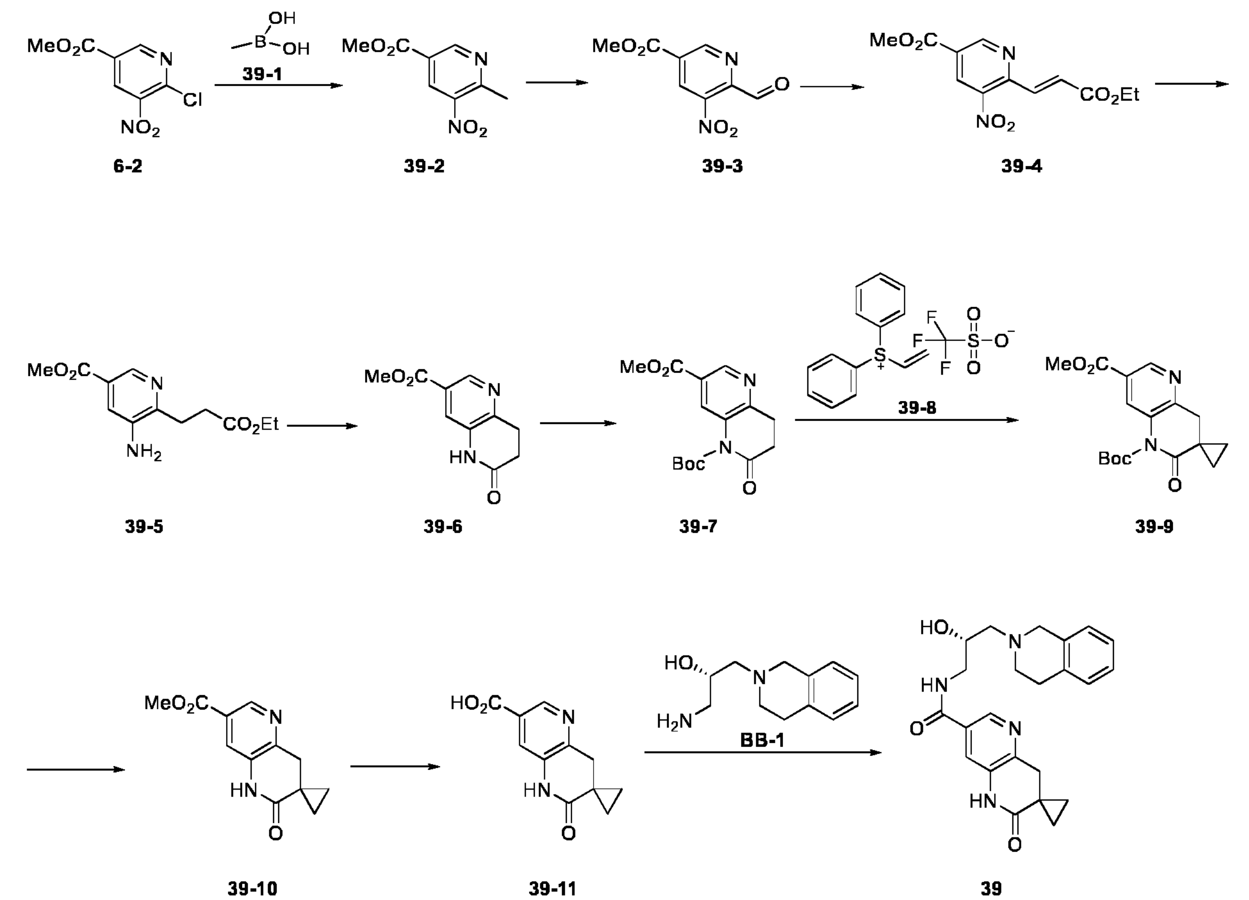


6-2, 39-2, 39-3, 39-4, 39-5, 39-6, 39-7, 39-9, 39-10, 39-11, 39

**Step 1: Synthesis of compound 39-2:**

Compound **6-2** (2.7 g, 12.47 mmol, 1 *eq*), compound **39-1** (820.88 mg, 13.71 mmol, 1.1 *eq*) and potassium carbonate (5.17 g, 37.40 mmol, 3 *eq*) were dissolved in a solution of anhydrous 1,4-dioxane (30 mL), added with tetrakis(triphenylphosphine)palladium (1.44 g, 1.25 mmol, 0.1 *eq*) and the reaction solution 120 and liquid 4 were stirred for 12 hours under nitrogen atmosphere. The reaction mixture was filtered to obtain a filtrate. The filtrate was concentrated under reduced pressure, and purified by thin-layer chromatography (silica gel, petroleum ether/ethyl acetate = 10/1 to 5/1) to obtain compound **39-2.**

$^1H$ NMR (400 MHz, $CDCl_3$): δ 9.29 (d, *J* = 1.6 Hz, 1H), 8.83 (d, *J* = 2.0 Hz, 1H), 4.01 (s, 3H), 2.94 (s, 3H).

**Step 2: Synthesis of compound 39-3:**

Compound **39-2** (1.67 g, 8.51 mmol, 1 *eq*) was dissolved in a solution of 1,4-dioxane (30 mL), then selenium dioxide (1.89 g, 17.03 mmol, 1.85 mL, 2 *eq*) was added, and the reaction mixture was stirred and reacted at 100°C for 20 hours. The reaction mixture was filtered, and the filtrate was concentrated under reduced pressure to obtain compound **39-3.**

**Step 3: Synthesis of compound 39-4:**

Compound **39-3** (0.5 g, 2.38 mmol, 1 *eq*) was dissolved in a solution of anhydrous tetrahydrofuran (40 mL), and diethyl (2-ethoxyacetyl)phosphonate (640.11 mg, 2.86 mmol, 566.47 μL, 1.2 *eq*) and cesium carbonate (930.28 mg, 2.86 mmol, 1.2 *eq*) were added. The reaction mixture was stirred at 20°C for 4.5 hours, added with water (50 mL), and extracted with ethyl acetate (50 mL) three times. The combined organic phases were dried over anhydrous sodium sulfate, filtered, and the filtrate was concentrated under reduced pressure to obtain compound **39-4.**

**Step 4: Synthesis of compound 39-5:**

Compound **39-4** (0.75 g, 2.68 mmol, 1 *eq*) was dissolved in a solution of anhydrous ethanol (10 mL), then palladium on carbon (0.5 g, 3.57 mmol, purity: 10%) was added under nitrogen atmosphere, and the reaction mixture was stirred and reacted at 20°C for 10 hours under hydrogen atmosphere. The reaction mixture was filtered, and the filtrate was concentrated under reduced pressure to obtain compound **39-5.**

**Step 5: Synthesis of compound 39-6:**

Compound **39-5** (0.65 g, 2.58 mmol, 1 *eq*) was dissolved in a solution of anhydrous tetrahydrofuran (20 mL), then glacial acetic acid (773.66 mg, 12.88 mmol, 736.82 μL, 5 *eq*) was added, and the reaction mixture was stirred and reacted at 60°C for 1.5 hours. The reaction mixture was concentrated under reduced pressure to remove the solvent to obtain a crude product. The crude product was added with petroleum ether (20 mL), filtered, and the filter cake was collected, concentrated under reduced pressure and dried to obtain compound **39-6.**

**Step 6: Synthesis of compound 39-7:**

Compound **39-6** (0.25 g, 1.21 mmol, 1 *eq*) was dissolved in anhydrous dichloromethane (5 mL), then di-*tert*-butyl dicarbonate (291.07 mg, 1.33 mmol, 306.39 μL, 1.1 *eq)* and 4-dimethylaminopyridine (74.06 mg, 606.21 μmol, 0.5 *eq*) were added, and the reaction mixture was stirred and reacted at 20°C for 10 min. The reaction mixture was washed once with saturated citric acid aqueous solution (10 mL) and once with saturated brine (10 mL) successively, dried over anhydrous sodium sulfate, filtered, and the filtrate was concentrated under reduced pressure to obtain compound **39-7.**

**Step 7: Synthesis of compound 39-9:**

Compound **39-7** (0.6 g, 1.96 mmol, 1 *eq)* and compound **39-8** (1.06 g, 2.94 mmol, 1.5 *eq*) were dissolved in a solution of anhydrous *N*,*N*-dimethylformamide (3 mL), then 1,8-diazabicycloundec-7-ene (894.60 mg, 5.88 mmol, 885.74 μL, 3 *eq*) was added, and the reaction mixture was stirred at 20°C for 1 hour. The reaction mixture was poured into water (10 mL), and extracted three times with ethyl acetate (10 mL × 3). The combined organic phases were washed three times with saturated brine (10 mL × 3), dried over anhydrous sodium sulfate, and filtered. The filtrate was concentrated under reduced pressure, and purified by silica gel thin-layer chromatography (petroleum ether: ethyl acetate = 1:1) to obtain compound **39-9.**

**Step 8: Synthesis of compound 39-10 hydrochloride:**

Compound **39-9** (80 mg, 240.71 μmol, 1 *eq*) was dissolved in anhydrous 1,4-dioxane (2 mL), then a solution of hydrochloric acid/1,4-dioxane (4 M, 4 mL, 66.47 *eq*) was added, and the reaction mixture was stirred and reacted at 20°C for 0.5 hours. The reaction mixture was concentrated under reduced pressure to obtain compound **39-10** hydrochloride.

**Step 9: Synthesis of compound 39-11:**

Compound **39-10** (50 mg, hydrochloride) was dissolved in a mixed solution of anhydrous tetrahydrofuran (1 mL) and water (1 mL), then lithium hydroxide monohydrate (10.84 mg, 258.36 μmol, 1.2 *eq*) was added, and the reaction mixture was stirred at 20°C for 10 min. The reaction mixture was added with a solution of hydrochloric acid/1,4-dioxane (4 M) to adjust the pH to 6 to 7, and concentrated under reduced pressure to obtain compound **39-11.**

**Step 10: Synthesis of compound 39:**

Compound **39-11** (30 mg, 137.48 μmol, 1 *eq*) was dissolved in *N,N*-dimethylformamide (1 mL), then 2-(7-azabenzotriazol-1-yl)-*N,N,N',N'*-tetramethyluronium hexafluorophosphate (57.50 mg, 151.23 μmol, 1.1 *eq*) and *N,N*-diisopropylethylamine (17.77 mg, 137.48 μmol, 23.95 μL, 1 *eq*) were added. The reaction mixture was stirred at 20°C for 0.5 hours, then compound **BB-1** (36.87 mg, 178.73 μmol, 1.3 *eq*) was added, and the reaction mixture was stirred at 20°C for 1 hour. The reaction mixture was purified by preparative HPLC (chromatographic column: Phenomenex Gemini-NX C18 75×30 mm×3 μm; mobile phase: [water (0.05% ammonia water (v/v))-acetonitrile]; acetonitrile%: 12% to 40%, the retention time of the HPLC column was 7 min) to obtain compound **39.**

$^1$H NMR (400 MHz, $CD_3OD$): δ 8.55 (d, *J* = 2.0 Hz, 1H), 7.64 (d, *J* = 2.0 Hz, 1H), 7.15-7.07 (m, 3H), 7.06-7.00 (m, 1H), 4.20-4.09 (m, 1H), 3.79 (s, 2H), 3.60 (dd, *J* = 5.0, 13.6 Hz, 1H), 3.45 (dd, *J* = 6.8, 13.6 Hz, 1H), 3.12 (s, 2H), 2.97-2.87 (m, 4H), 2.74-2.66 (m, 2H), 1.31 (q, *J* = 3.6, 2H), 0.90 (q, *J* = 3.6, 2H); MS (ESI) m/z: 407.4 $[M+1]^+$.

**Embodiment 33: Preparation of compounds 40 and 41**

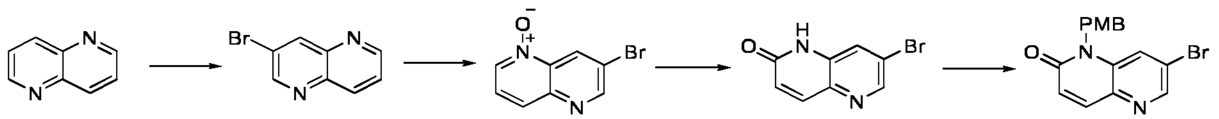

**40-1** **40-2** **40-3** **40-4** **40-5**

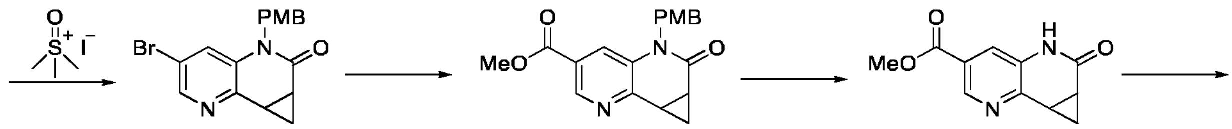

**40-6** **40-7** **40-8**

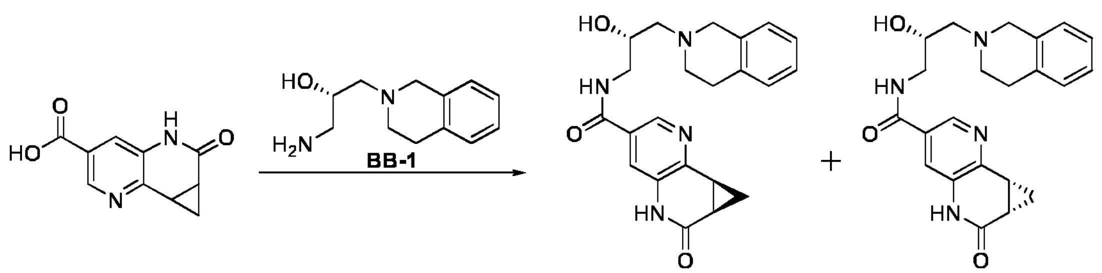


**40-9** **40 or 41** **41 or 40**

**Step 1: Synthesis of compound 40-2:**

Compound **40-1** (5 g, 38.42 mmol, 1 *eq*) and sodium acetate (6.30 g, 76.84 mmol, 2 *eq*) were dissolved in glacial acetic acid (25 mL). Liquid bromine (7.37 g, 46.10 mmol, 2.38 mL, 1.2 *eq*) was weighed and dissolved in glacial acetic acid (10 mL). A solution of liquid bromine in glacial acetic acid was added dropwise to the above reaction mixture at 80°C, and the reaction mixture was stirred and reacted in an oil bath at 80°C for 3 hours. The reaction mixture was added with dichloromethane (50 mL), washed once with water (50 mL), dried over anhydrous sodium sulfate, filtered, and the filtrate was concentrated under reduced pressure. The crude product was purified by silica gel column chromatography (petroleum ether: ethyl acetate = 10:1) to obtain compound **40-2.**

$^1$H NMR (400 MHz, $CDCl_3$): δ 9.05-8.97 (m, 2H), 8.62-8.59 (m, 1H), 8.41 (dd, *J* = 1.2, 8.2 Hz, 1H), 7.68 (dd, *J* = 4.2, 8.6 Hz, 1H).

**Step 2: Synthesis of compound 40-3:**

Compound **40-2** (6.60 g, 31.57 mmol, 1 *eq*) was dissolved in anhydrous dichloromethane (75 mL), then *m*-chloroperoxybenzoic acid (7.69 g, 37.89 mmol, purity: 85%, 1.2 *eq*) was added at 0°C, and the reaction mixture was stirred and reacted at 20°C for 12 hours. The reaction mixture was filtered, and the filtrate was washed with saturated sodium sulfite aqueous solution (50 mL) and sodium bicarbonate aqueous solution (50 mL) successively, dried over anhydrous sodium sulfate, and filtered. The filtrate was concentrated under reduced pressure, and the crude product was purified by silica gel column chromatography (petroleum ether: ethyl acetate = 1:1) to obtain compound **40-3.**

$^1$H NMR (400 MHz, DMSO-$d_6$): δ 9.17 (d, *J* = 2.4 Hz, 1H), 9.05 (d, *J*= 2.0 Hz, 1H), 8.70 (d, *J* = 6.0 Hz, 1H), 8.02 (d, *J* = 8.4 Hz, 1H), 7.76 (dd, *J* = 6.2, 8.8 Hz, 1H).

**Step 3: Synthesis of compound 40-4:**

Compound **40-3** (3.00 g, 13.33 mmol, 1 *eq*) and potassium carbonate (6.45 g, 46.66 mmol, 3.5 *eq*) were dissolved in chloroform (45 mL) and water (15 mL), then *p*-toluenesulfonyl chloride (3.05 g, 16.00 mmol, 1.2 *eq*) was added, and the reaction mixture was stirred and reacted at 20°C for 48 hours. The reaction mixture was concentrated under reduced pressure to remove chloroform, added with water (50 mL), filtered to obtain a filter cake, and concentrated under reduced pressure and dried to obtain compound **40-4.**

$^1$H NMR (400 MHz, DMSO-$d_6$): δ 11.96 (br s, 1H), 8.55 (d, *J* = 2.0 Hz, 1H), 7.92 (d, *J* = 10 Hz, 1H), 7.85 (d, *J* = 1.6 Hz, 1H), 6.78 (d, *J* = 9.6 Hz, 1H).

**Step 4: Synthesis of compound 40-5:**

Compound **40-4** (1.5 g, 6.67 mmol, 1 *eq*) was dissolved in a solution of anhydrous *N,N*-dimethylformamide (10 mL), then sodium hydride (279.92 mg, 7.00 mmol, purity: 60%, 1.05 *eq*) was added, and the reaction mixture was reacted in an oil bath at 40°C for 1 hour. *p*-Methoxybenzyl chloride (1.30 g, 8.33 mmol, 1.13 mL, 1.25 *eq*) was added at 40°C. After the addition was completed, the reaction mixture was reacted in an oil bath at 50°C for 2 hours. The reaction mixture was poured into water (50 mL), and extracted three times with ethyl acetate (50 mL × 3). The combined organic phases were washed three times with saturated brine (50 mL × 3), dried over anhydrous sodium sulfate, and filtered. The filtrate was concentrated under reduced pressure, and purified by column chromatography (silica gel, petroleum ether: ethyl acetate = 1:1) to obtain compound **40-5.**

**Step 5: Synthesis of compound 40-6:**

Sodium hydride (236.37 mg, 5.91 mmol, purity: 60%, 2 *eq*) was dissolved in a solution of dimethyl sulfoxide (15 mL), then trimethylsulfoxonium iodide (1.30 g, 5.91 mmol, 2 *eq*) was added at 20°C, and the reaction mixture was stirred and reacted at 60°C for 1 hour. The reaction mixture was added with compound **40-5** (1.02 g, 2.95 mmol, 1 *eq*), and stirred and reacted at 60°C for 0.5 hours. The reaction mixture was poured into water (50 mL), and extracted twice with ethyl acetate (50 mL × 2). The combined organic phases were washed twice with saturated brine (50 mL × 2), dried over anhydrous sodium sulfate, and filtered. The filtrate was concentrated under reduced pressure, and purified by silica gel column chromatography (petroleum ether: ethyl acetate = 1:3 to 1:1) to obtain compound **40-6.**

$^1$H NMR (400 MHz, $CDCl_3$): δ 8.20 (d, *J* = 1.8 Hz, 1H), 7.25 (d, *J* = 1.8 Hz, 1H), 7.13-7.07 (d, *J* = 8.6 Hz, 2H), 6.86 (d, *J* = 8.6 Hz, 2H), 5.16 (br d, *J* = 15.8 Hz, 1H), 4.90 (br d, *J* = 16.0 Hz, 1H), 3.78 (s, 3H), 2.86-2.79 (m, 1H), 2.54 (m, 1H), 1.80 (m, 1H) , 0.80 (q, *J*= 5.0 Hz, 1H).

**Step 6: Synthesis of compound 40-7:**

Compound **40-6** (0.71 g, 1.98 mmol, 1 *eq*) was dissolved in anhydrous *N,N*-dimethylformamide (10 mL) and anhydrous methanol (15 mL), then [1,1'-bis(diphenylphosphino)ferrocene]palladium dichloride (144.62 mg, 197.65 μmol, 0.1 *eq*) and sodium acetate (486.40 mg, 5.93 mmol, 3 *eq*) were added under argon atmosphere. The reaction mixture was replaced with carbon monoxide three times, and then reacted at 120°C under a pressure of 2 MPa for 18 hours. The reaction mixture was concentrated under reduced pressure, and purified by silica gel column chromatography (petroleum ether: ethyl acetate = 1:3 to 1:1) to obtain compound **40-7.**

**Step 7: Synthesis of compound 40-8:**

Compound **40-7** (0.82 g, 2.42 mmol, 1 *eq)* was dissolved in trifluoroacetic acid (5 mL) and anhydrous dichloromethane (5 mL), then the reaction mixture was cooled to 0°C, and added with trifluoromethanesulfonic acid (1.82 g, 12.12 mmol, 1.07 mL, 5 *eq)*. The reaction mixture was stirred and reacted at 20°C for 10 hours. The reaction mixture was concentrated under reduced pressure to obtain compound **40-8.**

$^1$H NMR (400 MHz, $CDCl_3$): δ 8.83 (d, *J* = 1.6 Hz, 1H), 8.24 (br s, 1H), 7.65 (d, *J* = 1.6 Hz, 1H), 3.96 (s, 3H), 2.90-2.86 (m, 1H), 2.43-2.32 (m, 1H), 1.89 (dt, *J* = 5.2, 9.2 Hz, 1H), 0.93 (q, *J* = 5.2 Hz, 1H).

**Step 8: Synthesis of compound 40-9:**

Compound **40-8** (200 mg, 916.56 μmol, 1 *eq*) was dissolved in anhydrous tetrahydrofuran (10 mL) and water (1 mL), then lithium hydroxide monohydrate (76.92 mg, 1.83 mmol, 2 *eq*) was added, and the reaction mixture was stirred and reacted at 20°C for 1 hour. The reaction mixture was concentrated under reduced pressure to obtain compound **40-9.**

**Step 9: Synthesis of compounds 40 and 41:**

Compound **40-9** (190 mg, 930.54 μmol, 1 *eq*) was dissolved in *N,N*-dimethylformamide (3 mL), then HATU (389.20 mg, 1.02 mmol, 1.1 *eq*) and diisopropylethylamine (132.29 mg, 1.02 mmol, 178.29 μL, 1.1 *eq*) were added. The reaction mixture was stirred at 20°C for 10 min, then compound **BB-1** (191.96 mg, 930.54 μmol, 1 *eq*) was added thereto, and the reaction mixture was stirred at 20°C for 0.5 hours. The reaction mixture was filtered to obtain a filtrate. The filtrate was purified by preparative HPLC (chromatographic column: Waters Xbridge BEH C18 250×50 mm×10 μm; mobile phase: [water (0.05% ammonium hydroxide (v/v))-acetonitrile]; acetonitrile%: 5% to 35%), and then purified by SFC (chromatographic column: DAICEL CHIRALPAK OJ (250 mm×30 mm, 10 μm); mobile phase: 0.1% ammonia water in methanol: 55% to 55%) to obtain compounds **40** and **41.**

Compound **40:** SFC analysis conditions: chromatographic column: Chiralcel OJ-3 50×4.6 mm I.D., 3 μm, mobile phase: mobile phase A was $CO_2$, mobile phase B was methanol (0.05% diethylamine); methanol (0.05% diethylamine): 55% to 55%; flow rate: 3 mL/min; detector: PDA; retention time: 2.216 min, chiral purity = 99.73%. $^1$H NMR (400 MHz, $CDCl_3$): δ 8.64 (br s, 1H), 8.56 (d, *J* =1.6 Hz, 1H), 7.75 (d, *J* = 1.2 Hz, 1H), 7.37 (br s, 1H), 7.20-7.11 (m, 3H), 7.00 (d, *J* = 7.2 Hz, 1H), 4.16-4.11 (m, 1H), 3.90 (br d, *J* = 14.8 Hz, 1H), 3.79-3.71 (m, 2H), 3.59-3.52 (m, 1H), 3.06-2.98 (m, 1H), 2.95 (br d, *J* = 5.6 Hz, 2H), 2.89-2.65 (m, 5H), 2.35-2.24 (m, 1H), 1.84-1.78 (m, 1H), 0.84 (q, *J* = 5.2 Hz,1H); MS (ESI) m/z: 393.3$[M+1]^+$.

Compound **41:** SFC analysis conditions: chromatographic column: Chiralcel OJ-3 50×4.6 mm I.D., 3 μm, mobile phase: mobile phase A was $CO_2$, mobile phase B was methanol (0.05% diethylamine); methanol (0.05% diethylamine): 55% to 55%; flow rate: 3 mL/min; detector: PDA; retention time: 2.508 min, chiral purity = 96.59%. $^1$H NMR (400 MHz, $CDCl_3$): δ 8.60 (s, 1H), 8.42 (br s, 1H), 7.71 (s, 1H), 7.48 (br s, 1H), 7.23-7.10 (m, 3H), 7.02 (d, *J* = 7.6 Hz, 1H), 4.19 (br s, 1H), 4.01 (d, *J* = 15.2 Hz, 1H), 3.89-3.72 (m, 2H), 3.60-3.43 (m, 1H), 3.09 (br s, 1H), 3.03-2.91 (m, 3H), 2.87-2.73 (m, 3H), 2.32-2.24 (m, 1H), 1.84-1.78 (m, 1H), 0.83 (br d, *J* = 4.8 Hz, 1H); MS (ESI) m/z: 393.3$[M+1]^+$.

**Embodiment 34: Preparation of compound 42**

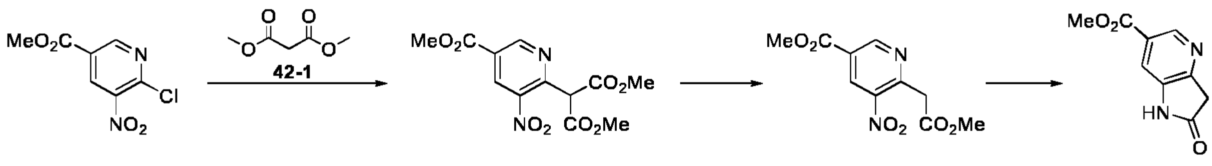


6-2 42-2 42-3 42-4

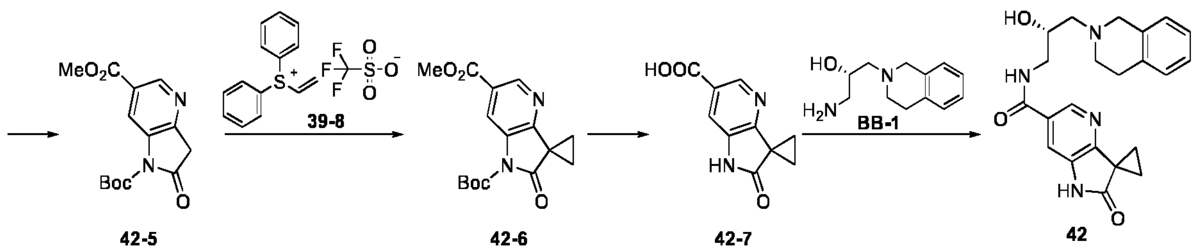


42-5 42-6 42-7 42

**Step 1: Synthesis of compound 42-2:**

Compound **6-2** (5 g, 23.09 mmol, 1 *eq*) was dissolved in *N,N*-dimethylformamide (50 mL), then cesium carbonate (11.28 g, 34.64 mmol, 1.5 *eq*) was added thereto, and the reaction mixture was stirred at 20°C for 30 min. **42-1** (3.66 g, 27.71 mmol, 3.18 mL, 1.2 *eq*) was then added, and the reaction mixture was stirred at 20°C for 10 hours. The reaction mixture was poured into water (150 mL), and extracted with ethyl acetate (100 mL × 3). The organic phase was separated, washed with saturated brine (100 mL × 3), dried over anhydrous sodium sulfate, filtered, and the filtrate was concentrated under reduced pressure to obtain a crude product. The resulting crude product was purified by silica gel column chromatography (petroleum ether: ethyl acetate = 1:0 to 10:1) to obtain compound **42-2.**

**Step 2: Synthesis of compound 42-3**

Compound **42-2** (3 g, 9.61 mmol, 1 *eq*) was dissolved in dimethyl sulfoxide (30 mL) and water (3 mL), then lithium chloride (1.02 g, 24.02 mmol, 491.90 μL, 2.5 *eq*) was added. The reaction mixture was stirred at 120 to 130°C for 2 hours. The reaction mixture was cooled to 20°C, then added with water (100 mL) and ethyl acetate (100 mL). The organic phase was separated, washed with saturated brine (50 mL), dried over anhydrous sodium sulfate, filtered, and the filtrate was concentrated under reduced pressure. The residue was purified by silica gel column chromatography (petroleum ether: ethyl acetate = 10:1 to 4:1) to obtain compound **42-3.**

**Step 3: Synthesis of compound 42-4:**

To a solution of compound **42-3** (800 mg, 3.15 mmol, 1 *eq*) in toluene (15 mL) and acetic acid (0.3 mL) was added Pd/C (500 mg, purity: 10%) under nitrogen atmosphere, then the reaction mixture was replaced with hydrogen three times, and stirred for 10 hours at 112°C under hydrogen (15 psi) atmosphere. The reaction mixture was cooled to 80°C, filtered, and the filter cake was washed with methanol (10 mL). The filtrate was added with saturated sodium bicarbonate aqueous solution (50 mL), and extracted with ethyl acetate (50 mL × 6). The organic phases were combined, washed with water, dried over anhydrous sodium sulfate, filtered, and the filtrate was concentrated under reduced pressure to obtain compound **42-4.**

**Step 4: Synthesis of compound 42-5**

Compound **42-4** (210 mg, 1.09 mmol, 1 *eq*) was dissolved in tetrahydrofuran (5 mL), then di-*tert*-butyl dicarbonate (476.99 mg, 2.19 mmol, 502.09 μL, 2 *eq*) and DMAP (66.75 mg, 546.39 μmol, 0.5 *eq*) were added, and the reaction mixture was stirred at 25°C for 30 min. The reaction mixture was added with water (15 mL) to quench, and extracted with ethyl acetate (15 mL). The organic phase was washed with 1 N hydrochloric acid (10 mL), saturated sodium bicarbonate aqueous solution (10 mL) and saturated brine (10 mL), dried over anhydrous sodium sulfate, filtered, and the filtrate was concentrated under reduced pressure to obtain compound **42-5.**

**Step 5: Synthesis of compound 42-6**

Compound **42-5** (500.00 mg, 2.30 mmol, 1 *eq)* was dissolved in dimethyl sulfoxide (5 mL), then compound **39-8** (409.14 mg, 1.13 mmol, 1.1 *eq*) and 1,8-diazabicycloundec-7-ene (468.76 mg, 3.08 mmol, 464.12 μL, 3 *eq*) were added, and the reaction mixture was stirred at 25°C for 10 min. The reaction mixture was added with water (15 mL) to quench, and extracted with ethyl acetate (15 mL). The organic phase was separated, dried over anhydrous sodium sulfate, filtered, and the filtrate was concentrated under reduced pressure to obtain a crude product. The resulting crude product was purified by preparative thin-layer chromatography (petroleum ether: ethyl acetate = 3:1) to obtain compound **42-6.**

**Step 6: Synthesis of compound 42-7 hydrochloride**

Compound **42-6** (100 mg, 314.15 μmol, 1 *eq*) was dissolved in acetic acid (1 mL) and concentrated hydrochloric acid (1 mL), and the reaction mixture was stirred at 100°C for 10 hours. The reaction mixture was concentrated under reduced pressure to obtain compound **42-7** hydrochloride.

**Step 7: Synthesis of compound 42**

Compound **42-7** (50 mg, hydrochloride) was dissolved in *N,N*-dimethylformamide (2 mL), then 2-(7-azabenzotriazol-1-yl)-*N,N,N',N'*-tetramethyluronium hexafluorophosphate (111.73 mg, 293.86 μmol, 1.2 *eq*) was added, and the reaction mixture was stirred at 25°C for 10 min. The reaction mixture was then added with compound **BB-1** (50.51 mg, 244.88 μmol, 1 *eq*) and *N,N*-diisopropylethylamine (158.25 mg, 1.22 mmol, 213.27 μL, 5 *eq*), and stirred at 25°C for 20 min. The reaction mixture was added with water (10 mL) to quench, and extracted with ethyl acetate (10 mL × 2). The organic phases were combined, dried over anhydrous sodium sulfate, filtered, and the filtrate was concentrated under reduced pressure to obtain a crude product. The resulting crude product was purified by preparative HPLC (chromatographic column: Phenomenex Gemini-NX C18 75×30 mm×3 μm; mobile phase: [water (0.05% ammonia water v/v)-acetonitrile]; acetonitrile%: 10% to 40%, the retention time of the HPLC column was 7 min) to obtain compound **42.**

$^1$H NMR (400 MHz, $CD_3OD$): δ 8.51 (d, *J* = 1.6 Hz, 1H), 7.63 (d, *J* = 2.0 Hz, 1H), 7.03-7.14 (m, 3H), 6.96-7.03 (m, 1H), 4.07-4.18 (m, 1H), 3.72 (s, 2H), 3.57 (dd, *J* = 13.6, 8.4 Hz, 1H), 3.44 (dd, *J* = 13.6, 6.8 Hz, 1H), 2.83-2.94 (m, 4H), 2.61-2.71 (m, 2H), 1.86-1.91 (m, 2H), 1.74-1.80 (m, 2H); MS (ESI) m/z: 393.4[M+1]$^+$.

**Embodiment 35: Preparation of compounds 43 and 44**

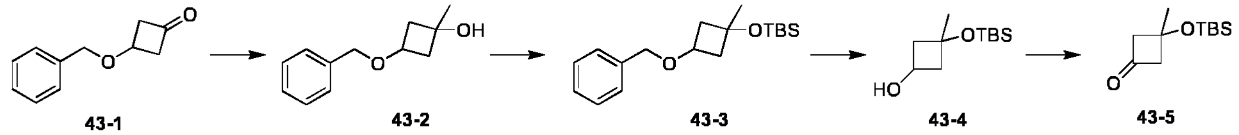

43-1 43-2 43-3 43-4 43-5

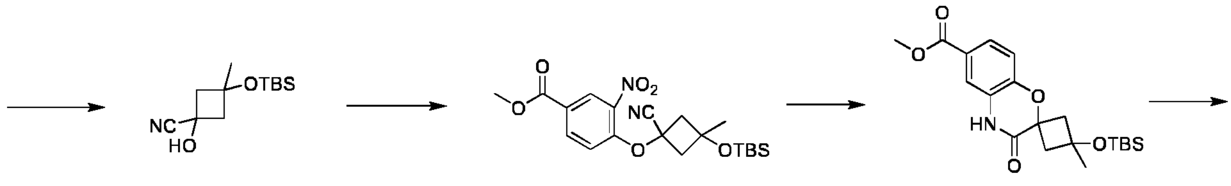

43-6 43-7 43-8

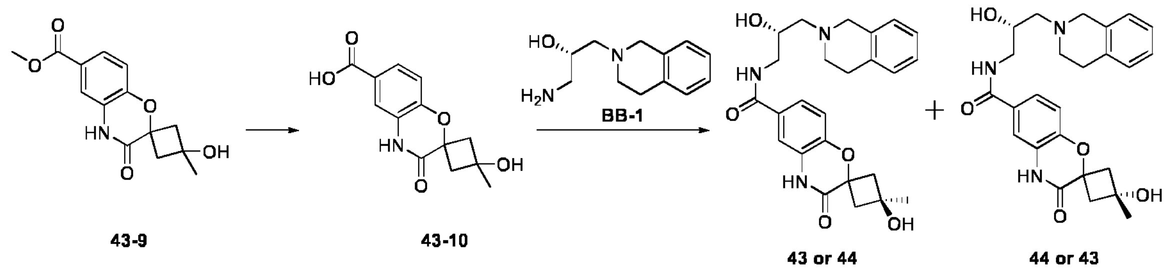

BB-1

+

43-9 43-10 43 or 44 44 or 43

**Step 1: Synthesis of compound 43-2:**

To a solution of compound **43-1** (20 g, 113.50 mmol, 1 *eq*) in tetrahydrofuran (100 mL) was added methylmagnesium bromide (3 M, 113.50 mL, 3 *eq*) at -78°C, and the reaction mixture was warmed to 25°C and stirred continuously for 2 hours. The reaction mixture was slowly added with saturated ammonium chloride solution (100 mL) in an ice bath to quench, and extracted with ethyl acetate (50 mL × 3). The organic phases were combined, washed with saturated brine (50 mL × 2), dried over anhydrous sodium sulfate, filtered, and the filtrate was concentrated under reduced pressure to obtain a crude product of compound **43-2.**

$^1$H NMR (400 MHz, DMSO-$d_6$): δ 7.34-7.26 (m, 5H), 4.44-4.27 (m, 2H), 3.79-3.57 (m, 1H), 3.02-2.76 (m, 1H), 2.31-2.18 (m, 1H), 2.09-1.84 (m, 2H), 1.41-1.13 (m, 3H).

**Step 2: Synthesis of compound 43-3**

To a solution of compound **43-2** (22.45 g, 116.77 mmol, 1 *eq*) and imidazole (23.85 g, 350.32 mmol, 3.0 *eq*) in dichloromethane (200 mL) was added *tert*-butyldimethylsilyl chloride (26.40 g, 175.16 mmol, 21.46 mL, 1.5 *eq)* at 0°C, and the reaction mixture was stirred continuously at 25°C for 12 hours. The reaction mixture was poured into ice water (100 mL), and extracted with dichloromethane (100 mL × 3). The organic phases were combined, washed with saturated brine (100 mL × 2), dried over anhydrous sodium sulfate, filtered, and the filtrate was concentrated under reduced pressure. The crude product was purified by column chromatography (petroleum ether: ethyl acetate = 1:0) to obtain compound **43-3.**

$^1$H NMR (400 MHz, $CDCl_3$): δ 7.36-7.33 (m, 5H), 4.76 (s, 2H), 4.42 (s, 1H), 1.55 (s, 2H), 1.31-1.27 (m, 2H), 0.99-0.94 (m, 9H), 0.12-0.10 (m, 6H), 0.09-0.07 (m, 3H).

**Step 3: Synthesis of compound 43-4**

A mixture of compound **43-3** (19 g, 61.99 mmol, 1 *eq*) and palladium on carbon (1.9 g, purity: 10%) in methanol (4 mL) was stirred and reacted at 25°C for 12 hours under hydrogen atmosphere. The reaction mixture was filtered through diatomite, and the filtrate was concentrated under reduced pressure. The crude product was purified by column chromatography (petroleum ether: ethyl acetate = 3:1) to obtain compound **43-4.**

**Step 4: Synthesis of compound 43-5**

To a solution of compound **43-4** (4 g, 18.49 mmol, 1 *eq*) in dichloromethane (80 mL) was added Dess-Martin periodinane (9.41 g, 22.18 mmol, 6.87 mL, 1.2 *eq*) at 25°C, and the reaction mixture was stirred continuously at 25°C for 12 hours. The reaction mixture was added with a mixed solution of saturated sodium sulfite and saturated sodium bicarbonate (100 mL, 1:1) to quench, and extracted with dichloromethane (40 mL × 3). The organic phases were combined, washed with saturated brine (40 mL × 2), dried over anhydrous sodium sulfate, filtered, and the filtrate was concentrated under reduced pressure to obtain a crude product of compound **43-5.**

$^1H$ NMR (400 MHz, $CDCl_3$): δ 3.24-3.16 (m, 2H), 3.04-2.95 (m, 2H), 1.60 (s, 3H), 0.91-0.89 (m, 9H), 0.14-0.11 (m, 6H).

**Step 5: Synthesis of compound 43-6**

To a solution of compound **43-5** (4.35 g, 20.29 mmol, 1 *eq*) in dichloromethane (50 mL) was added trimethylsilyl cyanide (2.42 g, 24.35 mmol, 3.05 mL, 1.2 *eq*) at 0°C, then boron trifluoride diethyl etherate (2.88 g, 20.29 mmol, 2.50 mL, 1 *eq*) was added, and the reaction mixture was stirred for 1 hour. The reaction mixture was added with saturated sodium bicarbonate solution to adjust the pH to 10, filtered through diatomite, and the filtrate was extracted with dichloromethane (50 mL × 3). The organic phases were combined, washed with saturated brine (50 mL × 2), dried over anhydrous sodium sulfate, filtered, and the filtrate was concentrated under reduced pressure. The crude product was purified by column chromatography (petroleum ether: ethyl acetate = 1:0 to 10:1) to obtain compound **43-6.**

$^1H$ NMR (400 MHz, $CDCl_3$): δ 2.88-2.72 (m, 2H), 2.60-2.49 (m, 1H), 2.47-2.35 (m, 1H), 1.53 (d, *J* = 0.6 Hz, 3H), 0.90-0.87 (m, 9H), 0.11-0.08 (m, 6H).

**Step 6: Synthesis of compound 43-7**

To a solution of compound methyl 4-fluoro-3-nitrobenzoate (0.22 g, 1.10 mmol, 1 *eq*) and compound **43-6** (733.41 mg, 3.04 mmol, 2.75 *eq*) in tetrahydrofuran (20 mL) was added cesium carbonate (899.89 mg, 2.76 mmol, 55.23 μL, 2.5 *eq*) at 25°C, and the reaction mixture was stirred for 12 hours, then heated to 60°C, and stirred continuously for 2 hours. The reaction mixture was diluted with water (20 mL), and extracted with ethyl acetate (20 mL × 3). The organic phases were combined, washed with saturated brine (20 mL × 2), dried over anhydrous sodium sulfate, filtered, and the filtrate was concentrated under reduced pressure. The crude product was purified by column chromatography (petroleum ether: ethyl acetate = 10:1) to obtain compound **43-7.**

$^1H$ NMR (400 MHz, $CDCl_3$): δ 8.57-8.52 (m, 1H), 8.28-8.21 (m, 1H), 7.13 (d, *J* = 8.8 Hz, 1H), 3.96 (s, 3H), 3.21 (br d, *J* = 8.6 Hz, 2H), 3.00-2.97 (m, 2H), 1.64 (s, 3H), 0.87-0.85 (m, 9H), 0.10-0.09 (m, 6H).

**Step 7: Synthesis of compound 43-8**

To a solution of compound **43-7** (0.550 g, 1.31 mmol, 1 *eq*) and ammonium chloride (349.80 mg, 6.54 mmol, 5 *eq*) in ethanol (20 mL) and water (10 mL) was added iron powder (365.19 mg, 6.54 mmol, 5 *eq)* at 80°C, and the reaction mixture was stirred continuously for 12 hours. The reaction mixture was filtered through diatomite, and the filtrate was concentrated under reduced pressure to obtain a crude product of compound **43-8.**

**Step 8: Synthesis of compound 43-9**

To a solution of compound **43-8** (0.320 g, 817.30 μmol, 1 *eq*) in tetrahydrofuran (10 mL) was added dilute hydrochloric acid (2 M, 2.04 mL, 5 *eq*) at 25°C, and the reaction mixture was stirred for 12 hours. The reaction mixture was diluted with water (20 mL), and extracted with ethyl acetate (15 mL × 3). The organic phases were combined, washed with saturated brine (15 mL × 2), dried over anhydrous sodium sulfate, filtered, and the filtrate was concentrated under reduced pressure. The crude product was purified by column chromatography (petroleum ether: ethyl acetate = 5:1 to 1.5:1) to obtain compound **43-9.**

**Step 9: Synthesis of compound 43-10**

To a solution of compound **43-9** (0.1 g, 360.66 μmol, 1 *eq*) in methanol (5 mL) and water (1 mL) was added lithium hydroxide monohydrate (75.67 mg, 1.80 mmol, 5 *eq)* at 25°C, and the reaction mixture was heated to 45°C and stirred for 12 hours. The reaction mixture was added with dilute hydrochloric acid (1 M) to adjust the pH to 5, and extracted with ethyl acetate (10 mL × 3). The organic phases were combined, washed with saturated brine (10 mL × 2), dried over anhydrous sodium sulfate, filtered, and the filtrate was concentrated under reduced pressure to obtain a crude

product of compound **43-10.**

**Step 10: Synthesis of compounds 43 and 44**

To a solution of compound **43-10** (0.1 g, 379.87 μmol, 1 *eq*) in *N,N*-dimethylformamide (5 mL) was added 2-(7-azabenzotriazol-1-yl)-*N,N,N',N'*-tetramethyluronium hexafluorophosphate (173.33 mg, 455.85 μmol, 1.2 *eq*) and *N,N*-diisopropylethylamine (73.64 mg, 569.81 μmol, 99.25 μL, 1.5 *eq*) respectively at 25°C, then the reaction mixture was stirred for 10 min, then compound **BB-1** (117.54 mg, 569.81 μmol, 1.5 *eq*) was added thereto, and the reaction mixture was stirred continuously for 30 min. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile%: 16% to 46%, the retention time of the HPLC column was 10 min) to obtain compounds **43** and **44.**

Compound **43:** SFC analysis conditions: chromatographic column: Chiralpak AD-3 50×4.6 mm I.D., 3 μm, mobile phase: mobile phase A was $CO_2$, mobile phase B was methanol+acetonitrile (0.05% diethylamine); 60% methanol+acetonitrile (0.05% diethylamine); flow rate: 3 mL/min; detector: PDA; retention time: 0.793 min, chiral purity = 100%. $^1H$ NMR (400 MHz, $CD_3OD$): δ 7.37-7.32 (m, 2H), 7.16-7.07 (m, 3H), 7.05-7.00 (m, 1H), 6.97-6.92 (m, 1H), 4.12-4.06 (m, 1H), 3.74 (s, 2H), 3.55-3.42 (m, 2H), 2.95-2.82 (m, 4H), 2.78-2.72 (m, 2H), 2.71-2.61 (m, 2H), 2.45-2.38 (m, 2H), 1.43 (s, 3H); MS (ESI) m/z: 452.3 $[M+1]^+$.

Compound **44:** SFC analysis conditions: chromatographic column: Chiralpak AD-3 50×4.6 mm I.D., 3 μm, mobile phase: mobile phase A was $CO_2$, mobile phase B was methanol+acetonitrile (0.05% diethylamine); 60% methanol+acetonitrile (0.05% diethylamine); flow rate: 3 mL/min; detector: PDA; retention time: 2.872 min, chiral purity = 98.43%. $^1H$ NMR (400 MHz, $CD_3OD$): δ 7.43-7.33 (m, 2H), 7.15-7.07 (m, 3H), 7.04-6.99 (m, 1H), 6.93 (d, *J* = 8.0 Hz, 1H), 4.12-4.06 (m, 1H), 3.73 (s, 2H), 3.56-3.39 (m, 2H), 2.98-2.79 (m, 4H), 2.76-2.58 (m, 4H), 2.38-2.25 (m, 2H), 1.56 (s, 3H); MS (ESI) m/z: 452.3 $[M+1]^+$.

**Embodiment 36: Preparation of compound 45**

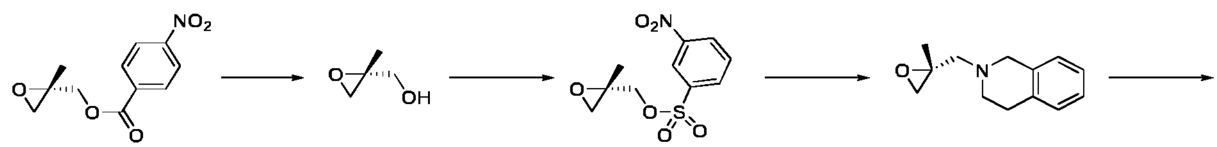

45-1 45-2 45-3 45-4

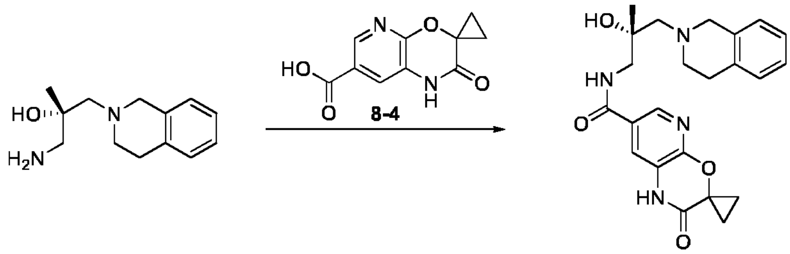

45-5 45

**Step 1: Synthesis of compound 45-2:**

To a mixed solution of compound **45-1** (100.0 mg, 421.57 μmol, 1 *eq*) in tetrahydrofuran (1 mL) and water (0.2 mL) was added lithium hydroxide monohydrate (26.5 mg, 632.36 μmol, 1.5 *eq*), and the reaction mixture was stirred at 20°C for 1 hour. The reaction mixture was added with dichloromethane (5 mL) and anhydrous sodium sulfate (5.00 g), stirred and dried for 20 min, and filtered to obtain a crude filtrate containing compound **45-2,** which was directly used in the next reaction step.

**Step 2: Synthesis of compound 45-3:**

To a solution of compound **45-2** (crude filtrate) in dichloromethane (5 mL) was added triethylamine (84.9 mg, 839.91 μmol, 116.91 μL, 2 *eq),* 4-dimethylaminopyridine (513.0 μg, 4.20 μmol, 0.01 *eq)* and 3-nitrobenzenesulfonyl chloride (93.0 mg, 419.95 μmol, 1 *eq),* and the reaction mixture was stirred at 20°C for 16 hours. The reaction mixture was diluted with water (30 mL), and extracted with dichloromethane (30 mL × 2). The organic phases were combined, washed with water (30 mL), dried over anhydrous sodium sulfate, filtered, and the filtrate was concentrated under reduced pressure. The crude product was purified by preparative thin-layer chromatography (silica gel, petroleum ether: ethyl acetate = 1:1) to obtain compound **45-3.**

**Step 3: Synthesis of compound 45-4**

To a solution of tetrahydroisoquinoline (30.0 mg, 225.24 μmol, 28.30 μL, 1 *eq*) and compound **45-3** (67.7 mg, 247.77 μmol, 1.1 *eq*) in tetrahydrofuran (3 mL) was added potassium fluoride (52.3 mg, 900.97 μmol, 21.11 μL, 4 *eq*), and the reaction mixture was stirred at 30°C for 16 hours. The reaction mixture was filtered, then the filtrate was concentrated under reduced pressure, and the crude product was purified by silica gel column chromatography (petroleum ether: ethyl acetate = 5:1) to obtain compound **45-4.**

**Step 4: Synthesis of compound 45-5**

Ammonia gas was introduced into ethanol (10 mL) at -70°C for 15 min, then compound **45-4** (40.0 mg, 196.77 μmol, 1 *eq*) was added, and the reaction mixture was stirred at 80°C for 2 hours. The reaction mixture was concentrated under reduced pressure to obtain a crude product of **45-5,** which was directly used in the next reaction step.

**Step 5: Synthesis of compound 45**

To a solution of compound **8-4** (15.0 mg, 68.13 μmol, 1 *eq*) in *N,N*-dimethylformamide (1.00 mL) was added HATU (28.4 mg, 74.94 μmol, 1.1 *eq*) and diisopropylethylamine (17.6 mg, 136.25 μmol, 23.73 μL, 2 *eq*), then the reaction mixture was stirred at 19°C for 0.2 hours, then compound **45-5** (20.0 mg, 90.78 μmol, 1.33 *eq*) was added, and the reaction mixture was stirred continuously for 0.1 hours. The reaction mixture was diluted with water (10 mL), and extracted with ethyl acetate (10 mL × 2). The organic phases were combined, washed with water (10 mL), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: water (0.05% ammonia water)-acetonitrile; acetonitrile%: 18% to 48%) to obtain compound **45.**

$^1H$ NMR (400 MHz, $CDCl_3$) δ = 8.27 (d, *J* = 2.0 Hz, 2H), 7.32-7.28 (m, 1H), 7.28-7.27 (m, 1H), 7.23-7.17 (m, 2H), 7.06-7.02 (m, 1H), 4.16-4.07 (m, 1H), 3.86 (dd, *J* = 6.4, 13.6 Hz, 1H), 3.78 (br d, *J* = 15.2 Hz, 1H), 3.46-3.38 (m, 1H), 3.12-3.04 (m, 1H), 3.01-2.89 (m, 3H), 2.80-2.71 (m, 2H), 1.54-1.50 (m, 2H), 1.41-1.35 (m, 2H), 1.30 (s, 3H). MS (ESI) m/z: 423.3 $[M+1]^+$.

**Embodiment 37: Preparation of compounds 46a and 46b**

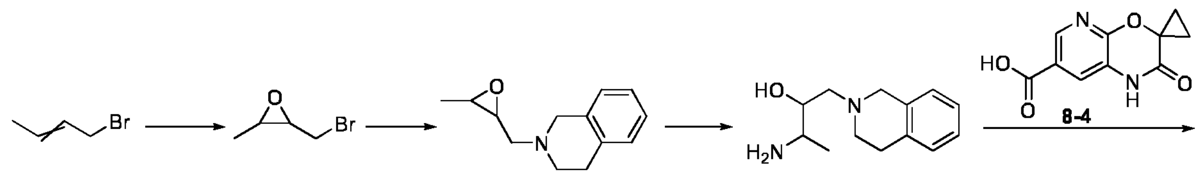


46-1 46-2 46-3 46-4

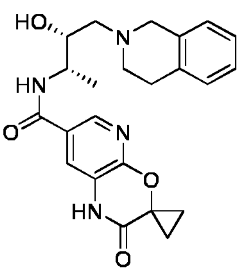

46a or 46b or 46c or 46d

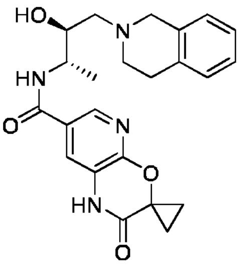

46b or 46a or 46c or 46d

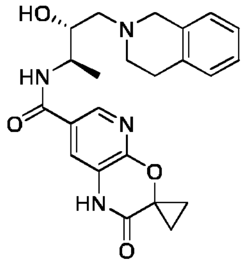

46c or 46a or 46b or 46d

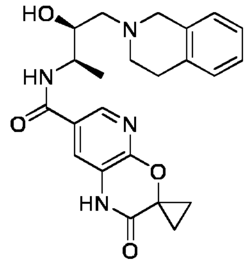

46d or 46a or 46b or 46c

### Step 1: Synthesis of compound 46-2

To a solution of compound **46-1** (20 g, 148.15 mmol, 15.27 mL, 1 *eq)* in anhydrous dichloromethane (100 mL) was added m-chloroperoxybenzoic acid (33.08 g, 162.96 mmol, content: 85%, 1.1 *eq)* at 0°C, and the reaction mixture was stirred at 20°C for 12 hours. The reaction mixture was filtered, then the filtrate was washed with saturated sodium sulfite (100 mL) and sodium bicarbonate (100 mL × 2), and concentrated under reduced pressure to remove most of the solvent to obtain a solution of compound **46-2** in dichloromethane.

$^1H$ NMR (400 MHz, $CDCl_3$) δ = 3.41-3.35 (m, 1H), 3.34-3.28 (m, 1H), 2.97 (dt, *J* = 1.6, 6.0 Hz, 1H), 2.92 (dq, *J* = 1.6, 5.2 Hz, 1H), 1.32 (d, *J* = 5.2 Hz, 3H).

### Step 2: Synthesis of compound 46-3

Sodium hydroxide (3.00 g, 75.08 mmol, 2 *eq*) was dissolved in a solution of *N,N*-dimethylformamide (20 mL) at 0°C, then tetrahydroisoquinoline (5 g, 37.54 mmol, 4.72 mL, 1 *eq*) was added, and the reaction mixture was stirred at 0°C for 10 min. Compound **46-2** (18.38 g, 45.05 mmol, 37% dichloromethane solution, 1.2 *eq*) was then added, and the reaction mixture was stirred at 20°C for 1 hour. The reaction mixture was poured into water (100 mL), and extracted with ethyl acetate (50 mL × 3). The combined organic phases were washed with water (50 mL × 3), dried over anhydrous sodium sulfate, filtered, and the filtrate was concentrated under reduced pressure. The crude product was purified by silica gel column chromatography (petroleum ether: ethyl acetate = 1:1) to obtain compound **46-3.**

### Step 3: Synthesis of compound 46-4

Compound **46-3** (2.2 g, 10.82 mmol, 1 *eq*) was dissolved in a solution of anhydrous ethanol (10 mL), then ammonia water (27.30 g, 194.75 mmol, 30 mL, purity: 25%, 17.99 *eq)* was added, and the reaction mixture was put into a tank and stirred at 100°C for 10 hours. The reaction mixture was concentrated under reduced pressure, and purified by reversed-phase preparative HPLC (chromatographic column: Waters Xbridge BEH C18 250×50 mm×10 μm; mobile phase: [water (0.05% ammonium hydroxide (v/v))-acetonitrile]; acetonitrile%: 25% to 55%, the retention time of the HPLC column was 17 min) to obtain a crude product of compound **46-4.**

### Step 4: Synthesis of compounds 46a and 46b

Compound **8-4** (0.1 g, 454.17 μmol, 1 *eq*) was dissolved in *N,N*-dimethylformamide (1 mL), then HATU (189.96 mg, 499.59 μmol, 1.1 *eq*) and diisopropylethylamine (70.44 mg, 545.01 μmol, 94.93 μL, 1.2 *eq*) were added. The reaction mixture was stirred at 20°C for 10 min, then compound **46-4** (150.09 mg, 681.26 μmol, 1.5 *eq)* was added thereto, and the reaction mixture was stirred at 20°C for 0.5 hours. The crude product was purified by preparative HPLC (chromatographic column: Phenomenex Gemini-NX C18 75×30 mm×3 μm; mobile phase: [water (0.05% ammonium hydroxide (v/v))-acetonitrile]; acetonitrile%: 12% to 42%, the retention time of the HPLC column was 10 min), for the first time was chirally purified (chiral chromatography column: DAICEL CHIRALCEL OJ (250 mm×30 mm, 10 μm); mobile phase: mobile phase A was $CO_2$, mobile phase B was a solution of 0.1% ammonia water in methanol; gradient: B%: 30% to

30%, cycle time: 2 min, duration: 100 min) to obtain a mixture of compounds **46a** and **46b** (analysis conditions: chiral chromatographic column: CHIRALCEL OJ-3 (50 mm×4.6 mm, 3 μm); mobile phase: mobile phase A was $CO_2$, mobile phase B was a solution of 0.05% diethylamine in methanol; gradient: B%: 5% to 40% , the retention times of the two peaks were analyzed to be 1.784 min and 1.849 min respectively, which were too close to be completely separated); for the second time was chirally purified (chiral chromatographic column: DAICEL CHIRALPAK AD (250 mm×30 mm, 10 μm, mobile phase: mobile phase A was $CO_2$, mobile phase B was a solution of 0.1% ammonia water in ethanol; gradient: B%: 70% to 70%, cycle time: 5.7 min, duration: 60 min) to obtain compound **46a** and compound **46b.**

Compound **46b** (chiral analysis conditions: chromatographic column: Chiralpak AD-3 50×4.6 mm, 3 μm, mobile phase: mobile phase A was $CO_2$, mobile phase B was ethanol (0.05% diethylamine); gradient: B%: 60%, retention time: 1.078 min, chiral purity = 100%): $^1$H NMR (400 MHz, $CDCl_3$) δ = 8.27 (s, 1H), 7.84 (s, 1H), 7.70 (d, *J*= 8.2 Hz, 1H), 7.23-7.08 (m, 4H), 7.03 (br d, *J* = 7.4 Hz, 1H), 4.33-4.29 (m, 1H), 4.17-4.12 (m, 1H), 4.06 (d, *J* = 15.2 Hz, 1H), 3.83 (d, *J* = 15.2 Hz, 1H), 3.14-3.04 (m, 2H), 3.01-2.95 (m, 2H), 2.86-2.81 (m, 2H), 1.50-1.46 (m, 2H), 1.36-1.32 (m, 2H), 1.28 (d, *J* = 6.8 Hz, 3H); MS (ESI) m/z: 423.4 $[M+H]^+$.

Compound **46a** (chiral analysis conditions: chromatographic column: Chiralpak AD-3 50×4.6 mm, 3 μm, mobile phase: mobile phase A was $CO_2$, mobile phase B was ethanol (0.05% diethylamine); gradient: B%: 60%, retention time: 2.380 min, chiral purity = 100%): $^1$H NMR (400 MHz, $CDCl_3$) δ = 8.35 (s, 1H), 7.88 (s, 1H), 7.82 (brs, 1H), 7.25-7.14 (m, 3H), 7.07 (d, *J* = 7.2 Hz, 1H), 4.32-4.22 (m, 3H), 4.06 (d, *J* = 14.4 Hz, 1H), 3.32-3.18 (m, 2H), 3.10-2.96 (m, 4H), 1.50-1.38 (m, 2H), 1.34-1.24 (m, 5H); MS (ESI) m/z: 423.4 $[M+H]^+$.

**Embodiment 38: Preparation of compound 47**

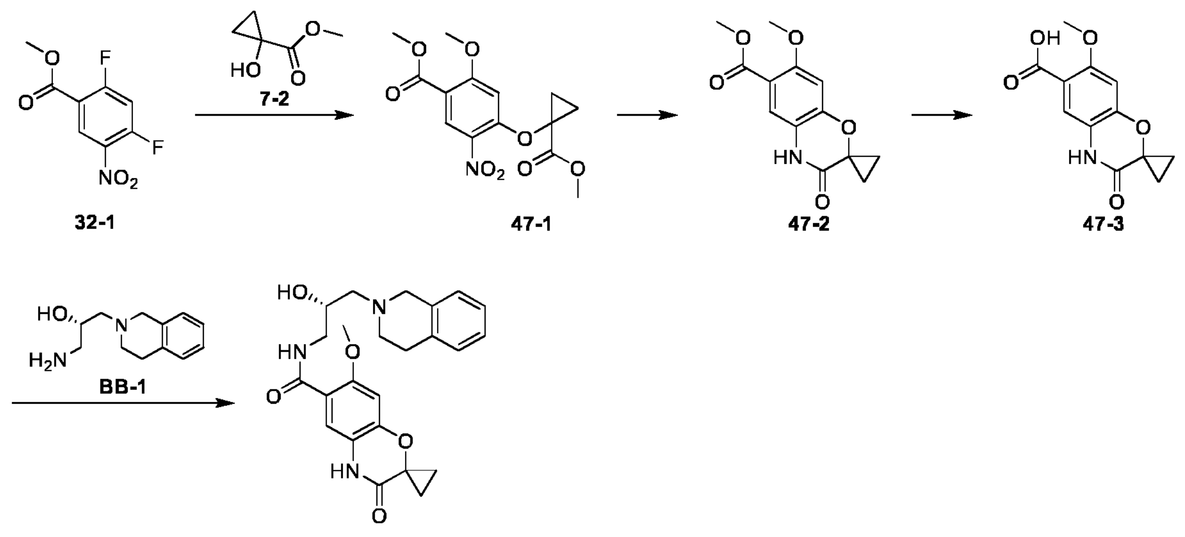


32-1 47-1 47-2 47-3

47

**Step 1: Synthesis of compound 47-1**

To a solution of compound **32-1** (0.5 g, 2.30 mmol, 1 *eq*) and compound **7-2** (267.39 mg, 2.30 mmol, 1 *eq*) in tetrahydrofuran (10 mL) was added cesium carbonate (2.25 g, 6.91 mmol, 3.0 *eq*) at 25°C, and the reaction mixture was heated to 45°C and stirred for 2 hours, then sodium methoxide (373.22 mg, 6.91 mmol, 3.0 *eq)* was added thereto, and the reaction mixture was heated to 60°C and stirred for 24 hours. The reaction mixture was filtered through diatomite, and the filtrate was concentrated under reduced pressure to obtain a crude product of compound **47-1.**

**Step 2: Synthesis of compound 47-2**

To a solution of compound **47-1** (0.630 g, 1.94 mmol, 1 *eq*) and ammonium chloride (518.02 mg, 9.68 mmol, 5 *eq*) in ethanol (200 mL) and water (10 mL) was added iron powder (540.82 mg, 9.68 mmol, 5 *eq*) at 80°C, and the reaction mixture was stirred and reacted for 12 hours. The reaction mixture was filtered through diatomite, then the filtrate was diluted with water (15 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by column chromatography (petroleum ether: ethyl acetate =

5/1 to 1/1) to obtain compound **47-2.**

**Step 3: Synthesis of compound 47-3**

To a solution of compound **47-2** (0.5 g, 1.90 mmol, 1 *eq*) in methanol (20 mL) and water (2 mL) was added lithium hydroxide monohydrate (398.49 mg, 9.50 mmol, 5 *eq)* at 25°C, and the reaction mixture was heated to 45°C and stirred for 12 hours. The reaction mixture was added with dilute hydrochloric acid (1 mol/L) to adjust the pH to 3, and extracted with ethyl acetate (15 mL × 3). The organic phases were combined, washed with saturated brine (15 mL × 2), dried over anhydrous sodium sulfate, filtered, and the filtrate was concentrated under reduced pressure to obtain a crude product of compound **47-3.**

$^1H$ NMR (400 MHz, DMSO-$d_6$) δ = 12.42 (br s, 1H), 10.73 (s, 1H), 7.34 (s, 1H), 6.68 (s, 1H), 3.74 (s, 3H), 1.32-1.25 (m, 2H), 1.21-1.15 (m, 2H).

**Step 4: Synthesis of compound 47**

To a solution of compound **47-3** (0.050 g, 200.63 μmol, 1 *eq*) in *N,N*-dimethylformamide (2 mL) was added HATU (91.54 mg, 240.75 μmol, 1.2 *eq*) and diisopropylethylamine (38.89 mg, 300.94 μmol, 52.42 μL, 1.5 *eq)* at 25°C, and the reaction mixture was stirred for 10 min, then compound **BB-1** (62.08 mg, 300.94 μmol, 1.5 *eq*) was added thereto, and the reaction mixture was stirred continuously for 12 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Phenomenex Gemini-NX C18 75×30 mm×3 μm; mobile phase: [water (10 mM ammonium bicarbonate)-acetonitrile]; acetonitrile%: 2% to 52%) to obtain compound **47.**

MS (ESI) m/z: 438.0 $[M+H]^+$; $^1H$ NMR (400 MHz, DMSO-$d_6$) δ = 8.23 (t, *J* = 5.2 Hz, 1H), 7.55 (s, 1H), 7.13-7.07 (m, 3H), 7.05-6.98 (m, 1H), 6.72 (s, 1H), 4.94 (d, *J* = 4.8 Hz, 1H), 3.95-3.84 (m, 1H), 3.79 (s, 3H), 3.65-3.57 (m, 2H), 3.55-3.46 (m, 1H), 3.28-3.21 (m, 1H), 2.86-2.76 (m, 3H), 2.75-2.65 (m, 3H), 1.34-1.27 (m, 2H), 1.22-1.13 (m, 2H).

**Embodiment 39: Preparation of compounds 48a and 48b**

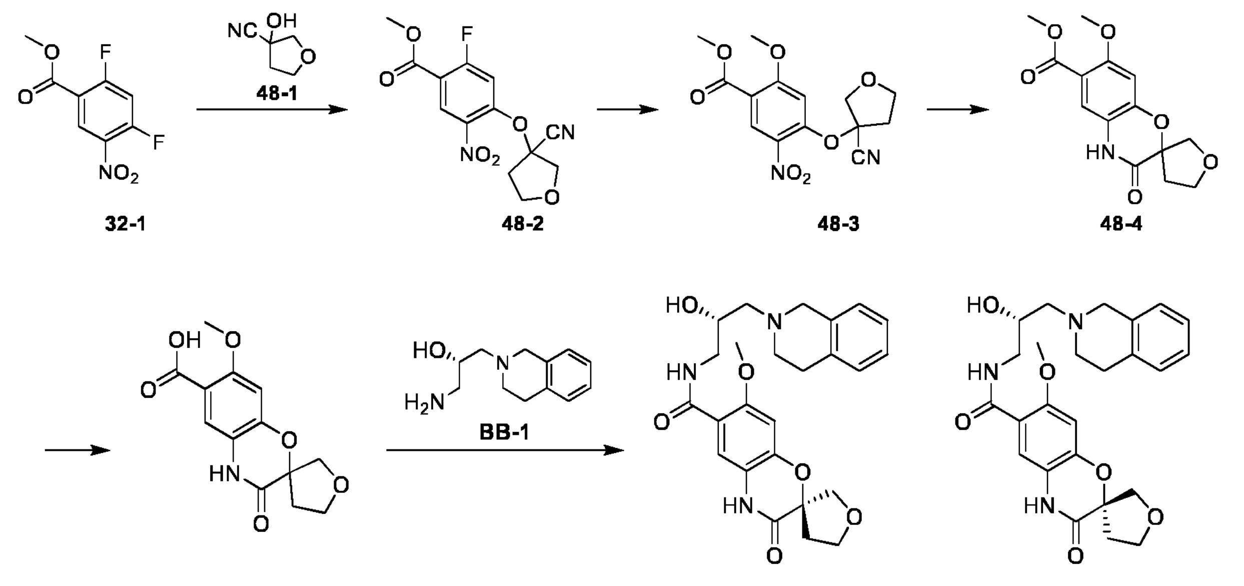


32-1 48-2 48-3 48-4

48-5 48a or 48b 48b or 48a

**Step 1: Synthesis of compound 48-2**

To a solution of compound **32-1** (0.5 g, 2.30 mmol, 1 *eq)* and compound **48-1** (390.24 mg, 3.45 mmol, 1.5 *eq)* in tetrahydrofuran (10 mL) was added cesium carbonate (1.50 g, 4.60 mmol, 2.0 *eq)* at 25°C, and the reaction mixture was stirred for 12 hours, then heated to 45°C, and stirred for 12 hours. The reaction mixture was diluted with water (10

mL), and extracted with ethyl acetate (10 mL × 3). The organic phases were combined, washed with saturated brine (10 mL × 3), dried over anhydrous sodium sulfate, filtered, and the filtrate was concentrated under reduced pressure. The crude product was purified by column chromatography (petroleum ether/ethyl acetate = 3/1 to 1/1) to obtain compound **48-2.**

$^1H$ NMR (400 MHz, $CDCl_3$) δ = 8.62 (d, *J* = 7.6 Hz, 1H), 7.15 (d, *J* = 10.8 Hz, 1H), 4.34 (s, 2H), 4.13 (dd, *J* = 6.4, 7.4 Hz, 2H), 3.98 (s, 3H), 2.77-2.70 (m, 2H).

**Step 2: Synthesis of compound 48-3**

To a solution of compound **48-2** (0.166 g, 535.08 μmol, 1 *eq)* in tetrahydrofuran (10 mL) was added sodium methoxide (144.53 mg, 2.68 mmol, 5 *eq)* at 25°C, and the reaction mixture was heated to 45°C and stirred for 12 hours. The reaction mixture was filtered through diatomite, and the filtrate was concentrated under reduced pressure. The crude product was purified by column chromatography (petroleum ether: ethyl acetate = 3:1 to 1:1) to obtain compound **48-3.**

**Step 3: Synthesis of compound 48-4**

To a solution of compound **48-3** (0.158 g, 490.27 μmol, 1 *eq*) in ethanol (5 mL) and water (5 mL) was added ammonium chloride (131.13 mg, 2.45 mmol, 5 *eq*) at 25°C, and the reaction mixture was heated to 80°C, then iron powder (136.90 mg, 2.45 mmol, 5 *eq)* was slowly added thereto, and the reaction mixture was stirred at 80°C for 12 hours. The reaction mixture was filtered through diatomite, and the filtrate was extracted with ethyl acetate (10 mL × 3). The organic phases were combined, washed with saturated brine (10 mL), dried over anhydrous sodium sulfate, filtered, and the filtrate was concentrated under reduced pressure to obtain a crude product of compound **48-4.**

$^1H$ NMR (400 MHz, DMSO-$d_6$) δ = 10.86 (s, 1H), 7.33 (s, 1H), 6.83 (s, 1H), 4.04-3.95 (m, 2H), 3.94-3.85 (m, 2H), 3.81 (s, 3H), 3.75 (s, 3H), 2.46-2.38 (m, 1H), 2.26-2.09 (m, 1H).

**Step 4: Synthesis of compound 48-5**

To a solution of compound **48-4** (0.107 g, 364.85 μmol, 1 *eq)* in tetrahydrofuran (4 mL), water (2 mL) and methanol (5 mL) was added lithium hydroxide monohydrate (45.93 mg, 1.09 mmol, 3 *eq)* at 25°C, and the reaction mixture was heated to 45°C and stirred for 12 hours. The pH of the reaction mixture was adjusted to 6, and the reaction mixture was extracted with ethyl acetate (20 mL × 2). The organic phases were combined, dried over anhydrous sodium sulfate, filtered, and the filtrate was concentrated under reduced pressure to obtain a crude product of compound **48-5.**

**Step 5: Synthesis of compounds 48a and 48b**

To a solution of compound **48-5** (0.045 g, 161.15 μmol, 1 *eq)* in *N,N*-dimethylformamide (2 mL) was added HATU (91.54 mg, 240.75 μmol, 1.2 *eq)* and diisopropylethylamine (31.24 mg, 241.72 μmol, 42.10 μL, 1.5 *eq)* at 25°C, the reaction mixture was stirred for 10 min, then compound **BB-1** (49.86 mg, 241.72 μmol, 1.5 *eq)* was added thereto, and the reaction mixture was stirred continuously for 12 hours. The reaction mixture was diluted with water (10 mL), and extracted with ethyl acetate (10 mL × 3). The organic phases were combined, washed with saturated brine (10 mL × 2), dried over anhydrous sodium sulfate, filtered, and the filtrate was concentrated under reduced pressure. The crude product was purified by preparative HPLC (chromatographic column: Waters Xbridge 150×25 mmx5 μm; mobile phase: [water (10 mM ammonium bicarbonate)-acetonitrile]; acetonitrile%: 25% to 55%), and then purified by SFC (chromatographic column: DAICEL CHIRALCEL OJ (250 mm×30 mm, 10 μm); mobile phase: phase A: $CO_2$, phase B: [0.1% ammonia water-methanol]; gradient: B%: 35% to 35%, 3.0 min; 130 min) to obtain compounds **48a** and **48b.**

Compound **48a:** (chiral analysis conditions: chromatographic column: OJ-3 50×4.6 mm, I.D. 3 μm, mobile phase: mobile phase A was $CO_2$, mobile phase B was methanol (0.05% diethylamine); gradient: B%: 5% to 40%, retention time: 2.075 min, chiral purity = 100%); MS (ESI) m/z: 468.0 $[M+H]^+$; $^1H$ NMR (400 MHz, $CD_3OD$) δ = 7.59 (s, 1H), 7.17-7.05 (m, 3H), 7.03-6.98 (m, 1H), 6.81 (s, 1H), 4.15-4.04 (m, 3H), 4.02-3.96 (m, 2H), 3.88 (s, 3H), 3.72 (s, 2H), 3.62 (dd, *J* = 4.8, 13.6 Hz, 1H), 3.43 (dd, *J*= 6.6, 13.6 Hz, 1H), 2.94-2.88 (m, 2H), 2.87-2.81 (m, 2H), 2.64 (d, *J* = 6.4 Hz, 2H), 2.57- 2.44 (m, 1H), 2.32-2.19 (m, 1H).

Compound **48b:** (chiral analysis conditions: chromatographic column: OJ-3 50×4.6 mm, I.D. 3 μm, mobile phase: mobile phase A was $CO_2$, mobile phase B was methanol (0.05% diethylamine); gradient: B%: 5% to 40%, retention time: 2.185 min, chiral purity = 98.19%); MS (ESI) m/z: 468.0 $[M+H]^+$; $^1H$ NMR (400 MHz, $CD_3OD$) δ = 7.59 (s, 1H), 7.14-7.06 (m, 3H), 7.01 (br d, *J* = 6.0 Hz, 1H), 6.80 (s, 1H), 4.15-4.04 (m, 3H), 4.02-3.95 (m, 2H), 3.88 (s, 3H), 3.71 (s, 2H), 3.63 (dd, *J* = 4.6, 13.6 Hz, 1H), 3.42 (dd, *J* = 6.8, 13.6 Hz, 1H), 2.93-2.80 (m, 4H), 2.63 (d, *J* = 6.0 Hz, 2H), 2.56-2.42 (m, 1H), 2.31-2.21 (m, 1H).

**Embodiment 40: Preparation of compounds 49a and 49b**

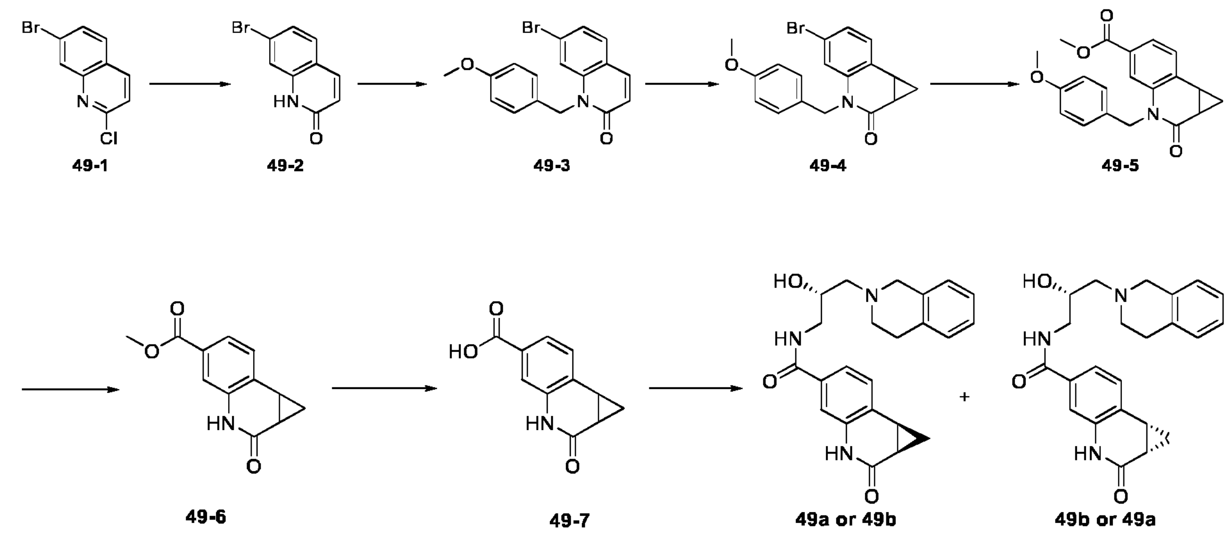

**49-1** **49-2** **49-3** **49-4** **49-5**

**49-6** **49-7** **49a or 49b** **49b or 49a**

**Step 1: Synthesis of compound 49-2**

A solution of compound **49-1** (3.5 g, 14.43 mmol, 1 *eq)* in hydrochloric acid (50 mL, 5 mol/L) and dioxane (5 mL) was stirred at 100°C for 12 hours. The reaction mixture was cooled to room temperature, filtered, and the filter cake was washed with water and dried to obtain compound **49-2.**

**Step 2: Synthesis of compound 49-3**

To a solution of compound **49-2** (3 g, 13.39 mmol, 1 *eq)* in *N,N*-dimethylformamide (50 mL) was added sodium hydride (803.30 mg, 20.08 mmol, purity: 60%, 1.5 *eq*) and *p*-methoxybenzyl chloride (2.52 g, 16.07 mmol, 2.19 mL, 1.2 *eq)* in batches at 0°C under nitrogen atmosphere, and the reaction mixture was warmed to 25°C and stirred for 12 hours. The reaction mixture was added with saturated ammonium chloride solution (30 mL) to quench, and extracted with ethyl acetate (20 mL × 3). The organic phases were combined, washed with saturated brine (20 mL × 2), dried over anhydrous sodium sulfate, filtered, and the filtrate was concentrated under reduced pressure. The crude product was purified by column chromatography (petroleum ether/ethyl acetate = 5/1 to 1/1) to obtain compound **49-3.**

$^1$H NMR (400 MHz, $CDCl_3$) $\delta$ = 7.67 (d, *J* = 9.6 Hz, 1H), 7.49 (d, *J* = 1.6 Hz, 1H), 7.41 (d, *J* = 8.0 Hz, 1H), 7.30 (dd, *J*= 1.8, 8.0 Hz, 1H), 7.23-7.15 (m, 2H), 6.89-6.83 (m, 2H), 6.79 (d, *J* = 9.6 Hz, 1H), 5.44 (br s, 2H), 3.78 (s, 3H).

**Step 3: Synthesis of compound 49-4**

To a solution of trimethylsulfoxonium iodide (1.92 g, 8.72 mmol, 3 *eq)* in tetrahydrofuran (10 mL) was added n-butyllithium (2.5 M, 3.49 mL, 3 *eq)* at 0°C, and the reaction mixture was stirred for 30 min, then a solution of compound **49-3** (1 g, 2.91 mmol, 1 *eq)* in tetrahydrofuran (10 mL) was added thereto, and the reaction mixture was heated to 25°C and stirred for 12 hours. The reaction mixture was added with saturated ammonium chloride solution (40 mL) to quench, and extracted with ethyl acetate (30 mL × 3). The organic phases were combined, washed with saturated brine (30 mL × 2), dried over anhydrous sodium sulfate, filtered, and the filtrate was concentrated under reduced pressure. The crude product was purified by column chromatography (petroleum ether/ethyl acetate = 3/1 to 1/1) to obtain compound **49-4.**

$^1$H NMR (400 MHz, $CDCl_3$) $\delta$ = 7.21 (d, *J* = 8.0 Hz, 1H), 7.13 (d, *J* = 8.8 Hz, 2H), 7.09 (dd, *J* = 2.0, 8.0 Hz, 1H), 7.02 (d, *J* = 1.6 Hz, 1H), 6.89-6.82 (m, 2H), 5.29-5.12 (m, 1H), 5.02-4.83 (m, 1H), 3.79 (s, 3H), 2.59-2.48 (m, 1H), 2.46-2.36 (m, 1H), 1.71-1.60 (m, 1H), 0.67 (q, *J* = 5.2 Hz, 1H).

**Step 4: Synthesis of compound 49-5**

To an autoclave containing a solution of compound **49-4** (1 g, 2.79 mmol, 1 *eq)* and triethylamine (621.44 mg, 6.14 mmol, 854.79 μL, 2.2 *eq*) in *N,N*-dimethylformamide (10 mL) and methanol (5 mL) was added Pd(dppf)$Cl_2$ (306.38 mg, 418.73 μmol, 0.15 *eq)* under nitrogen atmosphere, then the reaction mixture was replaced with carbon monoxide,

and finally stirred and reacted continuously at 100°C for 24 hours under carbon monoxide atmosphere (2 MPa). The reaction mixture was filtered through diatomite, then the filtrate was diluted with water (20 mL), and extracted with ethyl acetate (20 mL × 3). The organic phases were combined, washed with saturated brine (20 mL × 3), dried over anhydrous sodium sulfate, filtered, and the filtrate was concentrated under reduced pressure. The crude product was purified by column chromatography (petroleum ether/ethyl acetate = 3/1) to obtain compound **49-5.**

**Step 5: Synthesis of compound 49-6**

A solution of compound **49-5** (0.75 g, 2.22 mmol, 1 *eq*) in trifluoroacetic acid (10 mL) was stirred and reacted at 70°C for 12 hours. The reaction mixture was slowly poured into saturated sodium bicarbonate solution (150 mL), and extracted with ethyl acetate (40 mL × 3). The organic phases were combined, washed with saturated brine (40 mL × 2), dried over anhydrous sodium sulfate, filtered, and the filtrate was concentrated under reduced pressure to obtain a crude product of compound **49-6.**

$^1$H NMR (400 MHz, $CDCl_3$) δ = 7.69 (dd, *J* = 1.6, 8.0 Hz, 1H), 7.53 (br s, 1H), 7.44 (d, *J* = 8.0 Hz, 1H), 7.37 (d, *J* = 1.6 Hz, 1H), 3.92 (s, 3H), 2.62-2.50 (m, 1H), 2.31-2.20 (m, 1H), 1.78-1.69 (m, 1H), 0.79 (q, *J* = 4.8 Hz, 1H).

**Step 6: Synthesis of compound 49-7**

To a solution of compound **49-6** (0.7 g, 3.22 mmol, 1 *eq)* in methanol (12 mL), tetrahydrofuran (12 mL) and water (3 mL) was added lithium hydroxide monohydrate (676.09 mg, 16.11 mmol, 5 *eq)* at 25°C, and the reaction mixture was heated to 45°C and stirred for 1 hour. The reaction mixture was added with dilute hydrochloric acid (1 mol/L) to adjust the pH to 5, and extracted with ethyl acetate (25 mL × 3). The organic phases were combined, washed with saturated brine (25 mL × 2), dried over anhydrous sodium sulfate, filtered, and the filtrate was concentrated under reduced pressure to obtain a crude product of compound **49-7.**

**Step 7: Synthesis of compounds 49a and 49b**

To a solution of compound **49-7** (0.7 g, 3.44 mmol, 1 *eq)* in *N,N*-dimethylformamide (10 mL) was added HATU (1.57 g, 4.13 mmol, 1.2 *eq*) and diisopropylethylamine (667.86 mg, 5.17 mmol, 900.08 μL, 1.5 *eq)* at 25°C, and the reaction mixture was stirred for 10 min, then compound **BB-1** (746.18 mg, 3.62 mmol, 1.05 *eq)* was added thereto, and the reaction mixture was stirred continuously for 12 hours. The reaction mixture was concentrated under reduced pressure. The crude product was purified by preparative HPLC (chromatographic column: Waters Xbridge BEH C18 250×50 mm×10 μm; mobile phase: [water (10 mM ammonium bicarbonate)-acetonitrile]; acetonitrile%: 20% to 50%), and then purified by SFC (chromatographic column: DAICEL CHIRALPAK AS (250 mm×30 mm, 10 μm); mobile phase: phase A: $CO_2$, phase B: [0.1% ammonia water-methanol]; gradient: B%: 55% to 55%, 4.1 min; 80 min) to obtain compounds **49a** and **49b.**

Compound **49a** (chiral analysis conditions: chromatographic column: Chiralpak AS-3 50×4.6 mm I.D 3 μm, mobile phase: mobile phase A was $CO_2$, mobile phase B was methanol (0.05% diethylamine); gradient: B%: 5% to 40%, retention time: 1.785 min, chiral purity = 100%), $^1$H NMR (400 MHz, $CD_3OD$) δ = 7.35 (d, *J* = 1.0 Hz, 2H), 7.28 (s, 1H), 7.15-7.07 (m, 3H), 7.05-7.00 (m, 1H), 4.10 (quin, *J* = 6.0 Hz, 1H), 3.74 (s, 2H), 3.48 (dq, *J* = 6.0, 13.6 Hz, 2H), 2.94-2.83 (m, 4H), 2.71-2.57 (m, 3H), 2.20-2.08 (m, 1H), 1.78 -1.69 (m, 1H), 0.59 (q, *J* = 4.8 Hz, 1H), MS (ESI) m/z: 392.0 $[M+H]^+$.

Compound **49b** (chiral analysis conditions: chromatographic column: Chiralpak AS-3 50×4.6 mm I.D 3 μm, mobile phase: mobile phase A was $CO_2$, mobile phase B was methanol (0.05% diethylamine); gradient: B%: 5% to 40%, retention time: 2.246 min, chiral purity = 99.78%), $^1$H NMR (400 MHz, $CD_3OD$) δ = 7.35 (s, 2H), 7.28 (s, 1H), 7.15-7.07 (m, 3H), 7.05-6.98 (m, 1H), 4.09 (quin, *J* = 6.0 Hz, 1H), 3.73 (s, 2H), 3.56-3.39 (m, 2H), 3.00-2.80 (m, 4H), 2.74-2.56 (m, 3H), 2.19-2.09 (m, 1H), 1.79-1.69 (m, 1H), 0.59 (q, *J* = 4.8 Hz, 1H), MS (ESI) m/z: 392.0 $[M+H]^+$.

**Embodiment 41: Single crystal X-ray diffraction detection and analysis of compound 46a dihydrate**

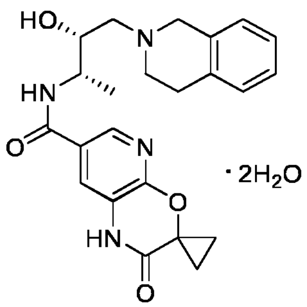

**46a dihydrate**

Compound **46a** (16 mg) was dissolved in dichloromethane/toluene/ethanol (1.2 mL, 1:2:1, v/v/v) at room temperature, then the solution was placed in a 4 mL semi-sealed vial, and slowly evaporated at room temperature to obtain a colorless needle-like crystal the next day. Crystals were collected and diffraction intensity data were collected with an XtaLAB Synergy-S diffractometer. The single crystal data showed that the single crystal was a dihydrate of compound **46a,** and the absolute configuration of compound **46a** could be determined. The three-dimensional ellipsoid diagram of compound **46a** dihydrate is shown in FIG. 10. The crystal structure data and parameters of compound **46a** dihydrate are shown in Tables 7, 8, 9, 10, 11 and 12.

Table 7: Crystal data of compound **46a** dihydrate

| | |
|---|---|
| Crystal size/$mm^3$ | 0.30 × 0.04 × 0.04 |
| Radiation Type | Cu Kα (λ = 1.54184) |
| Crystal system | orthorhombic |
| Space group | P212121 |
| a/Å | 4.94120(10) |
| b/Å | 18.2900(3) |
| c/Å | 24.7640(3) |
| α/° | 90 |
| β/° | 90 |
| γ/° | 90 |
| Cell Volume/Å3 | 2238.04(6) |
| Cell Formula Units Z | 4 |
| Crystal Density calc g/$cm^3$ | 1.361 |
| Crystal F(000) | 976.0 |
| Absorption Coefficient μ/mm-1 | 0.823 |
| Index ranges | -5 ≤ h ≤ 5, -18 ≤ k ≤ 21, -29 ≤ 1 ≤ 29 |
| Cell Measurement Temperature/K | 149.99(13) |
| 2θ range for data collection/° | 6.008 to 133.186 |
| Goodness-of-fit on F2 | 1.067 |
| Final R indexes [I>=2σ (I)] | R1 = 0.0288, wR2 = 0.0731 |
| Final R indexes [all data] | R1 = 0.0305, wR2 = 0.0740 |
| Largest diff. peak/hole/e Å-3 | 0.17/-0.18 |
| Reflections collected/unique | 55055/3939 [Rint = 0.0712] |
| Flack parameter | 0.09(7) |

Table 8: Atomic coordinates (×$10^4$) and equivalent isotropic displacement parameters (Å$^2$×$10^3$) of the crystals of compound 46a dihydrate

| Atom | x | y | z | U(eq) |
|---|---|---|---|---|
| O(2) | 7182(3) | 2849.3(8) | 3030.7(6) | 27.5(3) |
| O(1) | 1970(3) | 4587.4(7) | 3626.5(5) | 24.5(3) |
| O(3) | 1232(3) | 18.6(7) | 3798.1(6) | 30.2(3) |
| O(5) | -3350(3) | 2533.0(8) | 4714.5(6) | 29.2(3) |
| O(4) | -4203(3) | 499.3(8) | 4719.6(6) | 31.9(3) |
| N(3) | 4041(3) | 732.6(9) | 3316.8(6) | 23.6(3) |
| N(1) | 7488(3) | 5566.0(9) | 3401.1(6) | 20.7(3) |
| N(2) | 2872(3) | 3265.1(9) | 3020.2(6) | 22.0(3) |
| O(6) | -994(4) | 3765.1(10) | 4360.4(6) | 52.3(5) |
| N(4) | -1345(3) | 1235.6(9) | 4258.0(6) | 23.8(4) |
| C(14) | 4741(4) | 2755.7(11) | 3122.0(7) | 21.0(4) |
| C(21) | -937(4) | -76.2(11) | 4170.3(7) | 22.6(4) |
| C(18) | 758(4) | 1322.6(11) | 3881.6(7) | 20.8(4) |
| C(16) | 3724(4) | 2043.9(10) | 3338.9(7) | 20.6(4) |
| C(17) | 1598(4) | 1999.7(10) | 3710.3(7) | 20.7(4) |
| C(2) | 9985(4) | 6751.4(11) | 3493.1(8) | 23.6(4) |
| C(19) | 2022(4) | 705.0(11) | 3662.5(7) | 20.9(4) |
| C(10) | 5314(4) | 5238.4(10) | 3071.2(7) | 20.3(4) |
| C(20) | -2305(4) | 575.9(11) | 4405.5(7) | 22.9(4) |
| C(1) | 8183(4) | 6273.4(10) | 3152.4(8) | 23.3(4) |
| C(9) | 6575(4) | 5689.4(11) | 3963.8(7) | 24.1(4) |
| C(15) | 4889(4) | 1400.3(11) | 3163.3(7) | 22.0(4) |
| C(6) | 11904(4) | 7103.6(11) | 4346.7(9) | 29.9(4) |
| C(12) | 3588(4) | 3985.3(10) | 2807.4(7) | 21.2(4) |
| C(11) | 4267(4) | 4504.7(10) | 3277.0(7) | 20.2(4) |
| C(4) | 13004(4) | 7787.2(12) | 3550.3(10) | 34.6(5) |
| C(7) | 10253(4) | 6633.9(11) | 4046.8(8) | 24.5(4) |
| C(3) | 11349(4) | 7332.4(11) | 3250.4(9) | 28.4(4) |
| C(5) | 13286(4) | 7668.6(12) | 4103.9(9) | 34.1(5) |
| C(13) | 1329(4) | 4268.1(12) | 2448.8(8) | 28.5(4) |
| C(8) | 8886(4) | 5989.6(12) | 4304.2(7) | 27.1(4) |
| C(22) | -698(5) | -732.8(13) | 4526.7(9) | 38.6(5) |
| C(23) | -2631(4) | -732.1(12) | 4070.4(10) | 36.7(5) |

Table 9: Bond lengths (Å) of compound 46a dihydrate

| Atom Atom | Length/Å | Atom Atom | Length/Å |
|---|---|---|---|
| O(2) C(14) | 1.239(2) | C(18) C(17) | 1.373(3) |
| O(1) C(11) | 1.435(2) | C(18) C(19) | 1.400(3) |
| O(3) C(21) | 1.424(2) | C(16) C(17) | 1.399(3) |
| O(3) C(19) | 1.357(2) | C(16) C(15) | 1.381(3) |
| O(4) C(20) | 1.227(2) | C(2) C(1) | 1.506(3) |
| N(3) C(19) | 1.316(2) | C(2) C(7) | 1.394(3) |
| N(3) C(15) | 1.346(3) | C(2) C(3) | 1.394(3) |
| N(1) C(10) | 1.477(2) | C(10) C(11) | 1.526(3) |
| N(1) C(1) | 1.474(2) | C(9) C(8) | 1.522(3) |
| N(1) C(9) | 1.482(2) | C(6) C(7) | 1.398(3) |
| N(2) C(14) | 1.336(3) | C(6) C(5) | 1.377(3) |
| N(2) C(12) | 1.462(2) | C(12) C(11) | 1.539(3) |
| N(4) C(18) | 1.405(2) | C(12) C(13) | 1.517(3) |
| N(4) C(20) | 1.347(3) | C(4) C(3) | 1.383(3) |
| C(14) C(16) | 1.495(3) | C(4) C(5) | 1.395(3) |
| C(21) C(20) | 1.490(3) | C(7) C(8) | 1.500(3) |
| C(21) C(22) | 1.495(3) | C(22) C(23) | 1.479(4) |
| C(21) C(23) | 1.484(3) | | |

Table 10: Bond angles (°) of compound **46a** dihydrate

| Atom Atom | Angle/° | Atom Atom | Angle/° |
|---|---|---|---|
| C(19) O(3) C(21) | 119.29(15) | O(3) C(19) C(18) | 121.47(16) |
| C(19) N(3) C(15) | 117.05(16) | N(3) C(19) O(3) | 114.50(16) |
| C(10) N(1) C(9) | 111.13(14) | N(3) C(19) C(18) | 124.03(17) |
| C(1) N(1) C(10) | 107.15(14) | N(1) C(10) C(11) | 114.76(15) |
| C(1) N(1) C(9) | 109.28(15) | O(4) C(20) N(4) | 122.93(18) |
| C(14) N(2) C(12) | 121.93(15) | O(4) C(20) C(21) | 120.23(17) |
| C(20) N(4) C(18) | 122.80(16) | N(4) C(20) C(21) | 116.84(16) |
| O(2) C(14) N(2) | 122.83(18) | N(1) C(1) C(2) | 114.41(15) |
| O(2) C(14) C(16) | 120.87(18) | N(1) C(9) C(8) | 110.33(15) |
| N(2) C(14) C(16) | 116.27(16) | N(3) C(15) C(16) | 123.68(16) |
| O(3) C(21) C(20) | 119.81(16) | C(5) C(6) C(7) | 121.3(2) |
| O(3) C(21) C(22) | 114.87(17) | N(2) C(12) C(11) | 109.67(15) |
| O(3) C(21) C(23) | 114.52(16) | N(2) C(12) C(13) | 109.87(15) |
| C(20) C(21) C(22) | 116.62(16) | C(13) C(12) C(11) | 113.10(16) |
| C(23) C(21) C(20) | 117.17(17) | O(1) C(11) C(10) | 112.14(15) |
| C(23) C(21) C(22) | 59.56(16) | O(1) C(11) C(12) | 110.40(14) |
| C(17) C(18) N(4) | 122.03(18) | C(10) C(11) C(12) | 111.37(15) |

(continued)

| **Atom Atom** | Angle/° | **Atom Atom** | Angle/° |
|---|---|---|---|
| C(17) C(18) C(19) | 118.23(16) | C(3) C(4) C(5) | 119.6(2) |
| C(19) C(18) N(4) | 119.72(17) | C(2) C(7) C(6) | 118.87(19) |
| C(17) C(16) C(14) | 122.61(17) | C(2) C(7) C(8) | 119.75(18) |
| C(15) C(16) C(14) | 119.27(16) | C(6) C(7) C(8) | 121.31(18) |
| C(15) C(16) C(17) | 118.12(17) | C(4) C(3) C(2) | 120.8(2) |
| C(18) C(17) C(16) | 118.82(17) | C(6) C(5) C(4) | 119.7(2) |
| C(7) C(2) C(1) | 121.16(18) | C(7) C(8) C(9) | 112.69(16) |
| C(7) C(2) C(3) | 119.72(19) | C(23) C(22) C(21) | 59.84(14) |
| C(3) C(2) C(1) | 119.10(18) | C(22) C(23) C(21) | 60.60(14) |

Table 11: Torsion angles (°) of compound **46a** dihydrate

| A-B-C-D | Angle/° | A-B-C-D | Angle/° |
|---|---|---|---|
| O(2) -C(14) -C(16) - C(17) | 143.69(19) | C(10) -N(1) -C(9) -C(8) | 177.03(16) |
| O(2) -C(14) -C(16) - C(15) | -37.2(3) | C(20) -N(4) -C(18) - C(17) | 175.83(17) |
| O(3) -C(21) -C(20) -O(4) | 178.80(17) | C(20) -N(4) -C(18) - C(19) | -2.9(3) |
| O(3) -C(21) -C(20) -N(4) | -1.5(3) | C(20) -C(21) -C(22) - C(23) | 107.3(2) |
| O(3) -C(21) -C(22) - C(23) | - 104.96(19) | C(20) -C(21) -C(23) - C(22) | - 106.41(19) |
| O(3) -C(21) -C(23) - C(22) | 105.6(2) | C(1) -N(1) -C(10) -C(11) | - 178.48(15) |
| N(1) -C(10) -C(11) -O(1) | 92.81(18) | C(1) -N(1) -C(9) -C(8) | -64.9(2) |
| N(1) -C(10) -C(11) - C(12) | - 142.91(16) | C(1) -C(2) -C(7) -C(6) | - 178.57(17) |
| N(1) -C(9) -C(8) -C(7) | 50.4(2) | C(1) -C(2) -C(7) -C(8) | 4.4(3) |
| N(2) -C(14) -C(16) - C(17) | -38.2(2) | C(1) -C(2) -C(3) -C(4) | 179.46(19) |
| N(2) -C(14) -C(16) - C(15) | 140.91(18) | C(9) -N(1) -C(10) -C(11) | -59.2(2) |
| N(2) -C(12) -C(11) -O(1) | -60.76(19) | C(9) -N(1) -C(1) -C(2) | 48.3(2) |
| N(2) -C(12) -C(11) - C(10) | 173.98(14) | C(15) -N(3) -C(19) -O(3) | 179.26(16) |
| N(4) -C(18) -C(17)-C(16) | 179.35(16) | C(15) -N(3) -C(19) - C(18) | -1.0(3) |
| N(4) -C(18) -C(19) -O(3) | 12(3) | C(15) -C(16) -C(17) - C(18) | -0.3(3) |
| N(4) -C(18) -C(19) -N(3) | 178.55(17) | C(6) -C(7) -C(8) -C(9) | 162.85(18) |
| C(14) -N(2) -C(12) - C(11) | -87.1(2) | C(12) -N(2) -C(14) -O(2) | -2.0(3) |
| C(14) -N(2) -C(12) - C(13) | 148.02(17) | C(12) -N(2) -C(14) - C(16) | 179.97(15) |
| C(14) -C(16) -C(17) - C(18) | 178.87(16) | C(7) -C(2) -C(1) -N(1) | -18.7(3) |
| C(14) -C(16) -C(15) - N(3) | - 177.08(17) | C(7) -C(2) -C(3) -C(4) | 1.1(3) |
| C(21) -O(3) -C(19) -N(3) | 179.92(15) | C(7) -C(6) -C(5) -C(4) | 1.3(3) |
| C(21) -O(3) -C(19) - C(18) | 0.2(3) | C(3) -C(2) -C(1) -N(1) | 163.03(17) |
| C(18) -N(4) -C(20) -O(4) | 177.38(17) | C(3) -C(2) -C(7) -C(6) | -0.3(3) |
| C(18) -N(4) -C(20) - C(21) | 2.9(3) | C(3) -C(2) -C(7) -C(8) | - 177.31(18) |
| C(17) -C(18) -C(19) - O(3) | - 177.57(17) | C(3) -C(4) -C(5) -C(6) | -0.4(3) |

(continued)

| A-B-C-D | Angle/° | A-B-C-D | Angle/° |
|---|---|---|---|
| C(17) -C(18) -C(19) - N(3) | 2.7(3) | C(5) -C(6) -C(7) -C(2) | -0.9(3) |
| C(17) -C(16) -C(15) - N(3) | 2.1(3) | C(5) -C(6) -C(7) -C(8) | 176.05(19) |
| C(2) -C(7) -C(8) -C(9) | -20.2(3) | C(5) -C(4) -C(3) -C(2) | -0.8(3) |
| C(19) -O(3) -C(21) - C(20) | 0.0(3) | C(13) -C(12) -C(11) - O(1) | 62.2(2) |
| C(19) -O(3) -C(21) - C(22) | - 146.62(19) | C(13) -C(12) -C(11) - C(10) | -63.0(2) |
| C(19) -O(3) -C(21) - C(23) | 147.10(18) | C(22) -C(21) -C(20) - O(4) | -35.2(3) |
| C(19) -N(3) -C(15) - C(16) | -1.5(3) | C(22) -C(21) -C(20) - N(4) | 144.54(19) |
| C(19) -C(18) -C(17) - C(16) | -1.9(3) | C(23) -C(21) -C(20) - O(4) | 32.5(3) |
| C(10) -N(1) -C(1) -C(2) | 168.81(15) | C(23) -C(21) -C(20) - N(4) | - 147.78(18) |

Table 12: Hydrogen bonds of compound **46a** dihydrate

| D-H-A | d(D-H)/Å | d(H-A)/Å | d(D-A)/Å | D-H-A/° |
|---|---|---|---|---|
| O(1) -H(1) -N(1)1 | 0.82 | 2.12 | 2.902(2) | 160.2 |
| O(5) -H(5A) O(6) | 0.85 | 1.87 | 2.684(2) | 158.7 |
| O(5) -H(5B) O(5)2 | 0.85 | 2.05 | 2.8493(14) | 156.6 |
| N(2) -H(2) O(2)1 | 0.86 | 2.07 | 2.913(2) | 165.8 |
| O(6) -H(6A) O(4)3 | 0.85 | 1.96 | 2.790(2) | 163.9 |
| O(6) -H(6B) O(1) | 0.85 | 1.93 | 2.777(2) | 171.6 |
| N(4) -H(4) O(5) | 0.86 | 1.95 | 2.809(2) | 175.1 |

**Embodiment 42: Preparation of crystal form A and crystal form B of compounds 46a**

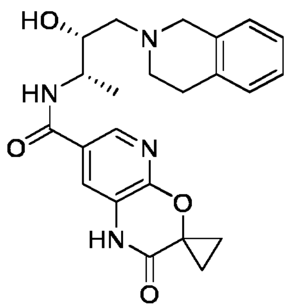

**46a**

Preparation of crystal form A: Compound **46a** (5 g) was dissolved in a solution of ethyl acetate (50 mL), then n-heptane (150 mL) was added, and the reaction mixture was stirred at 25°C for 72 hours. After filtration, the filter cake was collected and dried under vacuum to obtain a solid, which was detected as crystal form A of compound **46a** by XRPD.

Preparation of crystal form B: Compound **46a** (20.8 mg) was dissolved in tetrahydrofuran (3.0 mL), and slowly evaporated at room temperature to obtain a solid, which was detected as crystal form B of compound **46a** by XRPD.

**Embodiment 43: Study on hygroscopicity of crystal form A of compound 46a**

Instrument: SMS DVS intrinsic Dynamic Vapor Sorption. The crystal form A of compound **46a** (about 10 mg)

was taken and placed in a DVS sample tray for testing. The DVS pattern of crystal form A of compound **46a** is shown in FIG. 6. The moisture absorption weight gain of crystal form A of compound **46a** at 25°C/80% RH is: ΔW% = 0.8%, 2% > ΔW% ≥ 0.2%, slightly hygroscopic.

**Bioassay Data:**

**Experimental embodiment 1: PRMT5 enzyme inhibitory activity assay**

Experimental objective: To test the inhibitory effect of compounds on PRMT5 enzyme activity

Experimental materials: PRMT5 enzyme

Experimental operation: Test compounds were added at a certain concentration (0.17 nM to 99010 nM) to a white transparent bottomed 384-well plate using LABCYTE Echo 550, then PRMT5 was added, and a vehicle control (with DMSO and without compound) and a blank control (with DMSO and without PRMT5) were set up. The plate was incubated at 25°C for 30 min, then added with substrate, and reacted at 25°C for 90 min. After 90 min, the detection reagent was added to the 384-well plate using PerkinElmer LANCE Ultra TR-FRET standard method to combine with the enzyme-substrate reaction product. After 1 hour of reaction at 25°C, the signal was detected on a PerkinElmer EnVision 2105 Multimode Plate Reader. Raw data were used to calculate the inhibition rate of the test compounds using the following equation:

$$\textbf{Inhibition rate}\% = \frac{\textbf{RLU vehicle control} - \textbf{RLU compound}}{\textbf{RLU vehicle control} - \textbf{RLU blank control}} \times \textbf{100}\%$$

The inhibition rates were imported into Excel separately to calculate the inhibition rates of compounds at different concentrations, and then XLfit software was used to plot inhibition curves and calculate relevant parameters, including minimum inhibition rate, maximum inhibition rate and $IC_{50}$. The results are shown in Table 13.

**Table 13:** Results of PRMT5 enzyme inhibitory activity of the compounds of the present disclosure

| Test compound | $IC_{50}$ (nM) | Test compound | $IC_{50}$ (nM) |
|---|---|---|---|
| **Compound 1** | 20 | **Compound 35** | 11 |
| **Compound 2** | 128 | **Compound 36** | 7 |
| **Compound 3** | 18 | **Compound 37** | 8 |
| **Compound 4** | 22 | **Compound 39** | 34 |
| **Compound 8** | 15 | **Compound 40** | 34 |
| **Compound 9** | 36 | **Compound 46a** | 58 |
| **Compound 23** | 18 | **Compound 47** | 42 |
| **Compound 24** | 15 | **Compound 48a** | 46.2 |
| **Compound 29** | 16 | **Compound 48b** | 34.2 |
| **Compound 34** | 13 | **Compound 49b** | 26.2 |

Conclusion: The compounds of the present disclosure can effectively inhibit PRMT5 enzyme activity.

**Experimental embodiment 2: *In vitro* anti-proliferation assay of Z-138 cells**

Experimental objective: To test the anti-proliferation effect of compounds on Z-138 non-Hodgkin's lymphoma cells

Experimental materials: Z-138 non-Hodgkin's lymphoma cells (ATCC-CRL-3001)

Experimental operation: Z-138 cells were planted in a white transparent bottomed 384-well cell culture plate at a density of 800 cells per well, then test compounds were added to the cell culture wells at a certain concentration

(2.5 nM to 50000 nM) on the same day, and a vehicle control (with DMSO and without compound) and a blank control (with DMSO and without cells) were set up. The plate was incubated at 37°C, $CO_2$ and 100% relative humidity for 6 days. Samples were processed using Promega CellTiter-Glo Luminescent Cell Viability Assay Kit (Promega-G7571) standard method, and the luminescent signal was detected on a TECAN Spark®10M plate reader. Raw data were used to calculate the inhibition rate of the test compounds using the following equation:

$$\textbf{Inhibition rate}\% = \frac{\textbf{RLU vehicle control} - \textbf{RLU compound}}{\textbf{RLU vehicle control} - \textbf{RLU blank control}} \times \textbf{100}\%$$

The inhibition rates were imported into Excel separately to calculate the inhibition rates of compounds at different concentrations, and then XLfit software was used to plot inhibition curves and calculate relevant parameters, including minimum inhibition rate, maximum inhibition rate and $IC_{50}$. The results are shown in Table 14.

**Table 14:** Results of *in vitro* anti-proliferative activity of the compounds of the present disclosure on Z-138 tumor cells

| Test compound | $IC_{50}$ (nM) | Test compound | $IC_{50}$ (nM) |
|---|---|---|---|
| Compound 1 | 54 | Compound 26 | 22 |
| Compound 2 | 24 | Compound 27 | 4 |
| Compound 3 | 78 | Compound 28 | 10 |
| Compound 4 | 22 | Compound 29 | 6 |
| Compound 6 | 6 | Compound 32 | 19 |
| Compound 7 | 73 | Compound 34 | 27 |
| Compound 8 | 10 | Compound 35 | 22 |
| Compound 9 | 100 | Compound 36 | 7 |
| Compound 10 | 23 | Compound 37 | 8 |
| Compound 11 | 88 | Compound 38 | 18 |
| Compound 13 | 47 | Compound 39 | 12 |
| Compound 14 | 14 | Compound 40 | 4 |
| Compound 16 | 90 | Compound 41 | 124 |
| Compound 17 | 13 | Compound 42 | 53 |
| Compound 18 | 18 | Compound 43 | 17 |
| Compound 19 | 8 | Compound 44 | 18 |
| Compound 20 | 15 | Compound 46a | 11 |
| Compound 21 | 18 | Compound 47 | 66.8 |
| Compound 22 | 12 | Compound 48a | 29.3 |
| Compound 23 | 23 | Compound 48b | 11.5 |
| Compound 24 | 22 | Compound 49b | 13.1 |
| Compound 25 | 22 | - | - |

Conclusion: The compounds of the present disclosure can effectively inhibit the proliferation of non-Hodgkin's lymphoma cells.

**Experimental embodiment 3: Evaluation of anti-proliferative activity of MDA-MB-468 cells**

Experimental objective: To test the anti-proliferation effect of compounds on MDA-MB-468 human breast cancer cells

Experimental materials:

MDA-MB-468 cell line (purchased from Procell), penicillin/streptomycin antibiotics (purchased from Wisent), fetal bovine serum (purchased from Gibco). CellTiter-Glo (chemiluminescent cell viability detection reagent, purchased from Promega).

Experimental operation:

MDA-MB-468 cells were planted in a white 384-well plate with 40 μL of cell suspension per well, which contained 1000 MDA-MB-468 cells. The cell plate was cultured in a $CO_2$ incubator overnight. The compounds to be tested were diluted 3-fold with a row gun to the 10th concentration, that is, from 2000 μM to 101.61 nM, and a double-duplication experiment was set up. 78 μL of culture medium was added to a middle plate, and then 2 μL of gradient diluted compound per well was transferred to the middle plate according to the corresponding position. After being thoroughly mixed, 10 μL per well was transferred to the cell plate. The concentration range of the compound transferred to the cell plate was 10 μM to 0.51 nM. The cell plate was cultured in a $CO_2$ incubator for 7 days. Another cell plate was prepared, and the signal value was read on the day of administration as the maximum value (Max value in the equation below) to participate in data analysis. 10 μL of chemiluminescent cell viability detection reagent per well was added to the cell plate, and the plate was incubated at room temperature for 10 min to stabilize the luminescent signal. A multi-label analyzer was used for reading. After the incubation of the cell plate with the compound was completed, 10 μL of chemiluminescent cell viability detection reagent per well was added to the cell plate, and the plate was incubated at room temperature for 10 min to stabilize the luminescent signal. A multi-label analyzer was used for reading.

**Data analysis:**

The equation **(Sample-Min)/(Max-Min)×100%** was used to convert the raw data into inhibition rate, and the value of $IC_{50}$ might be obtained by curve fitting with four parameters (obtained by "log(inhibitor) vs. response -- Variable slope" mode in GraphPad Prism). The results are shown in Table 15.

**Table 15:** Results of *in vitro* anti-proliferative activity of the compounds of the present disclosure on MDA-MB-468 tumor cells

| Test compound | $IC_{50}$ (nM) | Test compound | $IC_{50}$ (nM) |
|---|---|---|---|
| **Compound 8** | 12 | **Compound 46a** | 5 |
| **Compound 39** | 12 | **Compound 49b** | 4.2 |
| **Compound 40** | 3 | - | - |

Conclusion: The compounds of the present disclosure can effectively inhibit the proliferation of triple-negative breast cancer cells.

**Experimental embodiment 4: Pharmacokinetic evaluation of the compounds of the present disclosure**

Experimental objective: To study the pharmacokinetic parameters of the compounds in CD-1 mice

Animal experimental design: Male CD-1 mice (28.08 to 29.01 g, 6 to 7 weeks old), purchased from Shanghai Family Planning Research Institute, n=2/group, 4 mice in total; blood collection time points (h): 0.083 (intravenous injection only), 0.25 (intragastric administration only), 0.5, 1, 2, 4, 8, 10 and 24; compound dose by tail vein injection: 1 mg/kg; single dose by intragastric administration: 5 mg/kg; intravenous and intragastric administration vehicles: 20% *N,N*-dimethylacetamide aqueous solution, 20% hydroxypropyl-β-cyclodextrin aqueous solution, 5% DMSO/50% PEG400/45% water for injection or 10% Solutol/30% PEG400/60% water for injection. All animals had free access to water during the experiment, fasted overnight before administration, and resumed food supply 4 hours after administration. The entire animal experiment complied with the Institutional Animal Care and Use Committee (IACUC) animal welfare.

Sample collection and storage: After administration, approximately 0.03 mL of blood was collected from the saphenous vein of the mouse at each time point into a pre-frozen commercial EDTA-K2 tube, and placed on wet ice until centrifugation. Blood samples were centrifuged at 4°C, 3,200 g for 10 min. Plasma was collected separately, transferred to a pre-labeled 96-well plate, fast frozen on dry ice, and stored at -70±10°C until LC-MS/MS analysis.

Pre-treatment of plasma samples: 3 μL of unknown, standard curve, QC, diluted QC and single and double blank samples were taken and placed in 1.5 mL tubes, and 60 μL of internal standard solution (internal standard: Labetalol 100 ng/mL acetonitrile solution, double blank with the same volume of pure acetonitrile) was added. The samples were vortexed for at least 15 seconds, and centrifuged at 4°C, 12,000 g for 15 min. 50 μL of the supernatant was taken and placed on a clean 96-well plate, and 200 μL of 70% acetonitrile was added. The plate was vortex at 800 rpm for 10 min, and centrifuged at 4°C, 3,220 g for 5 min. 2.0 μL of the supernatant was taken for LC-MS/MS analysis. 10-fold sample dilution procedure: 2 μL of the sample was added to 18 μL of blank matrix; 2-fold sample dilution procedure: 2 μL of the sample was added to 2 μL of blank matrix. Plasma was processed in the same manner as undiluted samples. The linear range was 4.00 to 6000 nM. Plasma concentrations would be analyzed with Triple Quad 6500 Plus, and pharmacokinetic parameters would be calculated with Phoenix WinNonlin 6.3. The results are shown in Table 16.

**Table 16:** Results of pharmacokinetic assay of the compounds of the present disclosure

| Test compound | Injection administration (1 mg/kg) | | | Oral administration (5 mg/kg) | |
|---|---|---|---|---|---|
| | Clearance rate CL (mL/min/ Kg) | Half-life $T_{1/2}$ (h) | Concentration integral AUC (nM.h) | Concentration integral AUC (nM.h) | Bioavailability F (%) |
| Compound 1 | 29.5 | 0.16 | 1335 | 930 | 13.9 |
| Compound 2 | 39.7 | 0.56 | 967 | 417 | 8.6 |
| Compound 3 | 78.1 | 0.16 | 474 | 171 | 7.2 |
| Compound 4 | 63.3 | 0.27 | 583 | 626 | 21.5 |
| Compound 6 | 42.1 | 0.24 | 944 | 773 | 16.4 |
| Compound 7 | 17.7 | 1.38 | 2313 | 7419 | 64.2 |
| Compound 8 | 53.3 | 0.91 | 875 | 1028 | 24.5 |
| Compound 9 | 107 | 2.15 | 336 | 168 | 10.0 |
| Compound 10 | 42.1 | 0.55 | 785 | 572 | 14.6 |
| Compound 17 | 59.3 | 0.62 | 676 | 314 | 9.93 |
| Compound 18 | 71.3 | 0.41 | 545 | 164 | 6.02 |
| Compound 19 | 86.8 | 0.23 | 502 | 201 | 8.82 |
| Compound 20 | 62.3 | 0.25 | 615 | 150 | 4.88 |
| Compound 29 | 37.9 | 0.59 | 981 | 441 | 8.99 |
| Compound 36 | 66.3 | 0.22 | 576 | 1092 | 37.9 |

(continued)

| Test compound | Injection administration (1 mg/kg) | | | Oral administration (5 mg/kg) | |
|---|---|---|---|---|---|
| | Clearance rate CL (mL/min/ Kg) | Half-life $T_{1/2}$ (h) | Concentration integral AUC (nM.h) | Concentration integral AUC (nM.h) | Bioavailability F (%) |
| **Compound 39** | 33.6 | 0.55 | 1214 | 1208 | 20.0 |

Conclusion: The compounds of the present disclosure exhibit good plasma exposure and reasonable oral bioavailability.

**Experimental embodiment 5: *In vivo* pharmacodynamic evaluation of the compounds of the present disclosure**

Experimental objective: To evaluate the antitumor effects of the compounds using the subcutaneous xenograft tumor CB17 SCID mouse model of human mantle cell lymphoma Z-138 cells in this experiment.

Experimental materials: CB17 SCID mice

Experimental methods and procedures

5.1 Cell culture

Cell culture: Human mantle cell lymphoma Z-138 cells (ATCC-CRL-3001) were cultured *in vitro* by suspension under the conditions of Improved Minimum Essential Medium with 10% fetal bovine serum and 1% double antibody, and cultured in a 37°C, 5% $CO_2$ incubator. Routine digestion was performed twice a week for passaging. When the cell saturation density was 80% to 90% and the number of cells reached the required level, the cells were collected, counted, and inoculated.

5.2 Tumor cell inoculation and group administration

(1) Cell inoculation: 0.2 mL ($5 \times 10^6$) Z-138 cells (plus Matrigel, volume ratio of 1:1) were subcutaneously inoculated at right side on the back of each mouse. When the average tumor volume reached 163 $mm^3$, random grouping method was adopted to start group administration. Experimental grouping and administration regimen are shown in Table **17** and Table **18** below.

**Table 17: Pharmacodynamic experimental animal grouping and administration regimen**

| Group | Number of mice | Compound for treatment | Dose (mg/kg) | Volume of administration ($\mu L/g$)$^2$ | Route of administration | Frequency of administration |
|---|---|---|---|---|---|---|
| 1 | 6 | Vehicle | -- | 10 | PO | QD $\times$ 21 |
| 2 | 6 | Compound 1 | 200 | 10 | PO | QD $\times$ 21 |
| 3 | 6 | Compound 4 | 200 | 10 | PO | QD $\times$ 21 |

**Table 18: Pharmacodynamic experimental animal grouping and administration regimen**

| Group | Number of mice | Compound for treatment | Volume of administration ($\mu L/g$)$^2$ | Route of administration |
|---|---|---|---|---|
| 1 | 6 | Vehicle | 10 | PO |
| 2 | 6 | Compound **8** | 10 | PO |

Method of administration of compound **8:** 200 mg/kg QD from day 0 to day 8; 100 mg/kg BID from day 9 to day 15; 150 mg/kg BID from day 16 to day 21; the frequency of administration of vehicle was consistent with that of compound **8.**
(2) Cell inoculation: 0.2 mL (5 × $10^6$) Z-138 cells (plus Matrigel, volume ratio of 1:1) were subcutaneously inoculated at right side on the back of each mouse. When the average tumor volume reached 107 $mm^3$, random grouping method was adopted to start group administration. Experimental grouping and administration regimen are shown in Table **19** below.

**Table 19: Pharmacodynamic experimental animal grouping and administration regimen**

| Group | Number of mice | Compound for treatment | Dose (mg/kg) | Volume of administration (μL/g)² | Route of administration | Frequency of administration |
|---|---|---|---|---|---|---|
| 1 | 6 | Vehicle | -- | 10 | PO | BID × 21 |
| 2 | 6 | Compound **39** | 80 | 10 | PO | BID × 21 |
| 3 | 6 | Compound **40** | 80 | 10 | PO | BID × 21 |
| 4 | 6 | Compound **46a** | 80 | 10 | PO | BID × 21 |

5.3 Tumor measurement and experimental indicators

Tumor diameters were measured twice a week with vernier calipers. The formula for calculating tumor volume was: V = 0.5a × $b^2$, where a and b are the long and short diameters of the tumor, respectively.
The antitumor efficacy of compounds was evaluated by TGI (%) or relative tumor proliferation rate T/C (%). Relative tumor proliferation rate T/C (%) = TRTV / CRTV × 100% (TRTV: RTV of the treatment group; CRTV: RTV of the negative control group). Relative tumor volume (RTV) was calculated according to the results of tumor measurement, and the formula was RTV = Vt / V0, where V0 was the average tumor volume measured at the time of group administration (that is, D0), Vt was the average tumor volume in a certain measurement, and the data of TRTV and CRTV were collected on the same day.
TGI (%) reflected the rate of tumor growth inhibition. TGI (%) = [1 - (Average tumor volume at the end of administration in a certain treatment group - Average tumor volume at the beginning of administration in this treatment group) / (Average tumor volume at the end of treatment in the solvent control group - Average tumor volume at the beginning of treatment in the solvent control group)] × 100%.
In this experiment, the efficacy of the compounds of the present disclosure in the subcutaneous xenograft model of human mantle cell lymphoma Z-138 cells was evaluated, and the solvent control group was used as a reference. The experimental results of compound **1** and compound **4** are shown in FIG. 1, and the experimental results of compound **8** are shown in FIG. 2, where each data point is marked by average tumor volume ± standard error of mean (SEM). The experimental results of compound **39,** compound **40** and compound **46a** are shown in Table 20.

**Table 20: Antitumor effect of the test compounds on xenograft tumor model of human mantle cell lymphoma Z-138**

| Group | Tumor volume ($mm^3$)* (Day 0) | Tumor volume ($mm^3$)* (Day 21) | RTV (Day 21) | TGI (%) (Day 21) | T/C (%) (Day 21) |
|---|---|---|---|---|---|
| Vehicle | 108 ± 5 | 1486 ± 105 | 13.79 ± 0.63 | -- | -- |
| **Compound 39** | 108 ± 7 | 179 ± 42 | 1.66 ± 0.42 | 94.8 | 12.1 |
| **Compound 40** | 107 ± 6 | 39 ± 9 | 0.36 ± 0.08 | 105.0 | 2.6 |
| **Compound 46a** | 107 ± 8 | 27 ± 13 | 0.24 ± 0.11 | 105.8 | 1.7 |
| Note*: Mean ± SEM, n = 6. | | | | | |

Conclusion: The compounds of the present disclosure exhibit excellent *in vivo* antitumor efficacy.

**Experimental embodiment 6: Evaluation of pharmacodynamic activity of the compounds of the present disclosure in human breast cancer MDA-MB-468 mouse model**

Experimental objective: To evaluate the antitumor effects of the test compounds in the subcutaneous xenograft tumor BALB/c mouse model of human breast cancer MDA-MB-468 cells.

Experimental materials: 6 to 8 weeks old Balb/c female mice

Experimental methods and procedures

6.1 Cell culture

Cell culture: Human breast cancer MDA-MB-468 cells (ATCC-HTB-132) were cultured *in vitro* in a monolayer under the conditions of McCoy's 5a Medium with 10% fetal bovine serum, 100 U/mL penicillin and 100 μg/mL streptomycin, and cultured in a 37°C, 5% $CO_2$ incubator. Routine digestion with trypsin-EDTA was performed twice a week for passaging. When the cell saturation density was 80% to 90% and the number of cells reached the required level, the cells were collected, counted, and inoculated.

6.2 Tumor cell inoculation and group administration

Cell inoculation: 0.2 mL ($10 \times 10^6$) MDA-MB-468 cells (plus Matrigel, volume ratio of 1:1) were subcutaneously inoculated at right side on the back of each mouse. Group administration was started when the average tumor volume reached 130 $mm^3$. Experimental grouping and administration regimen are shown in Table **21** below.

**Table 21: Pharmacodynamic experimental animal grouping and administration regimen**

| Group | Number of mice | Compound for treatment | Dose (mg/kg) | Volume of administration (μL/g)[2] | Route of administration | Frequency of administration |
|---|---|---|---|---|---|---|
| 1 | 6 | Vehicle | -- | 10 | PO | BID × 4W |
| 2 | 6 | Compound **40** | 100 | 10 | PO | BID × 4W |
| 3 | 6 | Compound **46a** | 100 | 10 | PO | BID × 4W |

6.3 Tumor measurement and experimental indicators

Tumor diameters were measured twice a week with vernier calipers. The formula for calculating tumor volume was: $V = 0.5a \times b^2$, where a and b are the long and short diameters of the tumor, respectively.

The antitumor efficacy of compounds was evaluated by TGI (%) or relative tumor proliferation rate T/C (%). Relative tumor proliferation rate T/C (%) = TRTV / CRTV × 100% (TRTV: RTV of the treatment group; CRTV: RTV of the negative control group). Relative tumor volume (RTV) was calculated according to the results of tumor measurement, and the formula was RTV = Vt / V0, where V0 was the average tumor volume measured at the time of group administration (that is, D0), Vt was the average tumor volume in a certain measurement, and the data of TRTV and CRTV were collected on the same day.

TGI (%) reflected the rate of tumor growth inhibition. TGI (%) = [1 - (Average tumor volume at the end of administration in a certain treatment group - Average tumor volume at the beginning of administration in this treatment group) / (Average tumor volume at the end of treatment in the solvent control group - Average tumor volume at the beginning of treatment in the solvent control group)] × 100%.

Experimental results:

In this experiment, the efficacy of the compounds of the present disclosure in the subcutaneous xenograft model of human breast cancer cells was evaluated, and the solvent control group was used as a reference. The experimental results are shown in Table 22.

**Table 22: Antitumor effect of the test compounds on subcutaneous xenograft tumor model of breast cancer MDA-MB-468 cells**

| **Group** | **Tumor volume ($mm^3$)[a] (Day 0)** | **Tumor volume ($mm^3$)[a] (Day 28)** | **RTV (Day 28)** | **T/C (%)** | **TGI (%)** |
|---|---|---|---|---|---|
| Vehicle | 131 ± 8.4 | 342 ± 14.5 | 2.64 ± 0.10 | -- | -- |
| Compound **46a** | 130 ± 10.5 | 115 ± 13.8 | 0.88 ± 0.06 | 33.29 | 107.01 |
| Compound **40** | 130 ± 10.0 | 140 ± 9.2 | 1.09 ± 0.06 | 41.23 | 95.15 |
| Note: a. Mean ± SEM. | | | | | |

Conclusion: The compounds of the present disclosure exhibit very excellent *in vivo* antitumor efficacy.

## Claims

1. A compound represented by formula (VII) or a pharmaceutically acceptable salt thereof,

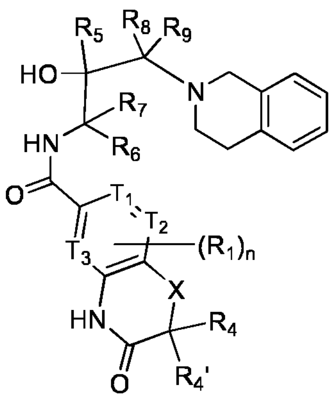

(VII)

wherein,

$T_1$, $T_2$ and $T_3$ are each independently selected from CH and N;
$R_1$ is each independently selected from H, F, Cl, Br, I, $OCH_3$ and $CH_3$, and the $OCH_3$ and $CH_3$ are optionally substituted by 1, 2 or 3 F;
$R_4$' is selected from H and $C_{1-3}$ alkyl;
$R_5$ is selected from H, F, Cl and $C_{1-3}$ alkyl, and the $C_{1-3}$ alkyl is optionally substituted by 1, 2 or 3 F;
$R_6$ and $R_8$ are each independently selected from H, F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
$R_7$ and $R_9$ are each independently selected from H, F, Cl and $C_{1-3}$ alkyl, and the $C_{1-3}$ alkyl is optionally substituted by 1, 2 or 3 F;
alternatively, $R_6$ and $R_7$ form $C_{3-6}$ cycloalkyl or 4- to 6-membered heterocycloalkyl together with the atoms to which they are attached, and the $C_{3-6}$ cycloalkyl and the 4- to 6-membered heterocycloalkyl are optionally substituted by 1, 2 or 3 F;
alternatively, $R_8$ and $R_9$ form $C_{3-6}$ cycloalkyl or 4- to 6-membered heterocycloalkyl together with the atoms to which they are attached, and the $C_{3-6}$ cycloalkyl and the 4- to 6-membered heterocycloalkyl are optionally substituted by 1, 2 or 3 F;
when X is selected from a single bond, $CH_2$ and O, $R_4$ and $R_4$' form moiety

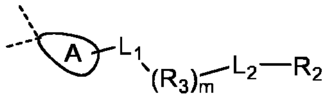

together with the atoms to which they are attached;
alternatively, X and $R_4$ are attached together to form a ring B;
the ring B is selected from $C_{3-6}$ cycloalkyl, and the $C_{3-6}$ cycloalkyl is optionally substituted by 1, 2 or 3 $R_c$;
$L_1$ and $L_2$ are each independently selected from a single bond and -C(=O)-;
$R_2$ is selected from H, OH,

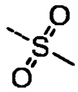,

$C_{1-3}$ alkyl, $C_{1-3}$ alkoxy, -NH-$C_{3-6}$ cycloalkyl, $C_{3-6}$ cycloalkyl and phenyl, and the $C_{1-3}$ alkyl, $C_{1-3}$ alkoxy, -NH-$C_{3-6}$ cycloalkyl, $C_{3-6}$ cycloalkyl and phenyl are optionally substituted by 1, 2 or 3 $R_a$;
$R_3$ is selected from $C_{3-6}$ cycloalkyl;
ring A is selected from $C_{3-6}$ cycloalkyl and 4- to 6-membered heterocycloalkyl, and the $C_{3-6}$ cycloalkyl and 4- to 6-membered heterocycloalkyl are optionally substituted by 1, 2 or 3 $R_b$;
m is selected from 0 and 1;
n is selected from 0, 1, 2 and 3;
$R_a$ and $R_c$ are each independently selected from H, F, Cl, Br, I, OH, $-OCH_3$ and $NH_2$;
$R_b$ is each independently selected from H, F, Cl, Br, I, =O, OH, $NH_2$, $C_{1-3}$ alkyl and $C_{1-3}$ alkoxy;
the 4- to 6-membered heterocycloalkyl contains 1, 2, 3 or 4 heteroatoms or heteroatom groups independently selected from -NH-, -O-, -S- and N.

2. The compound or the pharmaceutically acceptable salt thereof as claimed in claim 1, wherein, $R_1$ is each independently selected from H, F, $OCH_3$ and $CH_3$.

3. The compound or the pharmaceutically acceptable salt thereof as claimed in claim 1, wherein, $R_2$ is selected from H, OH,

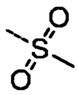,

$C_{1-3}$ alkyl, $C_{1-3}$ alkoxy, phenyl, cyclobutyl, -NH-cyclobutyl and -NH-cyclopropyl, and the $C_{1-3}$ alkyl, $C_{1-3}$ alkoxy, phenyl, cyclobutyl, -NH-cyclobutyl and -NH-cyclopropyl are optionally substituted by 1, 2 or 3 $R_a$.

4. The compound or the pharmaceutically acceptable salt thereof as claimed in claim 3, wherein, $R_2$ is selected from H, OH,

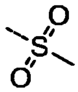,

$CH_3$,

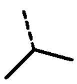, 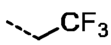, 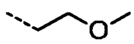, 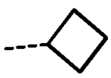,

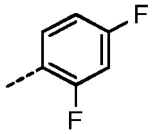 , 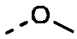 , 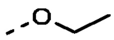 , 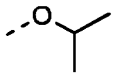 , 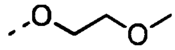 , 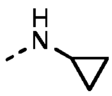 and 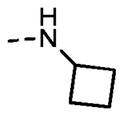 .

5. The compound or the pharmaceutically acceptable salt thereof as claimed in claim 1, wherein, $R_3$ is selected from cyclopropyl, cyclobutyl, cyclopentyl and cyclohexyl.

6. The compound or the pharmaceutically acceptable salt thereof as claimed in claim 5, wherein, $R_3$ is selected from

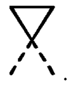 .

7. The compound or the pharmaceutically acceptable salt thereof as claimed in claim 1, wherein, structural moiety

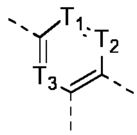

is selected from phenyl, pyridyl, pyrimidinyl, pyrazinyl and pyridazinyl.

8. The compound or the pharmaceutically acceptable salt thereof as claimed in claim 7, wherein, structural moiety

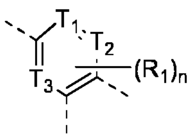

is selected from

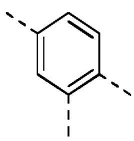 , 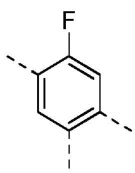 ,

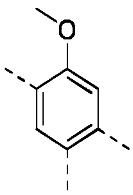 , 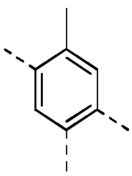 , 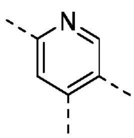 , 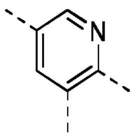 , 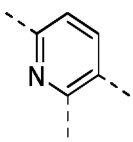 and 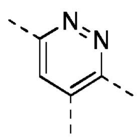 .

9. The compound or the pharmaceutically acceptable salt thereof as claimed in claim 1, wherein, ring A is selected from cyclopropyl, cyclobutyl, cyclopentyl, cyclohexyl, oxetanyl, tetrahydrofuranyl, tetrahydropyranyl, azetidinyl, piperidinyl and thietanyl, and the cyclopropyl, cyclobutyl, cyclopentyl, cyclohexyl, oxetanyl, tetrahydrofuranyl, tetrahydropyranyl, azetidinyl, piperidinyl and thietanyl are optionally substituted by 1, 2 or 3 $R_b$.

10. The compound or the pharmaceutically acceptable salt thereof as claimed in claim 9, wherein, ring A is selected from

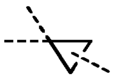, 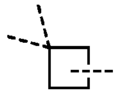, 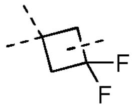, 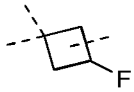, 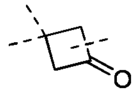,

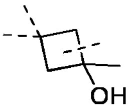, 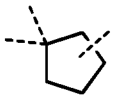, 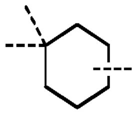, 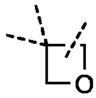, 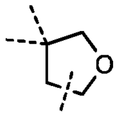, 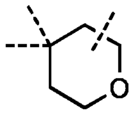, 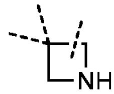, 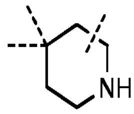,

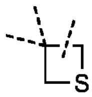 and 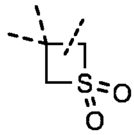.

**11.** The compound or the pharmaceutically acceptable salt thereof as claimed in claim 1, wherein, $R_5$ is selected from H and $CH_3$.

**12.** The compound or the pharmaceutically acceptable salt thereof as claimed in claim 1, wherein, $R_6$ is selected from H and $CH_3$, and $R_7$ is selected from H and $CH_3$.

**13.** The compound or the pharmaceutically acceptable salt thereof as claimed in claim 1, wherein, $R_8$ is selected from H and $CH_3$, and $R_9$ is selected from H and $CH_3$.

**14.** The compound or the pharmaceutically acceptable salt thereof as claimed in claim 1, wherein, ring B is selected from cyclopropyl, cyclobutyl, cyclopentyl and cyclohexyl, and the cyclopropyl, cyclobutyl, cyclopentyl and cyclohexyl are optionally substituted by 1, 2 or 3 $R_c$.

**15.** The compound or the pharmaceutically acceptable salt thereof as claimed in claim 14, wherein, ring B is selected from

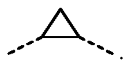.

**16.** The compound or the pharmaceutically acceptable salt thereof as claimed in claim 1, wherein, structural moiety

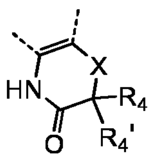

is selected from

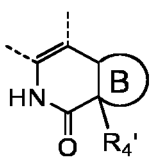, 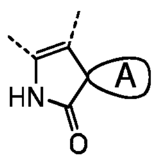,

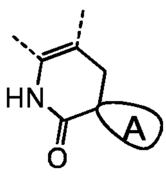 and 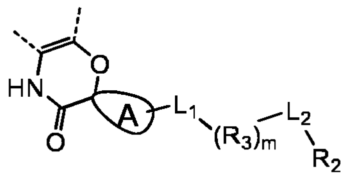,

and ring A, ring B, $R_2$, $R_3$, $R_4$', $L_1$, $L_2$ and m are as defined in claim 1.

**17.** The compound or the pharmaceutically acceptable salt thereof as claimed in claim 1, wherein, $-L_1-(R_3)_m-L_2-R_2$ is selected from H, OH,

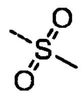,

$CH_3$,

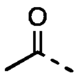, 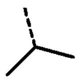, 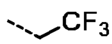,

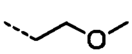, 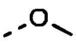, 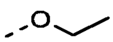, 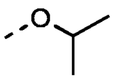, 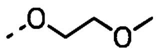, 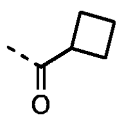, 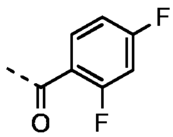, 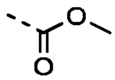,

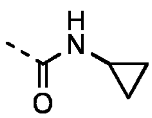 and 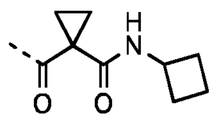.

**18.** The compound or the pharmaceutically acceptable salt thereof as claimed in any one of claims 1, 10, 15, 16 or 17, wherein, structural moiety

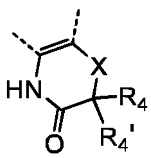

is selected from

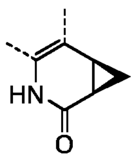, 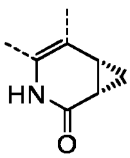, 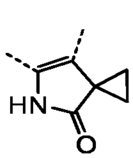, 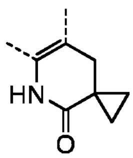, 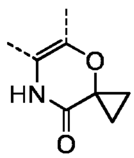, 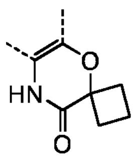, 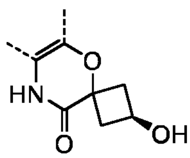,

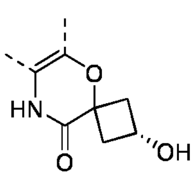, 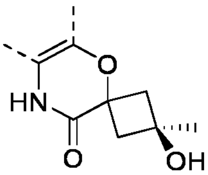, 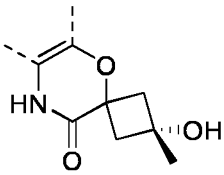, 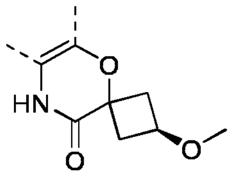, 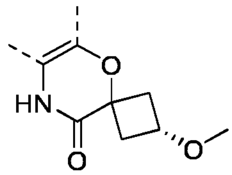,

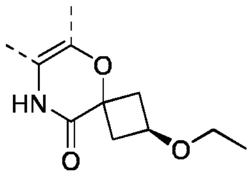, 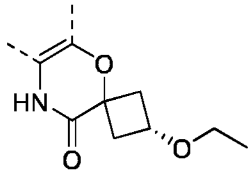, 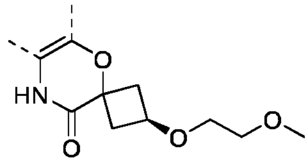, 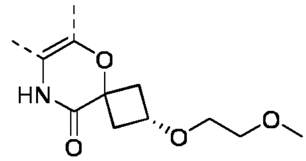,

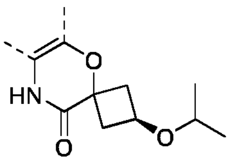, 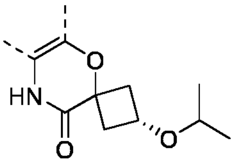, 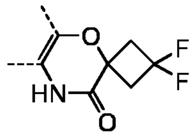, 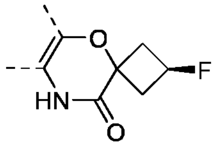, 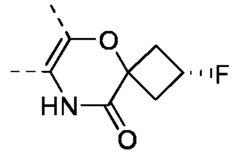,

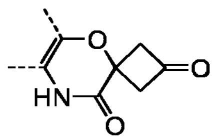, 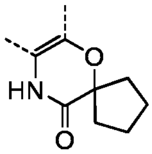, 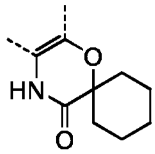, 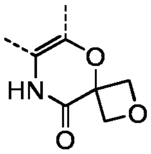, 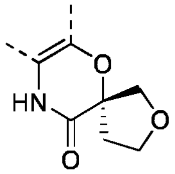, 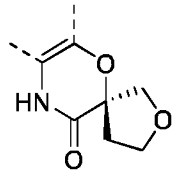,

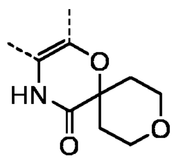, 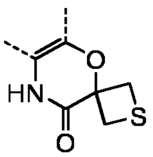, 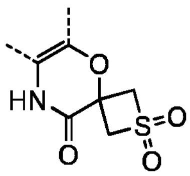, 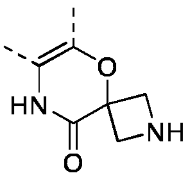, 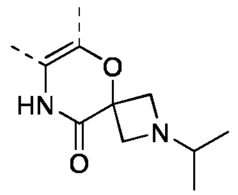,

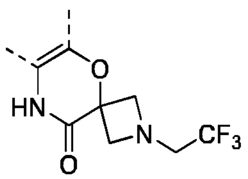, 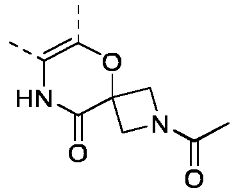, 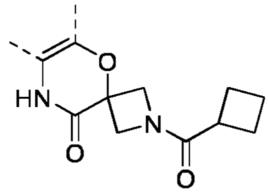, 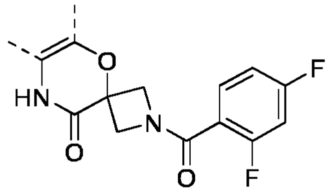,

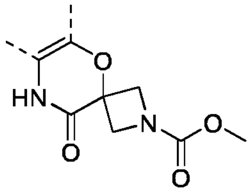, 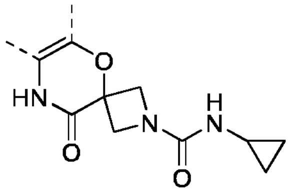, 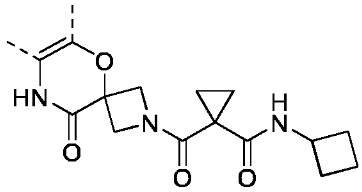, 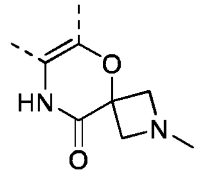,

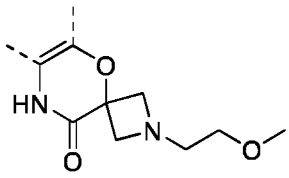
,
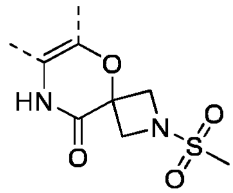
and
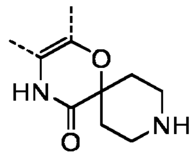
.

**19.** The compound or the pharmaceutically acceptable salt thereof as claimed in any one of claims 1 to 18, which is selected from:

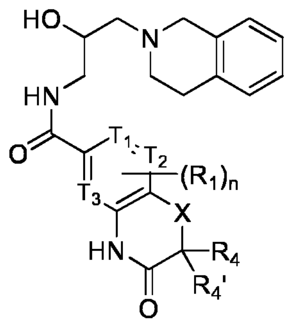

( IV)

,

wherein,
$T_1$, $T_2$, $T_3$, $R_1$, $R_4$, $R_4$', X and n are as defined in any one of claims 1 to 18.

**20.** The compound or the pharmaceutically acceptable salt thereof as claimed in any one of claims 1 to 18, which is selected from:

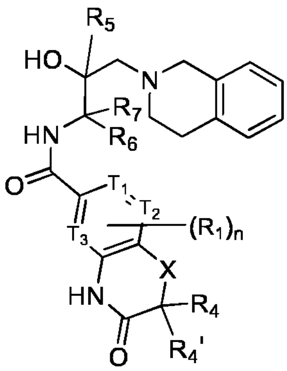

( V )

,

wherein,
$T_1$, $T_2$, $T_3$, $R_1$, $R_4$, $R_4$', $R_5$, $R_6$, $R_7$, X and n are as defined in any one of claims 1 to 18.

**21.** The compound or the pharmaceutically acceptable salt thereof as claimed in claim 1, which is selected from:

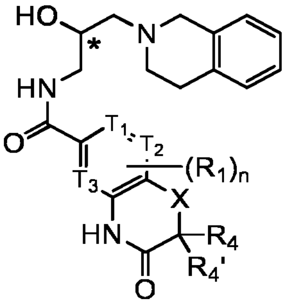
( IV-1) ,

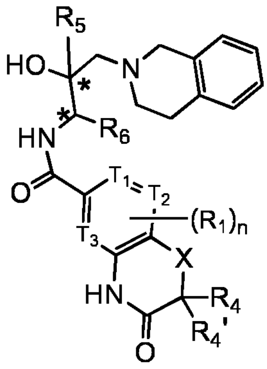
( V-1 ) and

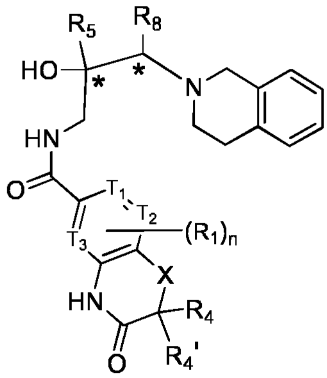
( VII-1 ) ,

wherein,

$T_1$, $T_2$, $T_3$, $R_1$, $R_4$, $R_4$', $R_5$, X and n are as defined in claim 1;
$R_6$ is selected from F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
$R_8$ is selected from F, Cl, $C_{3-6}$ cycloalkyl and $C_{1-3}$ alkyl, and the $C_{3-6}$ cycloalkyl and $C_{1-3}$ alkyl are optionally substituted by 1, 2 or 3 F;
the carbon atom with "*" is a chiral carbon atom, which exists in a (*R*) or (*S*) single enantiomer form or a (*R*) or (*S*) single enantiomer-rich form.

**22.** The compound or the pharmaceutically acceptable salt thereof as claimed in claim 21, which is selected from:

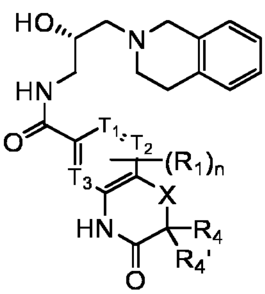
( IV-1-1) ,

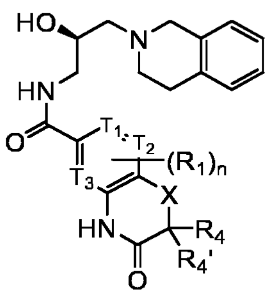
( IV-1-2) and

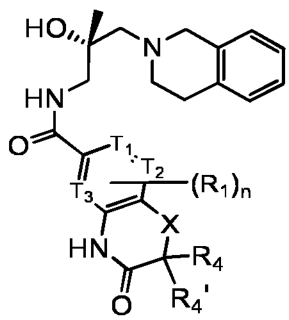
( V-1-1 ) ,

wherein,
$T_1$, $T_2$, $T_3$, $R_1$, $R_4$, $R_4$', X and n are as defined in claim 21.

**23.** The compound or the pharmaceutically acceptable salt thereof as claimed in claim 22, which is selected from:

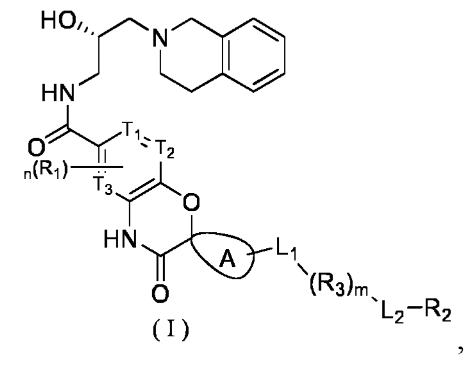
( I ) ,

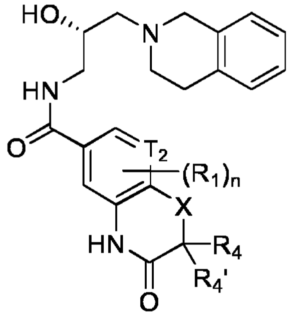
( IV-1-1-1) and

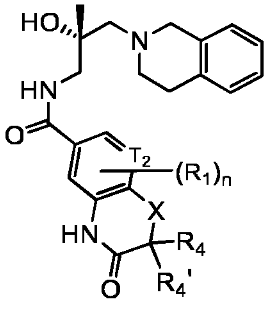
( V-1-1-1 ) ,

wherein, $T_1$, $T_2$, $T_3$, $R_1$, $R_2$, $R_3$, $R_4$, $R_4'$, $L_1$, $L_2$, X, ring A, m and n are as defined in claim 22.

**24.** The compound or the pharmaceutically acceptable salt thereof as claimed in any one of claims 21 to 23, which is selected from:

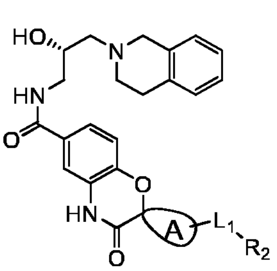
( I-a ) ,

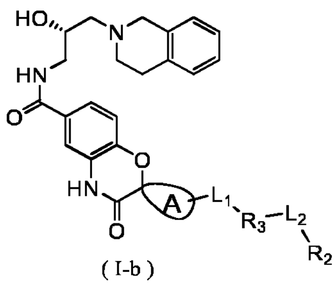
( I-b ) ,

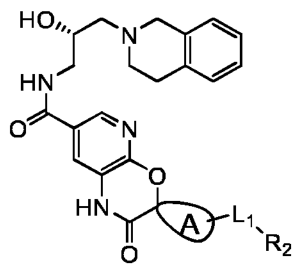
( II-a ) ,

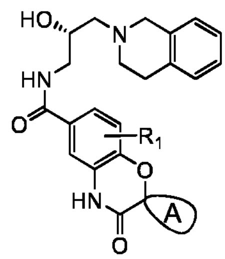
( IV-a ) ,

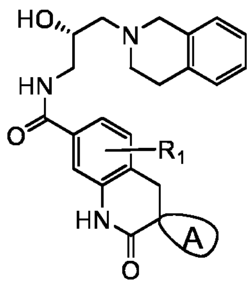
( IV-b ) ,

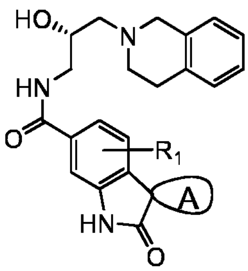
( IV-c ) ,

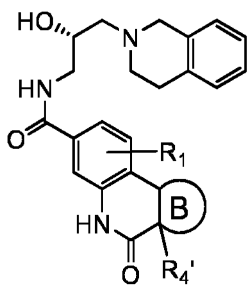
( IV-d ) ,

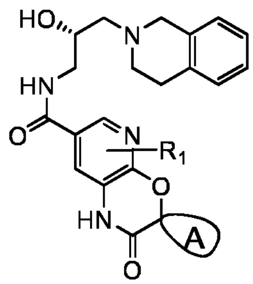
( IV-e ) ,

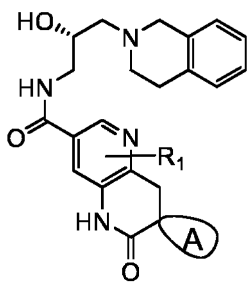
( IV-f ) ,

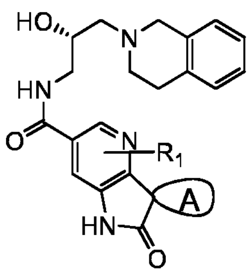
( IV-g ) ,

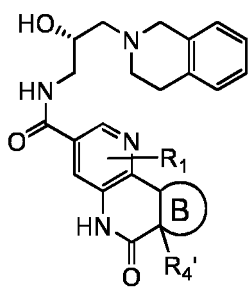
( IV-h ) ,

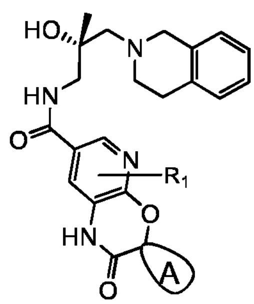
( V-a ) ,

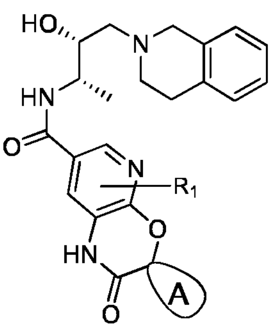
( VII-a ) ,

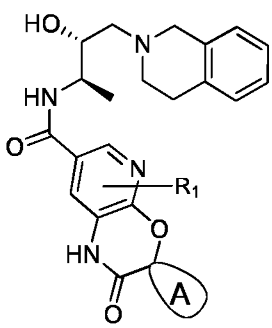
( VII-b ) ,

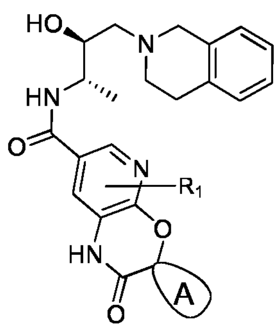
( VII-c ) ,

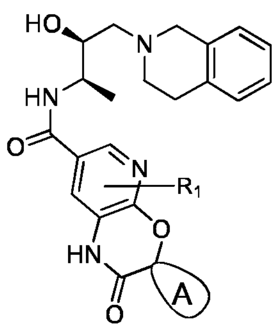
( VII-d ) ,

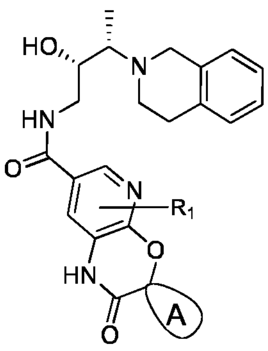

( VII-e ) ,

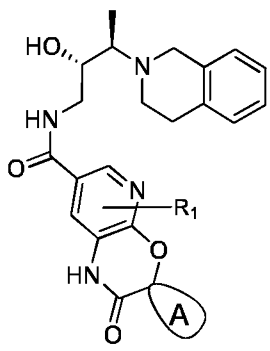

( VII-f ) ,

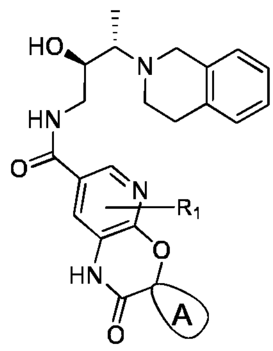

( VII-g )

and

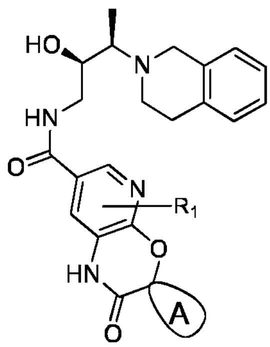

( VII-h ) ,

wherein, ring A, ring B, $R_1$, $R_2$, $R_3$, $R_4$', $L_1$ and $L_2$ are as defined in any one of claims 21 to 23.

**25.** A compound represented by the following formulas or a pharmaceutically acceptable salt thereof:

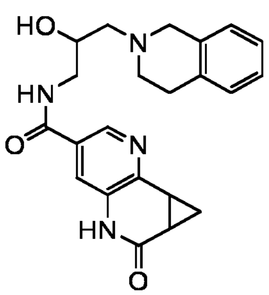

,

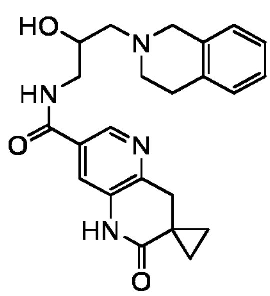

,

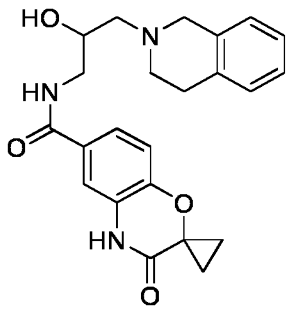

,

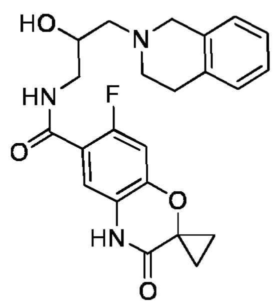

,

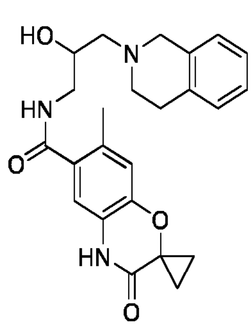 , 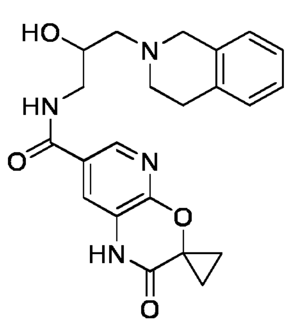 , 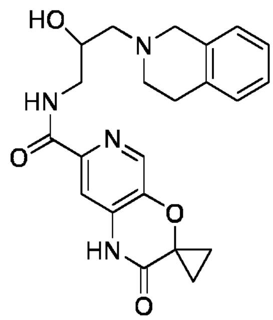 , 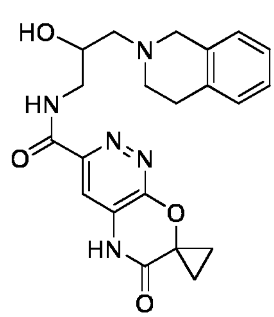 ,

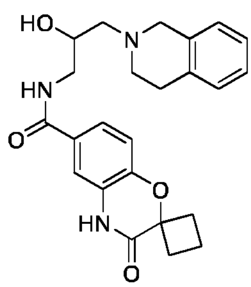 , 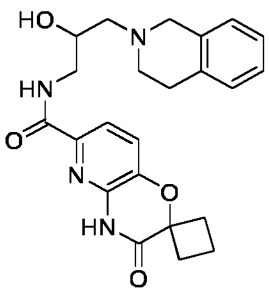 , 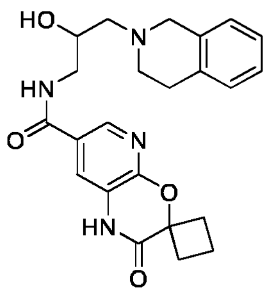 , 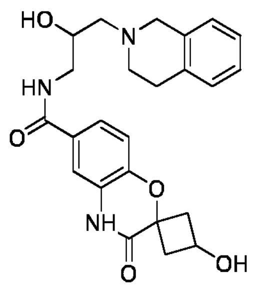 ,

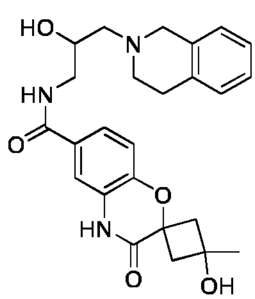 , 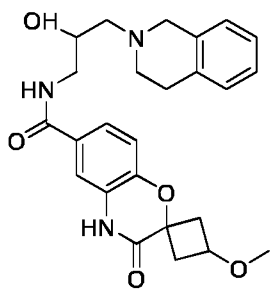 , 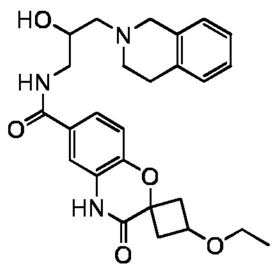 , 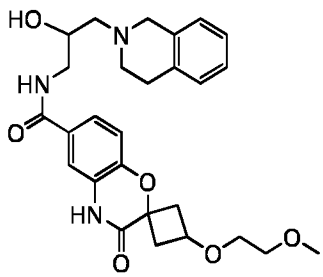 ,

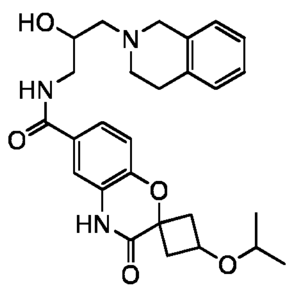 , 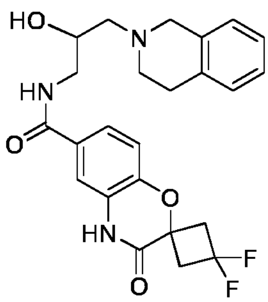 , 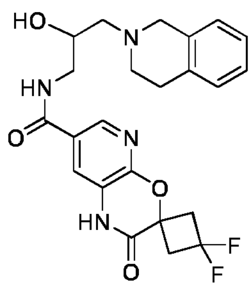 , 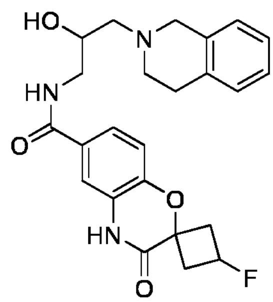 ,

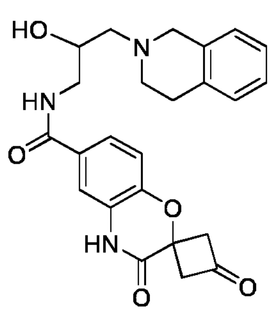 , 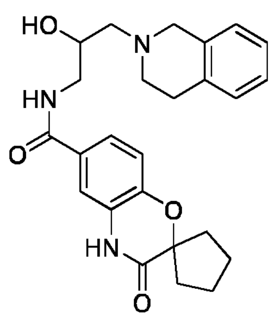 , 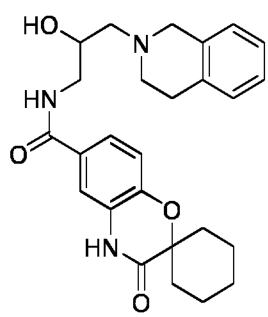 , 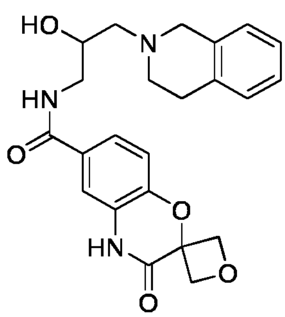 ,

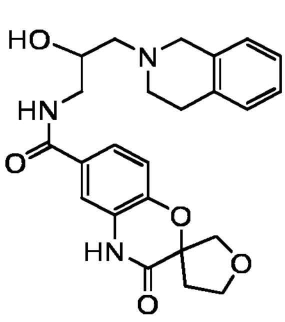,
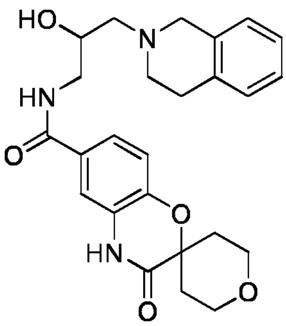,
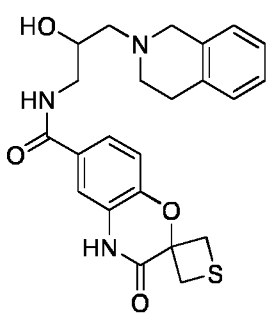,
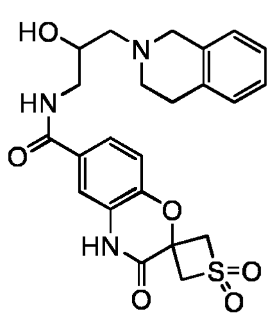,

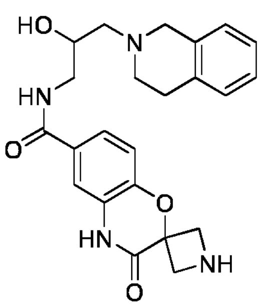,
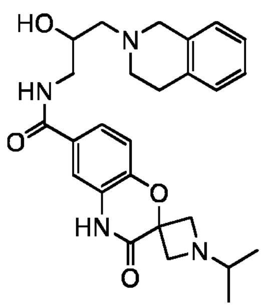,
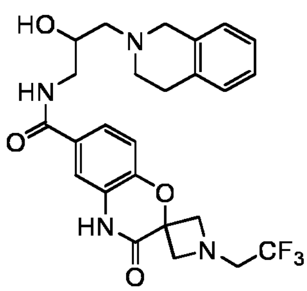,
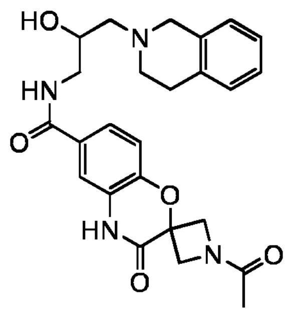,

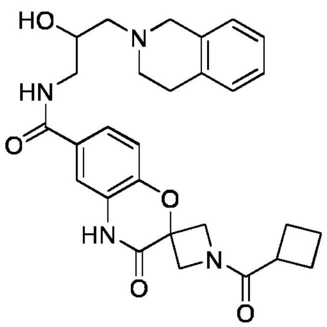,
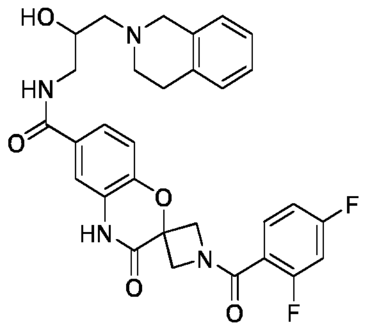,
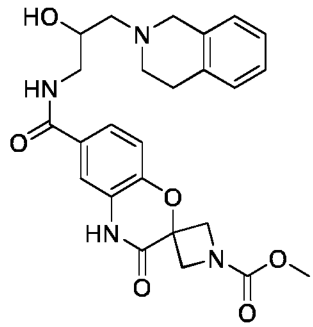,

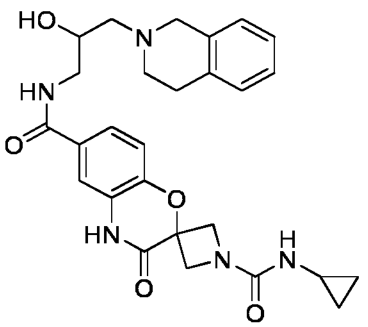,
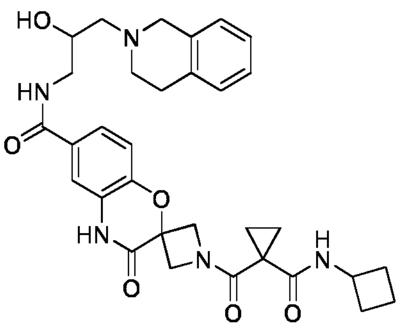,
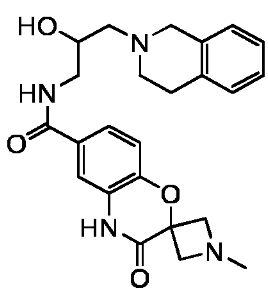,

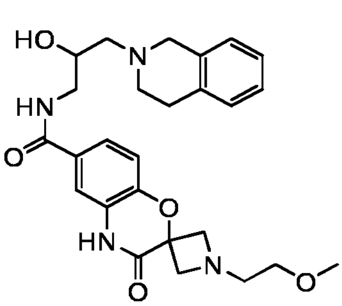,
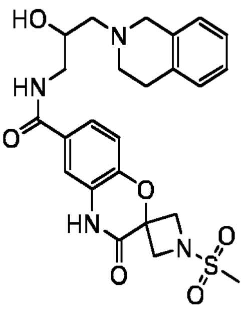,
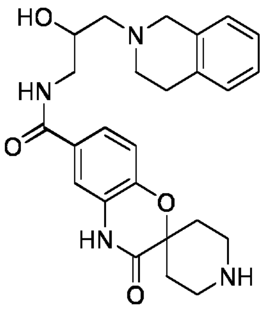,
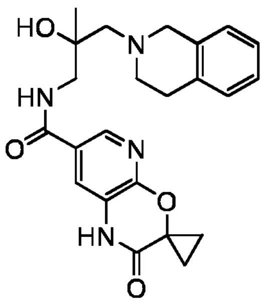,

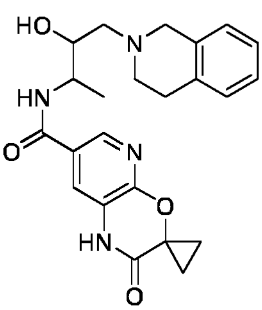,
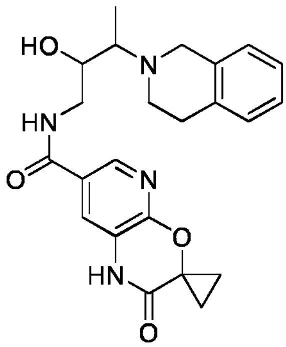,
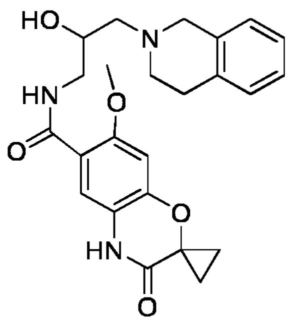,

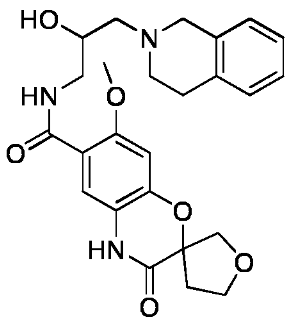
and
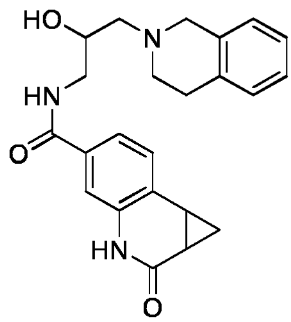.

**26.** A compound represented by the following formulas or a pharmaceutically acceptable salt thereof:

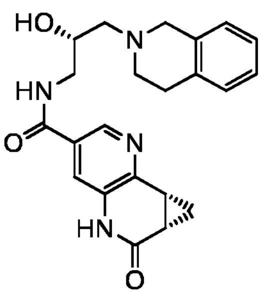,
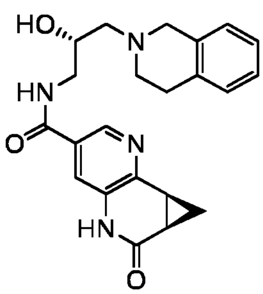,
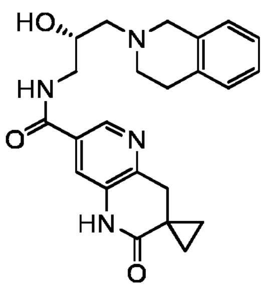,
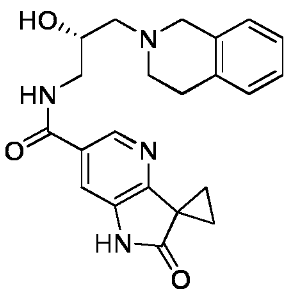,

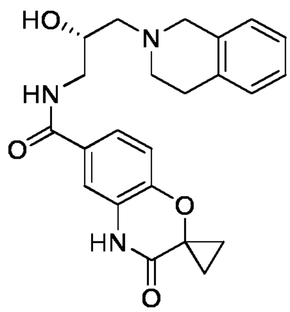,
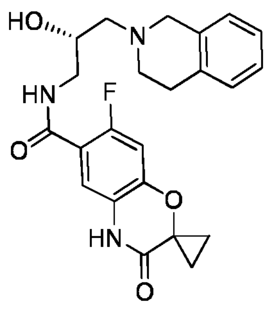,
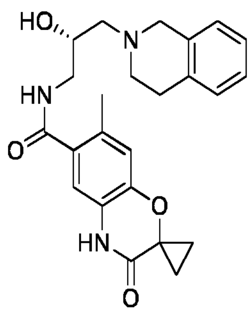,
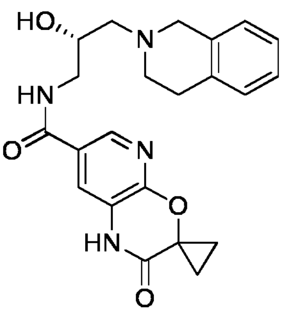,

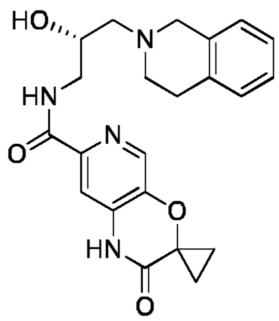,
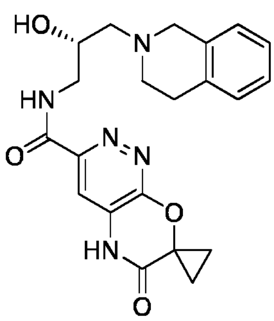,
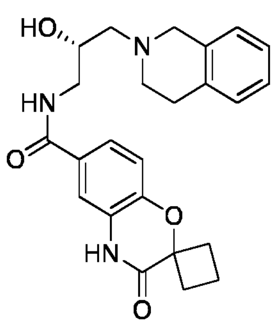,
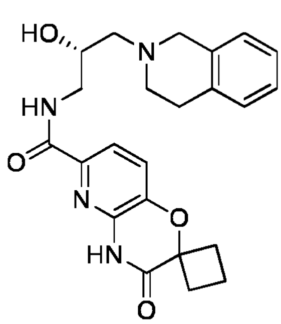,

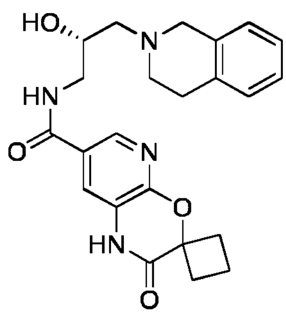,
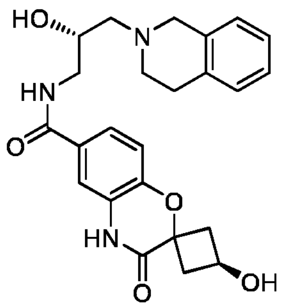,
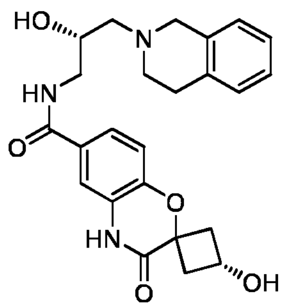,
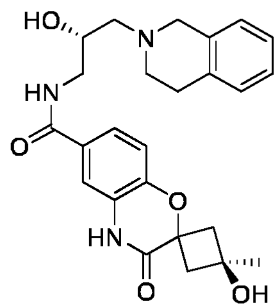,

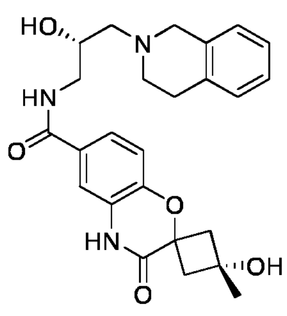,
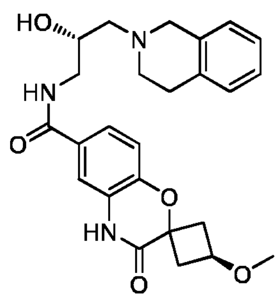,
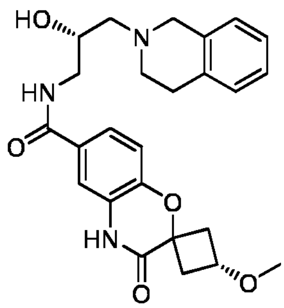,
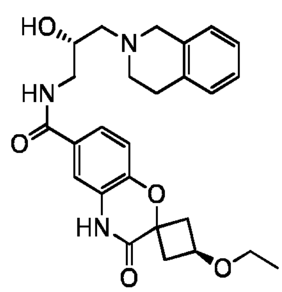,

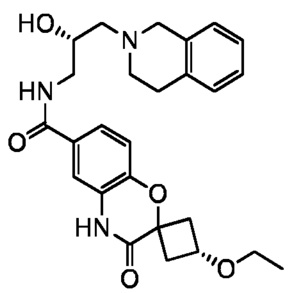,
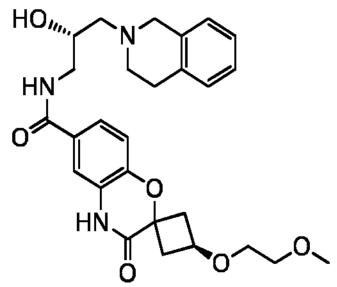,
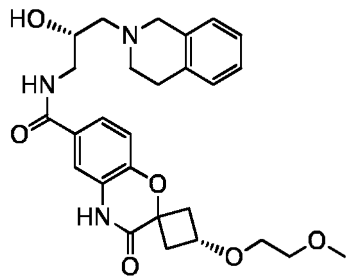,

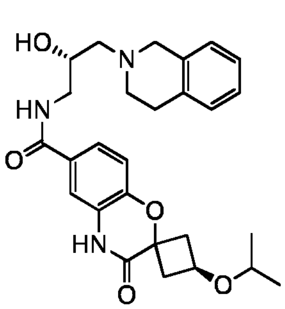 , 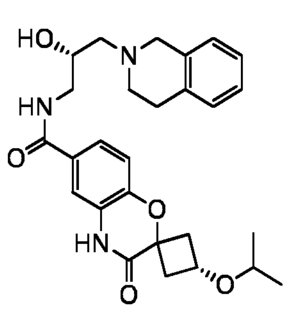 , 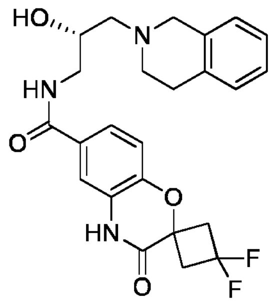 , 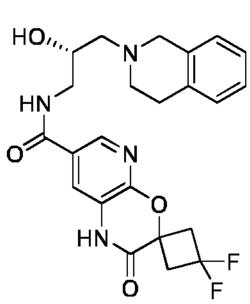 ,

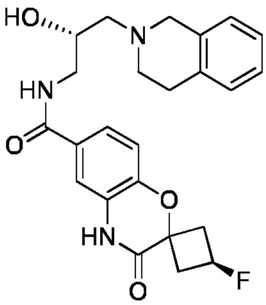 , 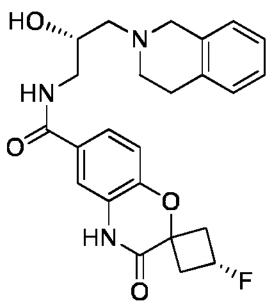 , 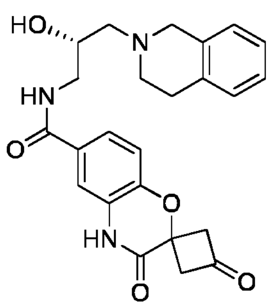 , 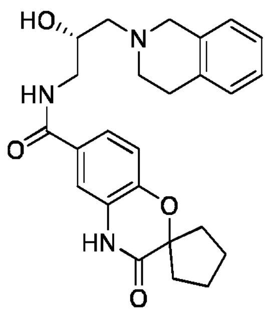 ,

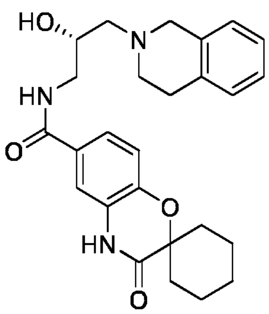 , 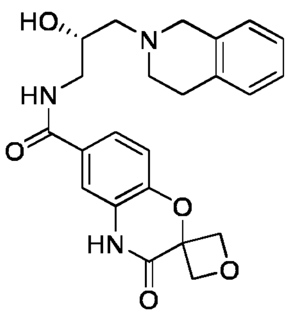 , 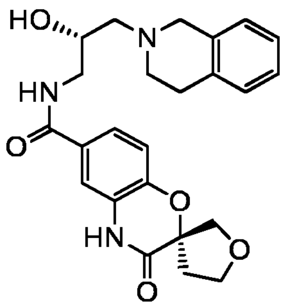 , 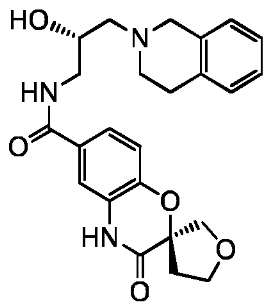 ,

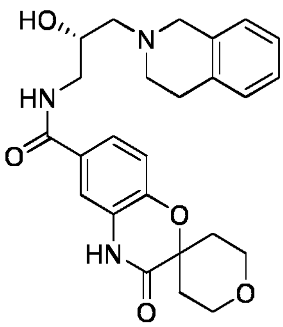 , 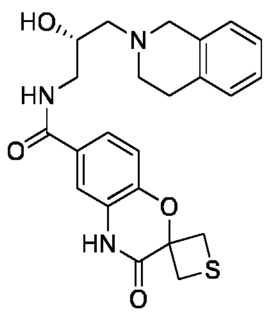 , 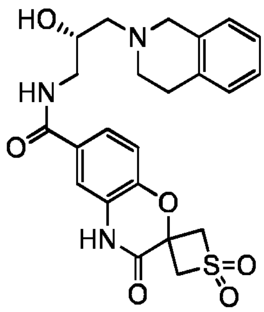 , 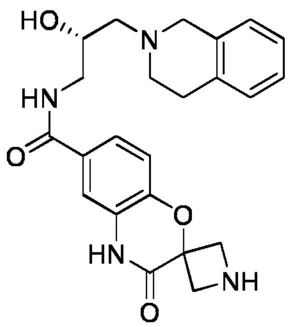 ,

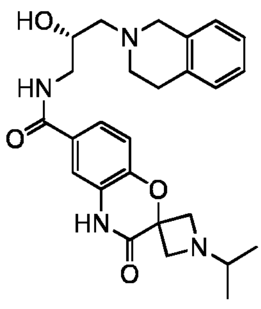 , 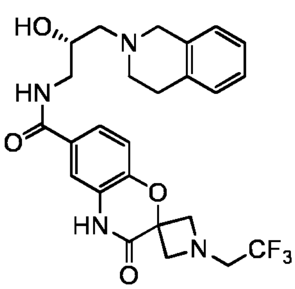 , 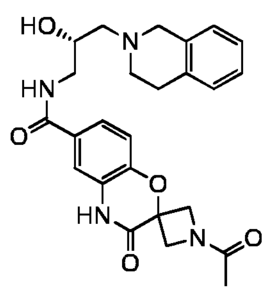 , 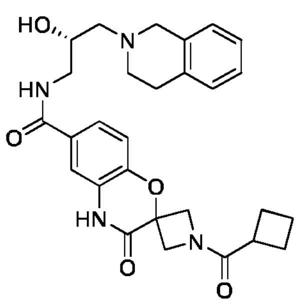 ,

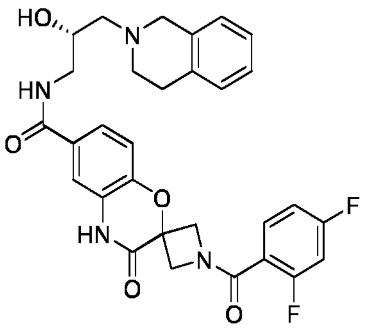
,
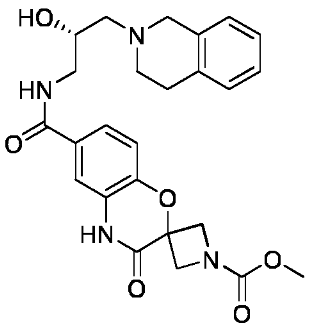
,
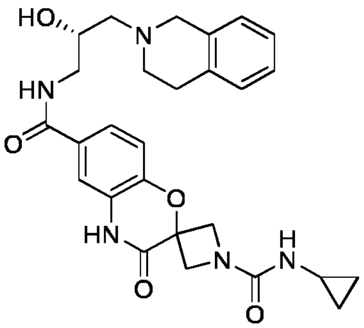
,
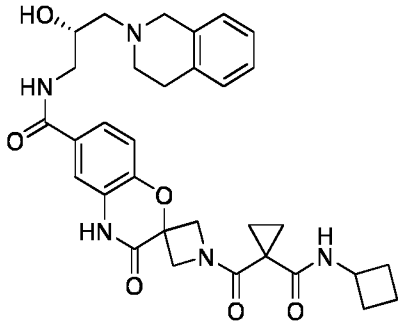
,
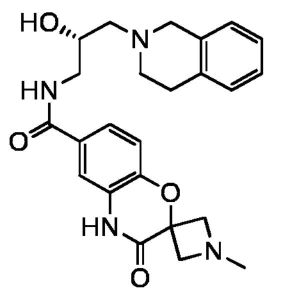
,
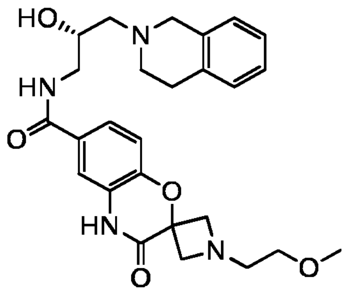
,
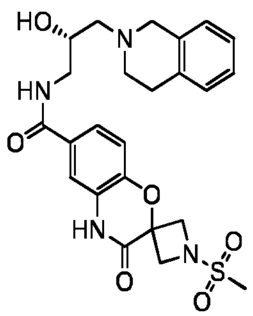
,
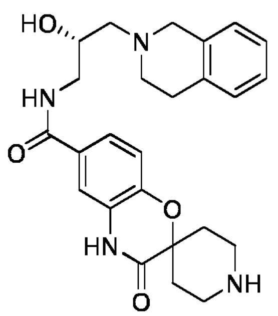
,
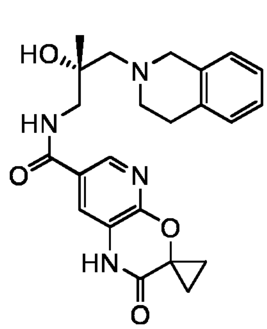
,
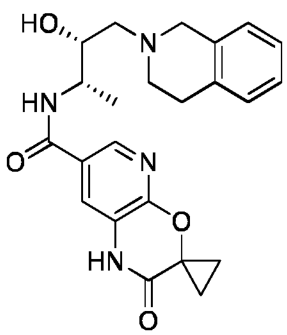
,
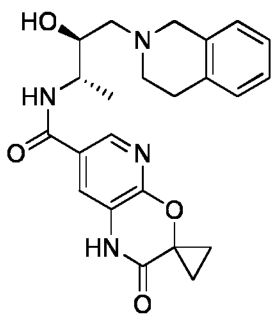
,
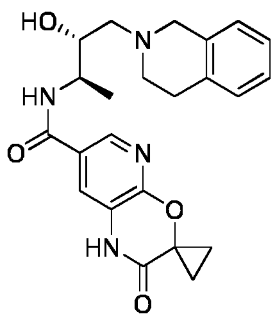
,
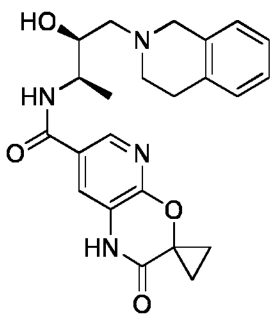
,
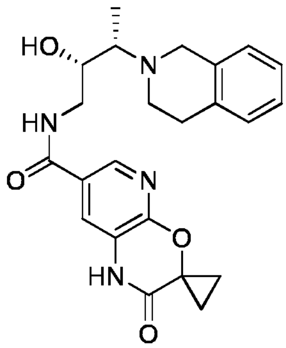
,

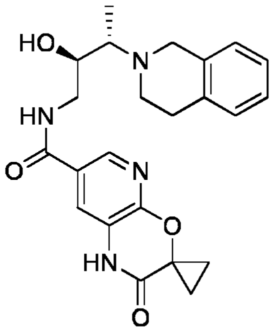,
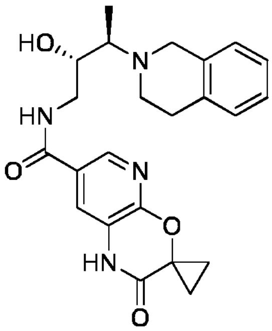,
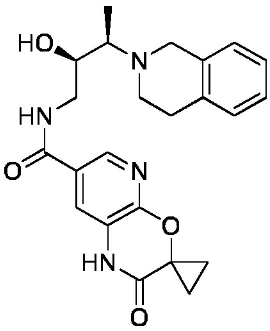,
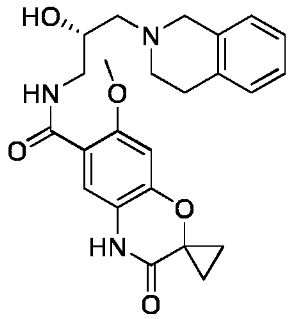,
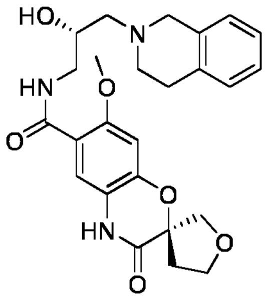,
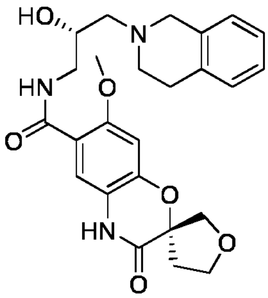,
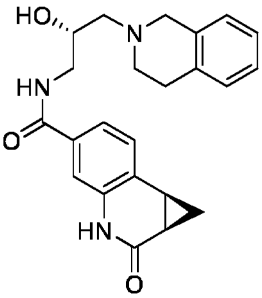 and
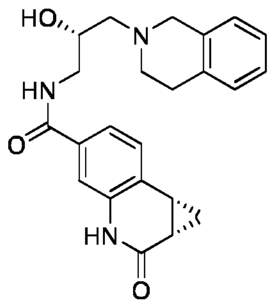.

**27.** A crystal form A of compound **46a,** wherein, the crystal form A has an X-ray powder diffraction pattern using Cu Kα radiation and having characteristic diffraction peaks at the following 2θ angles: 4.70±0.20°, 11.32±0.20°, 13.35±0.20°, 16.25±0.20°, 17.36±0.20°.

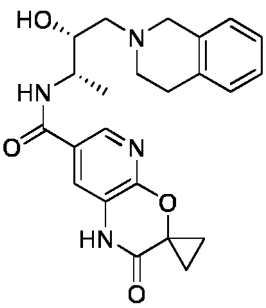

**46a**

**28.** The crystal form A as claimed in claim 27, wherein, the X-ray powder diffraction pattern of the crystal form A using Cu Kα radiation has characteristic diffraction peaks at the following 2θ angles: 4.70±0.20°, 8.68±0.20°, 9.35±0.20°, 11.32±0.20°, 13.35±0.20°, 16.25±0.20°, 17.36±0.20°, 23.22±0.20°.

**29.** The crystal form A as claimed in claim 28, wherein, the X-ray powder diffraction pattern of the crystal form A using Cu Kα radiation has characteristic diffraction peaks at the following 2θ angles: 4.70±0.20°, 8.68±0.20°, 9.35±0.20°, 9.99±0.20°, 11.32±0.20°, 13.35±0.20°, 14.05±0.20°, 16.25±0.20°, 17.36±0.20°, 20.08±0.20°, 23.22±0.20°, 25.65±0.20°.

**30.** The crystal form A as claimed in claim 29, wherein, the X-ray powder diffraction pattern of the crystal form A using Cu Kα radiation has characteristic diffraction peaks at the following 2θ angles: 4.70°, 8.68°, 9.35°, 9.99°, 11.32°, 13.35°, 14.05°, 16.25°, 17.36°, 17.65°, 20.08°, 23.22°, 23.80°, 24.13°, 25.65°, 28.34°, 30.32°, 33.54°.

**31.** The crystal form A as claimed in claim 30, wherein, the XRPD pattern of the crystal form A is shown in FIG. 3.

**32.** A crystal form B of compound **46a,** wherein, the crystal form B has an X-ray powder diffraction pattern using Cu Kα radiation and having characteristic diffraction peaks at the following 2θ angles: 7.12±0.20°, 10.69±0.20°, 12.74±0.20°, 14.28±0.20°, 15.18±0.20°.

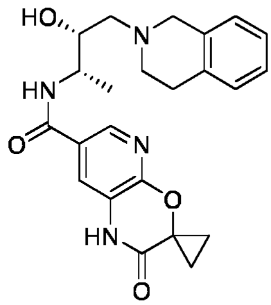

**46a**

**33.** The crystal form B as claimed in claim 32, wherein, the X-ray powder diffraction pattern of the crystal form B using Cu Kα radiation has characteristic diffraction peaks at the following 2θ angles: 7.12±0.20°, 10.15±0.20°, 10.69±0.20°, 12.74±0.20°, 14.28±0.20°, 15.18±0.20°, 17.87±0.20°, 25.12±0.20°.

**34.** The crystal form B as claimed in claim 33, wherein, the X-ray powder diffraction pattern of the crystal form B using Cu Kα radiation has characteristic diffraction peaks at the following 2θ angles: 3.61±0.20°, 7.12±0.20°, 10.15±0.20°, 10.69±0.20°, 12.74±0.20°, 14.28±0.20°, 15.18±0.20°, 17.87±0.20°, 20.39±0.20°, 21.09±0.20°, 23.86±0.20°, 25.12±0.20°.

**35.** The crystal form B as claimed in claim 34, wherein, the X-ray powder diffraction pattern of the crystal form B using Cu Kα radiation has characteristic diffraction peaks at the following 2θ angles: 3.61°, 7.12°, 10.16°, 10.69°, 10.95°, 12.08°, 12.74°, 14.28°, 15.18°, 17.07°, 17.87°, 20.08°, 20.39°, 21.09°, 22.79°, 23.29°, 23.86°, 24.77°, 25.12°, 26.37°, 30.02°, 30.68°, 31.23°, 32.46°, 34.80°, 36.18°, 37.13°.

**36.** The crystal form B as claimed in claim 35, wherein, the XRPD pattern of the crystal form B is shown in FIG. 7.

**37.** A use of the compound or the pharmaceutically acceptable salt thereof as claimed in any one of claims 1 to 25 in the manufacture of a medicament related to an inhibitor of treating protein arginine methyltransferase 5.

**38.** A use of the crystal form A as claimed in any one of claims 27 to 31 or the crystal form B as claimed in any one of claims 32 to 36 in the manufacture of a medicament related to an inhibitor of treating protein arginine methyltransferase 5.

**39.** The use as claimed in claim 37 or 38, wherein, the medicament related to the inhibitor of protein arginine methyltransferase 5 is a medicament for the prevention and/or treatment of diseases related to lymphoma and solid tumor.

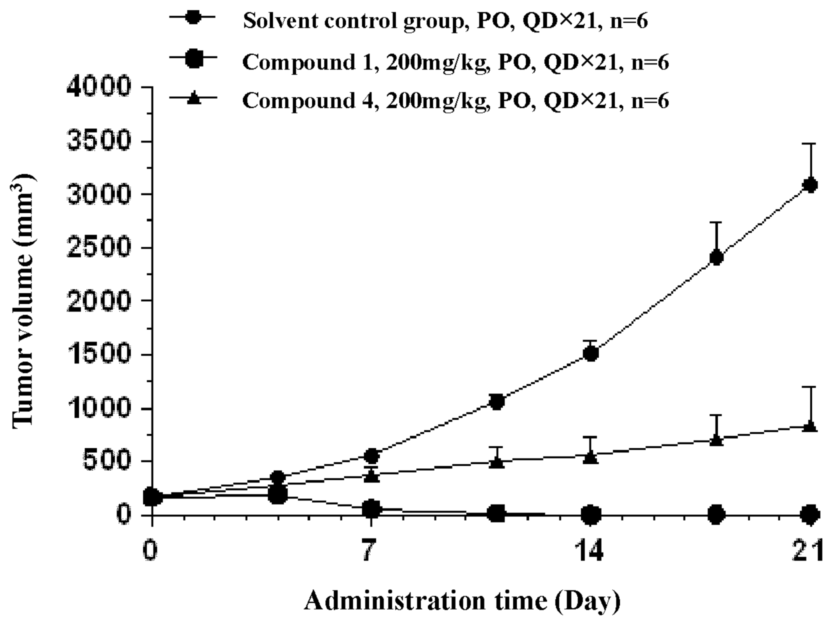
Solvent control group, PO, QD×21, n=6
Compound 1, 200mg/kg, PO, QD×21, n=6
Compound 4, 200mg/kg, PO, QD×21, n=6
4000
3500
3000
2500
2000
1500
1000
500
0
Tumor volume (mm3)
0
7
14
21
Administration time (Day)

FIG. 1

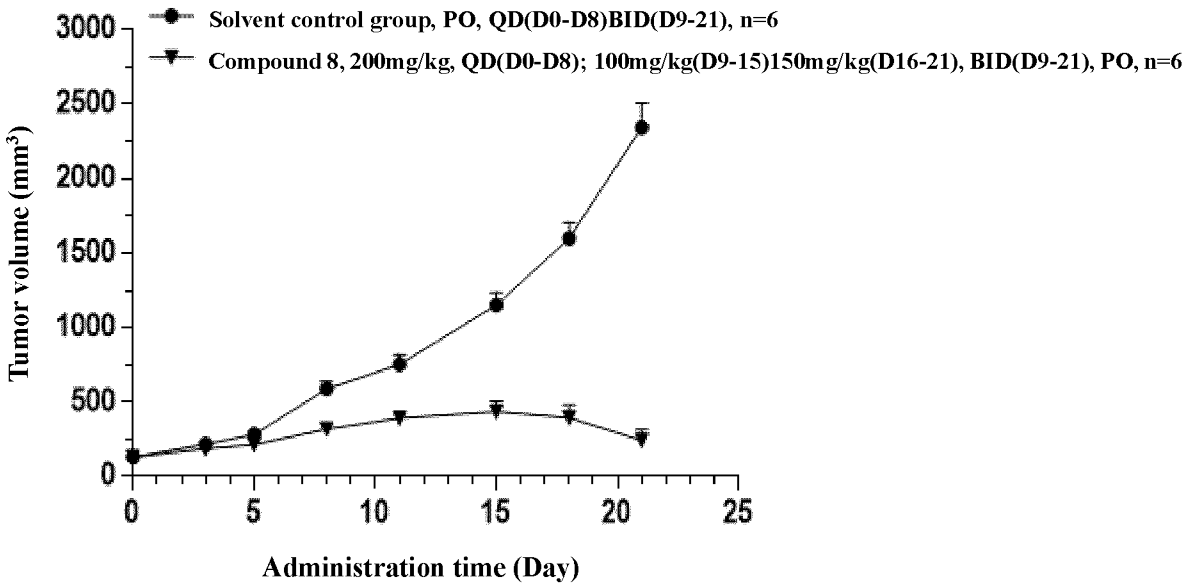
Solvent control group, PO, QD(D0-D8)BID(D9-21), n=6
Compound 8, 200mg/kg, QD(D0-D8); 100mg/kg(D9-15)150mg/kg(D16-21), BID(D9-21), PO, n=6
3000
2500
2000
1500
1000
500
0
Tumor volume (mm3)
0
5
10
15
20
25
Administration time (Day)

FIG. 2

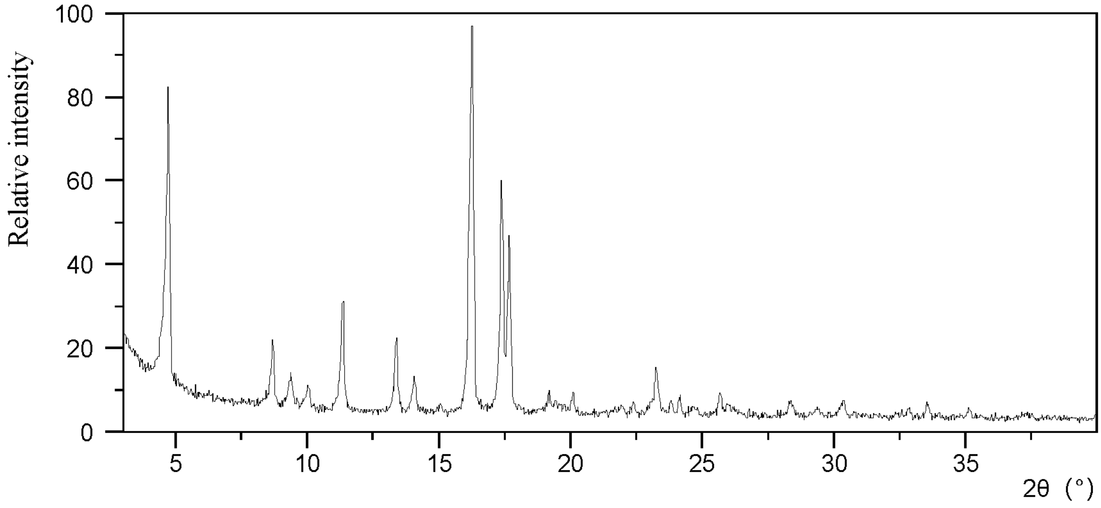
Relative intensity
100
80
60
40
20
0
5
10
15
20
25
30
35
2θ (°)

FIG. 3

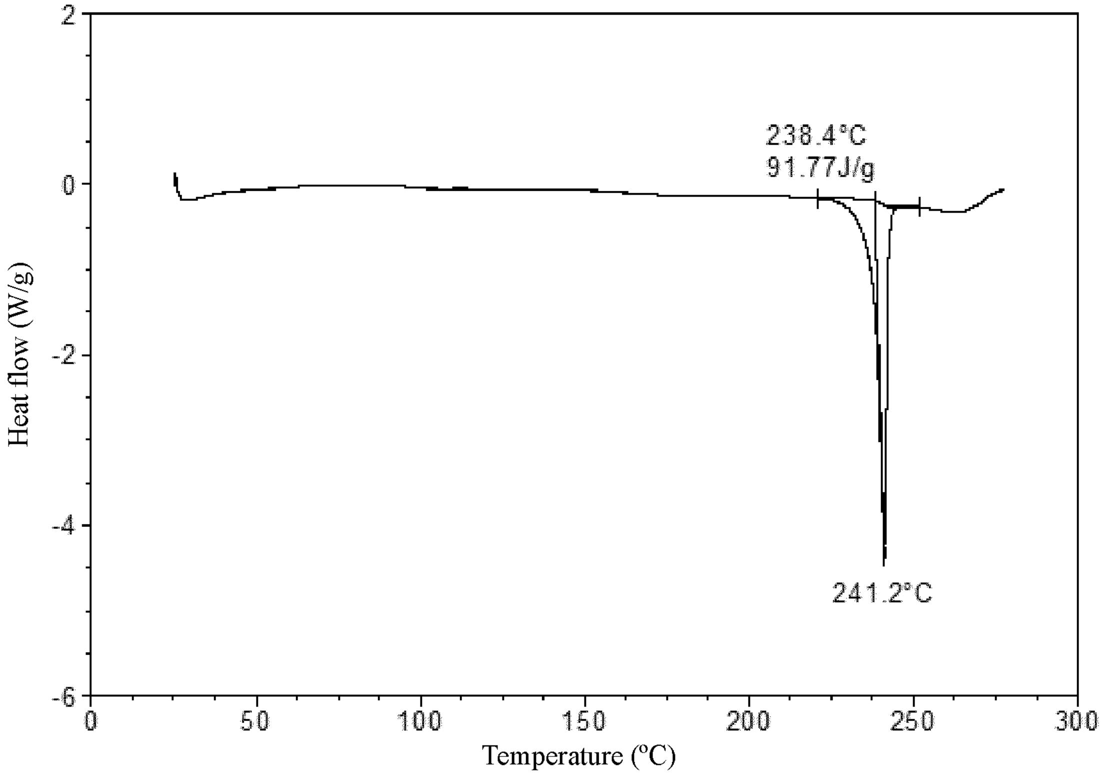
Heat flow (W/g)
2
0
-2
-4
-6
0
50
100
150
200
250
300
Temperature (ºC)
238.4°C
91.77J/g
241.2°C

FIG. 4

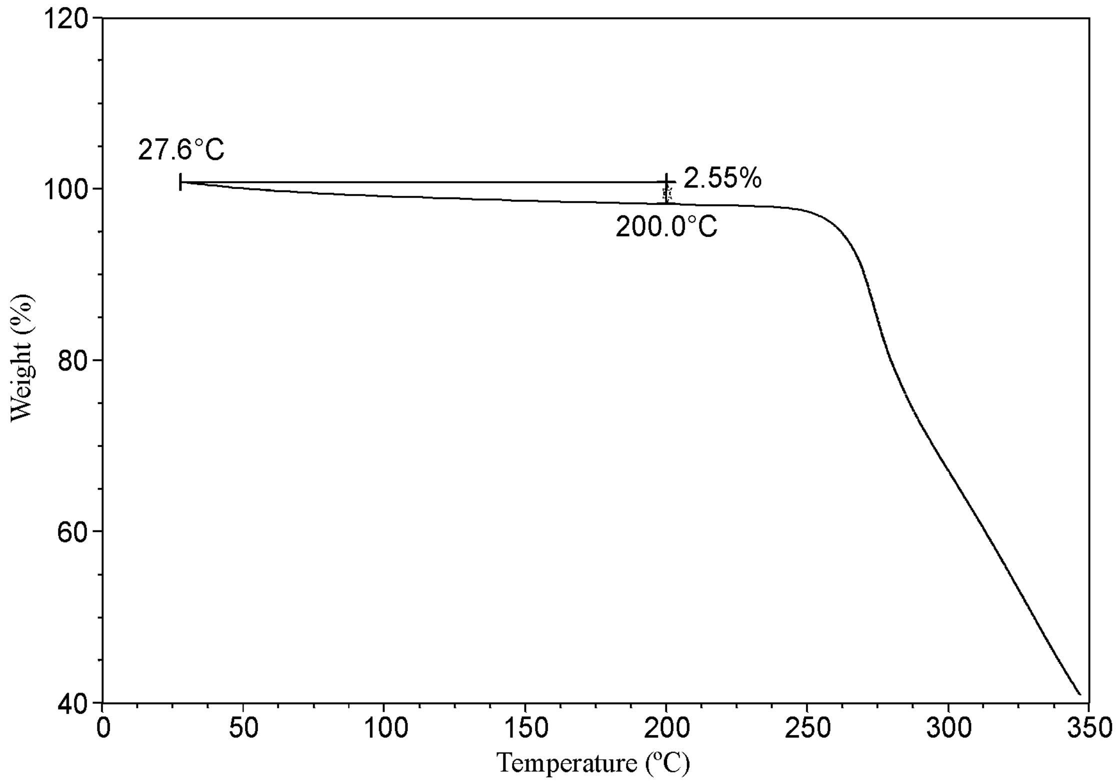
27.6°C
2.55%
200.0°C
Weight (%)
Temperature (°C)
120
100
80
60
40
0
50
100
150
200
250
300
350

FIG. 5

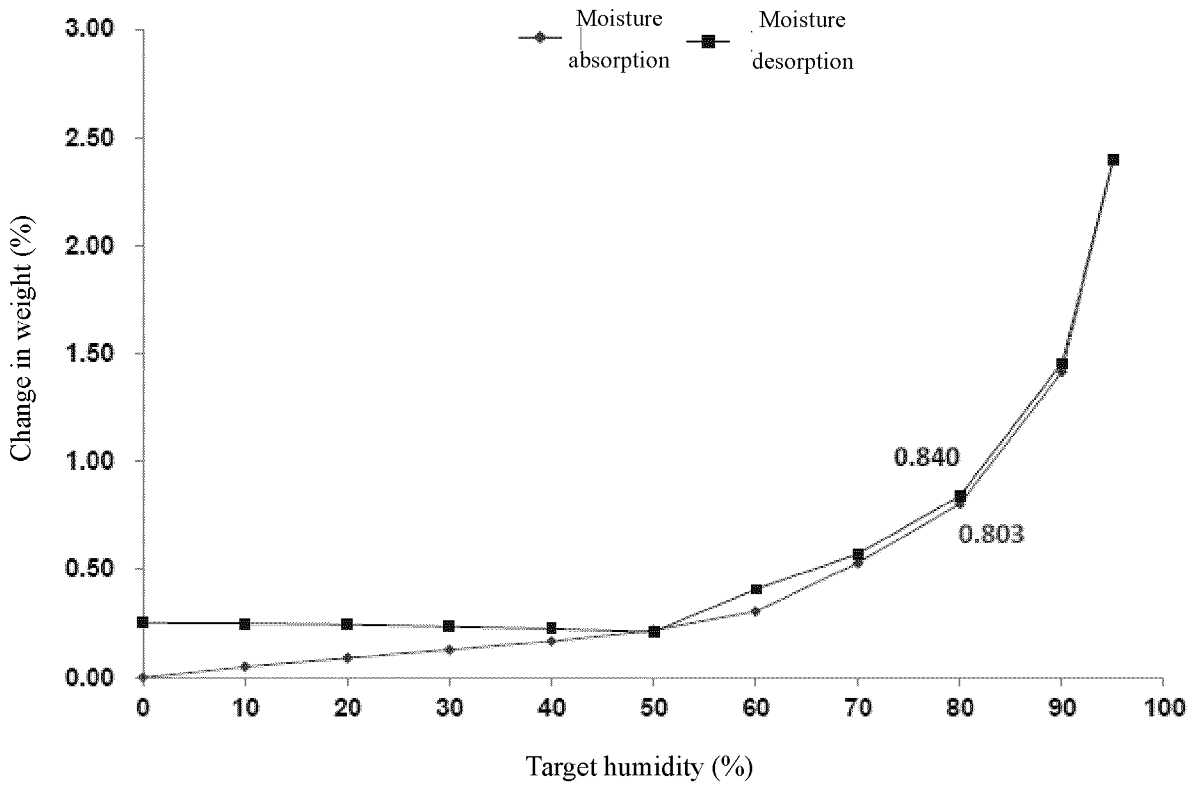
Moisture absorption
Moisture desorption
0.840
0.803
Change in weight (%)
Target humidity (%)
3.00
2.50
2.00
1.50
1.00
0.50
0.00
0
10
20
30
40
50
60
70
80
90
100

FIG. 6

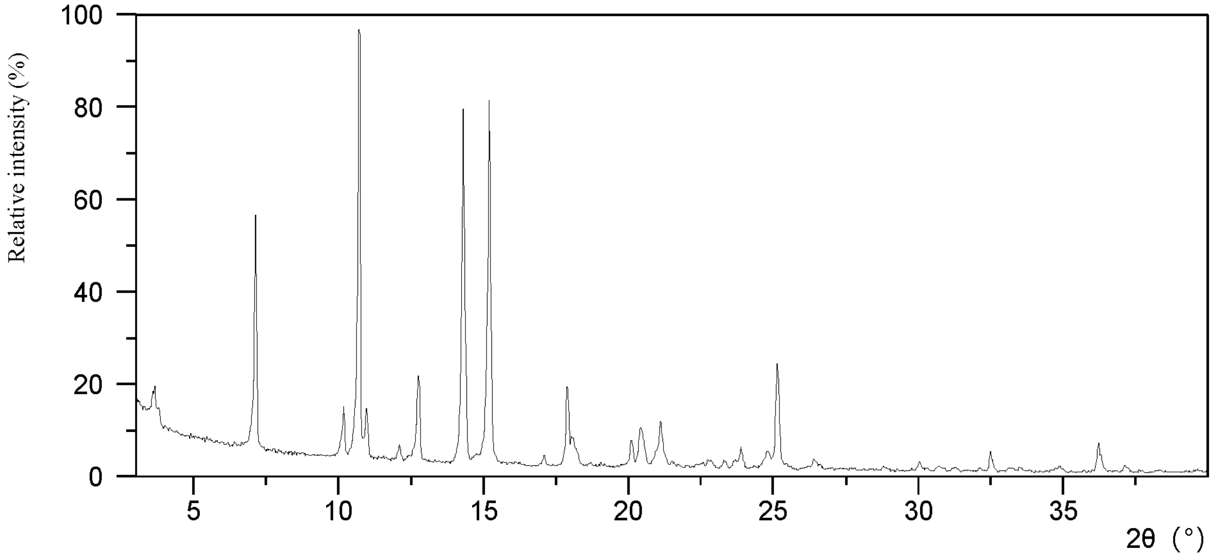
100
80
60
40
20
0
Relative intensity (%)
5
10
15
20
25
30
35
2θ (°)

FIG. 7

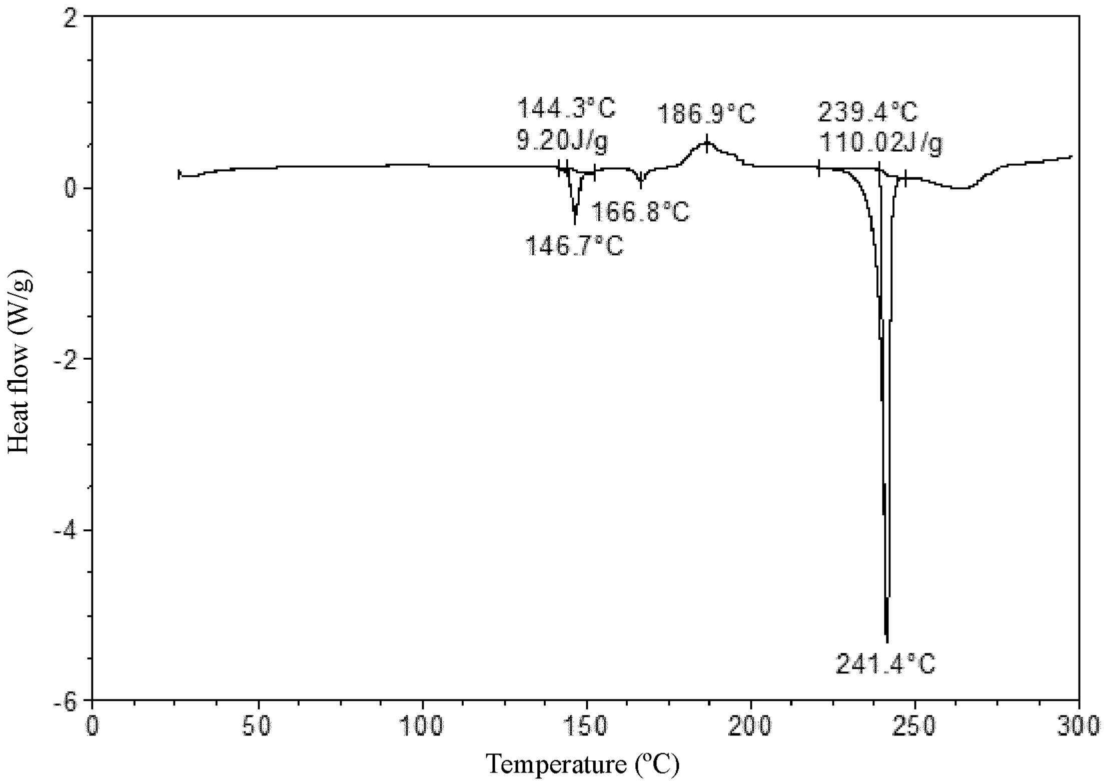
2
0
-2
-4
-6
Heat flow (W/g)
144.3°C
9.20J/g
146.7°C
166.8°C
186.9°C
239.4°C
110.02J/g
241.4°C
0
50
100
150
200
250
300
Temperature (°C)

FIG. 8

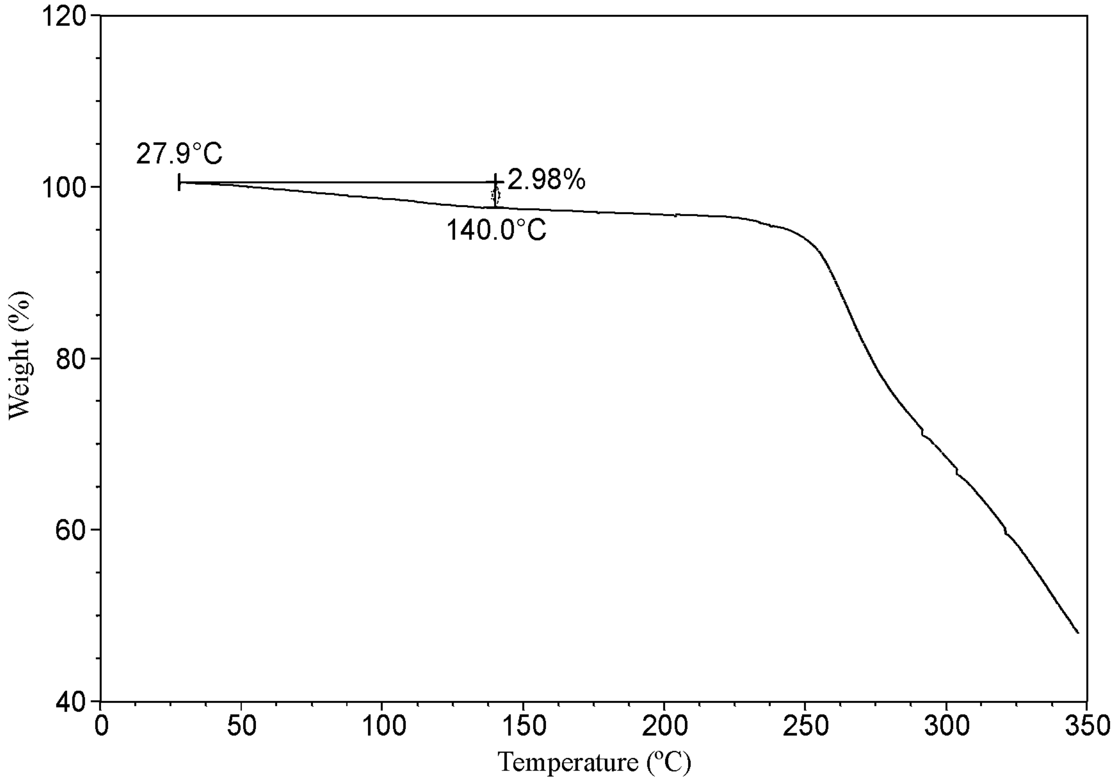
120
100
80
60
40
27.9°C
2.98%
140.0°C
Weight (%)
0
50
100
150
200
250
300
350
Temperature (°C)

FIG. 9

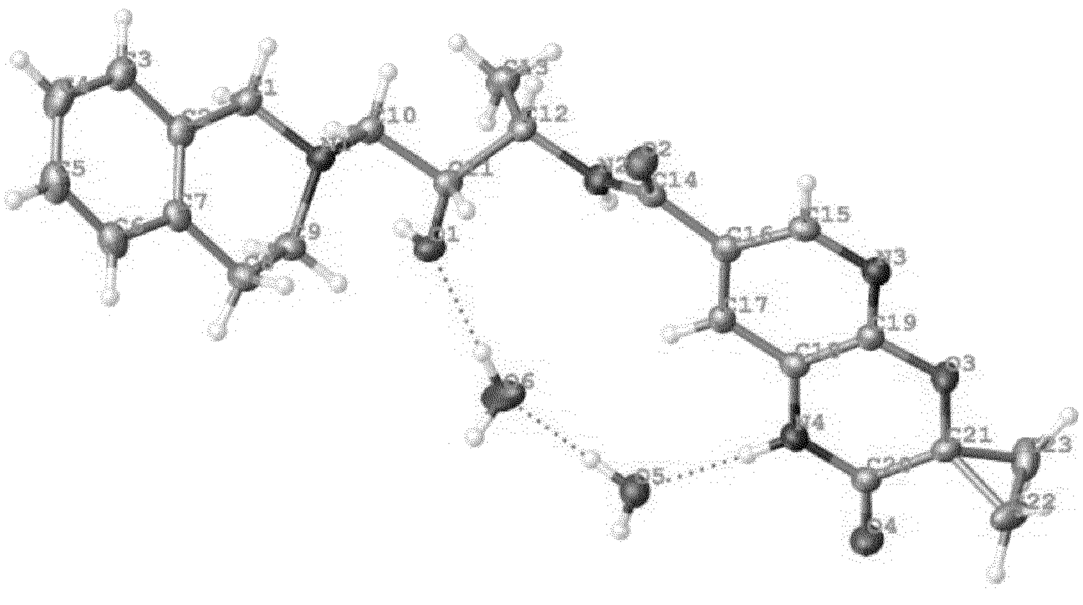

FIG. 10

## INTERNATIONAL SEARCH REPORT

International application No.
**PCT/CN2021/119445**

**A. CLASSIFICATION OF SUBJECT MATTER**

C07D 401/12(2006.01)i; C07D 413/12(2006.01)i; C07D 217/04(2006.01)i; A61K 31/4725(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT, CNKI, WPI, EPODOC, ISI Web of Knowledge, STN(REGISTRY, MARPAT, CAPLUS): 南京明德新药研发有限公司, 四氢异喹啉, 蛋白精氨酸甲基转移酶, tetrahydroisoquinoline, PRMT5, search structural formula

**C. DOCUMENTS CONSIDERED TO BE RELEVANT**

| Category* | Citation of document, with indication, where appropriate, of the relevant passages | Relevant to claim No. |
|---|---|---|
| A | WO 2014100695 A1 (EPIZYME, INC.) 26 June 2014 (2014-06-26) entire document | 1-38 |
| A | CN 105452226 A (EPIZYME, INC.) 30 March 2016 (2016-03-30) entire document | 1-38 |
| A | US 2004044031 A1 (BANYU PHARMA. CO. LTD.) 04 March 2004 (2004-03-04) entire document | 1-38 |
| A | CN 111542523 A (JUBILANT BIOSYS LIMITED) 14 August 2020 (2020-08-14) entire document | 1-38 |
| A | CN 108570059 A (SHANGHAI INSTITUTE OF MATERIA MEDICA, CHINESE ACADEMY OF SCIENCES) 25 September 2018 (2018-09-25) entire document | 1-38 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 December 2021** | **15 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/119445**

| Patent document cited in search report | | | Publication date (day/month/year) | Patent family member(s) | | | Publication date (day/month/year) |
|---|---|---|---|---|---|---|---|
| WO | 2014100695 | A1 | 26 June 2014 | US | 2014228343 | A1 | 14 August 2014 |
| | | | | US | 8906900 | B2 | 09 December 2014 |
| | | | | US | 2018186798 | A1 | 05 July 2018 |
| | | | | US | 2016214985 | A1 | 28 July 2016 |
| | | | | US | 9908887 | B2 | 06 March 2018 |
| | | | | CA | 2894126 | A1 | 26 June 2014 |
| | | | | EP | 2935240 | A1 | 28 October 2015 |
| | | | | US | 2017114061 | A1 | 27 April 2017 |
| | | | | US | 9777008 | B2 | 03 October 2017 |
| | | | | US | 2018298010 | A1 | 18 October 2018 |
| | | | | JP | 2016505000 | A | 18 February 2016 |
| | | | | US | 2015133427 | A1 | 14 May 2015 |
| | | | | US | 9388173 | B2 | 12 July 2016 |
| CN | 105452226 | A | 30 March 2016 | SI | 2935222 | T1 | 28 February 2019 |
| | | | | EP | 3498701 | A1 | 19 June 2019 |
| | | | | IL | 267364 | D0 | 29 August 2019 |
| | | | | HR | P20182037 | T1 | 08 February 2019 |
| | | | | US | 2021308121 | A1 | 07 October 2021 |
| | | | | US | 2018303822 | A1 | 25 October 2018 |
| | | | | US | 10391089 | B2 | 27 August 2019 |
| | | | | EA | 201590975 | A1 | 29 January 2016 |
| | | | | EA | 027908 | B1 | 29 September 2017 |
| | | | | CR | 20150371 | A | 09 November 2015 |
| | | | | PH | 12019500163 | A1 | 16 November 2020 |
| | | | | US | 2015252031 | A1 | 10 September 2015 |
| | | | | US | 9365519 | B2 | 14 June 2016 |
| | | | | DO | P2015000158 | A | 30 November 2015 |
| | | | | WO | 2014100719 | A2 | 26 June 2014 |
| | | | | WO | 2014100719 | A3 | 14 August 2014 |
| | | | | PT | 2935222 | T | 10 December 2018 |
| | | | | PE | 20151501 | A1 | 08 November 2015 |
| | | | | US | 2020022973 | A1 | 23 January 2020 |
| | | | | US | 10980794 | B2 | 20 April 2021 |
| | | | | DK | 2935222 | T3 | 07 January 2019 |
| | | | | PL | 2935222 | T3 | 28 February 2019 |
| | | | | ZA | 201505212 | B | 26 October 2016 |
| | | | | RS | 58040 | B1 | 28 February 2019 |
| | | | | MX | 2015008052 | A | 18 August 2016 |
| | | | | US | 2017027935 | A1 | 02 February 2017 |
| | | | | US | 9675614 | B2 | 13 June 2017 |
| | | | | IL | 260876 | A | 31 July 2019 |
| | | | | UA | 118548 | C2 | 11 February 2019 |
| | | | | EP | 2935222 | A2 | 28 October 2015 |
| | | | | EP | 2935222 | B1 | 05 September 2018 |
| | | | | ES | 2701069 | T3 | 20 February 2019 |
| | | | | US | 2014329794 | A1 | 06 November 2014 |
| | | | | US | 8993555 | B2 | 31 March 2015 |
| | | | | US | 2015344434 | A1 | 03 December 2015 |
| | | | | US | 9604930 | B2 | 28 March 2017 |
| | | | | PH | 12015501351 | A1 | 07 September 2015 |

Form PCT/ISA/210 (patent family annex) (January 2015)

**INTERNATIONAL SEARCH REPORT**
**Information on patent family members**

International application No.
**PCT/CN2021/119445**

| Patent document cited in search report | | | Publication date (day/month/year) | Patent family member(s) | | | Publication date (day/month/year) |
|---|---|---|---|---|---|---|---|
| | | | | PH | 12015501351 | B1 | 07 September 2015 |
| | | | | JP | 2016505596 | A | 25 February 2016 |
| | | | | JP | 6678455 | B2 | 08 April 2020 |
| | | | | US | 2019343826 | A1 | 14 November 2019 |
| | | | | KR | 20190109573 | A | 25 September 2019 |
| | | | | KR | 102173874 | B1 | 05 November 2020 |
| | | | | HU | E040323 | T2 | 28 February 2019 |
| | | | | US | 2017334861 | A1 | 23 November 2017 |
| | | | | US | 10307413 | B2 | 04 June 2019 |
| | | | | BR | 112015014590 | A2 | 21 November 2017 |
| | | | | CL | 2015001790 | A1 | 21 September 2015 |
| | | | | KR | 20150112953 | A | 07 October 2015 |
| US | 2004044031 | A1 | 04 March 2004 | AU | 5265701 | A | 20 November 2001 |
| | | | | AU | 779266 | B2 | 13 January 2005 |
| | | | | WO | 0185693 | A1 | 15 November 2001 |
| | | | | CA | 2408343 | A1 | 07 November 2002 |
| | | | | US | 6838465 | B2 | 04 January 2005 |
| | | | | EP | 1288202 | A1 | 05 March 2003 |
| | | | | EP | 1288202 | A4 | 02 July 2003 |
| | | | | JP | WO2001085693 | A1 | 08 January 2004 |
| CN | 111542523 | A | 14 August 2020 | | None | | |
| CN | 108570059 | A | 25 September 2018 | WO | 2018161922 | A1 | 13 September 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011000192
- CN 202110187630
- CN 202110251658X
- CN 202110529022
- CN 202110802850
- WO 2014100719 A